# EUROPEAN PATENT SPECIFICATION

(11) **EP 3 947 366 B1**
(45) Date of publication and mention of the grant of the patent: **21.05.2025**
(21) Application number: 20711972.8
(22) Date of filing: 24.03.2020
(51) Int. Cl.: C07D 401/14, C07D 405/14, C07D 413/14, C07D 403/14, C07D 417/14, C07D 498/04, A61P 35/00, A61K 31/444

(54) **HPK1 INHIBITORS**
HPK1-INHIBITOREN
INHIBITEURS D'HPK1

(30) Priority: 26.03.2019 US 201962823708 P; 08.04.2019 EP 19167820
(43) Date of publication of application: 09.02.2022
(73) Proprietor: Janssen Pharmaceutica NV, 2340 Beerse (BE)
(72) Inventor: MEVELLEC, Laurence, Anne, 92787 Issy-les Moulineaux Cedex 9 (FR); VIALARD, Jorge, Eduardo, 2340 Beerse (BE); COUPA, Sophie, 92787 Issy-les Moulineaux Cedex 9 (FR); ADELINET, Christophe, Denis, Pascal, 92787 Issy-les- Moulineaux Cedex 9 (FR); WROBLOWSKI, Berthold, 2340 Beerse (BE); EDWARDS, James, Patrick, Raritan, NJ 08869 (US)
(74) Representative: Schubert, Alexis
(86) International application number: PCT/EP2020/058081
(87) International publication number: WO 2020/193511

(56) References cited:
- WO-A1-2018/167147
- US-A1- 2019 076 401
- JAMES W. LEAHY ET AL: "Discovery of a Novel Series of Potent and Orally Bioavailable Phosphoinositide 3-Kinase [gamma] Inhibitors", JOURNAL OF MEDICINAL CHEMISTRY, vol. 55, no. 11, 14 June 2012 (2012-06-14), pages 5467 - 5482, XP055085698, ISSN: 0022-2623, DOI: 10.1021/jm300403a

## Description

### FIELD OF THE INVENTION

The present invention relates to pharmaceutical agents useful for therapy and/or prophylaxis in a mammal, pharmaceutical composition comprising such compounds, and their use as HPK1 inhibitors, useful for treating diseases such as cancer.

### BACKGROUND OF THE INVENTION

One of the hallmarks of cancer is its ability to evade the immune system by suppressing its ability to recognize malignant cells and mount an effective immune response against them. Therapeutic strategies designed to overcome these immunosuppressive effects include interfering with mechanisms that negatively regulate effector T cell function, such as the PD1/PDL1 immune checkpoint axis (Ribas and Wolchok, 2018). Antibodies that block PD1/PDL1 interaction and thereby eliminate the suppressive effect on T cells have demonstrated clinical benefit in several cancer types. Another example is the use of therapeutic antibodies that block the interaction between CD80/CD86 and the T-cell co-inhibitory receptor, CTLA-4, which facilitates T cell expansion in secondary lymphoid tissues. In addition to these cell-surface associated proteins, negative regulators of intracellular signalling required for an effective immune response have also been identified. One of these is Hematopoietic Progenitor Kinase 1 (HPK1), also known as MAP4K1, a serine/threonine kinase whose expression is restricted to hematopoietic cells (Hu et al., 1996; Kiefer et al., 1996).

HPK1 deficiency is associated with autoimmunity in humans and mice, suggesting a role in immune tolerance. HPK1 expression was down-regulated in PBMCs from patients with psoriatic arthritis (Batliwalla et al., 2005; Stoeckman et al., 2006) and in T cells from patients with systemic lupus erythematosus (Zhang et al., 2011), while HPK1-deficient mice displayed increased susceptibility to experimentally induced autoimmune encephalitis (Shui et al., 2007). T and B cells isolated from HPK1-deficient mice demonstrated enhanced antigen-dependent activation (Shui et al., 2007; Alzabin et al., 2010), highlighting its role as a negative regulator of T and B cell function. HPK1-deficient dendritic cells displayed enhanced antigen-presenting and T-cell activating properties, consistent with a negative regulatory role in these immune cells (Alzabin et al., 2009). HPK1 may also play a role in T and B cell adhesion by regulating integrin activation (Patzak et al., 2010; Königsberger et al., 2010). Overexpression studies suggested a role in activation-induced cell death through JNK pathway activation (Arnold et al., 2001; Brenner et al., 2005).

The HPK1 kinase is activated by T cell receptor and B cell receptor engagement. Upon TCR activation in T cells, Hpk1 is recruited to the TCR foci where it is phosphorylated and activated by Lck and Zap70 kinases (Arnold et al., 2005; Ling et al., 2001; Liou et al; 2000). Activated Hpk1 phosphorylates the adaptor proteins SLP76 and LAT, components of the TCR signalling complex that also includes GADS (Di Bartolo et al., 2007). Hpk1-dependent SLP76 phosphorylation on S376 creates a docking site for 14-3-3 proteins, which target SLP76 for degradation, thereby causing disruption of the TCR signalling complex and preventing further downstream signalling required for T cell activation (Lasserre et al., 2011). Hpk1 can also be activated by PGE2 in a PKA-dependent manner (Sawasdikosol et al., 2003, 2007; Alzabin et al., 2010) and possibly other immunosuppressive factors produced by tumors (Nagata et al., 1999; Zhou et al., 1999).

Growth of syngeneic Lewis Lung carcinoma tumors was reduced in HPK1^{-/-} compared to wild-type mice (Alzabin et al., 2010). Adoptive T cell transfer studies into T cell deficient mice demonstrated that the anti-tumor immune response in HPK1-/- mice was at least partially T-cell dependent (Alzabin et al., 2010). A potential contribution from dendritic cells to the anti-tumor effect in HPK1^{-/-} mice was demonstrated by reconstitution of irradiated mice with HPK1-deficent bone marrow derived dendritic cells (Alzabin et al., 2009). In more recent studies using mutant mice expressing a catalytically inactive form of HPK1, reduced GL261 glioblastoma tumor growth was observed and the anti-tumoral effect of anti-PD1 treatment on MC38 tumors was enhanced in mutant compared to wild-type mice (Hernanadez et al., 2018). These studies suggest that small molecule HPK1 kinase inhibitors could provide anti-tumoral effects as single agents, but also in combination with other immune modulators.

References referred to hereabove:
Alzabin S, Bhardwaj N, Kiefer F, Sawasdikosol S, Burakoff S. (2009). Hematopoietic progenitor kinase 1 is a negative regulator of dendritic cell activation. J. Immunol. 182:6187-94.
Alzabin S, Pyarajan S, Yee H, Kiefer F, Suzuki A, Burakoff S, Sawasdikosol S. (2010). Hematopoietic progenitor kinase 1 is a critical component of prostaglandin E2-mediated suppression of the anti-tumor immune response. Cancer Immunol. Immunother. 59:419-29.
Arnold R, Liou J, Drexler HC, Weiss A, Kiefer F. (2001). Caspase-mediated cleavage of hematopoietic progenitor kinase 1 (HPK1) converts an activator of NFkappaB into an inhibitor of NFkappaB. J Biol Chem. 276:14675-84.
Arnold R, Patzak IM, Neuhaus B, Vancauwenbergh S, Veillette A, Van Lint J, Kiefer F. (2005). Mol Cell Biol. 25:2364-83.
Batliwalla FM, Li W, Ritchlin CT, Xiao X, Brenner M, Laragione T, Shao T, Durham R, Kemshetti S, Schwarz E, Coe R, Kern M, Baechler EC, Behrens TW, Gregersen PK, Gulko PS. (2005). Microarray analyses of peripheral blood cells identifies unique gene expression signature in psoriatic arthritis. Mol Med. 11:21-9.
Brenner D, Golks A, Kiefer F, Krammer PH, Arnold R. (2005). Activation or suppression of NFkappaB by HPK1 determines sensitivity to activation-induced cell death. EMBO J. 24:4279-90.
Di Bartolo V, Montagne B, Salek M, Jungwirth B, Carrette F, Fourtane J, Sol-Foulon N, Michel F, Schwartz O, Lehmann WD, Acuto O. (2007). A novel pathway down-modulating T cell activation involves HPK-1-dependent recruitment of 14-3-3 proteins on SLP-76. J Exp Med. 204:681-91.
Hernandez S, Qing J, Thibodeau RH, et al. (2018). The kinase activity of Hematopoietic Progenitor Kinase 1 Is essential for the regulation of T cell function. Cell Rep. 25:80-94. Hu MC, Qiu WR, Wang X, Meyer CF, Tan TH. (1996). Human HPK1, a novel human hematopoietic progenitor kinase that activates the JNK/SAPK kinase cascade. Genes Dev. 10:2251-64.
Kiefer F, Tibbles LA, Anafi M, Janssen A, Zanke BW, Lassam N, Pawson T, Woodgett JR, Iscove NN. (1996). HPK1, a hematopoietic protein kinase activating the SAPK/JNK pathway. EMBO J. 15:7013-25.
Königsberger S, Peckl-Schmid D, Zaborsky N, Patzak I, Kiefer F, Achatz G. (2010). HPK1 associates with SKAP-HOM to negatively regulate Rap1-mediated B-lymphocyte adhesion. PLoS One. 5(9): e12468.
Lasserre R, Cuche C, Blecher-Gonen R, Libman E, Biquand E, Danckaert A, Yablonski D, Alcover A, Di Bartolo V. (2011). Release of serine/threonine-phosphorylated adaptors from signaling microclusters down-regulates T cell activation. J Cell Biol. 195:839-53. Ling P, Meyer CF, Redmond LP, Shui JW, Davis B, Rich RR, Hu MC, Wange RL, Tan TH. (2001). Involvement of hematopoietic progenitor kinase 1 in T cell receptor signaling. J Biol Chem. 276:18908-14.
Liou J, Kiefer F, Dang A, Hashimoto A, Cobb MH, Kurosaki T, Weiss A. (2000). HPK1 is activated by lymphocyte antigen receptors and negatively regulates AP-1. Immunity. 12:399-408.
Nagata Y, Kiefer F, Watanabe T, Todokoro K. (1999). Activation of hematopoietic progenitor kinase-1 by erythropoietin. Blood 93:3347-54.
Patzak IM, Königsberger S, Suzuki A, Mak TW, Kiefer F. (2010). HPK1 competes with ADAP for SLP-76 binding and via Rap1 negatively affects T-cell adhesion. Eur J Immunol. 40:3220-5.
Ribas A, Wolchok JD. (2018). Cancer immunotherapy using checkpoint blockade. Science 359:1350-1355.
Sawasdikosol S, Russo KM, and Burakoff SJ. (2003). Hematopoietic progenitor kinase 1 (HPK1) negatively regulates prostaglandin E2-induced fos gene transcription. Blood 101:3687-9.
Sawasdikosol S, Pyarajan S, Alzabin S, Matejovic G, and Burakoff SJ. (2007). Prostaglandin E2 activates HPK1 kinase activity via a PKA-dependent pathway. J Biol Chem. 282:34693-9.
Shui JW, Boomer JS, Han J, Xu J, Dement GA, Zhou G, Tan TH. (2007). Hematopoietic progenitor kinase 1 negatively regulates T cell receptor signaling and T cell-mediated immune responses. Nat Immunol. 8:84-91.
Stoeckman AK, Baechler EC, Ortmann WA, Behrens TW, Michet CJ, Peterson EJ. (2006). A distinct inflammatory gene expression profile in patients with psoriatic arthritis. Genes Immun. 7:583-91.
Zhang Q, Long H, Liao J, Zhao M, Liang G, Wu X, Zhang P, Ding S, Luo S, Lu Q. (2011). Inhibited expression of hematopoietic progenitor kinase 1 associated with loss of jumonji domain containing 3 promoter binding contributes to autoimmunity in systemic lupus erythematosus. J Autoimmun. 37:180-9.
Zhou G, Lee SC, Yao Z, Tan TH. (1999). Hematopoietic progenitor kinase 1 is a component of transforming growth factor beta-induced c-Jun N-terminal kinase signaling cascade. J Biol Chem. 274:13133-8.

WO2018049152, WO2018049214, WO2018049200, WO2018049191 and

WO2019051199 describe derivatives as HPK1 modulators and uses thereof for the treatment of cancer.

WO2018081531 describes methods for human T-cell activation.

WO2018102366 describes anilinopyrimidines as HPK1 inhibitors.

WO2016090300 describes methods and compositions for treating cancer using PD-1 axis antagonists and HPK1 antagonists.

WO2018167147, WO2018183956 and WO2018183964 describe compounds as inhibitors of HPK1.

WO2018215668 describes inhibitors of MAP4K1.

WO2018228920, WO2018228923 and WO2018228925 describe compounds useful in the treatment or prophylaxis of a disease, where the disease is cancer or conditions with dysregulated immune responses or other disorders associated with aberrant MAP4K1 signaling.

WO2016205942 describes HPK1 inhibitors.

### DESCRIPTION OF THE INVENTION

The present invention concerns novel compounds of Formula (I), 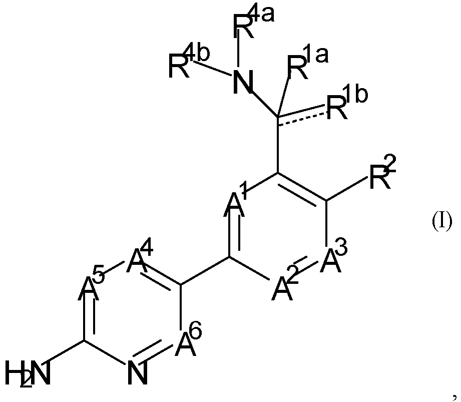
and the tautomers and stereoisomeric forms thereof, wherein
the dotted bond towards R^{1b} is an optional bond that may be present when R^{1b} and R^{4b} are taken together to form a monocyclic or bicyclic aromatic heterocyclyl as defined herein;
A¹ represents CH or N; A² represents CH; A³ represents CH or N;
provided that only one of A¹ and A³ represents N;
A⁴ represents CH or N; A⁵ represents CR^{3a}; A⁶ represents CH;
R^{1a} represents hydrogen;
R^{1b} represents hydrogen or CH₃;
R^{4a} represents hydrogen, C₁₋₄alkyl, or C₃₋₆cycloalkyl;
R^{4b} represents hydrogen, C₁₋₄alkyl, C₃₋₆cycloalkyl, or
a carbon linked monocyclic 4-, 5-, 6- or 7-membered non-aromatic heterocyclyl containing 1, 2 or 3 heteroatoms each independently selected from the group consisting of N, O, and S; wherein said S-atom might be substituted to form S(=O), S(=O)₂, or S(=O)(=NR^{x});
   or
R^{1b} and R^{4b} are taken together to form together with the atoms to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or a bicyclic 6- to 12- membered aromatic or fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, -C(=O)-NR^{6a}R^{6b}, and Het^{c};
wherein said monocyclic or bicyclic, aromatic or fully saturated heterocyclyl might be substituted on the optional additional nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
provided that in case R^{1b} and R^{4b} are taken together, R^{4a} represents hydrogen; and
R^{1a} represents hydrogen or R^{1a} is absent when the dotted bond towards R^{1b} is a bond;
   or
R^{4a} and R^{4b} are taken together to form together with the N-atom to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or
a bicyclic 6- to 12- membered aromatic or fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, -C(=O)-NR^{6a}R^{6b}, and Het^{c};
wherein said monocyclic or bicyclic, aromatic or fully saturated heterocyclyl might be substituted on the nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
in case R^{4a} and R^{4b} are taken together, R^{1a} represents hydrogen, and R^{1b} represents hydrogen;
R² is selected from the group consisting of cyano; halo; -C(=O)-NR^{8a}R^{8b};
-CH₂-NR^{8c}R^{8d}; Het^{b}; -P(=O)-(C₁₋₄alkyl)₂; -S(=O)₂-C₁₋₄alkyl; -S(=O)(=NR^{x})-C₁₋₄alkyl; C₁₋₆alkyl optionally substituted with 1 or 2 substituents each independently selected from the group consisting of halo, -OH, cyano, and -O-C₁₋₄alkyl;
C₃₋₆cycloalkyl optionally substituted with 1 or 2 substituents each independently selected from the group consisting of halo, -OH, cyano and -O-C₁₋₄alkyl; and
C₃₋₆cycloalkenyl optionally substituted with 1 or 2 substituents each independently selected from the group consisting of halo, -OH, cyano and -O-C₁₋₄alkyl;
R^{3a} represents hydrogen, halo, R⁷, -O-R⁷, cyano, -C(=O)-NR^{6e}R^{6f}, Het^{a}, or phenyl optionally substituted with 1, 2 or 3 substituents each independently selected from the group consisting of halo, cyano, C₁₋₄alkyl, -O-C₁₋₄alkyl, C₁₋₄alkyl substituted with one cyano, and C₁₋₄alkyl substituted with 1, 2 or 3 halo atoms;
Het^{a} represents a carbon linked monocyclic 5-, 6- or 7-membered aromatic heterocyclyl or carbon linked monocyclic 4-, 5-, 6- or 7-membered non-aromatic heterocyclyl, each containing 1, 2 or 3 heteroatoms each independently selected from the group consisting of N, O or S; wherein said S-atom might be substituted to form S(=O), S(=O)₂, or S(=O)(=NR^{x}); or
a carbon linked bicyclic 6- to 12-membered aromatic or non-aromatic heterocyclyl containing containing 1, 2 or 3 heteroatoms each independently selected from the group consisting of N, O or S; wherein said S-atom might be substituted to form S(=O), S(=O)₂, or S(=O)(=NR^{x});
wherein said monocyclic or bicyclic, aromatic or non-aromatic heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, -C(=O)-NR^{6a}R^{6b}, and Het^{c};
wherein said monocyclic or bicyclic, aromatic or non-aromatic heterocyclyl might be substituted on the nitrogen atoms with in total 1 or 2 substituents each independently selected from the group consisting of R⁷, -S(=O)₂-R⁷, -C(=O)-R⁷, -C(=O)-NR^{6a}R^{6b} and Het^{d};
R^{6a}, R^{6b}, R^{6c}, R^{6d}, R^{6e}, and R^{6f} are each independently selected from the group consisting of hydrogen; C₃₋₆cycloalkyl optionally substituted with one -OR⁵; and
C₁₋₄alkyl optionally substituted with one -OR⁵, wherein two hydrogen atoms on the same carbon atom of said C₁₋₄alkyl might be taken together to form C₃₋₆cycloalkyl;
R⁵ represents hydrogen or C₁₋₄alkyl;
R^{8a}, R^{8c}, and R^{8d} are each independently selected from the group consisting of hydrogen; C₁₋₄alkyl optionally substituted with one -OH or -O-C₁₋₄alkyl; and C₃₋₆cycloalkyl optionally substituted with one -OH or -O-C₁₋₄alkyl;
R^{8b} is selected from the group consisting C₁₋₄alkyl optionally substituted with one -OH or -O-C₁₋₄alkyl; and C₃₋₆cycloalkyl optionally substituted with one -OH or -O-C₁₋₄alkyl;
   or
R^{8a} and R^{8b}, or R^{8c} and R^{8d} are taken together to form together with the N-atom to which they are attached a monocyclic fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); wherein said monocyclic 4-, 5-, 6- or 7-membered fully saturated heterocyclyl might be substituted on one or more of the carbon atoms with in total 1, 2 or 3 substituents each independently selected from the group consisting of -OH, CN, halo, R⁷, - O-R⁷, -S(=O)₂-R⁷, -C(=O)-R⁷, -NR^{6c}R^{6d}, and
-C(=O)-NR^{6a}R^{6b};
wherein said monocyclic fully saturated heterocyclyl might be substituted on the nitrogen atoms with in total 1 or 2 substituents each independently selected from the group consisting of R⁷, -S(=O)₂-R⁷, -C(=O)-R⁷, and -C(=O)-NR^{6a}R^{6b};
each Het^{c} independently represents a monocyclic 4-, 5-, 6- or 7-membered fully saturated heterocyclyl containing 1, 2 or 3 heteroatoms each independently selected from the group consisting of N, O or S; wherein said S-atom might be substituted to form S(=O), S(=O)₂, or S(=O)(-NR^{x});
each Het^{d} independently represents a carbon linked monocyclic 4-, 5-, 6- or 7-membered fully saturated heterocyclyl containing 1, 2 or 3 heteroatoms each independently selected from the group consisting of N, O or S; wherein said S-atom might be substituted to form S(=O), S(=O)₂, or S(=O)(=NR^{x});
Het^{b} represents a monocyclic 4-, 5-, 6- or 7-membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x}); or
a bicyclic 6- to 12- membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x});
wherein said monocyclic or bicyclic non-aromatic heterocyclyl might be substituted on one or more of the carbon atoms with in total 1, 2 or 3 substituents each independently selected from the group consisting of -OH, CN, halo, R⁷, -O-R⁷, -S(=O)₂-R⁷,
-C(=O)-R⁷, -NR^{6c}R^{6d}, and -C(=O)-NR^{6a}R^{6b};
wherein said monocyclic or bicyclic non-aromatic heterocyclyl might be substituted on the nitrogen atoms with in total 1 or 2 substituents each independently selected from the group consisting of R⁷, -S(=O)₂-R⁷, -C(=O)-R⁷, and -C(=O)-NR^{6a}R^{6b};
each R⁷ independently represents C₃₋₆cycloalkyl optionally substituted with 1, 2 or 3 substituents each independently selected from the group consisting of halo, -OH, -O-C₁₋₄alkyl and cyano; or C₁₋₄alkyl optionally substituted with 1, 2 or 3 substituents each independently selected from the group consisting of halo, -OH, -O-C₁₋₄alkyl and cyano; each R^{x} independently represents hydrogen or C₁₋₄alkyl;
and the pharmaceutically acceptable addition salts, the N-oxides, and the solvates thereof.

The present invention also relates to a pharmaceutical composition comprising a therapeutically effective amount of a compound of Formula (I), a pharmaceutically acceptable addition salt, an N-oxide, or a solvate thereof, and a pharmaceutically acceptable carrier or excipient.

Additionally, the invention relates to a compound of Formula (I), a pharmaceutically acceptable addition salt, an N-oxide, or a solvate thereof, for use as a medicament, and to a compound of Formula (I), a pharmaceutically acceptable addition salt, an N-oxide or a solvate thereof, for use in the treatment or in the prevention of cancer.

In a particular embodiment, the invention relates to a compound of Formula (I), a pharmaceutically acceptable addition salt, an N-oxide, or a solvate thereof, for use in the treatment or in the prevention of cancer.

The invention also relates to the use of a compound of Formula (I), a pharmaceutically acceptable addition salt, an N-oxide, or a solvate thereof, in combination with an additional pharmaceutical agent for use in the treatment or prevention of cancer.

Furthermore, the invention relates to a process for preparing a pharmaceutical composition according to the invention, characterized in that a pharmaceutically acceptable carrier is intimately mixed with a therapeutically effective amount of a compound of Formula (I), a pharmaceutically acceptable addition salt, an N-oxide, or a solvate thereof.

The invention also relates to a product comprising a compound of Formula (I), a pharmaceutically acceptable addition salt, an N-oxide, or a solvate thereof, and an additional pharmaceutical agent, as a combined preparation for simultaneous, separate or sequential use in the treatment or prevention of cancer.

### DETAILED DESCRIPTION OF THE INVENTION

The term 'halo' or 'halogen' as used herein represents fluoro, chloro, bromo and iodo.

The prefix 'C_{x-y}' (where x and y are integers) as used herein refers to the number of carbon atoms in a given group. Thus, a C₁₋₆alkyl group contains from 1 to 6 carbon atoms, and so on.

The term 'C₁₋₄alkyl' as used herein as a group or part of a group represents a straight or branched chain fully saturated hydrocarbon radical having from 1 to 4 carbon atoms, such as methyl, ethyl, n-propyl, isopropyl, n-butyl, s-butyl, t-butyl and the like.

The term 'C₃₋₆cycloalkyl' as used herein as a group or part of a group defines a fully saturated, cyclic hydrocarbon radical having from 3 to 6 carbon atoms, such as cyclopropyl, cyclobutyl, cyclopentyl and cyclohexyl.

It will be clear for the skilled person that S(=O)₂ or SO₂ represents a sulfonyl moiety.

It will be clear for the skilled person that CO or C(=O) represents a carbonyl moiety.

In general, whenever the term 'substituted' is used in the present invention, it is meant, unless otherwise indicated or clear from the context, to indicate that one or more hydrogens, in particular from 1 to 4 hydrogens, more in particular from 1 to 3 hydrogens, preferably 1 or 2 hydrogens, more preferably 1 hydrogen, on the atom or radical indicated in the expression using 'substituted' are replaced with a selection from the indicated group, provided that the normal valency is not exceeded, and that the substitution results in a chemically stable compound, i.e. a compound that is sufficiently robust to survive isolation to a useful degree of purity from a reaction mixture.

Combinations of substituents and/or variables are permissible only if such combinations result in chemically stable compounds. 'Stable compound' is meant to indicate a compound that is sufficiently robust to survive isolation to a useful degree of purity from a reaction mixture.

The skilled person will understand that the term 'optionally substituted' means that the atom or radical indicated in the expression using 'optionally substituted' may or may not be substituted (this means substituted or unsubstituted respectively).

When two or more substituents are present on a moiety they may, where possible and unless otherwise indicated or clear from the context, replace hydrogens on the same atom or they may replace hydrogen atoms on different atoms in the moiety.

It will be clear for the skilled person that, unless otherwise is indicated or is clear from the context, a substituent on a heterocyclyl group may replace any hydrogen atom on a ring carbon atom or on a ring heteroatom (e.g. a hydrogen on a nitrogen atom may be replaced by a substituent).

A 'non-aromatic' group (e.g. a 'monocyclic non-aromatic heterocyclyl') embraces unsaturated ring systems without aromatic character, partially saturated and fully saturated carbocyclic and heterocyclic ring systems. The term 'partially saturated' refers to rings wherein the ring structure(s) contain(s) at least one multiple bond e.g. a C=C, N=C bond. The term 'fully saturated' refers to rings where there are no multiple bonds between ring atoms.

Unless otherwise specified or clear from the context, aromatic, non-aromatic or fully saturated heterocyclyl groups, can be attached to the remainder of the molecule of Formula (I) through any available ring carbon atom (carbon linked) or nitrogen atom (nitrogen linked).

Unless otherwise specified or clear from the context, aromatic, non-aromatic or fully saturated heterocyclyl groups, may optionally be substituted, where possible, on carbon and/or nitrogen atoms according to the embodiments.

The term 'monocyclic 4-, 5-, 6- or 7-membered non-aromatic heterocyclyl containing 1, 2 or 3 hetereoatoms each independently selected from the group consisting of N, O and S' as used herein alone or as part of another group, defines a monocyclic non-aromatic, cyclic hydrocarbon radical containing at least one nitrogen, oxygen or sulphur atom having from 4 to 7 ring members, as defined above. Non-limiting examples are:

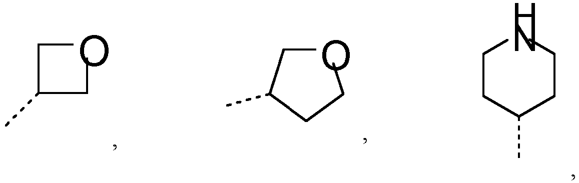

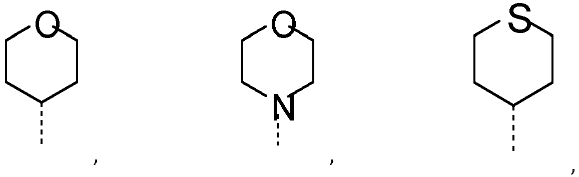

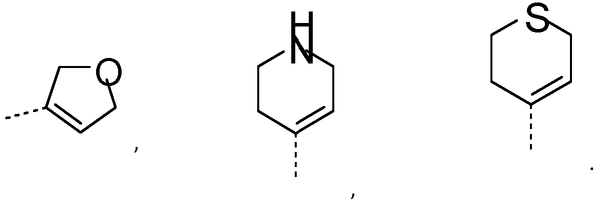

Within the context of this invention, bicyclic fully saturated heterocyclyl groups include fused, spiro and bridged saturated heterocycles.

Fused bicyclic groups are two cycles that share two atoms and the bond between these atoms.

Spiro bicyclic groups are two cycles that are joined at a single atom.

Bridged bicyclic groups are two cycles that share more than two atoms.

As defined in the scope, when R^{1b} and R^{4b} are taken together, R^{4a} represents hydrogen. This means that the nitrogen atom to which R^{4a} is attached, always has a hydrogen atom and is not substituted, nor is a double bond attached to said nitrogen atom.

Therefore, non-limiting examples of R^{1b} and R^{4b} taken together to form a monocyclic 5-membered aromatic heterocyclyl containing 1 N-atom and optionally 1 or 2 additional heteroatoms each independently selected from the group consisting of N, O, S, include, but are not limited to

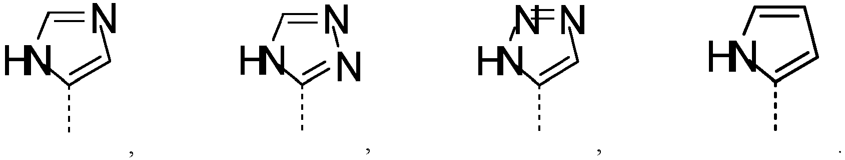

Therefore, non-limiting examples of R^{1b} and R^{4b} taken together to form a monocyclic 4-, 5-, 6- or 7-membered fully saturated heterocyclyl containing 1 N-atom and optionally 1 or 2 additional heteroatoms each independently selected from the group consisting of N, O, S, include, but are not limited to:

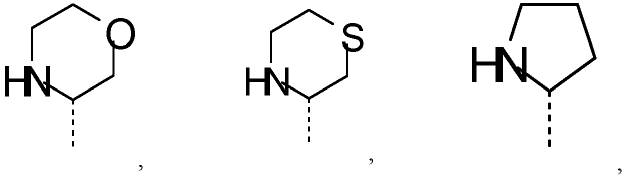

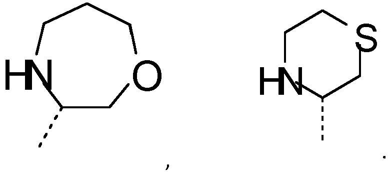

Therefore, non-limiting examples of R^{1b} and R^{4b} taken together to form a bicyclic 6- to 12-membered aromatic heterocyclyl containing 1 N-atom and optionally 1 or 2 additional heteroatoms each independently selected from the group consisting of N, O, S, include, but are not limited to

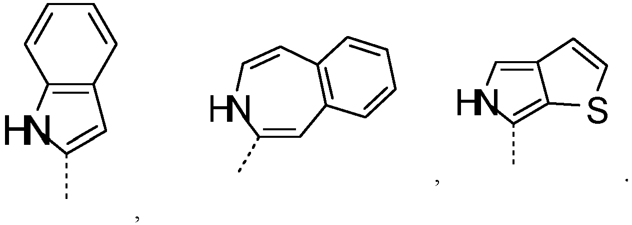

Therefore, non-limiting examples of R^{1b} and R^{4b} taken together to form a bicyclic 6- to 12-membered fully saturated heterocyclyl containing 1 N-atom and optionally 1 or 2 additional heteroatoms each independently selected from the group consisting of N, O, S, include, but are not limited to

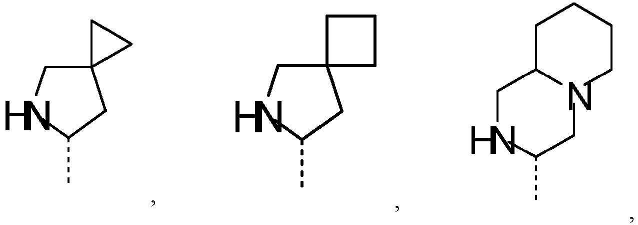

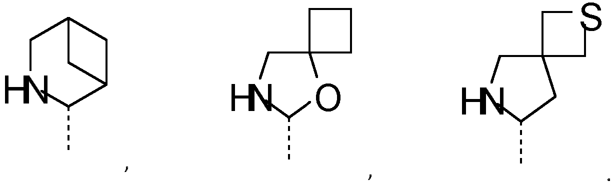

Non-limiting examples of R^{4a} and R^{4b} taken together to form together with the N-atom to which they are attached a monocyclic 5-membered aromatic heterocyclyl containing 1 N-atom and optionally 1 or 2 additional heteroatoms each independently selected from the group consisting of N, O, S, include, but are not limited to

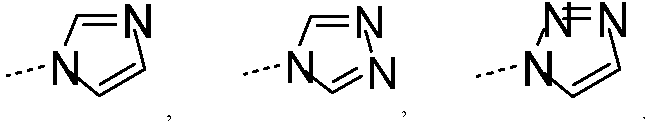

Non-limiting examples of R^{4a} and R^{4b} taken together to form together with the N-atom to which they are attached a monocyclic 4-, 5-, 6- or 7-membered fully saturated heterocyclyl containing 1 N-atom and optionally 1 or 2 additional heteroatoms each independently selected from the group consisting of N, O, S, include, but are not limited to:

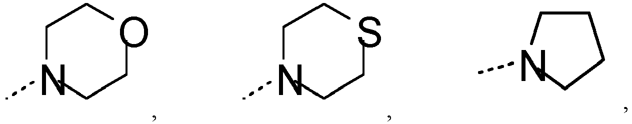

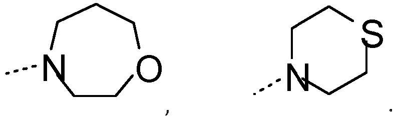

Non-limiting examples of R^{4a} and R^{4b} taken together to form together with the N-atom to which they are attached a bicyclic 6- to 12-membered aromatic heterocyclyl containing 1 N-atom and optionally 1 or 2 additional heteroatoms each independently selected from the group consisting of N, O, S, include, but are not limited to

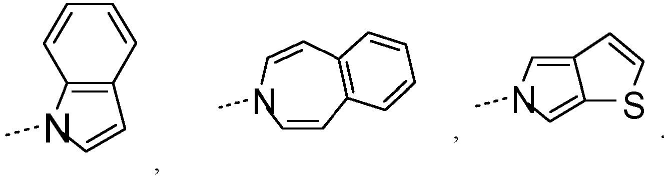

Non-limiting examples of R^{4a} and R^{4b} taken together to form together with the N-atom to which they are attached a bicyclic 6- to 12-membered fully saturated heterocyclyl containing 1 N-atom and optionally 1 or 2 additional heteroatoms each independently selected from the group consisting of N, O, S, include, but are not limited to

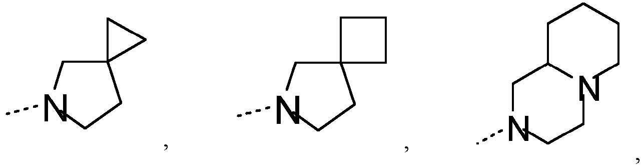

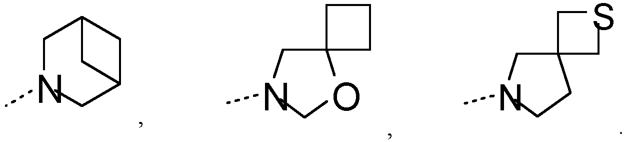

Non-limiting examples of carbon linked monocyclic 5-, 6- or 7-membered aromatic heterocyclyl containing 1, 2 or 3 hetereoatoms each independently selected from the group consisting of N, O and S, include, but are not limited to

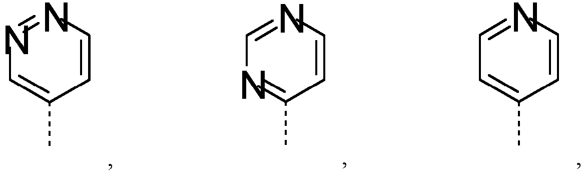

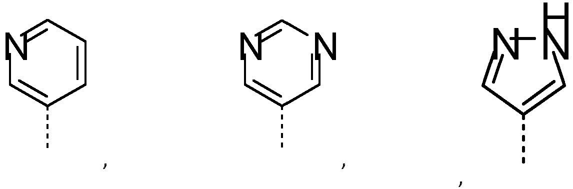

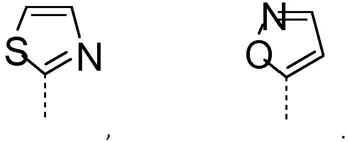

Non-limiting examples of carbon linked bicyclic 6- to 12-membered aromatic heterocyclyl containing 1, 2 or 3 hetereoatoms each independently selected from the group consisting of N, O and S, include, but are not limited to

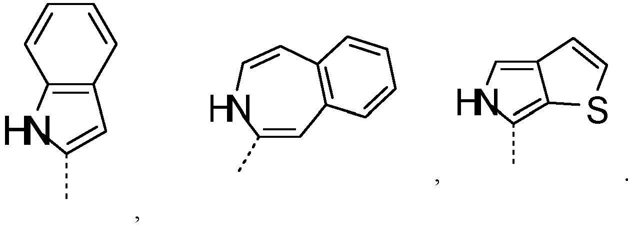

The term 'bicyclic 6- to 12-membered non-aromatic heterocyclyl containing 1, 2 or 3 hetereoatoms each independently selected from the group consisting of N, O and S' as used herein alone or as part of another group, defines a bicyclic non-aromatic, cyclic hydrocarbon radical containing at least one nitrogen, oxygen or sulphur atom having from 6 to 12 ring members, as defined above. Non-limiting examples are:

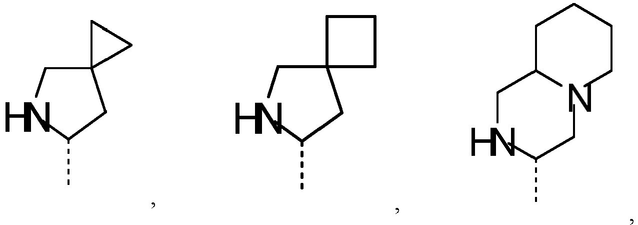

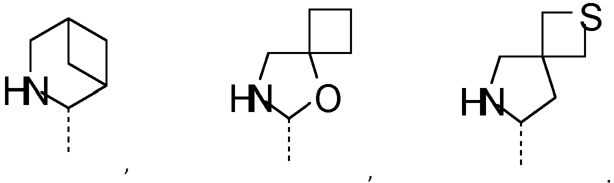

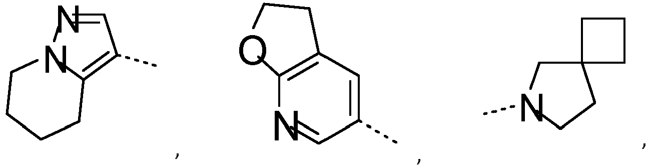

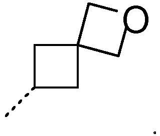

Non-limiting examples of monocyclic 4-, 5-, 6- or 7-membered fully saturated heterocyclyl containing 1, 2 or 3 heteroatoms each independently selected from the group consisting of N, O, S, include, but are not limited to:

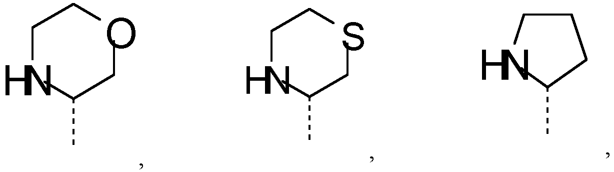

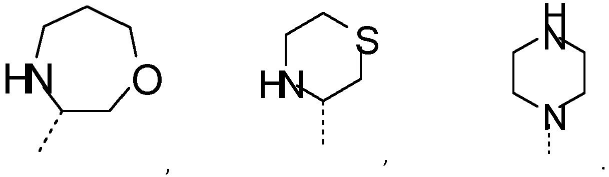

When any variable occurs more than one time in any constituent, each definition is independent.

When any variable occurs more than one time in any formula (e.g. Formula (I)), each definition is independent.

The term "subject" as used herein, refers to an animal, preferably a mammal (e.g. cat, dog, primate or human), more preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medicinal doctor or other clinician, which includes alleviation or reversal of the symptoms of the disease or disorder being treated.

The term "composition" is intended to encompass a product comprising the specified ingredients in the specified amounts, as well as any product which results, directly or indirectly, from combinations of the specified ingredients in the specified amounts.

The term "treatment", as used herein, is intended to refer to all processes wherein there may be a slowing, interrupting, arresting or stopping of the progression of a disease, but does not necessarily indicate a total elimination of all symptoms.

The term "compound(s) of the (present) invention" or "compound(s) according to the (present) invention" as used herein, is meant to include the compounds of Formula (I) and the pharmaceutically acceptable addition salts, the N-oxides, and the solvates thereof.

As used herein, any chemical formula with bonds shown only as solid lines and not as solid wedged or hashed wedged bonds, or otherwise indicated as having a particular configuration (e.g. R, S) around one or more atoms, contemplates each possible stereoisomer, or mixture of two or more stereoisomers.

Hereinbefore and hereinafter, the term "compound(s) of Formula (I)" is meant to include the tautomers thereof and the stereoisomeric forms thereof.

The terms "stereoisomers", "stereoisomeric forms" or "stereochemically isomeric forms" hereinbefore or hereinafter are used interchangeably.

The invention includes all stereoisomers of the compounds of the invention either as a pure stereoisomer or as a mixture of two or more stereoisomers.

Enantiomers are stereoisomers that are non-superimposable mirror images of each other. A 1:1 mixture of a pair of enantiomers is a racemate or racemic mixture.

Atropisomers (or atropoisomers) are stereoisomers which have a particular spatial configuration, resulting from a restricted rotation about a single bond, due to large steric hindrance. All atropisomeric forms of the compounds of Formula (I) are intended to be included within the scope of the present invention.

Diastereomers (or diastereoisomers) are stereoisomers that are not enantiomers, i.e. they are not related as mirror images. If a compound contains a double bond, the substituents may be in the E or the Z configuration.

Substituents on bivalent cyclic saturated or partially saturated radicals may have either the cis- or trans-configuration; for example if a compound contains a disubstituted cycloalkyl group, the substituents may be in the cis or trans configuration.

Therefore, the invention includes enantiomers, atropisomers, diastereomers, racemates, E isomers, Z isomers, cis isomers, trans isomers and mixtures thereof, whenever chemically possible.

The meaning of all those terms, i.e. enantiomers, atropisomers, diastereomers, racemates, E isomers, Z isomers, cis isomers, trans isomers and mixtures thereof are known to the skilled person.

The absolute configuration is specified according to the Cahn-Ingold-Prelog system. The configuration at an asymmetric atom is specified by either R or S. Resolved stereoisomers whose absolute configuration is not known can be designated by (+) or (-) depending on the direction in which they rotate plane polarized light. For instance, resolved enantiomers whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other stereoisomers. Thus, when a compound of Formula (I) is for instance specified as (R), this means that the compound is substantially free of the (S) isomer; when a compound of Formula (I) is for instance specified as E, this means that the compound is substantially free of the Z isomer; when a compound of Formula (I) is for instance specified as cis, this means that the compound is substantially free of the trans isomer.

The stereochemical configuration for centers in some compounds may be designated "R" or "*S*" when the mixture(s) was separated; for some compounds, the stereochemical configuration at indicated centers has been designated as "*R**" or *"S*"* when the absolute stereochemistry is undetermined (even if the bonds are drawn stereo specifically) although the compound itself has been isolated as a single stereoisomer and is enantiomerically pure.

For example, it will be clear that Compound 5b 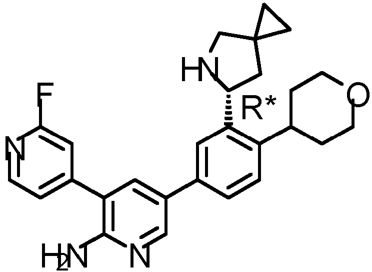 is

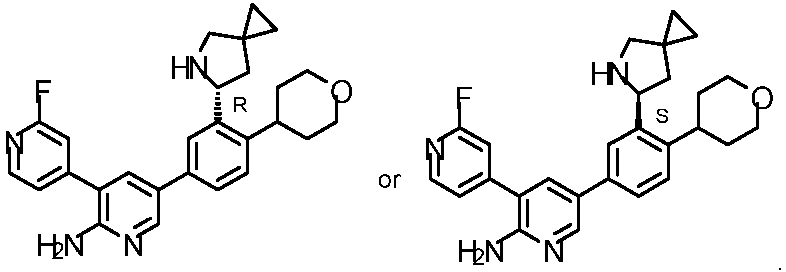

For compounds wherein the stereochemical configuration of two stereocentres is indicated by * (e.g. "*R**" or "*S**")*,* the absolute stereochemistry of the stereocentres is undetermined (even if the bonds are drawn stereospecifically), although the compound itself has been isolated as a single stereoisomer and is enantiomerically pure. In this case, the configuration of the first stereocentre is independent of the configuration of the second stereocentre in the same compound.

For example, for Compound 15a 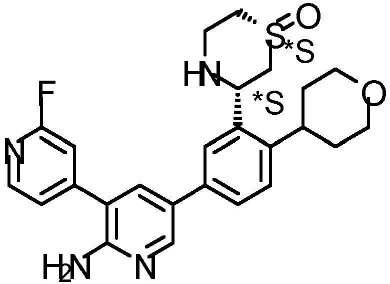 this means that the compound is 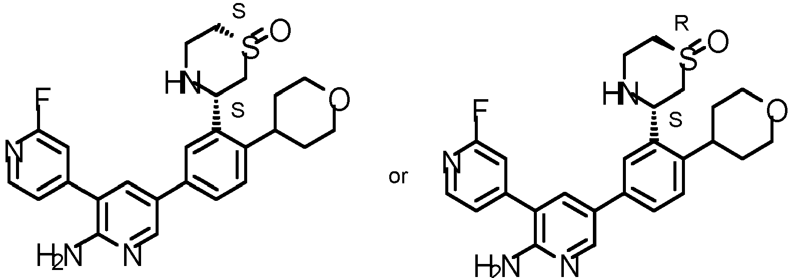 or

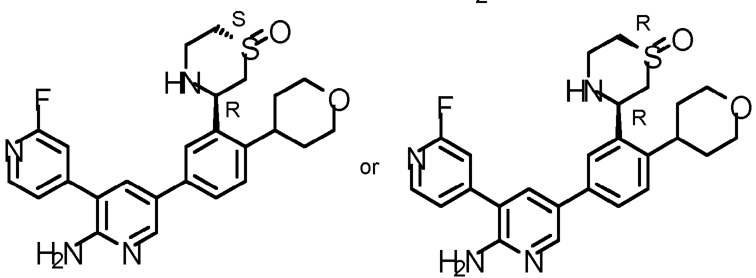

Some of the compounds according to Formula (I) may also exist in their tautomeric form. Such forms in so far as they may exist, although not explicitly indicated in the above Formula (I) are intended to be included within the scope of the present invention. It follows that a single compound may exist in both stereoisomeric and tautomeric form.

For example Co. 56 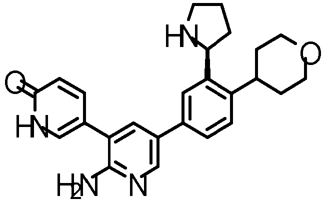 also covers the other tautomeric form

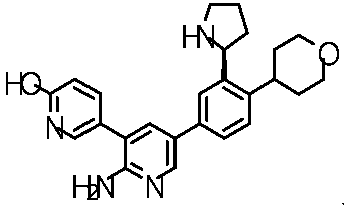

Pharmaceutically acceptable addition salts include acid addition salts and base addition salts. Such salts may be formed by conventional means, for example by reaction of a free acid or a free base form with one or more equivalents of an appropriate base or acid, optionally in a solvent, or in a medium in which the salt is insoluble, followed by removal of said solvent, or said medium, using standard techniques (e.g. *in vacuo,* by freeze-drying or by filtration). Salts may also be prepared by exchanging a counter-ion of a compound of the invention in the form of a salt with another counter-ion, for example using a suitable ion exchange resin.

The pharmaceutically acceptable addition salts as mentioned hereinabove or hereinafter are meant to comprise the therapeutically active non-toxic acid and base salt forms which the compounds of Formula (I), N-oxides and solvates thereof, are able to form.

Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic, tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds of Formula (I) and solvates thereof containing an acidic proton may also be converted into their non-toxic metal or amine salt forms by treatment with appropriate organic and inorganic bases.

Appropriate base salt forms comprise, for example, the ammonium salts, the alkali and earth alkaline metal salts, e.g. the lithium, sodium, potassium, cesium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline; the benzathine, N-methyl-D-glucamine, hydrabamine salts, and salts with amino acids such as, for example, arginine, lysine and the like. Conversely the salt form can be converted by treatment with acid into the free acid form.

The term solvate comprises the solvent addition forms as well as the salts thereof, which the compounds of Formula (I) are able to form. Examples of such solvent addition forms are e.g. hydrates, alcoholates and the like.

The compounds of the invention as prepared in the processes described below may be synthesized in the form of mixtures of enantiomers, in particular racemic mixtures of enantiomers, that can be separated from one another following art-known resolution procedures. A manner of separating the enantiomeric forms of the compounds of Formula (I), and pharmaceutically acceptable addition salts, N-oxides and solvates thereof, involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound would be synthesized by stereospecific methods of preparation. These methods will advantageously employ enantiomerically pure starting materials.

The term "enantiomerically pure" as used herein means that the product contains at least 80% by weight of one enantiomer and 20% by weight or less of the other enantiomer. Preferably the product contains at least 90% by weight of one enantiomer and 10% by weight or less of the other enantiomer. In the most preferred embodiment the term "enantiomerically pure" means that the composition contains at least 99% by weight of one enantiomer and 1% or less of the other enantiomer.

The present invention also embraces isotopically-labeled compounds of the present invention which are identical to those recited herein, but for the fact that one or more atoms are replaced by an atom having an atomic mass or mass number different from the atomic mass or mass number usually found in nature (or the most abundant one found in nature).

All isotopes and isotopic mixtures of any particular atom or element as specified herein are contemplated within the scope of the compounds of the invention, either naturally occurring or synthetically produced, either with natural abundance or in an isotopically enriched form. Exemplary isotopes that can be incorporated into compounds of the invention include isotopes of hydrogen, carbon, nitrogen, oxygen, phosphorus, sulfur, fluorine, chlorine and iodine, such as ²H, ³H , ¹¹C, ¹³C, ¹⁴C, ¹³N, ¹⁵O, ¹⁷O, ¹⁸O, ³²P, ³³P, ³⁵S, ¹⁸F, ³⁶Cl, ¹²²I, ¹²³I, ¹²⁵I, ¹³¹I, ⁷⁵Br, ⁷⁶Br, ⁷⁷Br and ⁸²Br. Preferably, the radioactive isotope is selected from the group of ²H, ³H, ¹¹C and ¹⁸F. More preferably, the radioactive isotope is ²H. In particular, deuterated compounds are intended to be included within the scope of the present invention.

Certain isotopically-labeled compounds of the present invention (e.g., those labeled with ³H and ¹⁴C) may be useful for example in substrate tissue distribution assays. Tritiated (³H) and carbon-l4 (¹⁴C) isotopes are useful for their ease of preparation and detectability. Further, substitution with heavier isotopes such as deuterium (i.e., ²H) may afford certain therapeutic advantages resulting from greater metabolic stability (e.g., increased *in vivo* half-life or reduced dosage requirements) and hence may be preferred in some circumstances. Positron emitting isotopes such as ¹⁵O, ¹³N, ¹¹C and ¹⁸F are useful for positron emission tomography (PET) studies. PET imaging in cancer finds utility in helping locate and identify tumours, stage the disease and determine suitable treatment. Human cancer cells overexpress many receptors or proteins that are potential disease-specific molecular targets. Radiolabelled tracers that bind with high affinity and specificity to such receptors or proteins on tumour cells have great potential for diagnostic imaging and targeted radionuclide therapy (Charron, Carlie L. et al. Tetrahedron Lett. 2016, 57(37), 4119-4127). Additionally, target-specific PET radiotracers may be used as biomarkers to examine and evaluate pathology, by for example, measuring target expression and treatment response (Austin R. et al. Cancer Letters (2016), doi: 10.1016/j .canlet.2016.05.008).

The present invention relates in particular to compounds of Formula (I) as defined herein, and the tautomers and the stereoisomeric forms thereof, wherein
the dotted bond towards R^{1b} is an optional bond that may be present when R^{1b} and R^{4b} are taken together to form a monocyclic aromatic heterocyclyl as defined herein;
A¹ represents CH or N; A² represents CH; A³ represents CH or N;
provided that only one of A¹ and A³ represents N;
A⁴ represents CH; A⁵ represents CR^{3a}; A⁶ represents CH;
R^{1a} represents hydrogen;
R^{1b} represents hydrogen;
R^{4a} represents hydrogen, or C₁₋₄alkyl;
R^{4b} represents C₁₋₄alkyl, C₃₋₆cycloalkyl, or
a carbon linked monocyclic 4-, 5-, 6- or 7-membered non-aromatic heterocyclyl containing 1, 2 or 3 oxygen atoms;
   or
R^{1b} and R^{4b} are taken together to form together with the atoms to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O); or
a bicyclic 6- to 12-membered fully saturated heterocyclyl containing 1 N-atom and optionally 1 or 2 additional heteroatoms each independently selected from the group consisting of N, O, S; wherein said optional S-atom might be substituted to form S(=O); wherein said monocyclic or bicyclic, aromatic or fully saturated heterocyclyl might be substituted on one or more of the carbon atoms with in total 1, 2 or 3 substituents each independently selected from the group consisting of halo and R⁷;
provided that in case R^{1b} and R^{4b} are taken together, R^{4a} represents hydrogen; and R^{1a} represents hydrogen or R^{1a} is absent when the dotted bond towards R^{1b} is a bond;
   or
R^{4a} and R^{4b} are taken together to form together with the N-atom to which they are attached a monocyclic 4-, 5-, 6- or 7-membered fully saturated heterocyclyl containing 1 N-atom; wherein said monocyclic fully saturated heterocyclyl might be substituted on one or more of the carbon atoms with in total 1, 2 or 3 halo substituents;
in case R^{4a} and R^{4b} are taken together, R^{1a} represents hydrogen, and R^{1b} represents hydrogen;
R² is selected from the group consisting of cyano; Het^{b}; and C₃₋₆cycloalkyl optionally substituted with 1 or 2 substituents each independently selected from the group consisting of halo and -O-C₁₋₄alkyl;
R^{3a} represents halo, R⁷, -O-R⁷, cyano, -C(=O)-NR^{6e}R^{6f}, Het^{a}, or phenyl optionally substituted with 1, 2 or 3 substituents each independently selected from the group consisting of halo and cyano;
Het^{a} represents a carbon linked monocyclic 5-, 6- or 7-membered aromatic heterocyclyl or carbon linked monocyclic 4-, 5-, 6- or 7-membered non-aromatic heterocyclyl, each containing 1, 2 or 3 heteroatoms each independently selected from the group consisting of N, O or S; wherein said S-atom might be substituted to form S(=O) or S(=O)₂; or
a carbon linked bicyclic 6- to 12-membered non-aromatic heterocyclyl containing containing 1, 2 or 3 heteroatoms each independently selected from the group consisting of N or O;
wherein said monocyclic or bicyclic, aromatic or non-aromatic heterocyclyl might be substituted on one or more of the carbon atoms with in total 1, 2 or 3 substituents each independently selected from the group consisting of -OH, CN, halo, R⁷, -O-R⁷, -NR^{6c}R^{6d}, and Het^{c};
wherein said monocyclic or bicyclic, aromatic or non-aromatic heterocyclyl might be substituted on the nitrogen atoms with in total 1 or 2 substituents each independently selected from the group consisting of R⁷, -S(=O)₂-R⁷, and -C(=O)-R⁷;
R^{6c}, R^{6d}, R^{6e}, and R^{6f} are each independently selected from the group consisting of hydrogen; and C₁₋₄alkyl optionally substituted with one -OR⁵;
R⁵ represents hydrogen;
each Het^{c} independently represents a monocyclic 4-, 5-, 6- or 7-membered fully saturated heterocyclyl containing 1, 2 or 3 nitrogen atoms;
Het^{b} represents a monocyclic 4-, 5-, 6- or 7-membered non-aromatic heterocyclyl containing 1, 2 or 3 heteroatoms each independently selected from the group consisting of N, O, S; wherein said S-atom might be substituted to form S(=O) or S(=O)₂; or
a bicyclic 6- to 12- membered non-aromatic heterocyclyl containing 1, 2 or 3 heteroatoms each independently selected from the group consisting of N, O, S; wherein said S-atom might be substituted to form S(=O) or S(=O)₂;
wherein said monocyclic or bicyclic non-aromatic heterocyclyl might be substituted on the nitrogen atoms with in total 1 or 2 substituents each independently selected from the group consisting of -S(=O)₂-R⁷,and -C(=O)-R⁷;
each R⁷ independently represents C₁₋₄alkyl optionally substituted with 1, 2 or 3 substituents each independently selected from the group consisting of halo, -OH, and cyano;
and the pharmaceutically acceptable addition salts, the N-oxides, and the solvates thereof.

The present invention relates in particular to compounds of Formula (I) as defined herein, and the tautomers and the stereoisomeric forms thereof, wherein
the dotted bond towards R^{1b} is absent;
A¹ represents CH or N; A² represents CH; A³ represents CH;
A⁴ represents CH; A⁵ represents CR^{3a}; A⁶ represents CH;
R^{1b} and R^{4b} are taken together to form together with the atoms to which they are attached a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O);
wherein said monocyclic fully saturated heterocyclyl might be substituted on one of the carbon atoms with 1 substituent selected from the group consisting of halo and R⁷; provided that R^{4a} represents hydrogen; and R^{1a} represents hydrogen;
R² represents Het^{b};
R^{3a} represents halo, cyano, or Het^{a};
Het^{a} represents a carbon linked monocyclic 5-, 6- or 7-membered aromatic heterocyclyl containing 1, 2 or 3 heteroatoms each independently selected from the group consisting of N, O or S; wherein said S-atom might be substituted to form S(=O) or S(=O)₂;
wherein said monocyclic aromatic heterocyclyl might be substituted on one of the carbon atoms with a halo substituent;
wherein said monocyclic aromatic heterocyclyl might be substituted on one nitrogen atom with R⁷;
Het^{b} represents a monocyclic 4-, 5-, 6- or 7-membered non-aromatic heterocyclyl containing 1 oxygen atom;
each R⁷ represents C₁₋₄alkyl;
and the pharmaceutically acceptable addition salts, the N-oxides, and the solvates thereof.

The present invention relates in particular to compounds of Formula (I) as defined herein, and the tautomers and the stereoisomeric forms thereof, wherein
the dotted bond towards R^{1b} is absent;
A¹ represents CH or N; A² represents CH; A³ represents CH;
A⁴ represents CH; A⁵ represents CR^{3a}; A⁶ represents CH;
R^{1b} and R^{4b} are taken together to form together with the atoms to which they are attached 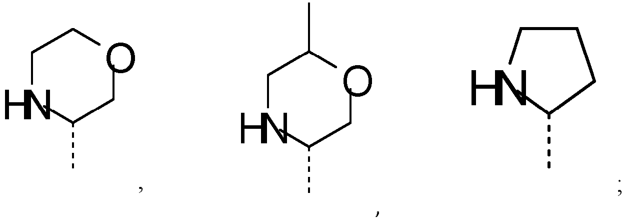
R^{4a} represents hydrogen; and R^{1a} represents hydrogen;
R² represents Het^{b};
R^{3a} represents halo, cyano, or Het^{a};
Het^{a} represents 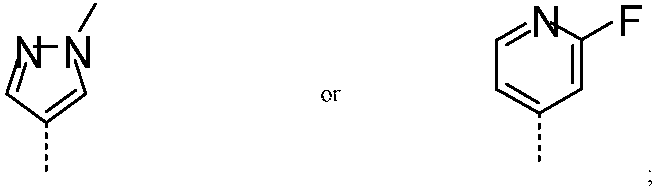
Het^{b} represents tetrahydropyranyl; in particular 4-tetrahydropyranyl;
and the pharmaceutically acceptable addition salts, the N-oxides, and the solvates thereof.

In an embodiment, the present invention relates to those compounds of Formula (I) and the pharmaceutically acceptable addition salts, the N-oxides, and the solvates thereof, or any subgroup thereof as mentioned in any of the other embodiments, wherein R² represents Het^{b}.

In an embodiment, the present invention relates to those compounds of Formula (I) and the pharmaceutically acceptable addition salts, the N-oxides, and the solvates thereof, or any subgroup thereof as mentioned in any of the other embodiments, wherein Het^{b} is attached the remainder of the molecule via a carbon atom (carbon linked).

In an embodiment, the present invention relates to those compounds of Formula (I) and the pharmaceutically acceptable addition salts, the N-oxides, and the solvates thereof, or any subgroup thereof as mentioned in any of the other embodiments, wherein
Het^{b} represents a carbon linked monocyclic 4-, 5-, 6- or 7-membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x}); or
a carbon linked bicyclic 6- to 12- membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x});
wherein said monocyclic or bicyclic non-aromatic heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, and -C(=O)-NR^{6a}R^{6b};
wherein said monocyclic or bicyclic non-aromatic heterocyclyl might be substituted on the nitrogen atoms with in total 1 or 2 substituents each independently selected from the group consisting of R⁷, -S(=O)₂-R⁷, -C(=O)-R⁷, and -C(=O)-NR^{6a}R^{6b}.

In an embodiment, the present invention relates to those compounds of Formula (I) and the pharmaceutically acceptable addition salts, the N-oxides, and the solvates thereof, or any subgroup thereof as mentioned in any of the other embodiments, wherein

In an embodiment, the present invention relates to those compounds of Formula (I) and the pharmaceutically acceptable addition salts, the N-oxides, and the solvates thereof, or any subgroup thereof as mentioned in any of the other embodiments, wherein
R² represents Het^{b};
Het^{b} represents a carbon linked monocyclic 4-, 5-, 6- or 7-membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x}); or
a carbon linked bicyclic 6- to 12- membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x});
wherein said monocyclic or bicyclic non-aromatic heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, and -C(=O)-NR^{6a}R^{6b}.

In an embodiment, the present invention relates to those compounds of Formula (I) and the pharmaceutically acceptable addition salts, the N-oxides, and the solvates thereof, or any subgroup thereof as mentioned in any of the other embodiments, wherein
Het^{b} represents a carbon linked monocyclic 4-, 5-, 6- or 7-membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x});
wherein said monocyclic non-aromatic heterocyclyl might be substituted on the nitrogen atoms with in total 1 or 2 substituents each independently selected from the group consisting of R⁷, -S(=O)₂-R⁷, -C(=O)-R⁷, and -C(=O)-NR^{6a}R^{6b}.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R² represents Het^{b};
Het^{b} represents a carbon linked monocyclic 4-, 5-, 6- or 7-membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x});
wherein said monocyclic non-aromatic heterocyclyl might be substituted on the nitrogen atoms with in total 1 or 2 substituents each independently selected from the group consisting of R⁷, -S(=O)₂-R⁷, -C(=O)-R⁷, and -C(=O)-NR^{6a}R^{6b}.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R² represents

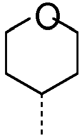

In an embodiment, the present invention relates to those compounds of Formula (I) and the pharmaceutically acceptable addition salts, the N-oxides, and the solvates thereof, or any subgroup thereof as mentioned in any of the other embodiments, wherein
R² represents 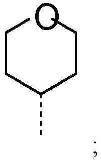
R^{1b} and R^{4b} are taken together to form together with the atoms to which they are attached 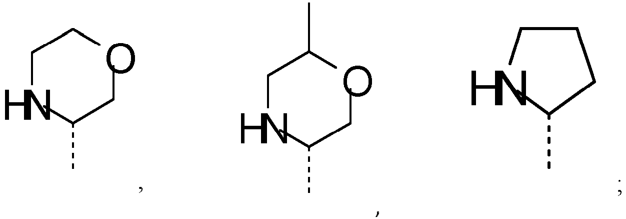
R^{4a} represents hydrogen; and
R^{1a} represents hydrogen.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together to form together with the atoms to which they are attached 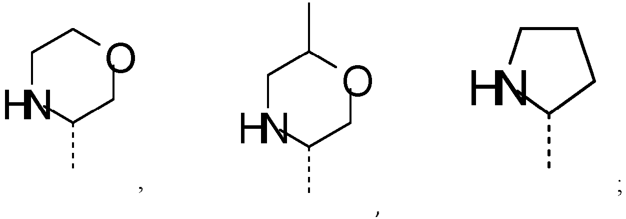
R^{4a} represents hydrogen; and
R^{1a} represents hydrogen.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together to form together with the atoms to which they are attached 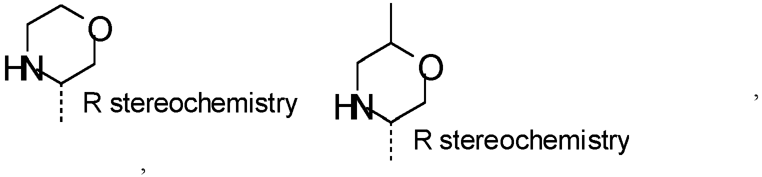 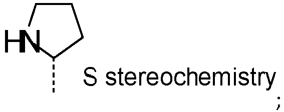
R^{4a} represents hydrogen; and R^{1a} represents hydrogen.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together to form together with the atoms to which they are attached 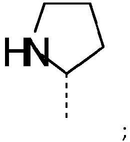
R^{4a} represents hydrogen; and R^{1a} represents hydrogen.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together to form together with the atoms to which they are attached 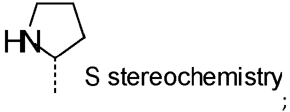
R^{4a} represents hydrogen; and R^{1a} represents hydrogen.

In an embodiment, the present invention relates to those compounds of Formula (I) and the pharmaceutically acceptable addition salts, the N-oxides, and the solvates thereof, or any subgroup thereof as mentioned in any of the other embodiments, wherein
A¹ represents CH or N; A² represents CH; A³ represents CH;
A⁴ represents CH; A⁵ represents CR^{3a}; A⁶ represents CH.

In an embodiment, the present invention relates to those compounds of Formula (I) and the pharmaceutically acceptable addition salts, the N-oxides, and the solvates thereof, or any subgroup thereof as mentioned in any of the other embodiments, wherein
A¹ represents CH or N; A² represents CH; A³ represents CH;
A⁴ represents CH; A⁵ represents CR^{3a}; A⁶ represents CH;
R² represents Het^{b};
R^{3a} represents halo, cyano, or Het^{a}.

In an embodiment, the present invention relates to those compounds of Formula (I) and the pharmaceutically acceptable addition salts, the N-oxides, and the solvates thereof, or any subgroup thereof as mentioned in any of the other embodiments, wherein
A¹ represents CH or N; A² represents CH; A³ represents CH;
A⁴ represents CH; A⁵ represents CR^{3a}; A⁶ represents CH;
R² represents Het^{b};
R^{3a} represents halo, cyano, or Het^{a};
Het^{a} represents a carbon linked monocyclic 5-, 6- or 7-membered aromatic heterocyclyl containing 1, 2 or 3 heteroatoms each independently selected from the group consisting of N, O or S; wherein said S-atom might be substituted to form S(=O) or S(=O)₂;
wherein said monocyclic aromatic heterocyclyl might be substituted on one of the carbon atoms with a halo substituent;
wherein said monocyclic aromatic heterocyclyl might be substituted on one nitrogen atom with R⁷;
Het^{b} represents a monocyclic 4-, 5-, 6- or 7-membered non-aromatic heterocyclyl containing 1 oxygen atom;
each R⁷ represents C₁₋₄alkyl.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1a} represents hydrogen;
R^{1b} represents hydrogen or CH₃;
R^{4a} represents hydrogen, C₁₋₄alkyl, or C₃₋₆cycloalkyl;
R^{4b} represents hydrogen, C₁₋₄alkyl, C₃₋₆cycloalkyl, or
a carbon linked monocyclic 4-, 5-, 6- or 7-membered non-aromatic heterocyclyl containing 1, 2 or 3 heteroatoms each independently selected from the group consisting of N, O, and S; wherein said S-atom might be substituted to form S(=O), S(=O)₂, or S(=O)(=NR^{x}).

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together to form together with the atoms to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or a bicyclic 6- to 12- membered aromatic or fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, -C(=O)-NR^{6a}R^{6b}, and Het^{c};
wherein said monocyclic or bicyclic, aromatic or fully saturated heterocyclyl might be substituted on the optional additional nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
provided that R^{4a} represents hydrogen; and
R^{1a} represents hydrogen or R^{1a} is absent when the dotted bond towards R^{1b} is a bond.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together to form together with the atoms to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, - NR^{6c}R^{6d}, -C(=O)-NR^{6a}R^{6b}, and Het^{c};
wherein said monocyclic aromatic or fully saturated heterocyclyl might be substituted on the optional additional nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
provided that R^{4a} represents hydrogen; and
R^{1a} represents hydrogen or R^{1a} is absent when the dotted bond towards R^{1b} is a bond.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together to form together with the atoms to which they are attached a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, - C(=O)-NR^{6a}R^{6b}, and Het^{c};
wherein said monocyclic fully saturated heterocyclyl might be substituted on the optional additional nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
provided that R^{4a} represents hydrogen; and
R^{1a} represents hydrogen or R^{1a} is absent when the dotted bond towards R^{1b} is a bond.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together;
provided that R^{4a} represents hydrogen; and
R^{1a} represents hydrogen or R^{1a} is absent when the dotted bond towards R^{1b} is a bond.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{4a} and R^{4b} are taken together to form together with the N-atom to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or a bicyclic 6- to 12- membered aromatic or fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, -C(=O)-NR^{6a}R^{6b}, and Het^{c};
wherein said monocyclic or bicyclic, aromatic or fully saturated heterocyclyl might be substituted on the nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d}.
R^{1a} represents hydrogen, and R^{1b} represents hydrogen.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1a} represents hydrogen;
R^{1b} represents hydrogen or CH₃;
R^{4a} represents hydrogen, C₁₋₄alkyl, or C₃₋₆cycloalkyl;
R^{4b} represents hydrogen, C₁₋₄alkyl, C₃₋₆cycloalkyl, or
a carbon linked monocyclic 4-, 5-, 6- or 7-membered non-aromatic heterocyclyl containing 1, 2 or 3 heteroatoms each independently selected from the group consisting of N, O, and S; wherein said S-atom might be substituted to form S(=O), S(=O)₂, or S(=O)(=NR^{x});
   or
R^{1b} and R^{4b} are taken together to form together with the atoms to which they are attached a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or
a bicyclic 6- to 12- membered fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, - NR^{6c}R^{6d}, -C(=O)-NR^{6a}R^{6b}, and Het^{c};
wherein said monocyclic or bicyclic, fully saturated heterocyclyl might be substituted on the optional additional nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
provided that in case R^{1b} and R^{4b} are taken together, R^{4a} represents hydrogen; and R^{1a} represents hydrogen or R^{1a} is absent when the dotted bond towards R^{1b} is a bond;
   or
R^{4a} and R^{4b} are taken together to form together with the N-atom to which they are attached a monocyclic 5-, 6- or 7-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S;
wherein said optional S-atom might be substituted to form S(=O), S(=O)₂, or S(=O)(=NR^{x}); or
a bicyclic 6- to 12- membered aromatic or fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, -NR^{6c}R^{6d}, -C(=O)-NR^{6a}R^{6b}, and Het^{c};
wherein said monocyclic or bicyclic, aromatic or fully saturated heterocyclyl might be substituted on the nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
in case R^{4a} and R^{4b} are taken together, R^{1a} represents hydrogen, and R^{1b} represents hydrogen.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1a} represents hydrogen;
R^{1b} represents hydrogen or CH₃;
R^{4a} represents hydrogen, C₁₋₄alkyl, or C₃₋₆cycloalkyl;
R^{4b} represents hydrogen, C₁₋₄alkyl, C₃₋₆cycloalkyl, or
a carbon linked monocyclic 4-, 5-, 6- or 7-membered non-aromatic heterocyclyl containing 1, 2 or 3 heteroatoms each independently selected from the group consisting of N, O, and S; wherein said S-atom might be substituted to form S(=O), S(=O)₂, or S(=O)(=NR^{x});
   or
R^{1b} and R^{4b} are taken together to form together with the atoms to which they are attached a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or
a bicyclic 6- to 12- membered fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, - NR^{6c}R^{6d}, -C(=O)-NR^{6a}R^{6b}, and Het^{c};
wherein said monocyclic or bicyclic, fully saturated heterocyclyl might be substituted on the optional additional nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
provided that in case R^{1b} and R^{4b} are taken together, R^{4a} represents hydrogen; and
R^{1a} represents hydrogen or R^{1a} is absent when the dotted bond towards R^{1b} is a bond.

In an embodiment, the present invention relates to those compounds of Formula (I) and the pharmaceutically acceptable addition salts, the N-oxides, and the solvates thereof, or any subgroup thereof as mentioned in any of the other embodiments, wherein
R^{1b} and R^{4b} are taken together to form together with the atoms to which they are attached a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or
a bicyclic 6- to 12- membered fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R⁷, - NR^{6c}R^{6d}, -C(=O)-NR^{6a}R^{6b}, and Het^{c};
wherein said monocyclic or bicyclic, fully saturated heterocyclyl might be substituted on the optional additional nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
provided that R^{4a} represents hydrogen; and
R^{1a} represents hydrogen or R^{1a} is absent when the dotted bond towards R^{1b} is a bond.

In an embodiment, the present invention relates to those compounds of Formula (I) and the pharmaceutically acceptable addition salts, the N-oxides, and the solvates thereof, or any subgroup thereof as mentioned in any of the other embodiments, wherein
Het^{c} represents oxetanyl.

In an embodiment, the present invention relates to those compounds of Formula (I) and the pharmaceutically acceptable addition salts, the N-oxides, and the solvates thereof, or any subgroup thereof as mentioned in any of the other embodiments, wherein the compounds of Formula (I) are restricted to compounds of Formula (I-a1):

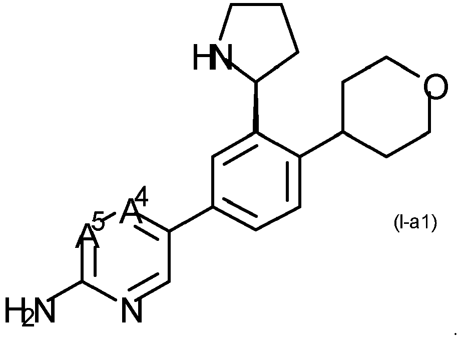

In an embodiment, the present invention relates to those compounds of Formula (I) and the pharmaceutically acceptable addition salts, the N-oxides, and the solvates thereof, or any subgroup thereof as mentioned in any of the other embodiments, wherein the compounds of Formula (I) are restricted to compounds of Formula (I-a2):

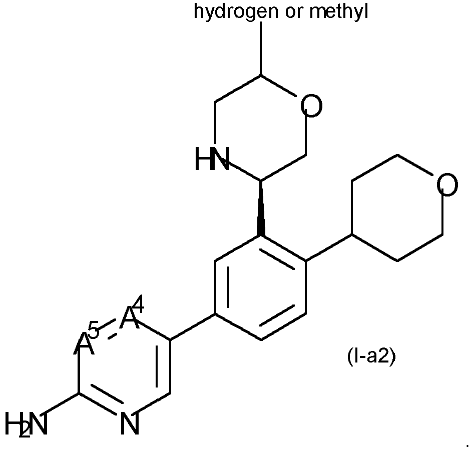

In an embodiment, the present invention relates to a subgroup of Formula (I) as defined in the general reaction schemes.

In an embodiment the compound of Formula (I) is selected from the group consisting of
any of the exemplified compounds,
tautomers and stereoisomeric forms thereof,
any pharmaceutically acceptable addition salts, the N-oxides, and the solvates thereof.

All possible combinations of the above indicated embodiments are considered to be embraced within the scope of the invention.

### METHODS FOR THE PREPARATION OF COMPOUNDS OF FORMULA (I)

In this section, as in all other sections unless the context indicates otherwise, references to Formula (I) also include all other sub-groups and examples thereof as defined herein.

The general preparation of some typical examples of the compounds of Formula (I) is described hereunder and in the specific examples and are generally prepared from starting materials which are either commercially available or prepared by standard synthetic processes commonly used by those skilled in the art of organic chemistry. The following schemes are only meant to represent examples of the invention and are in no way meant to be a limit of the invention.

Alternatively, compounds of the present invention may also be prepared by analogous reaction protocols as described in the general schemes below and the specific examples, combined with standard synthetic processes commonly used by those skilled in the art.

The skilled person will realize that in the reactions described in the Schemes, although this is not always explicitly shown, it may be necessary to protect reactive functional groups (for example hydroxy, amino, or carboxy groups) where these are desired in the final product, to avoid their unwanted participation in the reactions. In general, conventional protecting groups can be used in accordance with standard practice. The protecting groups may be removed at a convenient subsequent stage using methods known from the art.

The skilled person will realize that in the reactions described in the Schemes, it may be advisable or necessary to perform the reaction under an inert atmosphere, such as for example under N₂-gas atmosphere.

It will be apparent for the skilled person that it may be necessary to cool the reaction mixture before reaction work-up (refers to the series of manipulations required to isolate and purify the product(s) of a chemical reaction such as for example quenching, column chromatography, extraction).

The skilled person will realize that heating the reaction mixture under stirring may enhance the reaction outcome. In some reactions microwave heating may be used instead of conventional heating to shorten the overall reaction time.

The skilled person will realize that another sequence of the chemical reactions shown in the Schemes below, may also result in the desired compound of Formula (I).

The skilled person will realize that intermediates and final compounds shown in the Schemes below may be further functionalized according to methods well-known by the person skilled in the art. The intermediates and compounds described herein can be isolated in free form or as a salt, or a solvate thereof. The intermediates and compounds described herein may be synthesized in the form of mixtures of tautomers and stereoisomeric forms that can be separated from one another following art-known resolution procedures.

Hereinafter, "DCM" means dichloromethane; "LAH" means lithium aluminium hydride; "r.t." means room temperature; "Boc" means tert-butoxycarbonyl; "MeCN" means acetonitrile; "MeOH" means methanol; "TFA" means trifluoroacetic acid; "THF" means tetrahydrofuran; "Ti(OEt)₄" means titanium ethoxide; "Pd(PPh₃)₄" means tetrakis(triphenylphosphine)palladium; "[Ir(dtbbpy)(ppy)₂]PF₆" means (4,4'-Di-t-butyl-2, 2'-bipyridine)bis[2-(2-pyridinyl-kN)phenyl-kC]iridium(III) hexafluorophosphate; "SnAP" means tin amine protocol; "SLAP" means silicon amine protocol; "h" means hours; "PdCl₂(dppf)" means [1,1'-bis(diphenylphosphino-κP)ferrocene]dichloropalladium; "SFC" means Supercritical fluid chromatography; "LiHMDS" means Lithium bis(trimethylsilyl)amide; "SnBu₃" means tributyltin; "SiMe₃" means trimethylsilyl; "Cu(OTf)₂ means Copper (II) triflate; "PhBox" means 2,2'-Isopropylidenebis[(4R)-4-phenyl-2-oxazoline; "Bi(OTf)₃" means Bismuth(III) trifluoromethanesulfonate; "BF₃.2 MeOH means boron trifluoride in methanol; "TMSOTf" means trimethylsilyltriflate; "Me-THF" means methyltetrahydrofuran; "NiCl₂" means Nickel (II) chloride.

### Scheme 1

In general, compounds of Formula (I) wherein all variables are defined according to the scope of the present invention, can be prepared according to the following reaction Scheme 1.

In the scheme 1, the following definitions apply : X represents an halo. The following 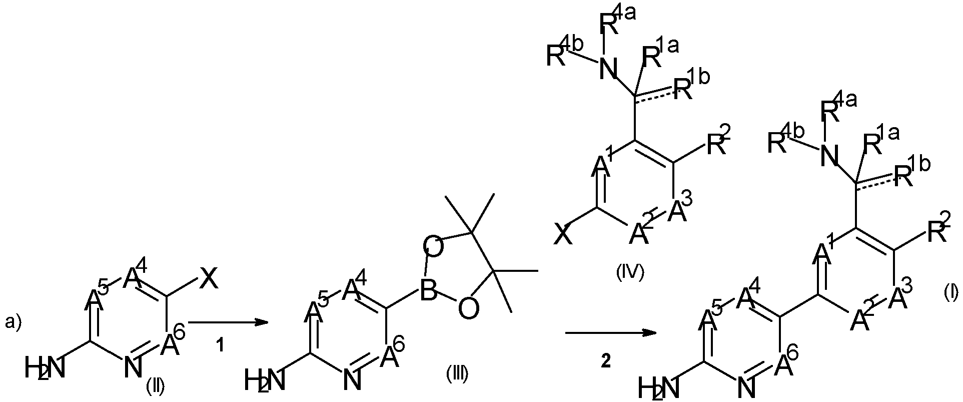 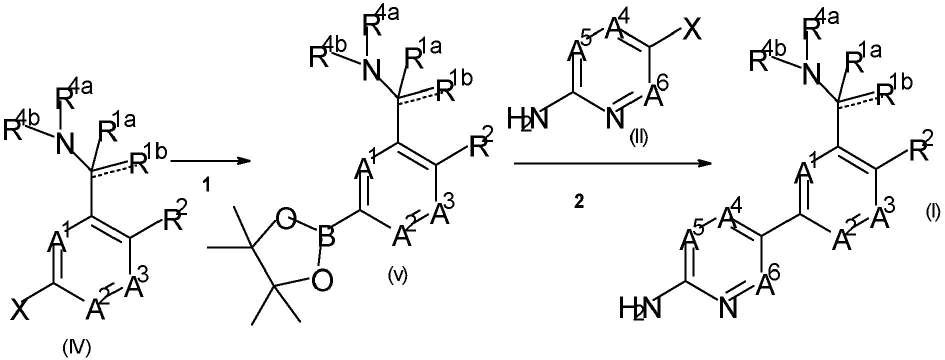 reaction conditions apply :
1 : at a suitable temperature such as for example at 80°C, in the presence of
   bis(pinacolatodiboron), a suitable catalyst as for example PdCl₂(dppf), a suitable basis such as potassium acetate, in a suitable solvent such 1,4-dioxane
2 : at a suitable temperature such as for example at 80°C, in the presence of a suitable catalyst as for example PdCl₂(dppf), a suitable basis such as sodium carbonate, in a suitable solvent or mixture of solvents such 1,4-dioxane and water In the preparation of compounds of the present invention, protection of remote functionality (e.g., primary or secondary amine) of intermediates may be necessary. The need for such protection will vary depending on the nature of the remote functionality and the conditions of the preparations methods. Suitable amino-protecting groups (NH-Pg) include t-butoxycarbonyl (Boc), acetyl, benzyl... The need for such protection is readily determined by one skilled in the art.

### Scheme 2

Intermediates of Formula (IV) wherein R^{1a} and R^{1b} represent hydrogen, and X represent halo can be prepared according to the following reaction Scheme 2. All other variables in Scheme 2 are defined according to the scope of the present invention. 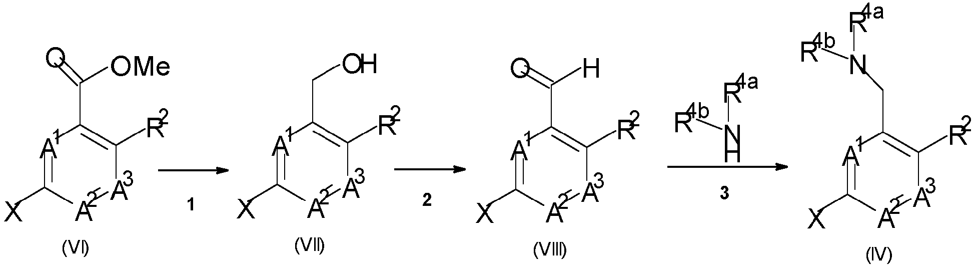
1: at a suitable temperature such as for example from 0°C to rt, in the presence of a suitable reducing agent such as LAH in a suitable solvent such as for example THF
2: at a suitable temperature such as rt, in the presence of an oxidizing agent such as for example manganese oxide or Dess-Martin periodinane, in a suitable solvent such as for example DCM
3 : at a suitable temperature such as for example rt, in the presence of a suitable reducing agent such as for example sodium triacetoxyborohydride in a suitable solvent such as for example DCM or THF.

### Scheme 3

Intermediates of Formula (IV) wherein R^{1a} represents hydrogen, R^{1b} and R^{4b} are taken together to form together with the atoms to which they attached a monocyclic 5-membered fully saturated heterocyclyl containing 1N-atom as for example intermediates of Formula (XII) can be prepared according to the following reaction Scheme 3. X represents halo, PG means protective group, all other variables in Scheme 3 are defined according to the scope of the present invention. 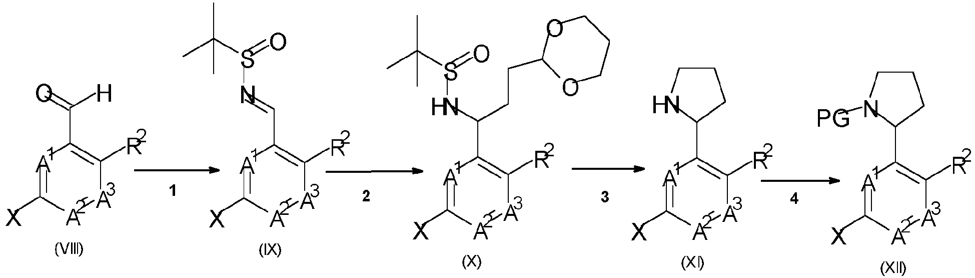
1: an intermediate of Formula (VIII) can react with t-Butylsulfinamide in the presence of Ti(OEt)₄ in a suitable solvent such as for example THF
2: Reaction with (1,3-dioxan-2-ylethyl) magnesium bromide in a suitable solvent, such as for example THF
3: in the presence of a suitable acid, such as for example TFA and a reductant triethylsilane in a suitable solvent such as for example water
4: an intermediate of Formula (XI) can be protected into a compound of Formula (XII) by reaction for example with di-tert-butyl dicarbonate in a suitable solvent such as for example DCM

The intermediates of Formula (XII) may be synthesized in the form of racemic mixtures of enantiomers which can be separated from one another such as liquid chromatography using a chiral stationary phase or SFC.

A skilled person will realize that similar chemistry can be used to prepare compounds wherein R^{1b} and R^{4b} are taken together to form together with the atoms to which they attached a monocyclic 4-, 6- or 7-membered fully saturated heterocyclyl containing 1N-atom.

### Scheme 4

Intermediates of Formula (IV) wherein R^{1a} represents hydrogen, R^{1b} and R^{4b} are taken together to form together with the atoms to which they attached a monocyclic 5-membered fully saturated heterocyclyl containing at least 1N-atom and substituted as for example in intermediates of Formula (XV) and (XVIII), can be prepared according to the following reaction Scheme 4. X represents halo and n and p = 1, 2, Y represents O or CH₂, PG means protective group. All other variables in Scheme 4 are defined according to the scope of the present invention.

A skilled person will realize that similar chemistry can be used to prepare compounds wherein R^{1b} and R^{4b} are taken together to form together with the atoms to which they attached other sizes of fully saturated heterocyclyls containing at least 1N-atom. 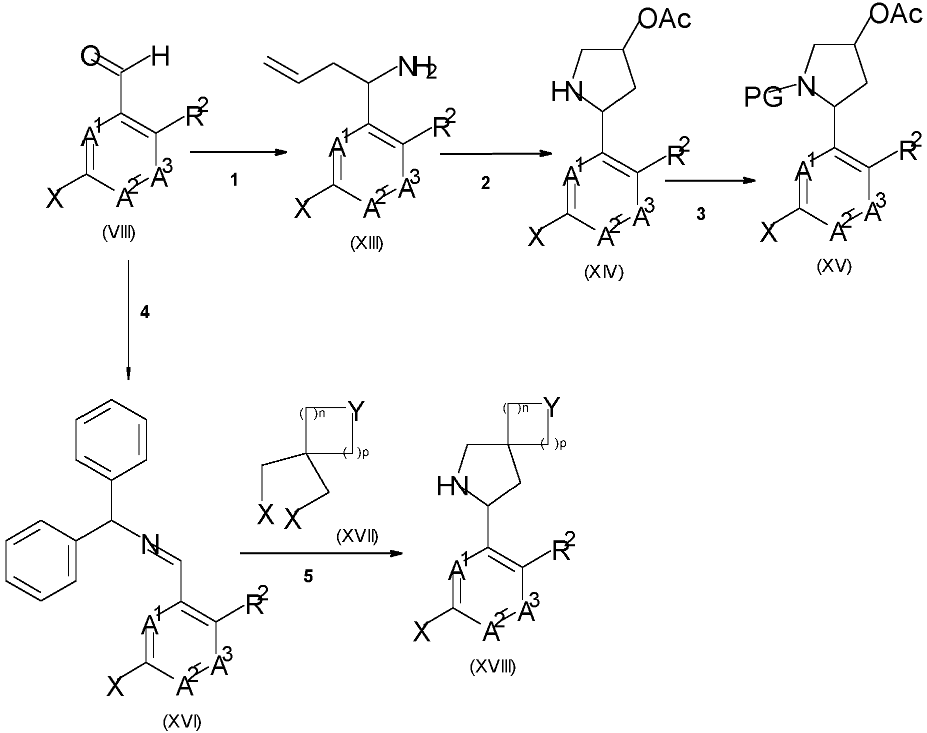
1 and 2: an intermediate of Formula (VIII) can be converted into a compound of formula (XIII) by reaction with Allylmagnesium chloride and LiHMDS in a suitable solvent, such as for example THF, followed by reaction with acetic anhydride in presence of a suitable basis such as triethylamine in a suitable solvent such as DCM.
3: an intermediate of Formula (XIV) can be protected into a compound of Formula (XV) by for example reaction with di-tert-butyl dicarbonate in a suitable solvent such as for example DCM
4: an intermediate of Formula (VIII) can react with Benzhydrylamine with magnesium sulfate in a suitable solvent such as DCM to give an intermediate of Formula (XVI)
5: an intermediate of Formula (XVI) can react with a compound of Formula (XVII) in presence of a suitable basis such as potassium tert-butoxide in a suitable solvent such as THF

Intermediates of Formula (XV) can be further functionalized by a person skilled in the art. The stereoisomers of intermediates of Formula (XV) and (XVIII) can be separated from one another such as liquid chromatography using a chiral stationary phase or SFC.

### Scheme 5

Intermediates of Formula (IV) wherein R^{1a} represents hydrogen, R^{1b} and R^{4b} are taken together to form together with the atoms to which they attached a monocyclic 6- or 7-membered fully saturated heterocyclyl containing at least 1 N-atom and substituted as for example intermediates of Formula (XX), can be prepared according to the following reaction Scheme 5. X represents halo, n and p represent 1 or 2 (but only one of n and p can represent 2), Y represents O , S, or N, Z represents SnBu₃ or SiMe₃. A skilled person will understand that R^{q} and R^{z} can be selected from the list of substituents as defined in the scope of the present invention and do also include bicyclic spiro moieties formed together with the N-containing heterocyclyls to which they are attached. A skilled person will understand that R^{q} and R^{z} for example will not represent cyano or a directly attached amine substituent.

All other variables in Scheme 5 are defined according to the scope of the present invention. 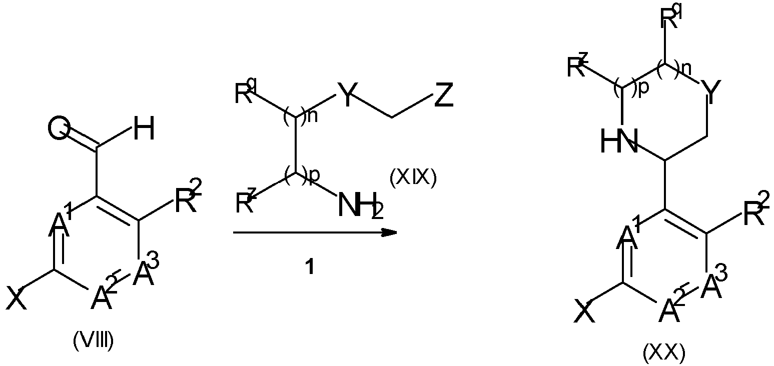
1: an intermediate of Formula (VIII) can react with a compound of formula (XIX, Y represents O, S or N, Z = SnBu₃) in the presence of an oxidant, such as for example Cu(OTf)₂, a suitable ligand, such as for example Lutidine or PhBox, and a suitable solvent or solvent mixture, such as hexafluoroisopropanol (HFIP) and DCM
or an intermediate of Formula (VIII) can react with a compound of Formula (XIX, Y = N, Z =SiMe₃) in the presence of a photocatalyst, such as for example Ir[ppy]₂(dtbbpy)PF₆, and a suitable solvent or solvent mixture, such as trifluoroethanol (TFE) and CH₃CN under blue light irradiation resulting
or an intermediate of Formula (VIII) can react with a compound of Formula (XIX, Y = S, Z = SiMe₃) in the presence of a photocatalyst, such as for example Ir[ppy]₂(dtbbpy)PF₆, a suitable acid or an acid mixture such as for example Bi(OTf)₃, Cu(OTf)₂, BF₃.2MeOH in a suitable solvent CH₃CN under blue light irradiation.
or an intermediate of Formula (VIII) can react with a compound of Formula (XIX, Y= O, Z = SiMe₃) in the presence of a photocatalyst, such as for example triphenylpyrilium (TPP), a suitable acid such as for example TMSOTf, and a suitable solvent or solvent mixture, such as hexafluoroisopropanol (HFIP) and CH₃CN under blue light irradiation.

### Scheme 6

Intermediates of Formula (IV) wherein R^{1a} is absent when the dotted towards R^{1b} is a bond as for example intermediates of Formula (XXIV) and (XXVI) can be prepared according to the following reaction Scheme 6. All other variables in Scheme 6 are defined according to the scope of the present invention. 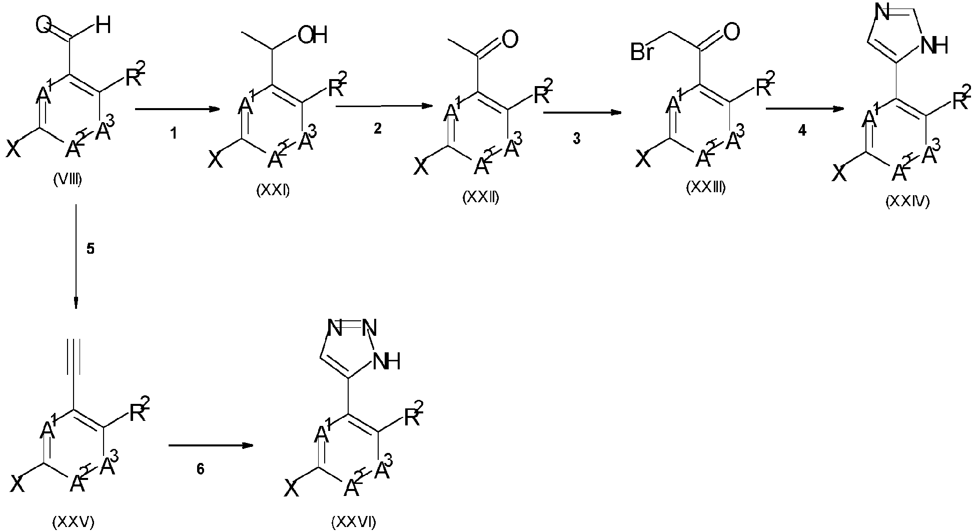
1 : an intermediate of Formula (VIII) can be converted into an intermediate of Formula (XXI) by reaction for example with methylmagnesium bromide in a suitable solvent, such as for example THF
2 : an intermediate of Formula (XXI) can be converted into an intermediate of Formula (XXII) by reaction for example with an oxidant such as for example manganese oxide in a suitable solvent, such as for example DCM
3: an intermediate of Formula (XXII) can be converted into an intermediate of Formula (XXIII) by reaction for example with a bromination agent such as for example tetra-n-butylammonium tribromide in a suitable solvent, such as for example acetonitrile
4: an intermediate of Formula (XXIII) can be converted into an intermediate of Formula (XXIV) by reaction for example with formamide as a suitable solvent
5: an intermediate of Formula (VIII) can be converted into an intermediate of Formula (XXV) by reaction for example with dimethyl(1-diazo-2-oxopropyl)phosphonate in presence of a suitable basis such as potassium carbonate in a suitable solvent, such as for example MeOH
6: an intermediate of Formula (XXV) can be converted into an intermediate of Formula (XXVI) by reaction for example with trimethylsilyl azide in the presence of a catalyst as for example copper iodide in a suitable solvent or mixture of solvents as for example DMF and MeOH.

### Scheme 7

Intermediates of Formula (II) wherein R^{3a} represents Het^{a} (or can also be used for R^{3a} represents phenyl optionally substituted) and X= Br can be prepared according to the following reaction Scheme 7. All other variables in Scheme 7 are defined according to the scope of the present invention. 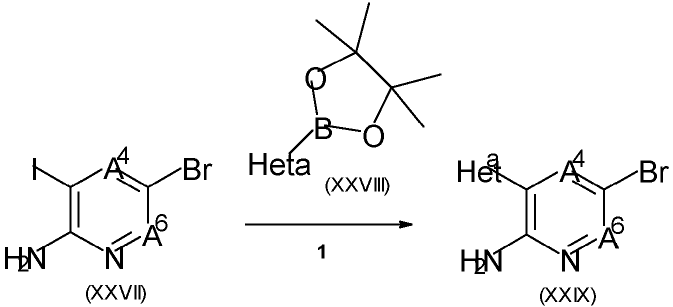
1: an intermediate of Formula (XXVII) can react with an intermediate of Formula (XXVIII) in the presence of a suitable catalyst, such as for example Pd(PPh₃)₄, a suitable base, such as sodium bicarbonate (Na₂CO₃), and a suitable solvent or solvent mixture, such as for example Me-THF or 1-4 dioxane and water.

### Scheme 8

An alternative to synthesis of compounds of Formula (I) wherein A⁵ represents Het^{a} and R² different from Br (compounds of Formula (XXXIII)): can be prepared according to the following reaction Scheme 8. All other variables are defined according to the scope of the present invention 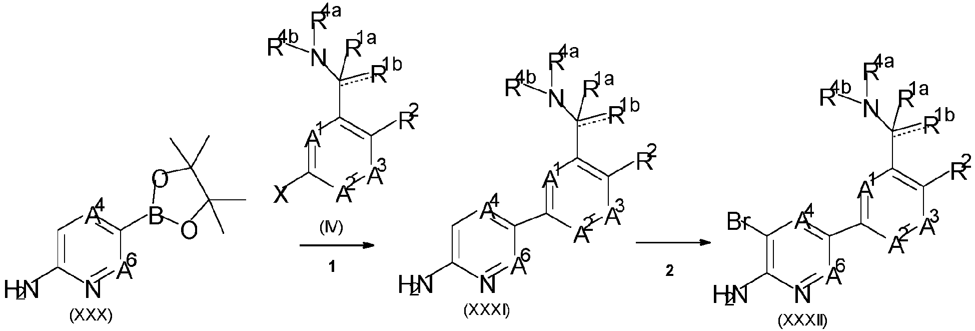 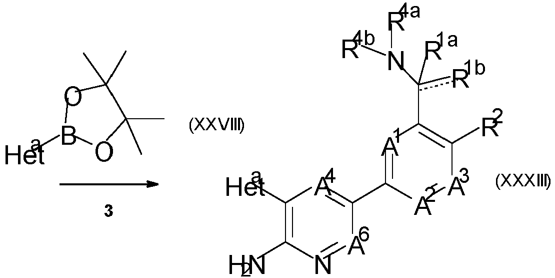
1: an intermediate of Formula (XXX) can react with an intermediate of Formula (IV) in the presence of suitable catalyst, such as for example Pd(PPh₃)₄, a suitable base, such as sodium bicarbonate (Na₂CO₃), in a suitable solvent or solvent mixture, such as for example Me-THF and water
2: an intermediate of Formula (XXXI) can be converted into an intermediate of Formula (XXXII) by reaction with bromine (Br₂) in the presence of a suitable solvent, such as for example acetic acid (AcOH)
3: an intermediate of Formula (XXXII) can react with an intermediate of Formula (XXVIII) in the presence of a suitable catalyst, such as for example Pd(PPh₃)₄, in the presence of a suitable base, such as sodium bicarbonate (Na₂CO₃), in a suitable solvent or solvent mixture, such as for example Me-THF and water.

### Scheme 9

An alternative to synthesis of compounds of Formula (I) wherein R^{3a} is different from halo, can be prepared according to the following reaction in Scheme 9, as for example, compounds of Formula (XXXV) wherein R² represents Het^{b} . All other variables are defined according to the scope of the present invention 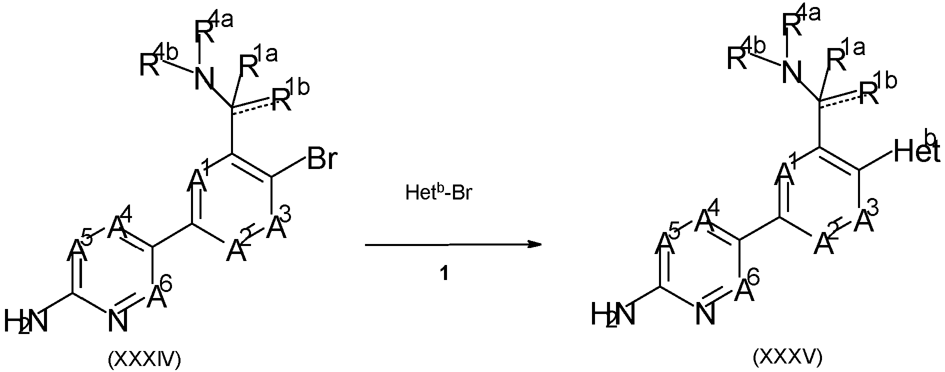
1: an intermediate of Formula (XXXIV) can react with Het^{b} bromides in the presence of a suitable photocatalyst, such as for example Ir[ppy]₂(dtbbpy)PF₆, a suitable Nickel source, such as for example NiCl₂ , a suitable ligand, such as for example di-tert-butylbispyridine (dtbbp), a suitable reductant, such as for example tris(trimethylsilyl)silane (TTMSS), a suitable base, such as sodium bicarbonate (Na₂CO₃), and a suitable solvent, such as DME, under blue light irradiation

### Scheme 10

An alternative to synthesis of compounds of Formula (I), can be prepared according to the following reaction in Scheme 10, as for example, compounds of Formula (XXXVII) wherein R^{1a} represents hydrogen, R^{1b} and R^{4b} are taken together to form together with the atoms to which they attached a monocyclic 6- or 7-membered fully saturated heterocyclyl containing at least 1N-atom and substituted; n and p represent 1 or 2 (but only one of n and p can represent 2), Y represents O , S, or N, and Z represents SnBu₃ or SiMe₃. A skilled person will understand that R^{q} and R^{z} can be selected from the list of substituents as defined in the scope of the present invention and do also include bicyclic spiro moieties formed together with the N-containing heterocyclyls to which they are attached. A skilled person will understand that R^{q} and R^{z} for example will not represent cyano or a directly attached amine substituent.

All other variables are defined according to the scope of the present invention 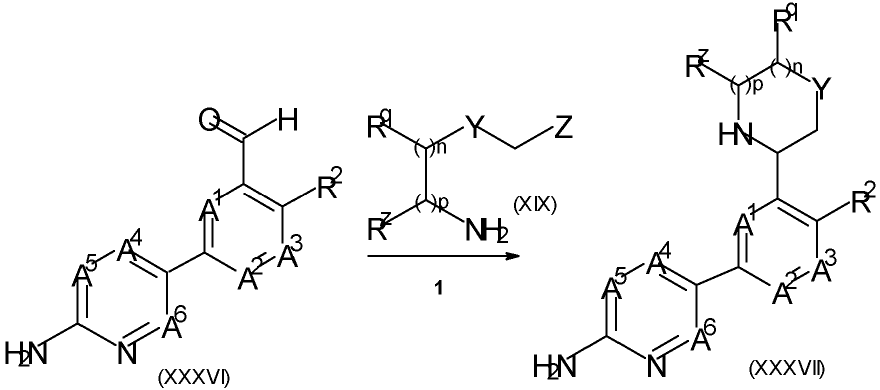
1: an intermediate of Formula (XXXVI) can react with a compound of Formula (XIX, Y represents O, S or N, Z = SnBu₃) in the presence of an oxidant, such as for example Cu(OTf)₂, a suitable ligand, such as for example Lutidine or PhBox, and a suitable solvent or solvent mixture, such as hexafluoroisopropanol (HFIP) and DCM
or an intermediate of Formula (XXXVI) can react with a compound of Formula (XIX, Y = N, Z =SiMe₃ ) in the presence of a photocatalyst, such as for example Ir[ppy]₂(dtbbpy)PF₆, and a suitable solvent or solvent mixture, such as trifluoroethanol (TFE) and CH₃CN under blue light irradiation
or an intermediate of Formula (XXXVI) can react with a compound of Formula (XIX, Y = S, Z = SiMe₃) in the presence of a photocatalyst, such as for example Ir[ppy]₂(dtbbpy)PF₆, a suitable acid or an acid mixture such as for example Bi(OTf)₃, Cu(OTf)₂, BF₃.2MeOH in a suitable solvent CH₃CN under blue light irradiation
or an intermediate of Formula (XXXVI) can react with a compound of Formula (XIX, Y= O, Z = SiMe₃) in the presence of a photocatalyst, such as for example triphenylpyrilium (TPP), a suitable acid such as for example TMSOTf, and a suitable solvent or solvent mixture, such as hexafluoroisopropanol (HFIP) and CH₃CN under blue light irradiation.

In the preparation of compounds of the present invention, protection of remote functionality (e.g., primary amine) of intermediates may be necessary. The need for such protection will vary depending on the nature of the remote functionality and the conditions of the preparations methods. Suitable amino-protecting groups (NH-Pg) include t-butoxycarbonyl (Boc), acetyl ... The need for such protection is readily determined by one skilled in the art.

It will be appreciated that where appropriate functional groups exist, compounds of various formulae or any intermediates used in their preparation may be further derivatised by one or more standard synthetic methods employing condensation, substitution, oxidation, reduction, or cleavage reactions. Particular substitution approaches include conventional alkylation, arylation, heteroarylation, acylation, sulfonylation, halogenation, nitration, formylation and coupling procedures.

The compounds of Formula (I) may be synthesized in the form of racemic mixtures of enantiomers which can be separated from one another following art-known resolution procedures. The racemic compounds of Formula (I) containing a basic nitrogen atom may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically.

In the preparation of compounds of the present invention, protection of remote functionality (e.g., primary or secondary amine) of intermediates may be necessary. The need for such protection will vary depending on the nature of the remote functionality and the conditions of the preparation methods. Suitable amino-protecting groups (NH-Pg) include acetyl, trifluoroacetyl, t-butoxycarbonyl (Boc), benzyloxycarbonyl (CBz) and 9-fluorenylmethyleneoxycarbonyl (Fmoc). The need for such protection is readily determined by one skilled in the art. For a general description of protecting groups and their use, see T. W. Greene and P. G. M. Wuts, Protective Groups in Organic Synthesis, 4th ed., Wiley, Hoboken, New Jersey, 2007.

### Pharmacology

It has been found that the compounds of the present invention inhibit Hpk1 activity.

An Hpk1 inhibitor is a compound that reduces Hpk1 functions, such as the ability to recruit proteins to the TCR and phosphorylate proteins like SLP76 and GADS.

The compounds of the present invention inhibit the Hpk1 kinase. Therefore, the compounds may be useful in the treatment or prevention, in particular in the treatment, of diseases that are susceptible to the effects of the immune system, such as cancer and viral infection.

The compounds of the present invention may enhance an immune response.

The compounds of the present invention may increase T cell activity.

The compounds of the present invention may have anti-tumoral properties through immune modulation.

In particular the compounds according to the present invention or pharmaceutical compositions thereof may be useful for treating or preventing, in particular treating, of diseases such as lung cancer, melanoma, head and neck cancer, esophageal cancer, bladder and urothelial cancer, liver cancer, kidney cancer, prostate cancer and hematopoietic cancer.

In some embodiments, the inhibition of HPK1 by a provided compound may be useful in treating or preventing, in particular treating, the following non-limiting list of cancers: breast cancer, lung cancer, esophageal cancer, bladder cancer, hematopoietic cancer, lymphoma, medulloblastoma, rectum adenocarcinoma, colon adenocarcinoma, gastric cancer, pancreatic cancer, liver cancer, adenoid cystic carcinoma, lung adenocarcinoma, head and neck squamous cell carcinoma, brain tumors, hepatocellular carcinoma, renal cell carcinoma, melanoma, oligodendroglioma, ovarian clear cell carcinoma, and ovarian serous cystadenoma.

Examples of cancers which may be treated or prevented, in particular treated, include, but are not limited to, acoustic neuroma, adenocarcinoma, adrenal gland cancer, anal cancer, angiosarcoma (e.g., lymphangio sarcoma, lymphangioendothelio sarcoma, hemangio sarcoma), appendix cancer, benign monoclonal gammopathy, biliary cancer (e.g., cholangiocarcinoma), bladder cancer, breast cancer (e.g., adenocarcinoma of the breast, papillary carcinoma of the breast, mammary cancer, medullary carcinoma of the breast), brain cancer (e.g., meningioma; glioma, e.g., astrocytoma, oligodendroglioma; medulloblastoma), bronchus cancer, carcinoid tumor, cervical cancer (e.g., cervical adenocarcinoma), chordoma, choriocarcinoma, craniopharyngioma, colorectal cancer (e.g., colon cancer, rectal cancer, colorectal adenocarcinoma), epithelial carcinoma, ependymoma, endothelio sarcoma (e.g., Kaposi's sarcoma, multiple idiopathic hemorrhagic sarcoma), endometrial cancer (e.g. , uterine cancer, uterine sarcoma), esophageal cancer (e.g., adenocarcinoma of the esophagus, Barrett' s adenocarinoma), Ewing sarcoma, eye cancer (e.g., intraocular melanoma, retinoblastoma), familiar hypereosinophilia, gall bladder cancer, gastric cancer (e.g., stomach adenocarcinoma), gastrointestinal stromal tumor (GIST), head and neck cancer (e.g., head and neck squamous cell carcinoma, oral cancer (e.g., oral squamous cell carcinoma (OSCC), throat cancer (e.g., pharyngeal cancer, laryngeal cancer, nasopharyngeal cancer, oropharyngeal cancer)), hematopoietic cancers (e.g., leukemia such as acute lymphocytic leukemia (ALL) (e.g., B-cell ALL, T-cell ALL), acute myelocytic leukemia (AML) (e.g., B-cell AML, T-cell AML), chronic myelocytic leukemia (CML) (e.g., B-cell CML, T-cell CML), and chronic lymphocytic leukemia (CLL) (e.g., B-cell CLL, T- cell CLL); lymphoma such as Hodgkin lymphoma (HL) (e.g., B-cell HL, T-cell HL) and non-Hodgkin lymphoma (NHL) (e.g., B-cell NHL such as diffuse large cell lymphoma (DLCL) (e.g., diffuse large B-cell lymphoma (DLBCL)), follicular lymphoma, chronic lymphocytic leukemia/small lymphocytic lymphoma (CLL/SLL), mantle cell lymphoma (MCL), marginal zone B-cell lymphomas (e.g., mucosa-associated lymphoid tissue (MALT) lymphomas, nodal marginal zone B-cell lymphoma, splenic marginal zone B-cell lymphoma), primary mediastinal B-cell lymphoma, Burkitt lymphoma, lymphoplasmacytic lymphoma (i.e., "Waldenstrom's macro globulinemia"), immunoblastic large cell lymphoma, hairy cell leukemia (HCL), precursor B -lymphoblastic lymphoma and primary central nervous system (CNS) lymphoma; and T-cell NHL such as precursor T-lymphoblastic lymphoma/leukemia, peripheral T-cell lymphoma (PTCL) (e.g., cutaneous T-cell lymphoma (CTCL) (e.g., mycosis fungiodes, Sezary syndrome), angioimmunoblastic T-cell lymphoma, extranodal natural killer T-cell lymphoma, enteropathy type T-cell lymphoma, subcutaneous panniculitis-like T-cell lymphoma, anaplastic large cell lymphoma); a mixture of one or more leukemia/lymphoma as described above; and multiple myeloma (MM)), heavy chain disease (e.g. , alpha chain disease, gamma chain disease, mu chain disease), hemangioblastoma, inflammatory myofibroblastic tumors, immunocytic amyloidosis, kidney cancer (e.g., nephroblastoma a.k.a. Wilms' tumor, renal cell carcinoma), liver cancer (e.g., hepatocellular cancer (HCC), malignant hepatoma), lung cancer (e.g., bronchogenic carcinoma, non-small cell lung cancer (NSCLC), squamous lung cancer (SLC), adenocarcinoma of the lung, *Lewis lung carcinoma,* lung neuroendocrine tumors: typical carcinoid, atypical carcinoid, small cell lung cancer (SCLC), and large cell neuroendocrine carcinoma), leiomyosarcoma (LMS), mastocytosis (e.g., systemic mastocytosis), myelodysplastic syndromes (MDS), mesothelioma, myeloproliferative disorder (MPD) (e.g., polycythemia Vera (PV), essential thrombocytosis (ET), agnogenic myeloid metaplasia (AMM) a.k.a. myelofibrosis (MF), chronic idiopathic myelofibrosis, chronic myelocytic leukemia (CML), chronic neutrophilic leukemia (CNL), hypereosinophilic syndrome (HES)), neuroblastoma, neurofibroma (e.g., neurofibromatosis (NF) type 1 or type 2, schwannomatosis), neuroendocrine cancer (e.g., gastroenteropancreatic neuroendoctrine tumor (GEP-NET), carcinoid tumor), osteosarcoma, ovarian cancer (e.g., cystadenocarcinoma, ovarian embryonal carcinoma, ovarian adenocarcinoma), papillary adenocarcinoma, pancreatic cancer (e.g., pancreatic andenocarcinoma, intraductal papillary mucinous neoplasm (IPMN), Islet cell tumors), penile cancer (e.g., Paget' s disease of the penis and scrotum), pinealoma, primitive neuroectodermal tumor (PNT), prostate cancer (e.g., prostate adenocarcinoma), rectal cancer, rhabdomyosarcoma, salivary gland cancer, skin cancer (e.g., squamous cell carcinoma (SCC), keratoacanthoma (KA), melanoma, basal cell carcinoma (BCC)), small bowel cancer (e.g., appendix cancer), soft tissue sarcoma (e.g., malignant fibrous histiocytoma (MFH), liposarcoma, malignant peripheral nerve sheath tumor (MPNST), chondrosarcoma, fibrosarcoma, myxosarcoma), sebaceous gland carcinoma, sweat gland carcinoma, synovioma, testicular cancer (e.g., seminoma, testicular embryonal carcinoma), thyroid cancer (e.g., papillary carcinoma of the thyroid, papillary thyroid carcinoma (PTC), medullary thyroid cancer), urethral cancer, vaginal cancer, and vulvar cancer (e.g. , Paget' s disease of the vulva).

The compounds according to the present invention or pharmaceutical compositions thereof, may also have therapeutic applications in combination with immune modulatory agents, such as inhibitors of the PD1/PDL1 immune checkpoint axis, for example antibodies (or peptides) that bind to and/or inhibit the activity of PD-1 or the activity of PD-L1.

The compounds according to the present invention or pharmaceutical compositions thereof, may also be combined with radiotherapy or chemotherapeutic agents.

The compounds according to the present invention or pharmaceutical compositions thereof, may may also be combined with other agents that stimulate or enhance the immune response, such as vaccines.

The compounds according to the present invention or pharmaceutical compositions thereof, may calso be used to enhance the antigen presenting properties of dendritic cells produced ex vivo.

The compounds according to the present invention or pharmaceutical compositions thereof may be useful for treating or preventing, in particular treating, of infectious diseases, such as viral, bacterial, fungal, and parasitic infections. The compounds according to the present invention or pharmaceutical compositions thereof may be useful for treating or preventing, in particular treating, of chronic infections. The compounds according to the present invention or pharmaceutical compositions thereof may be useful for treating or preventing, in particular treating, of chronic viral infections.

The invention relates to compounds of Formula (I) and pharmaceutically acceptable addition salts, N-oxides, and solvates thereof, for use as a medicament.

The invention relates to compounds of Formula (I) and pharmaceutically acceptable addition salts, N-oxides, and solvates thereof, for use in the inhibition of HPK1 activity.

The compounds of the present invention can be "anti-cancer agents", which term also encompasses "anti-tumor cell growth agents" and "anti-neoplastic agents".

The invention relates to compounds of Formula (I) and pharmaceutically acceptable addition salts, N-oxides, and solvates thereof, for use in the treatment of diseases mentioned above.

The invention relates to compounds of Formula (I) and pharmaceutically acceptable addition salts, N-oxides, and solvates thereof, for the treatment or prevention, in particular for the treatment, of said diseases.

The invention relates to compounds of Formula (I) and pharmaceutically acceptable addition salts, N-oxides, and solvates thereof, for the treatment or prevention, in particular in the treatment, of HPK1 mediated diseases or conditions.

The invention relates to compounds of Formula (I) and pharmaceutically acceptable addition salts, N-oxides, and solvates thereof, for the manufacture of a medicament.

The invention relates to compounds of Formula (I) and pharmaceutically acceptable addition salts, N-oxides, and solvates thereof, for the manufacture of a medicament for the inhibition of HPK1.

The invention relates to compounds of Formula (I) and pharmaceutically acceptable addition salts, N-oxides, and solvates thereof, for the manufacture of a medicament for the treatment or prevention, in particular for the treatment, of any one of the disease conditions mentioned hereinbefore.

The invention relates to compounds of Formula (I) and pharmaceutically acceptable addition salts, N-oxides, and solvates thereof, for the manufacture of a medicament for the treatment of any one of the disease conditions mentioned hereinbefore.

The invention relates to compounds of Formula (I) and pharmaceutically acceptable addition salts, N-oxides, and solvates thereof, can be administered to mammals, preferably humans, for the treatment or prevention of any one of the diseases mentioned hereinbefore.

One skilled in the art will recognize that a therapeutically effective amount of the compounds of the present invention is the amount sufficient to have therapeutic activity and that this amount varies *inter alias,* depending on the type of disease, the concentration of the compound in the therapeutic formulation, and the condition of the patient. An effective therapeutic daily amount would be from about 0.005 mg/kg to 100 mg/kg. In particular, an effective therapeutic daily amount would be 25 mg/kg BID (twice a day) or 50 mg/kg BID. In particular, an effective therapeutic daily amount would be 50 mg/kg QD (once a day) or 100 mg/kg QD. The amount of a compound according to the present invention, also referred to herein as the active ingredient, which is required to achieve a therapeutically effect may vary on case-by-case basis, for example with the particular compound, the route of administration, the age and condition of the recipient, and the particular disorder or disease being treated.

The present invention also provides compositions for preventing or treating the disorders referred to herein. Said compositions comprising a therapeutically effective amount of a compound of Formula (I), or a pharmaceutically acceptable addition salt, an N-oxide, or a solvate thereof, and a pharmaceutically acceptable carrier or diluent.

While it is possible for the active ingredient to be administered alone, it is preferable to present it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions may be prepared by any methods well known in the art of pharmacy, for example, using methods such as those described in Gennaro et al. Remington's Pharmaceutical Sciences (18th ed., Mack Publishing Company, 1990, see especially Part 8 : Pharmaceutical preparations and their Manufacture).

The compounds of the present invention may be administered alone or in combination with one or more additional therapeutic agents. Combination therapy includes administration of a single pharmaceutical dosage formulation which contains a compound according to the present invention and one or more additional therapeutic agents, as well as administration of the compound according to the present invention and each additional therapeutic agent in its own separate pharmaceutical dosage formulation.

Therefore, an embodiment of the present invention relates to a product containing as first active ingredient a compound according to the invention and as further active ingredient one or more anticancer agent, as a combined preparation for simultaneous, separate or sequential use in the treatment of patients suffering from cancer.

The one or more other medicinal agents and the compound according to the present invention may be administered simultaneously (e.g. in separate or unitary compositions) or sequentially in either order. In the latter case, the two or more compounds will be administered within a period and in an amount and manner that is sufficient to ensure that an advantageous or synergistic effect is achieved. It will be appreciated that the preferred method and order of administration and the respective dosage amounts and regimes for each component of the combination will depend on the particular other medicinal agent and compound of the present invention being administered, their route of administration, the particular condition, in particular tumour, being treated and the particular host being treated.

The following examples further illustrate the present invention.

### EXAMPLES

Several methods for preparing the compounds of this invention are illustrated in the following examples. Unless otherwise noted, all starting materials were obtained from commercial suppliers and used without further purification, or alternatively can be synthesized by a skilled person by using well-known methods.

Hereinafter, "DCM" means dichloromethane; "DME" means 1,2-dimethoxyethane; "DMF-DMA" means *N,N-*dimethylformamide dimethyl acetal; "ACN" means acetonitrile; "Ac" means acetyl; "LAH" means lithium aluminium hydride; "sol." means solution; "prep." means preparative; "aq." means aqueous; "Int." Means Intermediate; "Co." means compound; "r.t." means room temperature; "r.m." means reaction mixture; "KOAc" means potassium acetate; "AcONH₄" means ammonium acetate; "BisPin" means bis(pinacolato)diboron; "DCE" means 1,2-dichloroethane; "AcOEt" or "EtOAc" means ethylacetate; "DIPE" means diisopropyl ether; "Boc" means *tert*-butoxycarbonyl; "DMA" means dimethylacetamide; "HBr" means hydrogen bromide ; "MeCN" means acetonitrile; "HOBT" means 1-hydroxy-1H-benzotriazole; " TMS" means trimethylsilyl; "DIPE" means diisopropylether; " "DMAP" means 4-(dimethylamino)pyridine; "MeOH" means methanol; "LC" means liquid chromatography; "LCMS" means Liquid Chromatography/Mass spectrometry; "HPLC" means high-performance liquid chromatography; "NH₄Cl" means ammonium chloride; "H₂O" means water "TFA" means trifluoroacetic acid; "m.p." means melting point; "N₂" means nitrogen; " Na₂SO₄" means sodium sulfate; "AcOH" means acetic acid; "MeOD" means deuterated methanol;"D₂" means deuterium; "RP" means reversed phase; "min" means minute(s); "EtOAc" means ethyl acetate; "Et₃N" means triethylamine; "EtOH" means ethanol; "THF" means tetrahydrofuran; "MnO₂" means manganese dioxide; "Celite^{®}" means diatomaceous earth ; "MgSO₄" means magnesium sulfate; "NH₄OH" means ammonium hydroxide; "K₂CO₃" means potassium carbonate; "DMF" means *N,N-*dimethyl formamide; "Na₂SO₃" means sodium sulfite; "NaHCO₃" means sodium bicarbonate; "Na₂CO₃" means sodium carbonate ; "HCl" means hydrogen chloride "NaOH" means sodium hydroxide; "HCOOH" means formic acid; "DMSO" means dimethyl sulfoxide; 'iPrOH" means 2-propanol; "iPrNH₂" means isopropylamine; "SFC" means Supercritical Fluid Chromatography; "CO₂ " means carbon dioxide; "Et₃N" means triethylamine; "DIPEA" means N,N-diisopropylethylamine; "Pd(PPh₃)₄" means tetrakis(triphenylphosphine)palladium; "w/v" means weight/volume; "[Ir(dtbbpy)(ppy)2] PF₆" means (4,4'-Di-t-butyl-2,2'-bipyridine)bis[2-(2-pyridinyl-kN)phenyl-kC]iridium(III) hexafluorophosphate,"SLAP TM" means 2-(((Trimethylsilyl)methyl)thio)ethanamine; "SLAP" means silicon amine protocol; "SLAP hydropyridopyrazine" means (1-((trimethylsilyl)methyl)piperidin-2-yl)methanamine; "m-CPBA" means 3-chloroperbenzoic acid "PPh₃" means triphenylphosphine ; "Et₂O" means diethyl ether; "Pd/C" means palladium on carbon; "Et" means ethyl; "Me" means methyl; "h" means hours; "PdCl₂(dppf)" means [1,1'-bis(diphenylphosphino-κP)ferrocene]dichloropalladium; and "quant." means quantitative.

When a stereocentre is indicated with 'RS' this means that a racemic mixture was obtained at the indicated centre, unless otherwise indicated.

### Preparation of the Intermediates and the final Compounds

### Synthesis of intermediate 1:

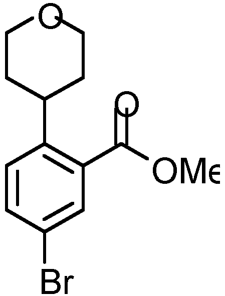

To a mixture of Zn (69.9 g, 1.07 mol) in DMA (500 mL) was added ethylene dibromide (16.5 g, 88 mmol, 6.64 mL) in one portion under N₂. Then TMSCl (6.83 g, 62.9 mmol, 8.0 mL) was added slowly and the mixture was stirred for 30 min at 25 °C. A solution of 4-iodotetrahydropyran (200 g, 943 mmol) in DMA (500 mL) was added dropwise slowly (30 min) to maintain temperature below 50°C, the resulting mixture was stirred at 25 °C for 1.5 h and then added via a cannula to a solution of methyl 5-bromo-2-iodobenzoate (214.4 g, 629 mmol), Pd(dppf)Cl₂.CH₂Cl₂ (25.7 g, 31.4 mmol) and CuI (12.0 g, 62.9 mmol) in DMA (2400 mL) under N₂, the color of the mixture turned brown, then the mixture was heated and stirred at 80 °C for 14 h under N₂. Six batches were combined together. The mixture was cooled to rt and diluted with EtOAc (10 L). A saturated aqueous solution of NH₄Cl (18 L) and H₂O (10 L) were added and the mixture was stirred for 30 mins, then filtered through a pad of Celite^{®}. The cake was washed with EtOAc (5 L* 2), the filtrate was extracted with EtOAc ( 8 L * 4), the combined organic phase was washed with brine (15 L* 2), dried with anhydrous Na₂SO₄, filtered and concentrated in vacuum to give the crude product. The crude product was purified by silica gel chromatography eluted with Petroleum ether : EtOAc =100:0-93:7 to give intermediate **1** (700 g, 61% yield) as a yellow solid.

The following intermediate was prepared via an analogous procedure :

| Intermediate | Structure | Quantity | Yield |
|---|---|---|---|
| Intermediate **2** | 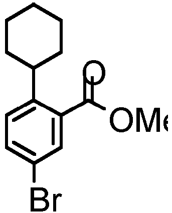 | | |
| Starting from iodocyclohexane | | 0.7 g | 40% |

### Synthesis of intermediate 3:

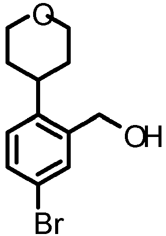

To a mixture of intermediate **1** (220 g, 735 mmol) in THF (2200 mL) was added portionwise LAH (27.9 g, 735 mmol) at 0-10 °C over 30 min. The grey suspension was stirred at 0-30 °C for 1 h. The mixture was cooled to 0 °C and H₂O (800 mL) was added dropwise and then the mixture was stirred for 30 min. The three batches were worked together. The mixture was filtered and the cake was washed with EtOAc (3 L * 3), the filtrated was extracted with EtOAc (2 L * 3), the combined organic phase was washed with brine (2 L *2), dried with Na₂SO₄, filtered and concentrated in vacuum. The crude product was used for next step without purification to give intermediate **3** (576 g, 87% yield) as a yellow oil.

The following intermediate was prepared via an analogous procedure :

| Intermediate | Structure | Quantity | Yield |
|---|---|---|---|
| Intermediate **4** | 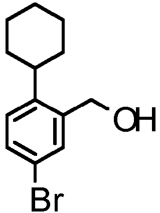 | | |
| Starting from intermediate 2 | | 0.53 g | 84% |

### Synthesis of intermediate 5 :

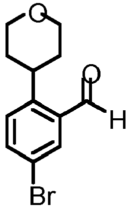

To a mixture of intermediate **3** (325 g, 1.2 mol) in CH₂Cl₂ (3200 mL) was added MnO₂ (834 g, 9.6 mol) in one portion. The black mixture was stirred at 30 °C for 34h. The two batches were worked together. The mixture was filtered through Celite^{®} and the cake was washed with CH₂Cl₂ (2 L *3), the filtrate was concentrated in vacuum. The crude product was purified by silica gel column chromatography (Petroleum ether/Ethyl acetate=100:0-85:15) to give 560 g as a crude product which was stirred with Petroleum ether (800 mL) at 30 °C for 4h, filtered and the cake was washed with Petroleum ether (200 mL * 2) and concentrated in high vacuum to give intermediate **5** (502 g, 1.85 mol, 77% yield) as a white solid.

The following intermediate was prepared via an analogous procedure :

| Intermediate | Structure | Quantity | Yield |
|---|---|---|---|
| Intermediate **6** | 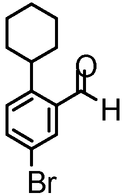 | | |
| Starting from intermediate 4 | | 0.35 g | 67% |

### Synthesis of intermediate 7 :

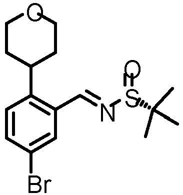

Titanium (IV) ethoxide (64.5 mL, 0.308 mol) was added dropwise to a solution of intermediate **5** (20.7 g, 77 mmol) and (S)-(-)-t-butylsulfinamide (18.06 g, 0.149 mol). The solution was stirred at rt overnight and the mixture was poured into brine and Ethyl acetate was added. The organic layer was separated, dried over MgSO₄, filtered and evaporated until dryness. The residue was purified br preparative LC (330g of irregular SiOH 35-40 µm GraceResolv, mobile phase : gradient from 100% DCM to DCM 95%/ MeOH 5%/NH₄OH 0.1%. The pure fractions were collected and the solvent evaporated until dryness to give intermediate **7** (30 g, y= quant.)

The following intermediates were prepared via an analogous procedure :

| Intermediate | Structure | Quantity | Yield |
|---|---|---|---|
| Intermediate **8** | 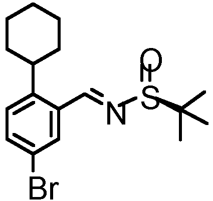 | | |
| Starting from intermediate 6 | | 0.32 g | 66% |
| Intermediate **9** | 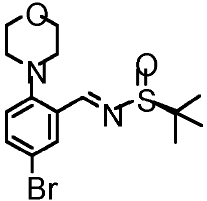 | | |
| Starting from 5-bromo-2-(N-morpholino)-benzaldehyde | | 3.2 g | 76% |
| Intermediate **10** | 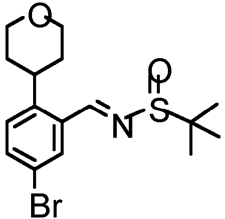 | | |
| Starting from intermediate 5 | | 9.5 g | 94% |

### Synthesis of intermediate 11 :

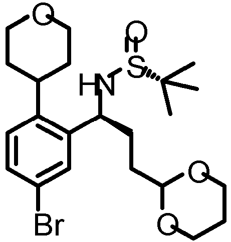

A solution of intermediate **7** (41 g, 0.11 mol) in THF (500 mL) was added dropwise at -78°C to a solution of (1,3-dioxan-2-ylethyl)magnesium bromide (96.6 g, 0.44 mol) in THF (550mL). The reaction mixture was stirred at -78°C for 0.5h. A saturated solution of NH₄Cl was added and the reaction mixture was extracted with ethyl acetate, the organic layer was separated, dried over MgSO₄, filtered and evaporated until dryness to give intermediate **11** (60 g, y= quant.)

The following intermediates were prepared via an analogous procedure :

| Intermediate | Structure | Quantity | Yield |
|---|---|---|---|
| Intermediate **12** | 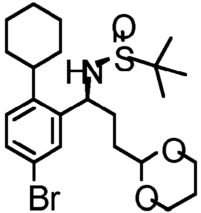 | | |
| Starting from intermediate 8 | | 0.28 g | 67% |
| Intermediate **13** | 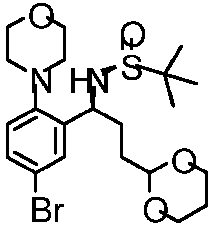 | | |
| Starting from intermediate 9 | | 1.23 g | 93% |

### Synthesis of intermediate 14 :

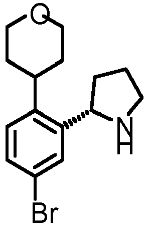

Intermediate **11** (66.4 g, 0.135 mol) was slowly added to a mixture of TFA (597 mL) and water (33 mL) to maintain temperature below 25°C. The reaction mixture was stirred at rt for 40 min. Triethylsilane (221 mL, 1.38 mol) was added and the reaction mixture vigorously stirred at rt overnight. The reaction mixture was evaporated till dryness . A purification was performed via preparative LC (stationary phase : irregular SiOH 35-40 µM 220+330g, GraceResolv, mobile phase : from DCM 100% to DCM 80%/MeOH/20%/NH4OH 0.1%). The pure fractions were evaporated and taken up in DCM and water, basified with K₂CO₃. The organic layer was separated , dried over MgSO₄, filtered and evaporated to give intermediate **14** (33.2g, 79%).
[α]_{d}: - 47.5° (589 nm, c 0.36 w/v %, DMF, 20 °C)

The following intermediates were prepared via an analogous procedure :

| Intermediate | Structure | Quantity | Yield |
|---|---|---|---|
| Intermediate **15** | 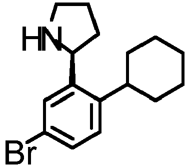 | | |
| Starting from intermediate 12 | | 0.13 g | 73% |
| Intermediate **16** | 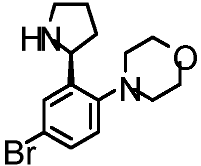 | | |
| Starting from intermediate 13 | | 0.18 g | 23% |

### Synthesis of intermediate 17 :

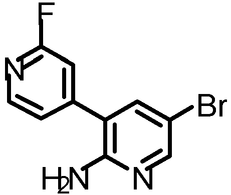

A solution of 2-amino-3-iodo-5-bromopyridine (25.12 g, 84.0 mmol), 2-fluoropyridine-4-boronic acid pinacol ester (18.75 g, 84.0 mmol) and sodium carbonate (17.81 g, 168 mmol) in 1,4-dioxane (240 mL) and water (35 mL) in a sealed tube was degassed during 10 min. After addition of PdCl₂(dppf) (3.44g, 4.2 mmol), the reaction mixture was stirred at 90°C overnight. Water, potassium carbonate and ethyl acetate were added. The organic layer was separated, dried over MgSO₄, filtered and evaporated until dryness. DCM was added to the residue, the insoluble was filtered, washed with DCM and dried yielding 13.6 g (60.4%) of intermediate **17.** The filtrate was purified by preparative LC (SiOH 30µm Interchim, from 100% DCM to DCM 90%/ MeOH 10%/ NH₄OH 0.1%). The pure fractions were collected and evaporated until dryness to give intermediate **17** (6 g, 26.6%).

### Synthesis of intermediate 18 :

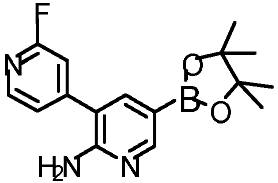

To a solution of intermediate **17** (13.6 g, 50.7 mmol), bis(pinacolato)diboron (14.2 g, 55.8 mmol) and potassium acetate (14.94 g, 152 mmol) in 1,4-dioxane (490 mL) was added under nitrogen atmosphere PdCl2(dppf) (1.66 g, 2.03 mmol). The reaction mixture was heated at 80°C for 20 hours, cooled to room temperature. Ethyl acetate was added and the mixture was filtered through a pad of celite and the filtrate was evaporated until dryness. Water and Ethyl acetate were added, the insoluble was filtered, taken up into diethtlether then filtered and dried to give 9.13 g of intermediate **18** (57.1%). The organic layer was separated, dried over MgSO₄, filtered and evaporated until dryness. The residue was taken up into diethylether, stirred for 30 min then filtered and dried to give 6 g of intermediate **18** (37.7%).

The following intermediates were prepared via an analogous procedure as for intermediate 18:

| Intermediate | Structure | Quantity | Yield |
|---|---|---|---|
| Intermediate **23** | 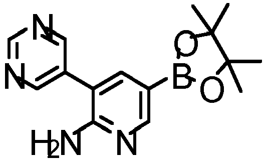 | | |
| Starting from 5-bromo-3-(pyrimidin-5-yl)pyridine-2-amine | | 0.527 g | 56% (80% purity) |
| Intermediate **24** | 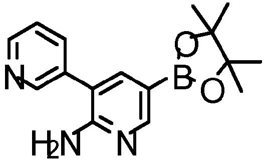 | | |
| Starting from 5-bromo-3-(pyridin-3-yl)pyridin-2-amine | | 0.263 g | 29% |
| Intermediate **25** | 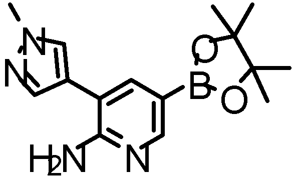 | | |
| Starting from 5-bromo-3-(1-methyl-1H-pyrazol-4-yl)pyridin-2-amine | | 0.465 g | 24% |
| Intermediate **26** | 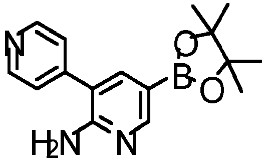 | | |
| Starting from 5-bromo-[3,4'-Bipyridin]-2-amine | | 2.2 g | 38% |

### Synthesis of intermediate 19 :

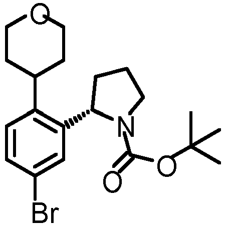

Di-tert-butyl dicarbonate (44.3 g, 0.203 mol) was added to a solution of intermediate **14** (21 g, 67.7 mmol) and 4-dimethylaminopyridine (1.65g 13.5 mmol) in DCM (680 mL). The reaction mixture was stirred at rt for 24h. Water and DCM were added, the organic layer was separated, dried over MgSO₄, filtered and evaporated until dryness. The residue was crystallized in diethylether, filtered and dried to give 22.2 g of intermediate **19** (80%).

The following intermediates were prepared via an analogous procedure :

| Intermediate | Structure | Quantity | Yield |
|---|---|---|---|
| Intermediate **20** | 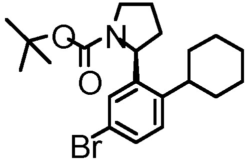 | | |
| Starting from intermediate 15 | | 0.2 g | Quant. |
| Intermediate **21** | 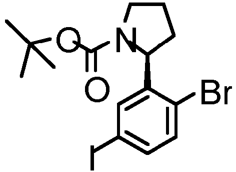 | | |
| Starting from Pyrrolidine,2-(2-bromo-5-iodophenyl), (2S) | | 27.5g | 58% |

### Synthesis of intermediate 22 :

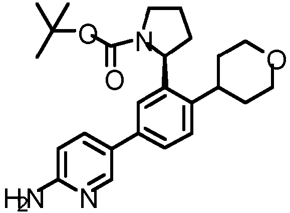

A mixture of 2-aminopyridine-5-boronic acid pinacol ester (5.92 g, 26.9 mmol), intermediate **19** (9.2g, 22.4 mmol), Pd(PPh₃)₄ (2.6 g, 2.25 mmol) and sodium carbonate (7.1 g, 67.2 mmol) in 2-methyltetrahydrofuran (250 mL) and water (25 mL) was stirred under N₂ in a sealed tube at 85°C for 15 hours. The reaction mixture was cooled down; water, potassium carbonate and ethyl acetate were added. The organic layer was separated, dried over MgSO_{4,}, filtered and evaporated until dryness. The residue was purified by preparative LC (SiOH 30µm Interchim, from 100% DCM to DCM 90%/ MeOH 10%/ NH₄OH 0.1%). The fractions were collected and evaporated until dryness to give 11.6 g of a residue which was purified again via preparative LC (Stationary phase: irregular SiOH 40 µm 220g, Mobile phase: 60% Heptane, 5% MeOH (+5% NH₄OH), 35% AcOEt). The pure fractions were collected and evaporated until dryness to give 6 g of intermediate **22** (63%).

### Synthesis of intermediate 27 :

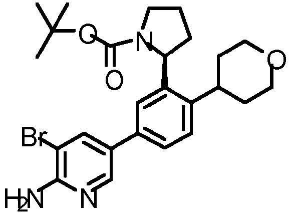

Bromine (0.73 mL, 14.2 mmol) was added dropwise to a solution of intermediate **22** (6 g, 14.2 mmol) in acetic acid (90 mL) at rt. After 20min, water and potassium carbonate were added. The aqueous phase was extracted with ethyl acetate and the organic layer was dried over MgSO_{4,}, filtered and evaporated until dryness. The residue was purified by preparative LC (SiOH 30µm Interchim, from 100% DCM to DCM 90%/ MeOH 10%/ NH₄OH 0.1%). The pure fractions were collected and evaporated until dryness to give 5.8 g of intermediate **27** (81%).

### Synthesis of intermediate 28 :

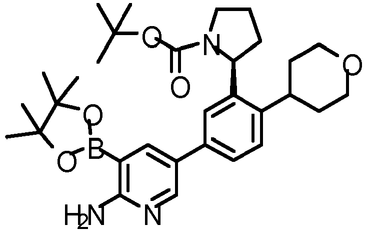

To a solution of intermediate **27** (0.51 g, 1 mmol), bis(pinacolato)diboron (0.3 g, 1.2 mmol) and potassium acetate (0.31 g, 3.1 mmol) in 1,4-dioxane (11 mL) was added under nitrogen atmosphere PdCl₂(dppf) (0.084 g, 0.1 mmol) in a schlenk. The reaction mixture was stirred and heated at 110°C for 20 hours, cooled at room temperature, then DCM was added and the mixture was filtered through a cellite pad and the filtrate was evaporated until dryness and the residue was used without purification for the next step.

### Synthesis of intermediate 29 :

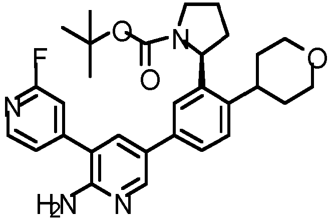

A mixture of intermediate **27** (6 g, 22.4 mmol), ), 2-fluoropyridine-4-boronic acid pinacol ester (3.2 g, 14.3 mmol), PdCl₂(dppf) (0.61 g, 0.75 mmol) and sodium carbonate (2.5 g, 23.9 mmol) in 1,4-dioxane (115 mL) and water (11.5 mL) was stirred under N₂ in a sealed tube at 90°C for 6 hours. The reaction mixture was cooled down, poured into water and potassium carbonate and the aqueous phase was extracted with ethyl acetate. The organic layer was separated, dried over MgSO_{4,}, filtered and evaporated until dryness. The residue was purified by preparative LC (220 g SiOH 30µm Interchim, from 100% DCM to DCM 90%/ MeOH 10%/ NH₄OH 0.1%). The pure fractions were collected and evaporated until dryness to give 6.8 g of intermediate **29** (quant.).

The following intermediates were prepared via an analogous procedure:

| Intermediate | Structure | Quantity | Yield |
|---|---|---|---|
| Intermediate **30** | 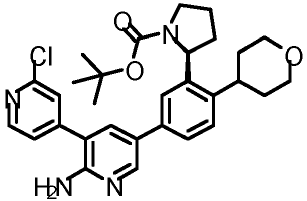 | 0.75 g | 71% |
| Starting from 2-Chloropyridine-4-boronic acid pinacol ester | | | |
| Intermediate **31** | 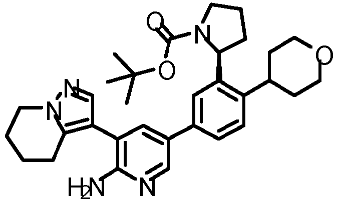 | 0.16 g | 59% |
| Starting from (4,5,6,7-TETRAHYDROPYRAZ OLO[1,5-A]PYRIDIN-3-YL)BORONIC ACID PINACOL ESTER | | | |
| Intermediate **32** | 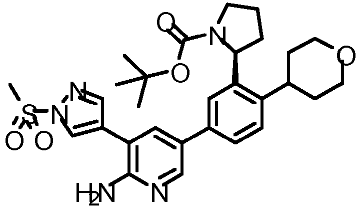 | 0.102 g | 58% |
| Starting from 1-(Methylsulfonyl) pyrazole-4-boronic acid pinacol ester | | | |
| Intermediate **33** | 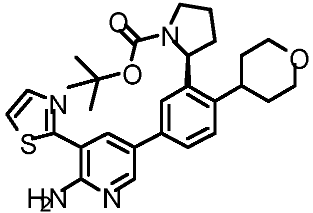 | | |
| Starting from 2-(Tributylstannyl) thiazole | | 0.042 g | 42% |
| Intermediate **34** | 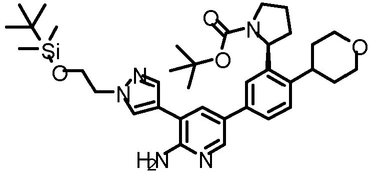 | 0.079 g | 41% |
| Starting from 1-(2-{ [tert-butyl(dimethyl) silyl]oxy }ethyl)-4-(4,4, 5,5-tetramethyl-1,3,2-dioxaborolan-2-yl)-1H-pyrazole | | | |
| Intermediate **35** | 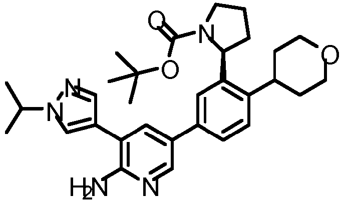 | | |
| Starting from 1-Isopropyl-1H-pyrazole-4-boronic acid pinacol ester | | 0.058 g | 36% |
| Intermediate **36** | 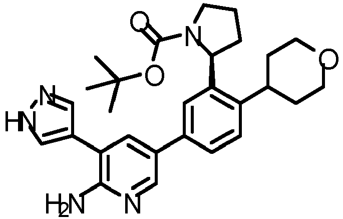 | | |
| Starting from 1-Boc-pyrazole-4-boronic acid pinacol ester | | 0.086 g | 59% |
| Intermediate **37** | 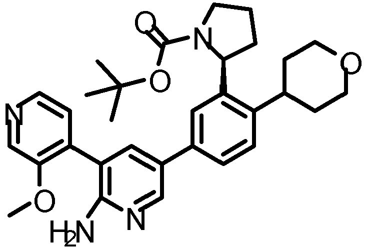 | | |
| Starting from 3-Methoxy-4-pyridineboronic acid pinacol ester | | 0.073 g | 99% |
| Intermediate **38** | 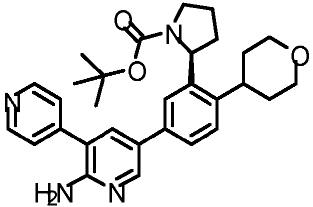 | | |
| Starting from Pyridine-4-boronic acid pinacol ester | | 0.127 g | 69% |
| Intermediate **39** | 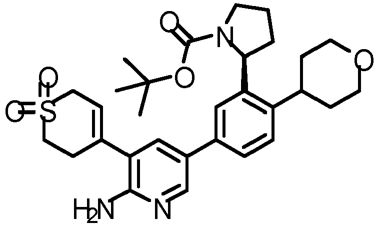 | | |
| Starting from 4-(4,4,5, 5-Tetramethyl-1,3,2-dioxaborolan-2-yl)-3,6-dihydro-2H-thiopyran 1,1-dioxide | | 0.38 g | Quant |
| Intermediate **40** | 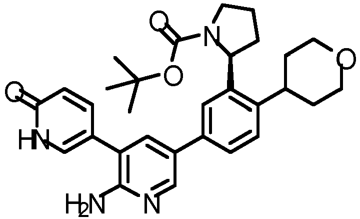 | | |
| Starting from 6-hydroxypyridine-3-boronic acid pinacol ester | | 0.086 g | 38% |
| Intermediate **41** | 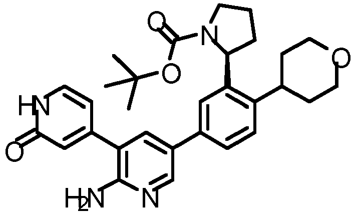 | | |
| Starting from 2-Hydroxypyridine-4-boronic acid pinacol ester | | 0.092g | 40% |
| Intermediate **42** | 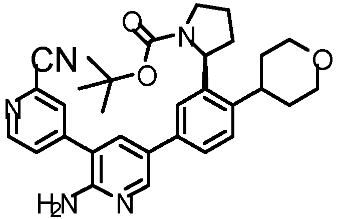 | | |
| Starting from 2-Cyanopyridine-4-boronic acid pinacol ester | | 0.23 g | 88% |
| Intermediate **43** | 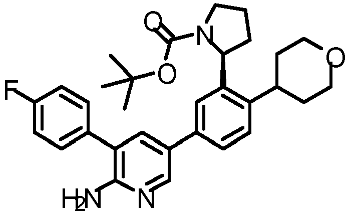 | | |
| Starting from 4-Fluorophenylboronic acid, pinacol ester | | 0.24 g | 94% |
| Intermediate **44** | 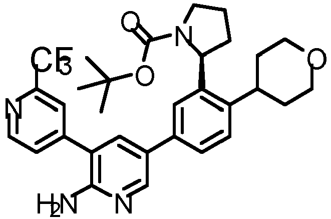 | | |
| Starting from 2-Trifluoromethyl-4-pyridineboronic acid pinacol ester | | 0.22 g | 78% |

The following intermediates were prepared via an analogous procedure as for intermediate 17 :

| Intermediate | Structure | Quantity | Yield |
|---|---|---|---|
| Intermediate **45** | 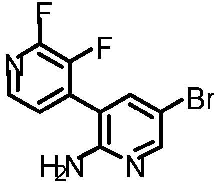 | | |
| Starting from 2,3-difluoropyridine-4-boronic acid | | 0.09 g | 31% |
| Intermediate **46** | 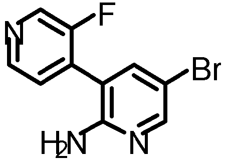 | | |
| Starting from 3-fluoropyridine-4-boronic acid | | 0.257g | 20% |
| Intermediate **47** | 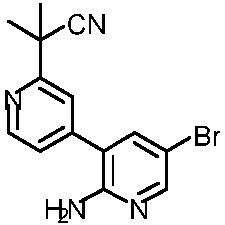 | 0.234 g | 70% |
| Starting from (2-(2-CYANOPROPAN-2-YL) PYRIDIN-4-YL)BORONIC ACID PINACOL ESTER | | | |
| Intermediate **48** | 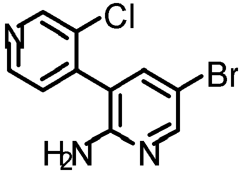 | | |
| Starting from 3-chloropyridine-4-boronic acid | | 0.139 g | 23% |
| Intermediate **49** | 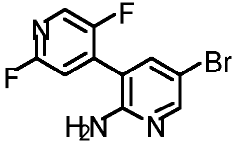 | | |
| Starting from 2,5-difluoropyridine-4-boronic acid | | 0.103 g | 28% |
| Intermediate **50** | 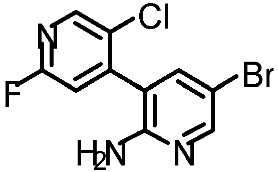 | | |
| Starting from 5-chloro-2-fluoropyridine-4-yl) boronic acid | | 0.179 g | 26% |
| Intermediate **51** | 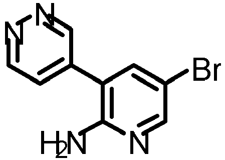 | | |
| Starting from 4-(tributylstannyl)-pyridazine | | 0.294 g | 69% |
| Intermediate **52** | 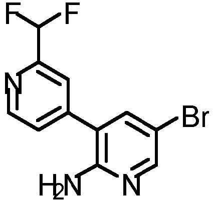 | 0.04 g | 31% |
| Intermediate **53** | 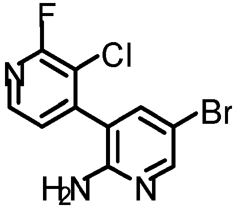 | 0.09 g | 30% |
| Starting from 3-chloro-2-fluoropyridine-4-boronic acid | | | |
| Intermediate **54** | 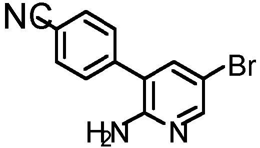 | 0.097 g | 52% |
| Starting from 4-Cyanophenylboronic acid | | | |
| Intermediate **55** | 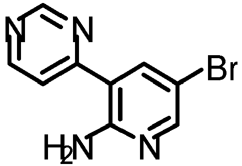 | 0.268 g | 41% |
| Starting from 4-(tributylstannyl)-pyrimidine | | | |
| Intermediate **56** | 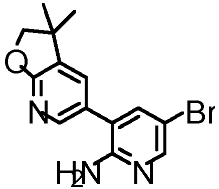 | 0.3g | 47% |
| Starting from intermediate 65 | | | |
| Intermediate **57** | 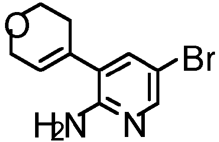 | 0.375g | 99% |
| Starting from 3,6-dihydro-2H-pyran-4-boronic acid pinacol ester | | | |
| Intermediate **58** | 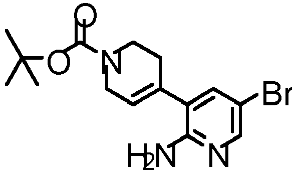 | 0.520g | 83% |
| Starting from N-Boc-1,2, 5,6-Tetrahydropyridine-4-Boronic Acid Pinacol Ester | | | |

### 1) Synthesis of intermediate 59 :

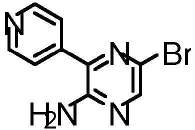

A mixture of 3,5-dibromopyrazin-2-amine (1.3 g, 5.14 mmol), 4-pyridinylboronic acid (0.948 g, 7.71 mmol) and sodium carbonate (1.09 g, 10.28 mmol) in 1,4-dioxane (50 mL) and water (12.5 mL) was degazed 10 min. After addition of PdCl₂(dppf) (0.21g, 0.257 mmol) the reaction mixture was heated at 90°C for 12h. The reaction mixture was filtered through a pad of Celite^{®}, water was added and the aqueous phase was extracted with DCM. The organic layer was dried over MgSO4, filtered and concentrated. The crude mixture was purified by flash chromatography using Heptane/Ethylacetate 0% to 100% as gradient. The desired fractions were collected and concentrated to give intermediate **59** (0.5 g, 38%).

2) The following intermediate was prepared via an analogous procedure :

| Intermediate | Structure | Quantity | Yield |
|---|---|---|---|
| Intermediate **60** | 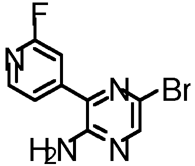 | 0.3 g | 28% |

### Synthesis of intermediate 61 :

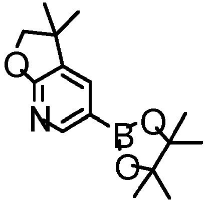

3) A mixture of 3,3-dimethyl-2,3-dihydrofuro[2,3-b]pyridine (2.8 g, 18.77 mmol), N-bromosuccinamide (10 g, 56.3 mmol) in acetonitrile (50 mL) was heated at 60°C for 18 hours. The mixture was concentrated and diluted with ethyl acetate (100 mL). The organic layer was washed with 1M Na₂SO₃ (30 mL, twice) and 1M NaHCO₃ (30 mL, twice). The organic layer was separated, dried over MgSO₄, filtered and evaporated until dryness. The residue was purified by column chromatography over silica gel (petroleum ether/ethyl acetate : 100/0 to 4/1). The pure fractions were collected and evaporated until dryness to give 3.7 g of 5-bromo-3,3-dimethyl-2H-furo[2,3-b] pyridine (86%.).

4) A mixture of 5-bromo-3,3-dimethyl-2H-furo[2,3-b] pyridine (0.5 g, 2.19 mmol), bis(pinacolato)diboron (0.72 g, 2.85 mmol), PdCl₂(dppf) (0.143 g, 0.175 mmol) and potassium acetate (0.645 g, 6.58 mmol) in Me-THF (7.5 mL) was stirred at 85°C under nitrogen atmosphere in a sealed tube overnight. The reaction mixture was cooled to rt, ethyl acetate was added and the reaction mixture was filtered through decalite. The filtrate was evaporated until dryness to give intermediate **61** used without purification for the next step.

### Synthesis of intermediate 62 :

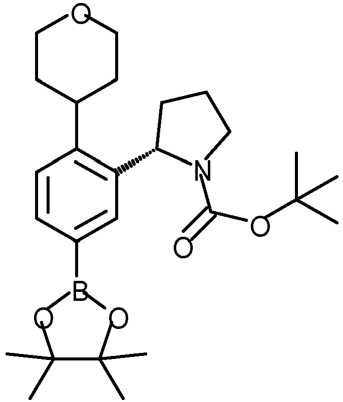

A mixture of intermediate **19** (4 g, 9.7 mmol), bis(pinacolato)diboron (3 g, 11.8 mmol), PdCl₂(dppf) (0.8 g, 0.98 mmol) and potassium acetate (2.87 g, 29.2 mmol) in 1,4-dioxane (90 mL) was stirred at 90°C under nitrogen atmosphere in a schlenk flask for 15 hours. The reaction mixture was cooled to rt, water was added. The aqueous layer was extracted with ethyl acetate. The organic layer was dried over MgSO₄, filtered and evaporated until dryness. The residue was purified by preparative LC (SiOH 35-40µm GraceResolv, from heptane/AcOEt 80/20 to 50/50). The fractions were collected and evaporated until dryness to give 5.3 g of intermediate **62** (quant.).

The following intermediate was prepared via an analogous procedure :

| Intermediate | Structure | Quantity | Yield |
|---|---|---|---|
| Intermediate **63** | 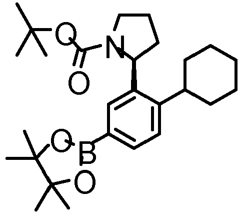 | Used without purification in next step | |
| Starting from intermediate 51 | | | |

### Synthesis of intermediate 64 :

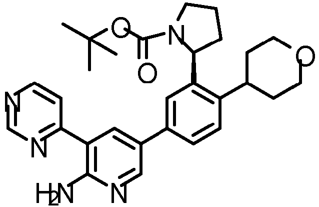

Sodium carbonate (60 mg, 0.57 mmol) was added to a solution of intermediate **55** (71 mg, 0.28 mmol), intermediate **62** (130 mg, 0.28 mmol) in water (1 mL) and 1,4-dioxane (4 mL). The mixture was degassed with a stream of nitrogen for 5 min., then PdCl2(dppf) (12 mg, 0.014 mmol) was added. The reaction mixture was stirred at 90°C overnight. The reaction mixture was cooled to rt, water (15 mL) and AcOEt (20 mL) were added. The aqueous layer was extracted with ethyl acetate (3 x 25 mL). The organic layer was dried over MgSO₄, filtered and evaporated until dryness. The residue was purified by silicagel chromatography (Heptane/EtOAc : 0-100%). The fractions were collected and evaporated until dryness to give 98 mg of intermediate **64** (68%).

The following intermediates were prepared via an analogous procedure :

| Intermediate | Structure | Quantity | Yield |
|---|---|---|---|
| Intermediate **65** | 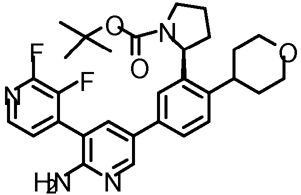 | 0.1g | 65% |
| Starting from Intermediate 45 | | | |
| Intermediate **66** | 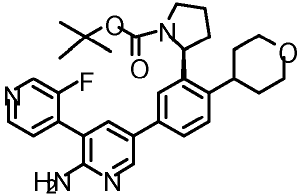 | 0.14g | 43% |
| Starting from Intermediate 46 | | | |
| Intermediate **67** | 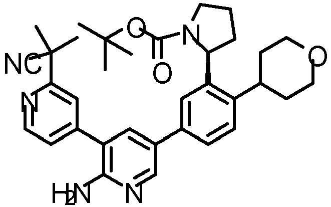 | 0.194 g | 67% |
| Starting from Intermediate 47 | | | |
| Intermediate **68** | 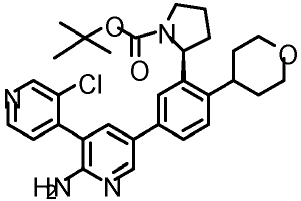 | 0.167g | 57% |
| Starting from Intermediate 48 | | | |
| Intermediate **69** | 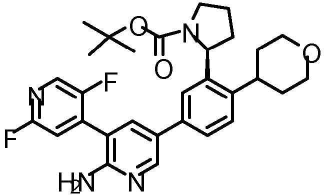 | 0.15g | 72% |
| Starting from Intermediate 49 | | | |
| Intermediate **70** | 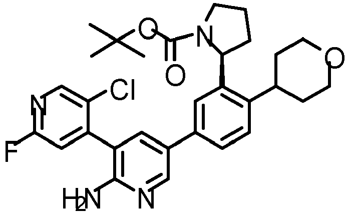 | 0.229g | 71% |
| Starting from intermediate 50 | | | |
| Intermediate **71** | 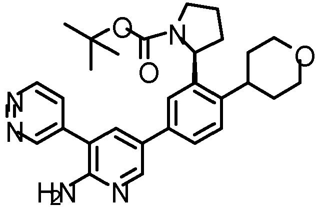 | 0.109g | 51% |
| Starting from Intermediate 51 | | | |
| Intermediate **72** | 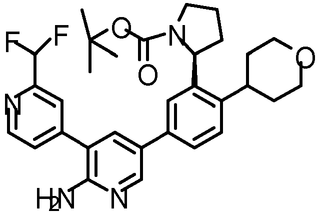 | 0.04 g | 73% |
| Starting from intermediate 52 | | | |
| Intermediate **73** | 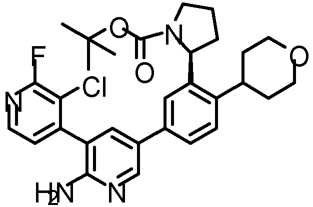 | 0.072 g | 45 % |
| Starting from Intermediate 53 | | | |
| Intermediate **74** | 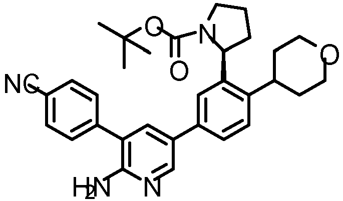 | 0.1 g | 66% |
| Starting from Intermediate 54 | | | |
| Intermediate **75** | 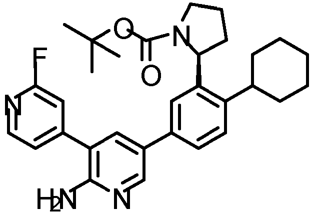 | 0.05 g | 20% |
| Starting from Intermediate 63 and 17 | | | |
| Intermediate **76** | 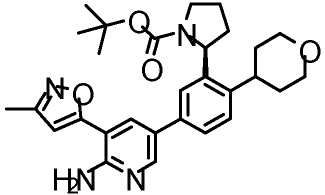 | 0.147g | 71% |
| Starting from 5-bromo-3-(3-methyl-1,2-oxazol-5-yl)pyridin-2-amine | | | |
| Intermediate **77** | 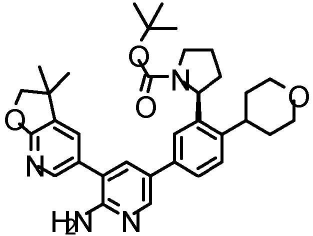 | 0.22g | 41% |
| Starting from Intermediate 56 | | | |
| Intermediate **78** | 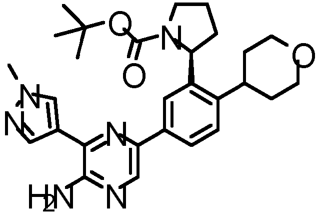 | 0.111g | 31% |
| Starting from 2-pyrazinamine, 5-bromo-3-(1-methyl-1H-pyrazol-4-yl) | | | |
| Intermediate **79** | 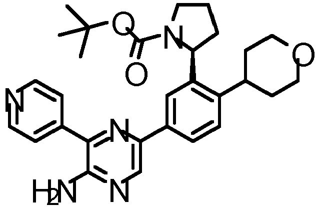 | 0.054g | 51% |
| Starting from Intermediate 59 | | | |
| Intermediate **80** | 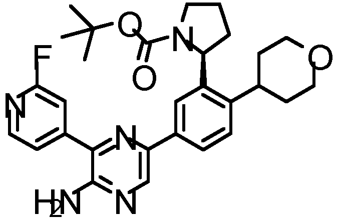 | 0.107 g | 65% |
| Starting from Intermediate 60 | | | |
| Intermediate **81** | 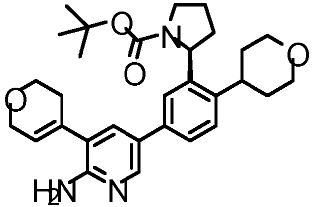 | 0.250 g | 92% (70% purity) |
| Starting from Intermediate 57 | | | |
| Intermediate **82** | 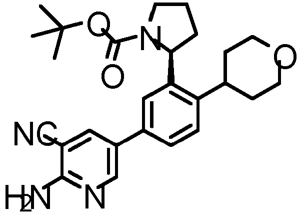 | 1.92 g | 98% |
| Starting from 2-amino-5-bromonicotinonitrile | | | |
| Intermediate **83** | 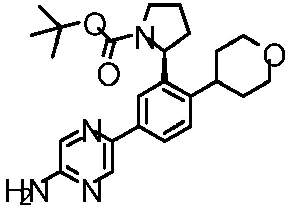 | 0.63 g | 86% |
| Starting from 2-Amino-5-bromopyrazine | | | |

### Synthesis of intermediate 84:

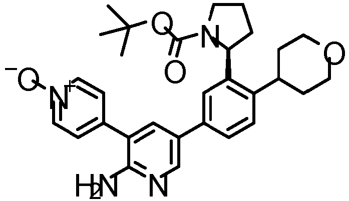

A mixture of intermediate **28** (0.55 g, 1 mmol), Pyridine, 4-bromo-, 1-oxide (0.174 g, 1 mmol), PdCl₂(dppf) (0.082 g, 0.1 mmol) and sodium carbonate (0.212 g, 2 mmol) in 1,4-dioxane (15 mL) and water (1.5 mL) was stirred under N₂ in a sealed tube at 90°C for 5 hours. The reaction mixture was cooled down, poured into water and potassium carbonate and the aqueous phase was extracted with ethyl acetate. The organic layer was separated, dried over MgSO_{4,}, filtered and evaporated until dryness. The residue was purified by preparative LC (12 g SiOH 30µm Interchim, from 100% DCM to DCM 90%/ MeOH 10%/ NH₄OH 0.1%). The pure fractions were collected and evaporated until dryness to give 0.274 g of intermediate **84** (53%).

### Synthesis of intermediate 85 :

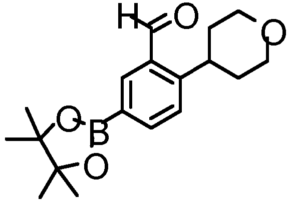

PdCl₂(dppf) (5.43 g, 7.43 mmol) was added to a solution of intermediate **5** (20 g, 74.3 mmol), bis(pinacolato)diboron (22.6 g, 89.2 mmol) and potassium acetate (21.88 g, 223 mmol) under a nitrogen atmosphere in 1,4-dioxane (600 mL). The reaction mixture was stirred at 100°C for 18h. The reaction mixture was cooled to rt, diluted with AcOEt (1800 mL) and filtered. The filtrate was washed with water (400 mL, three times). The organic layer was dried over MgSO₄, filtered and evaporated until dryness to give 23.5 g of intermediate **85** (100%).

### Synthesis of intermediate 86 :

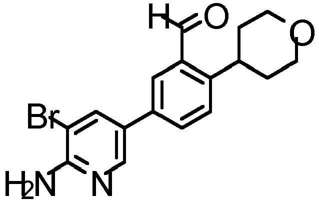

A solution of 2-amino-3-iodo-5-bromopyridine (42.92 g, 143.6 mmol), intermediate **85** (45.4 g, 143.6 mmol) and sodium carbonate (45.6 g, 430.7 mmol) in 1,4-dioxane (500 mL) and water (125 mL) was degassed during 10 min. After addition of PdCl₂(dppf) (11.72 g, 14.4 mmol), the reaction mixture was stirred at 75°C overnight. The mixture was filtered through a pad of celite and rinsed with DCM/MeOH (10/1, 500 mL). The filtrate was evaporated until dryness and purified by column chromatography over silica gel (mobile phase : DCM/MeOH 100/0 to 90/10). The pure fractions were collected and evaporated until dryness, washed with acetonitrile (75 mL, twice) to give intermediate **86** as a brown solid (27.5 g, 51%).

### Synthesis of intermediate 87 :

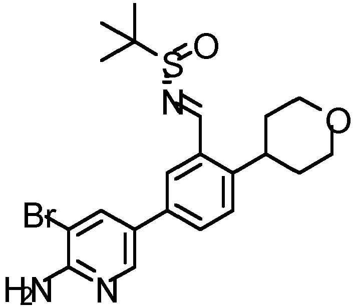

Titanium (IV) ethoxide (25.26 g, 110.7 mmol) was added dropwise to a solution of intermediate **86** (10 g, 27.7 mmol) and (S)-(-)-t-butylsulfinamide (6.71 g, 55.37 mmol). The solution was stirred at 45°C overnight and the mixture was poured into brine (400 mL) and DCM (500 mL) was added. The mixture was filtered through a pad of celite which was washed wuth DCM (500 mL).The organic layer was separated, dried over Na₂SO₄, filtered and evaporated until dryness. The residue was purified by column chromatography over silica gel (mobile phase : DCM/MeOH 100/0 to 90/10). The pure fractions were collected and evaporated until dryness, washed with acetonitrile (20 mL, twice) to give intermediate **87** as a pale brown solid (12 g, 93%).

### Synthesis of intermediate 88 :

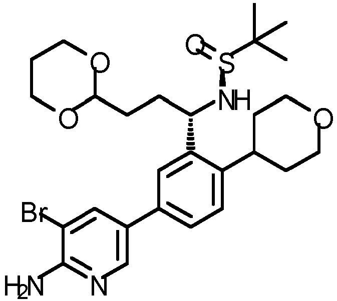

To a mixture of magnesium (9.77 g, 406.96 mmol) in THF (170 mL) were added catalytic amounts of I₂ under nitrogen atmosphere. (1,3-dioxan-2-ylethyl) bromide (31.75 g, 162.78 mmol) was added dropwise. The reaction mixture was periodically cooled in a room temperature water bath to prevent over-refluxing. The mixture was stirred at rt for 1h and then cooled to -65°C. A solution of intermediate **87** (12.6 g, 27.13 mmol) in THF (80 mL) was added dropwise and the reaction mixture was stirred at -65°C for 1h and then at rt overnight. A saturated solution of NH₄Cl (120 mL) and ethyl acetate (200 mL) were added. The mixture was filtered through a pad of celite which was washed with ethyl acetate (200 mL).The organic layer was separated, dried over Na₂SO₄, filtered and evaporated until dryness. The crude compound was washed with acetonitrile (20 mL, twice) and dried to give intermediate **88** as a brown solid (12.6 g, 80%).

### Synthesis of intermediate 89 :

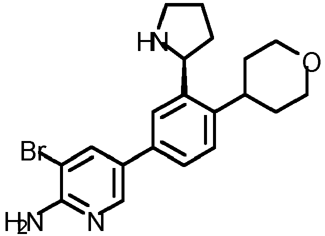

Intermediate **88** (0.35 g, 0.603 mmol) was slowly added to a mixture of TFA (2.66 mL, 34.8 mmol) and water (0.14 mL) to maintain temperature below 25°C. The reaction mixture was stirred at rt for 30 min. Triethylsilane (0.963 mL, 6.028 mmol) was added and the reaction mixture vigorously stirred at rt overnight. The reaction mixture was evaporated till dryness . A purification was performed via preparative LC (stationary phase : irregular SiOH 15-40 µM 4 g, Interchim, mobile phase : DCM /MeOH/NH₄OH 95/5/1 to 90/10/1). The pure fractions were evaporated to give intermediate **89** (0.2 g, 82%).

### Synthesis of intermediate 90 :

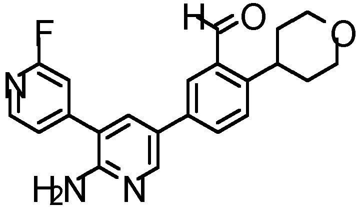

Sodium carbonate (0.41 g, 3.88 mmol) was added to a solution of intermediate **86** (1 g, 1.94 mmol), 2-fluoropyridine-4-boronic acid pinacol ester (0.52 g, 2.33 mmol) in water (1.75 mL) and 1,4-dioxane (18.7 mL). The mixture was degassed with a stream of nitrogen for 5 min. , then PdCl₂(dppf) (0.158 g, 0.194 mmol) was added. The reaction mixture was stirred at 90°C for 6 hours. The reaction mixture was cooled to rt, water and DCM were added. The aqueous layer was extracted DCM. The organic layer was dried over MgSO₄, filtered and evaporated until dryness. A purification was performed via preparative LC (stationary phase : irregular SiOH 15-40 µM 12 g, Grace, mobile phase : DCM /MeOH/NH₄OH 100/0/0 to 90/10/1). The pure fractions were evaporated to give intermediate **90** (0.66 g, 90%).

The following intermediate was prepared via an analogous procedure :

| Intermediate | Structure | Quantity | Yield |
|---|---|---|---|
| Intermediate **91** | | | |
| Starting from intermediate 58 and [3,4'-Bipyridin]-2-amine, 5-bromo | 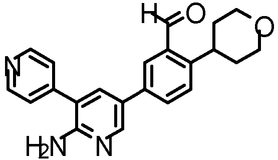 | 0.1 g | 49% |

### Synthesis of intermediate 92 :

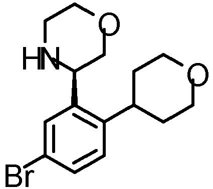

Intermediate **5** (7.39 g, 27.46 mmol) and molecular sieves 4 A (3.6g) were added to [(2-aminoethoxy)methyl]tributyl stannane (10 g, 27.46 mmol) in DCM (95 mL). The reaction mixture was stirred at rt for 6h, filtered over Celite^{®} and the filtrate was evaporated till dryness to give the imine. (R,R)-2,2'-Isopropylidenebis(4-phenyl-2-oxazoline) (1.84 g, 5.5 mmol) was added in one portion to a suspension of copper (II) triflate in hexafluoroisopropanol (50 mL). To this mixture was added the imine in hexafluoroisopropanol (50 mL). The reaction mixture was stirred at rt overnight. 1N NaOH was added, the mixture was stirred and extracted with DCM, the organic layer was separated, dried over MgSO4, filtered and evaporated till dryness. A purification was performed via preparative LC (Stationary phase: SiOH 35-40µm. 330g interchim, Mobile phase: 100% DCM to 50/50 DCM/AcOEt). The fractions were collected and evaporated until dryness (8.5 g, 95%). Both enantiomers were separated via chiral SFC (Stationary phase: CHIRALPAK AD-H 5µm 250*30mm, Mobile phase: 70% CO2, 30% iPrOH(0.3% iPrNH₂)) yielding 1.86 g of enantiomer A (20.8%, [α]_{d}: + 7.05° (589 nm, c 0.19 w/v %, DMF, 20 °C) and 4.63 g of enantiomer B (intermediate **92**, 51.7 %, .[α]_{d}: - 76.67° (589 nm, c 0.27 w/v %, DMF, 20 °C).

The following intermediates were prepared via an analogous procedure :

| Intermediate | Structure | Quantity | Yield |
|---|---|---|---|
| Intermediate **93a** | 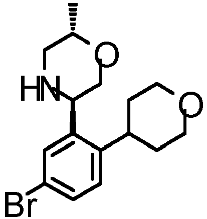 [α]_{d}: - 73.4° (589 nm, c 0.29 w/v %, DMF, 20 °C) | | |
| Starting from intermediate 5 and {[(1-aminopropan-2-yl) oxy]methyl}tributylstannane | | 2.6 g | 26% |
| Intermediate **93b** | 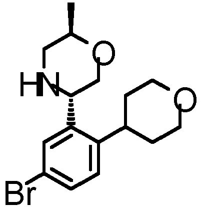 [α]_{d}: + 71.3° (589 nm, c 0.24 w/v %, DMF, 20 °C) | 2.8 g | 28% |
| Starting from intermediate 5 and {[(1-aminopropan-2-yl) oxy]methyl}tributylstannane | | | |
| Intermediate **94** | 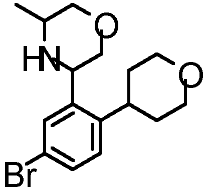 | 0.8 g | 63% |
| Starting from intermediate 5 and [(2-aminopropoxy) methyl]tributylstannane | | | |
| Intermediate **95** | 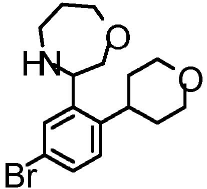 | 0.54 g | 35 % |
| Starting from intermediate 5 and 3-[(Tributylstannyl) methoxy]-1-propanamine | | | |

### Synthesis of intermediate 96 :

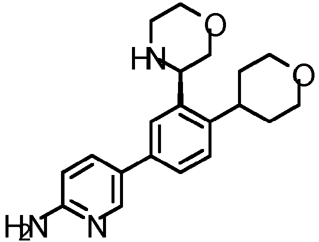

A mixture of 2-Aminopyridine-5-boronic acid pinacol ester (1.2 g, 5.45 mmol), intermediate **92** (1.44 g, 4.41 mmol), Pd(PPh₃)₄ (0.51 g, 0.44 mmol), Na₂CO₃ (1.4 g, 13.2 mmol) in Me-THF (50 mL) and water (5 mL) was heated under N₂ in a sealed tube at 85°C for 15 hours. H₂O, K₂CO₃ and AcOEt were added ; the organic layer was extracted, dried over MgSO4, filtered and evaporated until dryness. The residue was purified by preparative LC (SiOH 30µm Interchim, graduent from 100% DCM to 90% DCM 10% CH₃OH 0.1% NH4OH). The pure fractions were collected and evaporated until dryness yielding intermediate **96** (1.24 g, 83%).

The following intermediate was prepared via an analogous procedure :

| Intermediate | Structure | Quantity | Yield |
|---|---|---|---|
| Intermediate **97** | 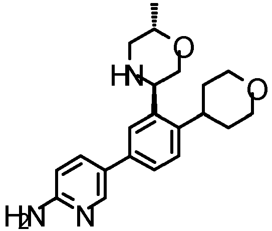 | | |
| Starting from intermediate 93a | | 2.3 g | 87% |

### Synthesis of intermediate 98 :

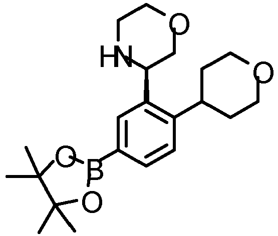

A mixture of intermediate **92** (0.28 g, 0.86 mmol), bis(pinacolato)diboron (0.261 g, 1 mmol), PdCl2(dppf) (0.045 g, 0.05 mmol), potassium acetate (0.252 mg, 2.6 mmol) in 1,4-dioxane (8 mL) was stirred at 120°C using one single mode microwave (Biotage Initiator EXP 60) with a power output ranging from 0 to 400 W for 45min. The reaction mixture was filtered over Celite^{®}, H₂O was added to the filtrate, the filtrate was extracted with ethyl acetate, the organic layer was separated, dried over MgSO4, filtered and evaporated. Compound **98** was used as it for next step.

The following intermediates were prepared via an analogous procedure :

| Intermediate | Structure | Quantity |
|---|---|---|
| Intermediate **99** | 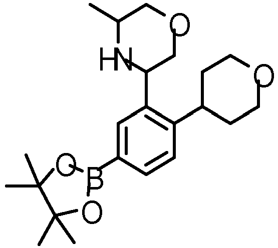 | Used without purification in next step |
| Starting from intermediate 94 | | |
| Intermediate **100a** | 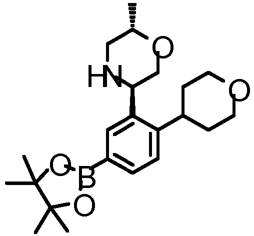 | Used without purification in next step |
| Starting from intermediate 93a | | |
| Intermediate **100b** | 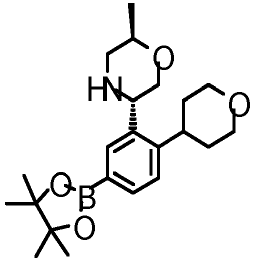 | Used without purification in next step |
| Starting from intermediate 93b | | |
| Intermediate **101** | 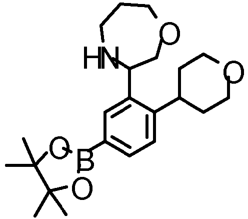 | Used without purification in next step |
| Starting from intermediate 95 | | |

### Synthesis of intermediate 102 :

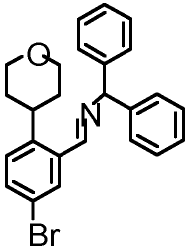

Magnesium sulfate (3.94 g, 32.7 mmol) and intermediate **5** ( 4 g, 14.86 mmol) were added successively to a stirred solution of Benhydrylamine (2.64 mL, 14.86 mmol) in DCM (52 mL). The resulting mixture was stirred at rt overnight, filtered. The filtrate was evaporated until dryness, dried under vacuum and used directly in the next step without further purification.

### Synthesis of intermediate 103:

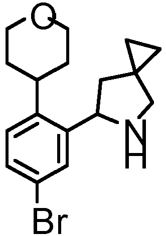

Intermediate **102** (1.079 g, 2.48 mmol) was dissolved in THF (17 mL) under nitrogen atmosphere and cooled to -78 °C. A 1M solution of Potassium tert-butoxide in dry THF (334.6 mg, 2.98 mmol) was added dropwise (changing of color to violet). After 30 min 1,1-bis(iodomethyl)cyclopropane (2.4 g, 7.45 mmol) was added rapidly. The mixture was stirred for 1h at -78 °C, then warmed to room temperature (after 5 min changing of color to grey) and stirred overnight. Water and DCM were added, the phases separated and the aqueous phase extracted with DCM (3 x 100 mL). The combined organic extracts were dried over MgSO4, filtered, the solvent removed under reduced pressure and the residue obtained dried under high vacuum. This residue was dissolved in acetone (10 mL), 3M HCl (3.56 mL, 10.67 mmol) was added and the mixture stirred at room temperature overnight. The mixture was basified with sat. Na₂CO₃ aqueous solution and extracted with DCM. The layers were separated and the aqueous phase extracted with DCM (5 x 50 mL). The combined organic extracts were dried over MgSO4, filtered and the solvent removed under reduced pressure. The crude mixture was purified by silicagel column chromatography (DCM/MeOH : 0-15%). The pure fractions were collected and evaporated until dryness to give intermediate **103** (425 mg, 51%)

The following intermediate was prepared via an analogous procedure :

| Intermediate | Structure | Quantity | Yield |
|---|---|---|---|
| Intermediate **104** | 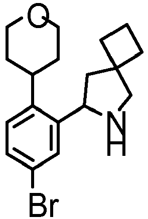 | 0.128 g | 15% |
| Starting from intermediate 102 and 1,1-Bis(iodomethyl) cyclobutane | | | |

### Synthesis of intermediate 105:

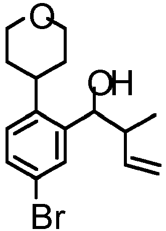

Zinc dust (1.21 g, 18.58 mmol) and crotyl bromide (0.9 mL, 7.43 mmol) were added to a solution of intermediate **5** (1g, 3.42 mmol) in THF (75 mL). A staturated aqueous solution of NH₄Cl (37.5 mL) was added dropwise to the previous mixture and the reaction stirred at room temperature for 50 min. The mixture was filtered through a pad of celite. The filtrate was acidified with 2N HCl and extracted with EtOAc (3 x 50 mL). The combined organic extracts were washed with brine, dried over MgSO₄, filtered and concentrated. The crude mixture was purified by silicagel column chromatography (Heptane:EtOAc; 0-40%). The pure fractions were collected and evaporated until dryness to give intermediate **105** (1.16 g, 94%).

### Synthesis of intermediate 106:

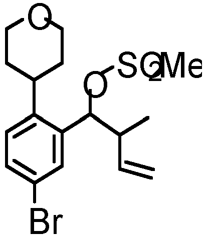

Triethylamine (0.944 mL, 6.8 mmol) was added at 0 °C to a solution of intermediate **105** (1.16 g, 3.57 mmol) in DCM (35 mL) under argon atmosphere. Then methane sulfonyl chloride (0.414 mL, 5.35 mmol) was added slowly to the previous solution and the mixture warmed to room temperature and stirred for 20 h. The mixture was diluted with water and the layers separated and the aqueous phase was extracted with DCM (3 x 40 mL). The combined organic extracts were dried over MgSO4, filtered and the solvent removed under reduced pressure. The crude intermediate **106** obtained was dried under high vacuum and used directly in the next step without further purification.

### Synthesis of intermediate 107:

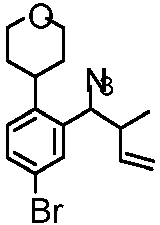

Sodium azide (0.616 g, 9.48 mmol) was added carefully to a solution of intermediate **106** (1.53 g, 3.79 mmol) in dry DMF (35 mL) and the mixture stirred at 45 °C overnight. The mixture was diluted with water (200 mL) and extracted with EtOAc (3 x 40 mL). The combined organic extracts were washed with brine (25 mL), dried over MgSO4, filtered and the solvent removed under reduced pressure. The crude mixture was purified by silicagel column chromatography (Heptane:EtOAc; 0-10%). The pure fractions were collected and evaporated until dryness to give intermediate **107** (0.929 g, 67%).

### Synthesis of intermediate 108 and 109:

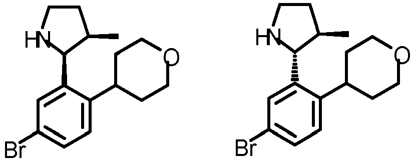

A solution of Borane dimethyl sulphide complex (0.550 mL, 5.8 mmol) in 2.9 mL of dry THF (2 M solution) was added dropwise at 0 °C to a solution of cyclohexene (1.18 mL, 11.6 mmol) in 1.7 mL of dry THF. The resulting white suspension was stirred at 0 °C for 1h, then cooled to -15 °C and a solution of intermediate **107** (0.677 g, 1.93 mmol) in dry THF (5.4 mL) was added dropwise to it. The reaction was allowed to warm to room temperature, stirred at this temperature for 3 h. The reaction was quenched with MeOH at 0 °C and evaporated to dryness. The crude mixture was dissolved in EtOAc and washed with 1M HCl aqueous solution. The layers were separated and the aqueous phase extracted with EtOAc (10 x 20 mL). The combined organic extracts were dried over MgSO₄, filtered and the solvent removed under reduced pressure. The crude mixture was purified by silicagel column chromatography (DCM:MeOH; 0-15%). All fractions containing product were combined and concentrated to afford a mixture of intermediates **108** and **109** (0.421 g, 67%) which was separated by reverse phase using a GILSON Semi-Prepeparative System, operated by Trilution software, equipped with a Phenomenex Gemini C18 100A column (100 mm long x 30 mm I.D.; 5 µm particles) at 25 °C, with a flow rate of 20 or 40 mL/min. A gradient elution (method ISO15-AF): [start: 85% H₂O (0.1% HCOOH) - 15% ACN-MeOH; finish: 85% H₂O (0.1% HCOOH) - 15% ACN-MeOH]. The desired fractions were collected and partially concentrated to removed the organic solvents. Then the fractions were basified with saturated Na₂CO₃ aqueous solution and extracted with EtOAc. The combined organic extracts were dried over MgSO4, filtered, the solvent removed under reduced pressure and dried under high vacuum to afford intermediate **108** (0.079 g, 12.6%) and intermediate **109** (0.180 g, 28.7%).

### Synthesis of intermediate 110:

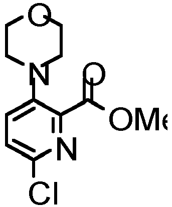

To a solution of Methyl 6-chloro-3-fluoropicolinate (1.64 g, 8.65 mmol) in DMSO (10 mL) were added morpholine (0.908 mL, 10.38 mmol) and N,N-diisopropylethylamine (4.52 mL, 25.9 mmol) . The reaction mixture was stirred at rt over the week-end. The mixture was diluted with AcOEt. The organic phase was washed with water and the aqueous phase extracted with AcOEt. The combined organic extracts were dried over MgSO4, filtered and the solvent removed under reduced pressure. The crude mixture was purified by silicagel column chromatography (Heptane:EtOAc; 0-20%). The pure fractions were collected and evaporated until dryness to give intermediate **110** (1.82 g, 82%).

### Synthesis of intermediate 111:

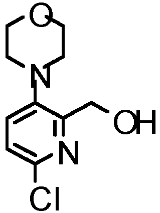

To a mixture of intermediate **110** (1.7g, 6.62 mmol) and calcium chloride (1.47 g, 13.25 mmol) in ethanol (17 mL) was added sodium borohydride (0.751 g, 19.9 mmol) at 0 °C. The reaction mixture was stirred for 2 h at 0 °C and 4 h at rt. Then water was added to the reaction mixture, and it was extracted with DCM. The organic layer was dried over anhydrous magnesium sulfate and filtered. The crude mixture was purified by flash column chromatography eluting with a mixture of Heptane/AcOEt (40%) to give intermediate **111** (1.37 g, 90%).

### Synthesis of intermediate 112:

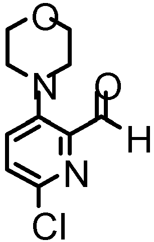

To a solution of intermediate **111** (1.37 g, 5.99 mmol) in dry DCM (30 mL) was added Dess-Martin Periodinane (3.81g, 8.99 mmol). The reaction mixture was stirred at rt for 3 h. The reaction was quenched with Na₂S₂O₃ and Na₂CO₃ was added until basic pH. Then it was extracted with DCM, the organic phase was dried over MgSO4, filtered and the solvent was concentrated in vacuo. The crude mixture was purified by flash column chromatography eluting with a mixture of Heptane/AcOEt (50%) to yield intermediate **112** (1.34 g, 99%).

### Synthesis of intermediate 113:

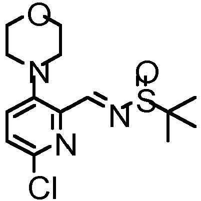

Titanium (IV) ethoxide (4.96 mL, 23.65 mmol) was added dropwise under nitrogen atmosphere to a solution of intermediate **112** (1.34 g, 5.91 mmol) and 2-methyl-2-propanesulfinamide in dry THF (17 mL). The mixture was stirred at room temperature for 16 h. The reaction mixture was diluted with DCM and brine. The resulting precipitate was removed by filtration through celite and washed with DCM. The layers were separated and the aqueous phase extracted with DCM. The combined organic extracts were dried over MgSO₄, filtered and the solvent removed under reduced pressure. The crude mixture was purified by flash column chromatography eluting with a mixture of Heptane/AcOEt (50%) to afford intermediate **113** (1.3 g, 67%) .

### Synthesis of intermediate 114:

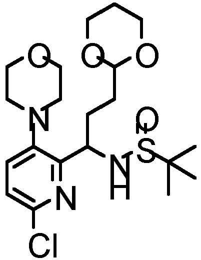

(1,3-dioxan-2-ylethyl)magnesium bromide (28.7 mL, 14.37 mmol) was added dropwise at -78 °C to a solution of intermediate **113** (1.58 g, 4.79 mmol) in dry THF (49 mL). The mixture was stirred at -78 °C for 1 h, quenched with a saturated NH₄Cl aqueous solution and slowly warmed to room temperature. The mixture was diluted with EtOAc and the aqueous phase was extracted with EtOAc. The combined organic extracts were dried over MgSO₄, filtered and the solvent removed under reduced pressure. The crude mixture was purified by flash column chromatography over silica gel, eluting with a mixture of Heptane /AcOEt (60%) to yield intermedaite **114** (1.82 g, 85%).

### Synthesis of intermediate 115:

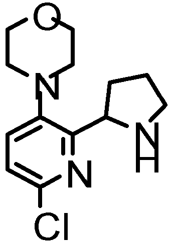

Intermediate **114** (2 g, 4.48 mmol) was slowly added to a mixture of TFA (19.8 mL, 259 mmol) and water (1.3 mL) to maintain temperature below 25°C. The reaction mixture was stirred at rt for 30 min. Triethylsilane (7.16 mL, 44.8 mmol) was added and the reaction mixture vigorously stirred at rt overnight. The reaction mixture was evaporated till dryness. The crude mixture was purified by silicagel column chromatography, gradient in DCM/DCM:MeOH (9:1) (50%) to yield intermediate **115** (1.09 g, 88%).

### Synthesis of intermediate 116:

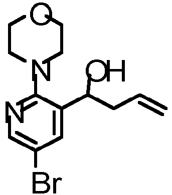

Zinc dust (1.27 g, 19.4 mmol) and allyl bromide (0.67 mL, 7.75 mmol) were added to a solution of 5-bromo-2-(morpholin-4-yl)pyridine-3-carbaldehyde (1.05 g, 3.9 mmol) in THF (75 mL). NH₄Cl (sat. aq. Solution, 39 mL) was added dropwise to the previous mixture and the reaction stirred at room temperature for 1 h. The mixture was filtered through a pad of celite. The filtrate was acidified with 2N HCl and extracted with EtOAc (3 x 50 mL). The combined organic extracts were washed with brine, dried over MgSO4, filtered and concentrated. The crude mixture was purified by silicagel column chromatography (Heptane:EtOAc; 0-40%) to yield intermediate **116** (1.09 g, 89%).

### Synthesis of intermediate 117:

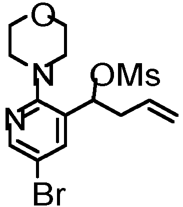

Triethylamine (0.922 mL, 6.6 mmol) was added at 0 °C to a solution of intermediate **116** (1.09 g, 3.48 mmol) in DCM (35 mL) under argon atmosphere. Then methanesulfonyl chloride (0.404 mL, 5.2 mmol) was added slowly to the previous solution and the mixture warmed to room temperature and stirred for 2 h. The mixture was diluted with water and the layers separated and the aqueous phase was extracted with DCM (3 x 40 mL). The combined organic extracts were dried over MgSO4, filtered and the solvent removed under reduced pressure. The yellow oil obtained was dried under high vacuum and used directly in the next step without further purification.

### Synthesis of intermediate 118:

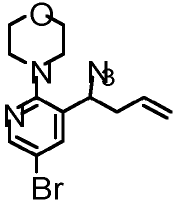

Sodium azide (0.429 g, 6.6 mmol) was added carefully to a solution of intermediate **117** (1.29 g, 3.3 mmol) in dry DMF (25 mL) and the mixture stirred at 60°C for 5 hours. The mixture was diluted with water (100 mL) and extracted with EtOAc (3 x 30 mL). The combined organic extracts were washed with brine (25 mL), dried over MgSO4, filtered and the solvent removed under reduced pressure. The crude mixture was purified by silicagel column chromatography (Heptane:EtOAc; 0-10%) yielding intermediate **118** (0.72 g, 64%).

### Synthesis of intermediate 119:

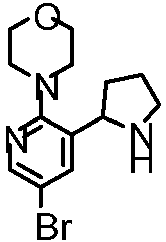

A solution of Borane dimethyl sulfide complex (0.6 mL, 6.1 mmol) in dry THF (2M solution, 3 mL) was added dropwise at 0 °C to a solution of cyclohexene (1.2 mL, 12.2 mmol) in dry THF (2 mL). The resulting white suspension was stirred at 0 °C for 1h, then cooled to -15 °C and a solution of intermediate **118** (0.691 g, 2.04 mmol) in dry THF (6 mL) was added dropwise. The reaction was allowed to warm to room temperature and stirred overnight at this temperature. The reaction was quenched with MeOH at 0 °C, the mixture was evaporated to dryness and the crude mixture was purified by silicagel column chromatography (DCM:MeOH; 0-15%) yielding intermediate **119** (0.47 g, 71%).

### Synthesis of intermediate 120:

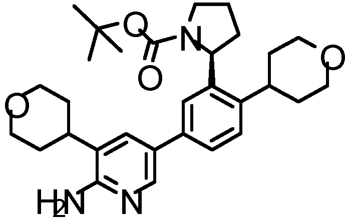

To a solution of intermediate **81** (0.335 g, 0.53 mmol, 80% purity) in methanol (20 mL) was added Pd/C 10% under nitrogen atmosphere. The resulting reaction mixture was stirred at room temperature under hydrogen atmosphere for 3 hours. After completion of the reaction, the catalyst was filtered off on Celite^{®} and washed with methanol. The filtrate was concentrated under reduced pressure to yield intermediate **120** (0.232 g, 60%, 70% purity)

### Synthesis of intermediate 121:

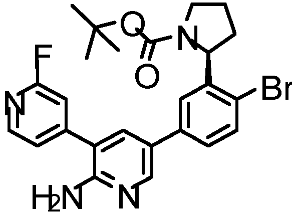

A mixture of intermediate **18** (13.45 g, 42.7 mmol), intermediate **21** (19.3 g, 42.7 mmol), PdCl₂(dppf) (3.49 g, 4.27 mmol) and sodium carbonate (13.6 g, 128.1 mmol) in 1,4-dioxane (386 mL) and water (64 mL) under N₂ was heated at 90°C for 3 hours. The reaction mixture was cooled to rt, diluted with ethyl acetate (1000 mL) and filtered. The filtrate was washed with water (3*300 mL), the aqueous layer was extracted with ethyl acetate. The organic layer was dried over MgSO₄, filtered and evaporated until dryness. The residue was purified by chromatography over silica gel (petroleum ether/ethyl acetate : from 100/0 to /100). The fractions were collected and evaporated until dryness to give intermediate **121** (15.7 g, 70%).

### Synthesis of intermediate 122:

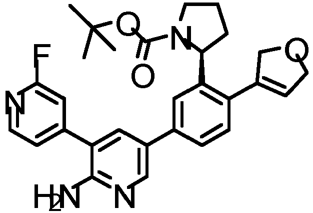

A mixture of intermediate **121** (0.846 g, 1.59 mmol), 2,5-dihydrofuran-3-ylboronic acid pinacol ester (2.43 g, 3.18 mmol), PdCl₂(dppf) (0.130 g, 0.159 mmol) and sodium carbonate (0.506 g, 4.77 mmol) in 1,4-dioxane (42 mL) and water (8 mL) under N₂ was heated at 95°C for 18 hours. The reaction mixture was cooled to rt, diluted with ethyl acetate (200 mL) and filtered. The filtrate was washed with water (3*50 mL), the aqueous layer was extracted with ethyl acetate. The organic layer was dried over MgSO₄, filtered and evaporated until dryness. The residue was purified by chromatography over silica gel (petroleum ether/ethyl acetate : from 100/0 to /100) followed by preparative HPLC (Phenomenex Gemini 150*25 mm*10µm ; water (0.05% ammonia hydroxide v/v) and MeCN : from 55/45 to 25/75). The fractions were collected and evaporated until dryness to give intermediate **122** (0.39 g, 47%).

The following intermediates were prepared via an analogous procedure :

| Intermediate | Structure | Quantity | Yield |
|---|---|---|---|
| Intermediate **123** | 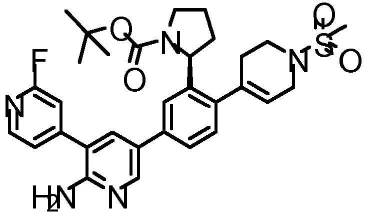 | 0.75 g | 98% |
| Starting from intermediates 121 and 1-Methanesulfonyl-4-(tetramethyl-1,3,2-dioxaborolan-2-yl)-1, 2,3,6-tetrahydropyridine | | | |
| Intermediate **124** | 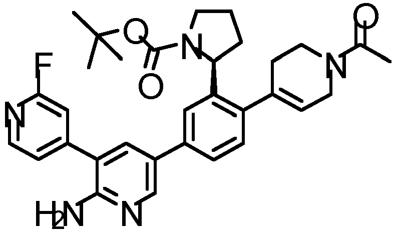 | 0.56 g | 78% |
| Starting from intermediates 121 and 1-Acetyl-5,6-dihydro-2H-pyridine-4-boronic acid, pinacol ester | | | |
| Intermediate **125** | 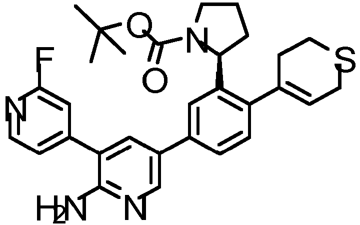 | 0.53 g | 75% |
| Starting from intermediates 121 and 3,6-Dihydro-2H-thiopyran-4-ylboronic acid pinacol ester | | | |
| Intermediate **126** | 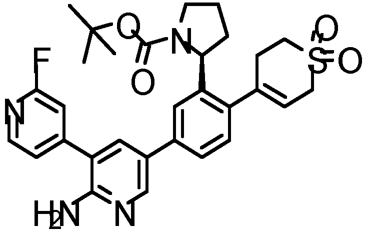 | 0.28 g | Quant. |
| Starting from intermediates 121 and 1,1-Dioxo-1,2,3,6-tetrahydro-2H-thiopyran-4-yl-boronic acid pinacol ester | | | |
| Intermediate **127** | 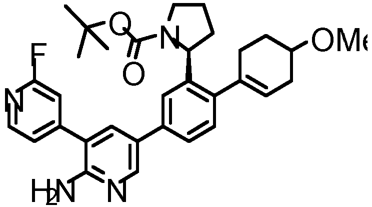 | 0.45 g | Quant. |
| Starting from intermediates 121 and 4-Methoxycyclohexene-1-boronic acid pinacol ester | | | |
| Intermediate **128** | 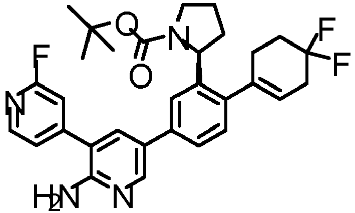 | 0.22 g | 67% |
| Starting from intermediates 121 and 4,4-Difluoro-1-cyclohexene-1-boronic acid pinacol ester | | | |

### Synthesis of intermediate 129:

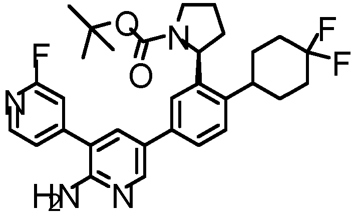

A mixture of intermediate **128** (0.15 g, 0.272 mmol), Pd/C 10% (0.15g, 0.272 mmol) in MeOH (3 mL) was hydrogenated under 5 bars pressure at 80°C overnight. The reaction mixture was filtered over celite, washed with DCM/MeOH and the filtrate was evaporated. The residue was purified by preparative LC (12 g SiOH 40 µm irregular, mobile phase : DCM/MeOH/NH₄OH 98/2/0 to 90/10/0.1) The pure fractions were collected and evaporated until dryness yielding 0.017 g of intermediate **129** (11%).

The following intermediate was prepared via an analogous procedure :

| Intermediate | Structure | Quantity | Yield |
|---|---|---|---|
| Intermediate **130** | 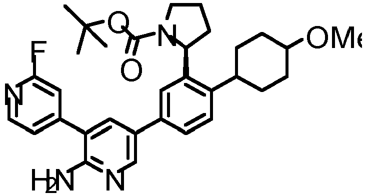 | 0.045g | 10% |
| Starting from intermediate 127 | | | |

### Synthesis of intermediate 131:

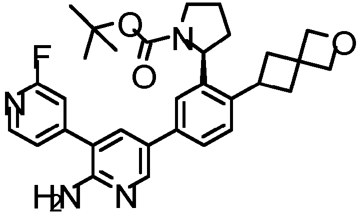

To a solution of intermediate **121** (0.2 g, 0.39 mmol), tris(trimethylsilyl)silane (0.145 g, 0.584 mmol), [Ir(dtbbpy)(ppy)₂]PF₆ (0.035 g, 0.039 mmol), sodium carbonate (0.124 g, 1.17 mmol), 6-Bromo-2-oxaspiro[3.3]heptane (0.138 g, 0.779 mmol) in DME (3.5 mL) was added a solution of Nickel (II) chloride ethylene glycol dimethyl ether complex (4.3 mg, 0.02 mmol) and 4'-di-tert-butyl-2, 2'-bipyridine (5.3 mg, 0.02 mmol) in DME (1.5 mL). The reaction mixture was degassed with nitrogen and irradiated under blue light in a Hepatochem device for 20 hours (Hepatochem photoreactor - irradiated with a Cree lamp 18W). Water and ethyl acetate were added, the organic layer was separated, dried over MgSO₄, filtered and evaporated until dryness. The residue was purified by preparative LC (irregular SiOH 40µm 12g, from 100% DCM to DCM 95%/ MeOH 5%/ NH₄OH 0.1%). The fractions were collected and evaporated until dryness to give intermediate **131** (0.094 g, 45%).

The following intermediates were prepared via an analogous procedure :

| Intermediate | Structure | Quantity | Yield |
|---|---|---|---|
| Intermediate **132** | 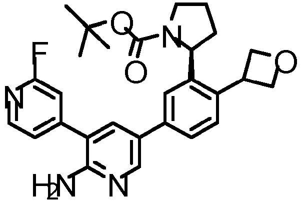 | 0.2 g | 70% |
| Starting from intermediate 121 | | | |
| Intermediate **132b** | 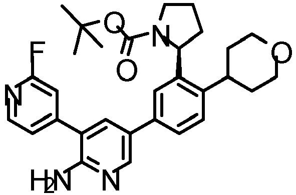 | 0.053 g | 26% |
| Starting from intermediate 121 | | | |

### Synthesis of intermediate 133:

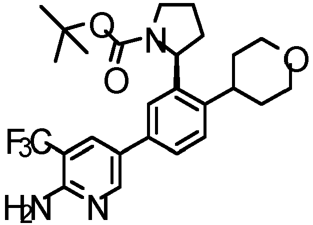

A mixture of intermediate **83** (0.25 g, 0.589 mmol), ammonium persulfate (0.205 g, 0.898 mmol), (Ir[dF(CF₃)ppy]2(dtbpy))PF₆ (10 mg, 0.009 mmol) and sodium trifluoromethanesulfinate (0.14 g, 0.897 mmol) in DMSO (12.5 mL) was irradiated with a Kessil lamp for 5 hours. The solution was poured into H₂O then extracted with DCM. The organic layer was dried over MgSO₄, filtered and evaporated until dryness. The residue was purified by preparative LC (12g of SiOH 40µm Buchi, graduent from 100% DCM to 90% DCM 10% CH₃OH 0.1% NH4OH). The fractions were collected and evaporated until dryness. Another purification was performed by preparative LC (12g of SiOH 15µm Buchi, graduent from 100% DCM to 90% DCM 10% MeOH 0.1% NH4OH). The fractions were collected and evaporated until dryness yielding intermediate **133** (0.064 g, 22%).

### Synthesis of intermediate 134:

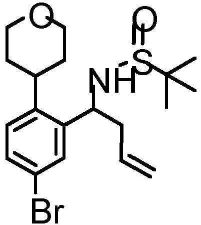

Allylmagnesium chloride (12 mL, 20.5 mmol) was added dropwise to a solution of intermediate **10** (1.9 g, 5.1 mmol) in THF (55 mL) at -70°C under N₂. The reaction mixture was stirred for 1 hour, poured into H₂O and NH₄Cl. The aqueous phase was extracted with AcOEt. The organic layer was dried over MgSO4, filtered and evaporated until dryness yielding intermediate **134** (2.07 g, 98%).

### Synthesis of intermediate 135:

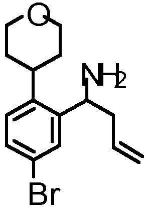

HCl 3M (35 mL) was added slowly to a solution of intermediate **134** (2.07 g, 5 mmol) in THF (18 mL) at 5°C. The reaction mixture was stirred for 20 hours at room temperature. Water was added and the aqueous phase was basified by addition of potassium carbonate, then extracted with AcOEt. The organic layer was dried over MgSO4, filtered and evaporated until dryness yielding intermediate **135** (1.6 g, Quant.).

### Synthesis of intermediate 136:

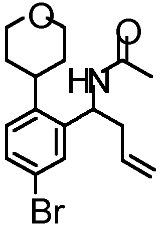

A mixture of intermediate **135** (1.6 g, 5.2 mmol) and Et₃N (2.9 mL, 20.9 mmol) in DCM (25 mL) was cooled at 5°C; Acetic anhydride (1.45 mL, 15.5 mmol) was added and the reaction mixture was stirred for 3 hours. Water and potassium carbonate were added and the organic layer was extracted, dried over MgSO₄, filtered and evaporated until dryness. The residue was purified by preparative LC (12g of SiOH 15µm Interchim, graduent from 100% DCM to 90% DCM 10% MeOH 0.1% NH4OH). The fractions were collected and evaporated until dryness yielding intermediate **136** (1.55 g, 85%).

### Synthesis of intermediate 137:

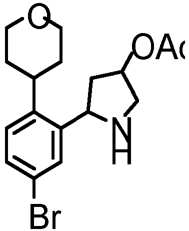

Iodine (10.8 g, 42.6 mmol) was added to a solution of intermediate **136** (5 g, 14.2 mmol) in THF (40 mL) and water (10 mL) at room temperature. The reaction mixture was heated at 50°C for 1 hour, then poured into a saturated solution of NaHCO₃. The aqueous phase was extracted with AcOEt. The organic layer was washed with a saturated solution of Na₂S₂O₃, dried over MgSO₄, filtered and evaporated until dryness. The residue was purified by preparative LC (80g of SiOH 35-40µm GraceResolv, graduent from 100% DCM to 90% DCM 10% CH₃OH 0.1% NH₄OH. The fractions were collected and evaporated until dryness yielding intermediate **137** (2.21 g, 42%).

### Synthesis of intermediate 138:

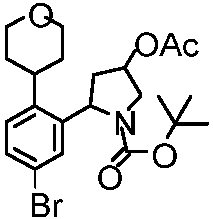

Di-tert-butyl dicarbonate (3.56 g, 16.3 mmol) was added to a solution of intermediate **137** (2 g, 5.43 mmol), 4-Dimethylaminopyridine (0.133 g, 1.09 mmol) in DCM (60 mL) at room temperature. The reaction mixture was stirred for 2 days, poured into water. The organic layer was extracted, dried over MgSO₄, filtered and evaporated until dryness. The residue was purified by preparative LC (12g of SiOH 35-40µm GraceResolv, graduent from 100% DCM to 95% DCM 5% CH3OH). The fractions were collected and evaporated until dryness yielding intermediate **138** (2.19 g, 86%).

### Synthesis of intermediate 139:

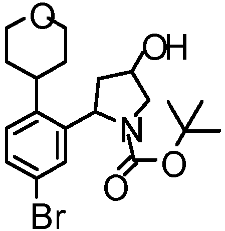

NaOH 1M (5 mL, 5 mmol) was slowly added to a mixture of intermediate **138** (2.1 g, 4.5 mmol) in MeOH (25 mL) at 5°C. The reaction mixture was stirred for 2 hours. MeOH was evaporated at room temperature and the residue was taken up with water then neutralized with HCl 1N. The insoluble was filtered and dried yielding intermediate **139** (1.92 g, Quantitatif).

### Synthesis of intermediate 140:

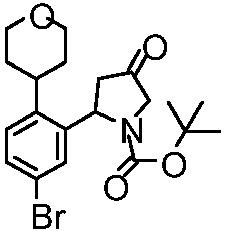

DMSO (0.5 mL, 7 mmol) was added dropwise to oxalyl chloride (1.76 mL, 3.52 mmol) in DCM (5 mL) at -78°C, the solution was stirred for 10 min then intermediate **139** (0.5 g, 1.17 mmol) in DCM (5 mL) was added dropwise and the reaction was stirred at -50°C for 30 min. After dropwise addition of Et₃N (1.5 mL, 10.8 mmol) , the reaction mixture was allowed to warm to room temperature over 30 min. The mixture was poured into NH₄Cl and H₂O. The organic layer was extracted, dried over MgSO4, filtered and evaporated until dryness. The residue was purified by preparative LC (24 g of SiOH 35-40µm GraceResolv, graduent from 100% DCM to 95% DCM 5% CH₃OH). The fractions were collected and evaporated until dryness yielding intermediate **140** (0.312 g, 63%).

### Synthesis of intermediate 141:

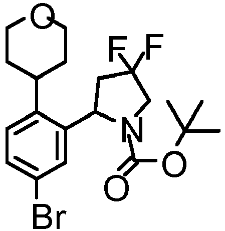

Diethylaminosulfur trifluoride (0.31 mL, 2.19 mmol) was added dropwise to a solution of intermediate **140** (0.31 g, 0.73 mmol) in DCM (6 mL) at -70°C under N2. The mixture was stirred for 15 hours at room temperature , cooled at -70°C then diethylaminosulfur trifluoride (0.31 mL, 2.19 mmol) was added dropwise. The reaction mixture was stirred for 2.5 hours at room temperature . Water was added and the aqueous phase was basified potassium carbonate. The organic layer was extracted, dried over MgSO4, filtered and evaporated until dryness. The residue was purified by preparative LC (12 g of SiOH 35-40 µm GraceResolv, graduent from 100% DCM to 95% DCM 5% CH₃OH). The fractions were collected and evaporated until dryness yielding intermediate **141** (0.3 g, 92%).

### Synthesis of intermediate 142:

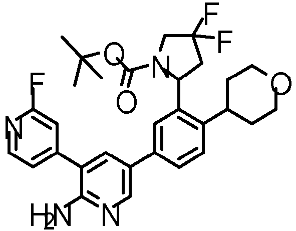

A mixture of intermediate **18** (0.233 g, 0.74 mmol), intermediate **141** (0.3 g, 0.67 mmol), PdCl₂(dppf) (0.071 g, 0.087 mmol) and sodium carbonate (0.143 g, 1.34 mmol) in 1,4-dioxane (10 mL) and water (1 mL) under N₂ was heated at 90°C for 8 hours. Water and potassium carbonate were added . The aqueous layer was extracted with ethyl acetate. The organic layer was dried over MgSO₄, filtered and evaporated until dryness. The residue was purified by preparative LC (24 g of SiOH 35-40 µm GraceResolv, graduent from 100% DCM to 90% DCM 10% CH₃OH 0.1% NH4OH). The fractions were collected and evaporated until dryness yielding intermediate **142** (0.225 g, 92%).

### Synthesis of intermediate 143:

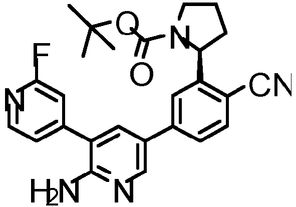

A mixture of intermediate **121** (300 mg, 0.58 mmol), Zinc cyanide (316 mg, 2.7 mmol), Pd (PPh₃)₄ (67.5 mg, 0.06 mmol) in DMF (2.9 mL) was stirred in a Biotage apparatus at 140°C for 45min. Water and AcOEt were added, the organic layer was separated, dried over MgSO4, filtered and evaporated. A purification was performed via preparative LC (Stationary phase: irregular SiOH 40 µm 25 g, Mobile phase: 98/2 to 90% DCM 10% CH₃OH 0.1% NH₄OH) yielding intermediate **143** (0.2 g, 74%).

### Synthesis of intermediate 144:

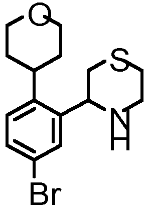

Intermediate **5** (0.5 g, 1.86 mmol) and molecular sieves 4A (0.1 g) were added to SLAP TM (0.38 g, 2.32 mmol) in DCM (8 mL). The reaction mixture was stirred at rt 5 h, filtered over celite^{©}, the filtrate was evaporated till dryness to afford the imine which was added to a solution of [Ir(dtbbpy)(ppy)₂]PF₆ (17 mg, 0.0186 mmol), Copper (II) trifluoromethanesulfonate (0.67 g, 1.86 mmol) and Bismuth(III) trifluoromethane sulfonate (0.612 g, 0.93 mmol) in MeCN (10 mL). The reaction mixture was stirred at rt for 16 h under blue light (34W - Hepatochem photoreactor - irraditated with a Kessil lamp). The recation mixture was poured into H₂O and K₂CO₃ and extracted with AcOEt. The organic layer was dried over MgSO4, filtered and evaporated until dryness. The residue was purified by preparative LC (Stationary phase: irregular SiOH 15-40µm 40g GraceResolv, graduent from 100% heptane to 50% heptane 50% AcOEt). The fractions were collected and evaporated until dryness yielding intermediate **144** (0.4 g, 63%).

### Synthesis of intermediate 145:

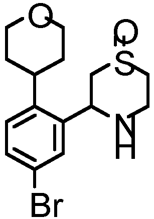

m-CPBA (0.221 g, 0.85 mmol) was added portionwise to intermediate **144** (0.291 g, 0.85 mmol) in DCM (12 mL) at 0°C. The reaction mixture was stirred at room temperature for 15 hours. H₂O and K₂CO₃ were added and the mixture was stirred for 1 hour. The organic layer was extracted, dried over MgSO₄, filtered and evaporated until dryness yielding intermediate **145** (0.3 g, 98%).

### Synthesis of intermediate 146:

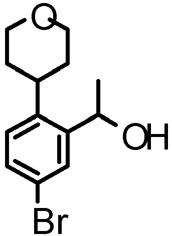

Methylmagnesium bromide (1.4 M in THF, 3.8 mL, 5.35 mmol) was added at 0 °C to a solution of intermediate **5** (1.2 g, 4.46 mmol) in dry THF (8 mL) under nitrogen atmosphere. The reaction was stirred at 0 °C for 30 min and then warmed to room temperature for 1.5 h. The reaction was quenched with a saturated NH4Cl aqueous solution. The aqueous phase was extracted with EtOAc (5 x 15 mL). The combined organic extracts were dried over MgSO₄, filtered and the solvent removed under reduced pressure. The crude mixture was purified by silica gel column chromatography gradient n-heptane/Ethyl Acetate from 100/0 to 65/35. The desired fractions were collected and evaporated in vacuo to afford intermediate **146** (1.18 g, 93%).

### Synthesis of intermediate 147:

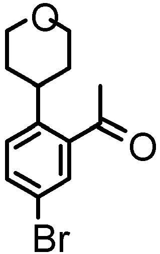

Manganese oxide (8.45 g, 86.6 mmol) was added to a solution of intermediate **146** (1.18 g, 4.13 mmol) in DCM (20 mL). The resulting suspension was stirred at room temperature overnight. The mixture was filtered through a pad of celite and was washed with DCM and MeOH. The solvents were evaporated under reduced pressure and the crude mixture was purified by flash silicagel column chromatography (Heptane:EtOAc; 0- 50%). The fractions were collected and concentrated to afford intermediate **147** (0.51 g, 44%).

### Synthesis of intermediate 148:

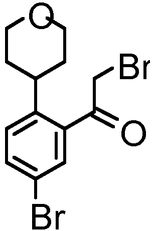

Tetra-n-butylammonium tribromide (1.6 g, 3.31 mmol) was added to a solution of intermediate **147** (0.94 g, 3.31 mmol) in acetonitrile (10 mL). The reaction was stirred at rt for 2h. The solvent was concentrated under reduced pressure, and the residue was redissolved in EtOAc. The solution was washed with saturated aqueous NaHCO3, dried over MgSO₄, and concentrated under reduced pressure. The crude mixture was purified by silicagel column chromatography (Heptane:EtOAc; 0-35%). All fractions were combined and concentrated to afford intermediate **148** (1.14 g, 95%).

### Synthesis of intermediate 149:

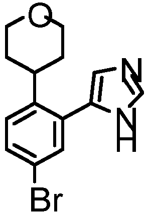

Intermediate **148** (0.6 g, 1.66 mmol) was dissolved in formamide (3 mL) and the reaction was stirred for 11 h at 165 °C. The mixture obtained was diluted with EtOAc, washed with saturated NaHCO₃ and the layers were separated. The aqueous phase was extracted with DCM (3 x 20 mL), the combined organic extracts dried over MgSO4, filtered and concentrated under reduced pressure. The crude mixture was purified by silicagel column chromatography with Heptane/EtOAc (gradient: 0-70% EtOAc). All fractions were combined and concentrated to afford intermediate **149** (0.250 g, 49%).

### Synthesis of intermediate 150:

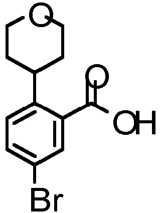

To a solution of intermediate **5** (0.6 g, 2.23 mmol) in DMF (20 mL) was added oxone (0.75g, 2.45 mmol). The reaction mixture was stirred at rt for 48 hours. 1N HCl was used to dissolve the salts and the aqueous phase was extracted with ethyl acetate. The organic layer was washed with 1N HCl (x 3) and brine , dried over Na₂SO₄ and the solvent was reduced under pressure. The crude mixture was purified by flash chromatography using Heptane/Ethyl acetate 0% to 100% as gradient. The desired fractions were collected and concentrated yielding intermediate **150** (0.46 g, 66%).

### Synthesis of intermediate 151:

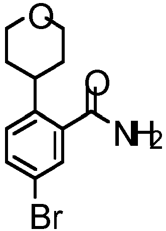

To a solution of intermediate **150** (0.46 g, 1.48 mmol) in dry THF (20 mL) was added portionwise 1,1-carbonylimidazole (0.36g, 2.22 mmol). The mixture was stirred at rt for 2 h. An ammonium hydroxide solution (0.91 g/mL, 11.4 mL, 74 mmol) was added and the reaction was stirred at rt overnight. The reaction mixture was diluted with ethyl acetate (50 ml) and washed with water. The phases were separated and the aqueous layer was extracted with Ethyl acetate (5x30 mL). The organic layer was dried over MgSO4, filtered and evaporated until dryness. The residue was purified by flash chromatography using Heptane/Ethyl acetate 0% to 100% as gradient. The desired fractions were collected and concentrated to afford intermediate **151** (0.349 g, 82%).

### Synthesis of intermediate 152:

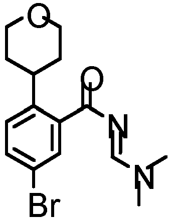

A solution of intermediate **151** (0.27 g, 0.95 mmol) in DMF-DMA (3.4 mL, 25.6 mmol) was stirred and heated at 110°C overnight. The reaction mixture was concentrated and used directly in the next reaction.

### Synthesis of intermediate 153:

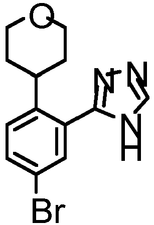

To a solution of intermediate **152** (0.32 g, 0.95 mmol) in AcOH (1.68 mL) was added hydrazine monohydrate (0.101 mL, 2.08 mmol). The reaction mixture was stirred and heated at 120°C for 1 hour. The reaction mixture was concentrated and was triturated with diethyl ether, filtered and the solid was dried under high vacuum yielding intermediate **150** (0.3 g, Quant.).

### Synthesis of intermediate 154:

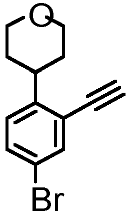

Dimethyl(1-diazo-2-oxopropyl)phosphonate (0.4 mL, 2.68 mmol) was added to a mixture of intermediate **5** (0.6g, 2.23 mmol) and potassium carbonate (0.62 g, 4.46 mmol) in methanol (30 mL). The mixture was stirred at r.t. for 4 h. Methanol was evaporated in vacuo. Half saturated aqueous solution of NaHCO₃ was added and the aqueous phase was extracted with dichloromethane (x4). The combined organic extracts were dried over MgSO₄, filtered and concentrated. The residue was purified by flash column chromatography (Heptane/EtOAc gradient from 100:0 to 80:20) yielding intermediate **154** (0.45 g, 76%).

### Synthesis of intermediate 155:

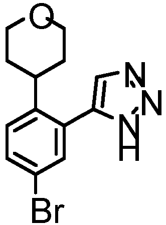

Intermediate **154** (0.25 g, 0.943 mmol) and copper iodide (9 mg, 0.047 mmol) were added in a vial which was sealed with a screw-cap septum. It was backfilled with nitrogen (3 times), then trimethylsilyl azide (0.19 mL, 1.41 mmol) was added via syringe, followed by addition of DMF/MeOH (4/1) (2 mL). The mixture was stirred at 80°C for 10 h. The solvents were evaporated and the mixture was diluted with water and AcOEt. The layers were separated and the aqueous phase extracted with AcOEt. The combined organic extracts were dried over MgSO₄, filtered and concentrated. The residue was purified by flash column chromatography (Heptane/ AcOEt gradient from 100:0 to 85:15) yielding intermediate **155** (0.15 g, 52%).

### Synthesis of intermediate 156:

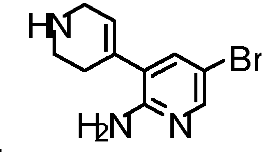

A mixture of intermediate **58** (0.52 g) and a hydrogen chloride solution (4 M in dioxane, 8 mL) in dioxane was stirred at rt overnight. The solvents were evaporated in vacuo and the crude mixture was used in the next step without further purification.

### Synthesis of intermediate 157:

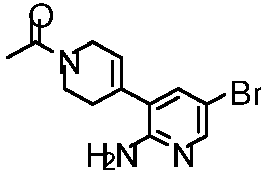

Triethylamine (0.51 mL, 3.67 mmol) was added to a mixture of intermediate **156** (0.427 g, 1.47 mmol), acetic anhydride (0.15 mL, 1.62 mmol) in DCM (15 mL) at room temperature. The mixture was stirred overnight. The solvents were evaporated and the crude mixture was purified by flash column chromatography over silica gel, gradient : DCM/DCM-MeOH (9:1) from 100/0 to 0/100% yielding intermediate **157** (0.41 g, 90%).

### Synthesis of intermediate 158:

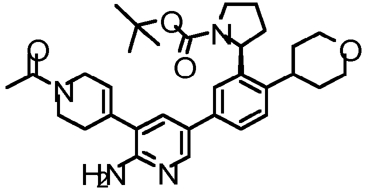

Sodium carbonate (139 mg, 1.31 mmol) was added to a solution of intermediate **157** (0.243 g, 0.66 mmol), intermediate **62** (0.3 g, 0.66 mmol) in water (2 mL) and 1,4-dioxane (12 mL). The mixture was degassed with a stream of nitrogen for 5 min. , then PdCl₂(dppf) (27 mg, 0.033 mmol) was added. The reaction mixture was stirred at 90°C overnight. The reaction mixture was cooled to rt, water and AcOEt were added. The aqueous layer was extracted with ethyl acetate. The organic layer was dried over MgSO₄, filtered and evaporated until dryness. The residue was purified by silicagel chromatography (DCM/DCM:MeOH (4:1) from 100/0 to 0/100. The fractions were collected and evaporated until dryness to give 0.252 g of intermediate **158** (53%, purity 75%).

### Synthesis of intermediate 159:

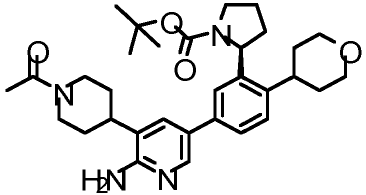

To a solution of intermediate **158** (0.252 g, 0.35 mmol, 75% purity) in methanol (15 mL) was added Pd/C 10% under nitrogen atmosphere. The resulting reaction mixture was stirred at room temperature under hydrogen atmosphere for 3 hours. After completion of the reaction, the catalyst was filtered off on celite and washed with methanol. The filtrate was concentrated under reduced pressure to get intermediate **159** (0.205 g, 97%, 90% purity).

### Synthesis of intermediate 160:

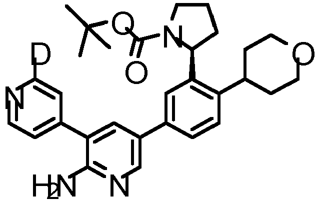

A mixture of intermediate **30** (0.1 g, 0.187 mmol), Pd/C (10 mg, 0.094 mmol) in MeOD (10 mL) was deuterated with D2 (1atm) at room temperature in a round bottom flask and stirred for 20 hours. The mixture was filtered through a pad of Celite^{®} and the filtrate was evaporated until dryness. The residue was purified by preparative LC (12 g of SiOH 30µm Interchim, graduent from 100% DCM to 90% DCM 10% CH3OH 0.1% NH₄OH). The fractions were collected and evaporated until dryness yielding intermediate **160** (0.067 g, 71%).

### Synthesis of intermediate 161:

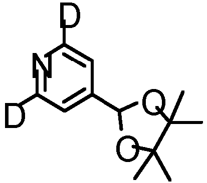

A mixture of 2,6-Dichloropyridine-4-boronic acid, pinacol ester (1 g, 3.65 mmol), Pd/C (0.194 g, 1.82 mmol) in MeOD (20 mL) was deuterated with D2 (1atm) at room temperature in a round bottom flask and stirred for 20 hours. The mixture was filtered through a pad of Celite^{®} and the filtrate was evaporated until dryness used directly in the next step.

### Synthesis of intermediate 162:

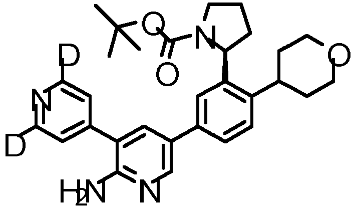

A mixture of intermediate **27** (0.5 g, 1 mmol), intermediate **161** (0.516 g, 2.5 mmol), sodium carbonate (0.21 g, 2 mmol), PdCl₂(dppf) (0.04 g, 0.05 mmol) in 1,4-dioxane (10 mL) and water (1 mL) was heated at 90°C under N₂ for 3 hours in a sealed tube. The reaction mixture was poured into water and potassium carbonate and extracted with ethyl acetate. The organic layer was dried over MgSO4, filtered and evaporated until dryness. The residue was purified by preparative LC (12 g of SiOH 35-40µm GraceResolv, graduent from 100% DCM to 88% DCM 12% CH₃OH 0.1% NH4OH) yielding intermediate **162** (0.204 g, 41%).

### Synthesis of intermediate 163:

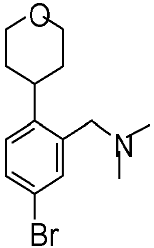

To a solution of Benzenemethanamine, 5-amino-*N,N*-dimethyl-2-(tetrahydro-2H-pyran-4-yl) (6.82 g, 26.75 mmol) in HBr (48% in water, 2 mL) cooled in an ice bath, was added dropwise sodium nitrite (2.03 g, 29.42 mmol) in water. The mixture was stirred for 30 min at 0-4°C. Then this mixture was added dropwise to a solution of copper bromide (2.12 g, 14.71 mmol) in HBr (48% in water, 5.5 mL) cooled in an ice bath. The reaction mixture was stirred at 100°C for 2 hours, then poured into aqueous NaOH (10 mol/L, 200mL) and was extracted with ethyl acetate (100mL*5). The organic layer was dried over MgSO₄, filtered and evaporated until dryness. The residue was purified by column chromatography over silica gel (eluent: petrol/ethyl acetate=100:0-2:1) yielding intermediate **163** (6.02 g, 76.6%) as a white solid.

### Synthesis of intermediate 164:

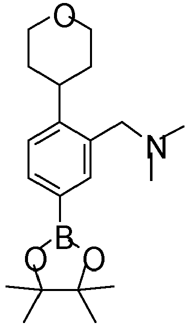

A mixture of intermediate **163** (1.5 g, 5.1 mmol), bis(pinacolato)diboron (1.5 g, 6.1 mmol), PdCl₂(dppf) (0.267 g, 0.3 mmol) and potassium acetate (1.5 g, 15.3 mmol) in 1,4-dioxane (20 mL) was stirred at 120°C using one single mode microwave (Biotage Initiator EXP 60) with a power output ranging from 0 to 400 W for 45min . The reaction mixture was cooled to rt, filtered over celite and water was added. The organic layer was dried over MgSO₄, filtered and evaporated until dryness. The residue was cristallized in DIPE/pentane, the precipitate was filtered off (catalyst derivatives) and the filtrate was evaporated and used as it for next step.

### Synthesis of intermediate 165:

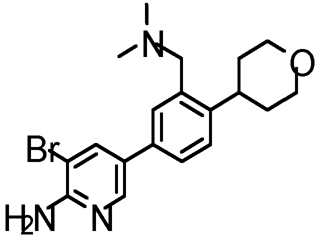

In a sealed tube, a mixture of intermediate **164** (1.75 g, 5.1 mmol), 3-Bromo-5-iodopyridin-2-amine (1.5 g, 5.1 mmol), sodium carbonate (1.6 g, 15.2 mmol), PdCl₂(dppf) (0.415 g, 0.51 mmol) in 1,4-dioxane (22.6 mL) and water (5.3 mL) was stirred at 90°C for 16h. The mixture was poured into H₂O and potassium carbonate and extracted with AcOEt. The organic layer was dried over MgSO4, filtered and evaporated until dryness. The residue was purified by preparative LC (Stationary phase: irregular 15-40µm 80g Merck, Mobile phase: 0.4% NH₄OH, 96% DCM, 4% MeOH) yielding intermediate **165** (0.685 g, 35%).

Intermediate 166 was prepared starting from intermediate 165 and following an analogous reaction protocol as was used for intermediate 158.

| Intermediate | Structure | Quantity | Yield |
|---|---|---|---|
| Intermediate **166** | 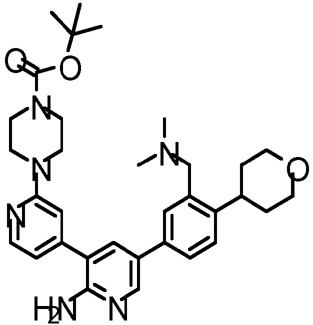 | 0.128 g | 87% |
| Starting from intermediate 165 and 2-(4-tert-Butoxycarbonylpiperazin-1-yl)pyridine-4-boronic acid, pinacol ester | | | |

### Synthesis of intermediate 167:

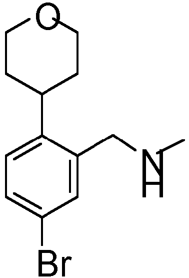

A mixture of intermediate **5** (0.4 g, 1.49 mmol) and methylamine (aqueous solution, 0.129 mL, 1.49 mmol) in MeOH (5 mL) and acetic acid (five drops) was stirred at room temperature for 15 min. Sodiumtriacetoxyborohydride (0.945 g, 4.46 mmol) was added to the resulting mixture and the mixture was stirred at room temperature for 2h. 1 eq of methylamine (0.130 ml) and 1.5 eq of sodiumtriacetoxyborohydride (470 mg) were added to the reaction mixture which was stirred at room temperature overnight. The mixture was evaporated under vacuo and diluted with water, extracted with dichloromethane three times. The combined organic phases were dried over MgSO₄, filtered and concentrated under reduced pressure. The residue was purified by flash chromatography (dichloromethane/(dichloromethane/methanol 9: 1) gradient 100:0 to 0:100). The desired fractions were collected and the solvent removed under reduced pressure to afford 0.15 g of intermediate **167** (0.15 g, 35%).

### Synthesis of intermediate 168:

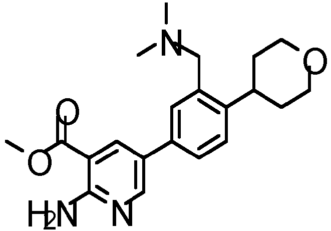

Sodium carbonate (2.36 g, 22.24 mmol) was added to a solution of intermediate **163** (3.35 g, 11.12 mmol) and methyl 2-amino-5-(4,4,5,5-tetramethyl-1,3,2-dioxaborolan-2-yl) nicotinate (4.02 g, 14.5 mmol) in 1,4-dioxane (60 mL) and water (12 mL). The mixture was degassed with a stream of nitrogen for 5 min, then PdCl₂(dppf) (0.455 g, 0.56 mmol) was added. The reaction mixture was stirred at 90 °C overnight. The reaction was diluted with water (20 mL). The layers were separated and the aqueous phase extracted with DCM (3 x 20 mL). The combined organic extracts were dried over MgSO₄, filtered and the solvent removed under reduced pressure. The crude mixture was purified by silicagel column chromatography (Heptane/ EtOAc : 0-40%). The desired fractions were collected and the solvent removed under reduced pressure to afford intermediate **168** (3.3 g, 79%).

### Synthesis of intermediate 169:

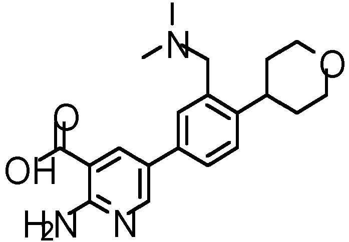

Lithium hydroxide (1.09 g, 17.9 mmol) was added to a solution of intermediate **168** in methanol (12 mL) and water (3 mL). The reaction mixture was stirred at 55 °C for 14h. Methanol was evaporated under reduced pressure and the residue obtained was diluted with EtOAc and water. Organic layer was discarded , the aqueous layer was acidified with HCl 6N and extracted with EtOAc (10 x 20 mL). The organic layer and aqueous layer were filtrated under reduce pressure to obtain a brown solid washed with DCM and dried to yield intermediate **169** (1.05 g, 65%).

### Synthesis of final compounds

### Preparation of Co. 1 (1^{st} approach)

### Synthesis of Co.1 :

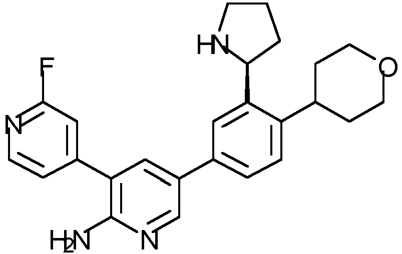

A mixture of intermediate **18** (21.7 g, 67.4 mmol), intermediate **14** (16.04 g, 51.7 mmol), PdCl₂(dppf) (6.78 g, 8.3 mmol) and sodium carbonate (11g, 103.4 mmol) in 1,4-dioxane (800 mL) and water (80 mL) under N₂ was heated at 80°C for 4 hours. The reaction mixture was cooled to rt, water and potassium carbonate were added. The aqueous layer was extracted with ethyl acetate. The organic layer was dried over MgSO₄, filtered and evaporated until dryness. The residue was purified by preparative LC (SiOH 30-40µm GraceResolv, from 100% DCM to DCM 80%/ MeOH 20%/ NH₄OH 1%). The fractions were collected and evaporated until dryness to give **Co.1** which was futher purified in multiple batches via preparative LC (Stationary phase : irregular SiOH 40µm 300 g, mobile phase : DCM 96%/ MeOH 4%/ NH₄OH 0.5%). The pure fractions were collected and evaporated until dryness to give **Co.1** (15.3 g, 71%). **Co.1** was taken up in acetonitrile, heated at 50°C 15 min, cooled down. The compound was filtered off and dried yielding 11.5 g of **Co.1** (53%) as an off-white powder.[α]_{d}: - 42.22° (589 nm, c 0.27 w/v %, DMF, 20 °C); m.p. = 218°C (DSC).

The following compound was prepared via an analogous procedure :

| Compound | Structure | Quantity | Yield |
|---|---|---|---|
| **Co. 1b** | 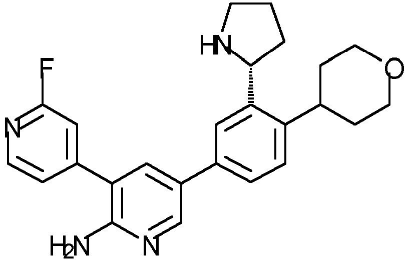 [α]_{d}: +20.3° (589 nm, c 0.093, w/v %, MeOH, 23 °C) | | |
| | | 0.085 g | 54% |

### Preparation of Co. 1 (2^{nd} approach)

### Synthesis of Co.1 :

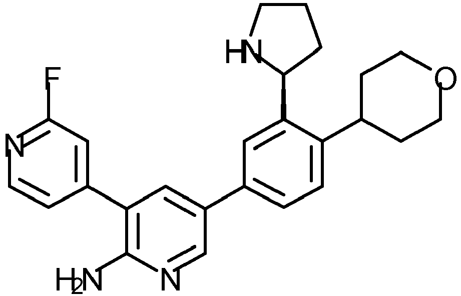

Trifluoroacetic acid (13 mL, 0.17 mol) was added dropwise to a solution of intermediate **29** (3.1 g, 6 mmol) in DCM (80 mL). The mixture was stirred at rt for 15h, poured into water, basified with potassium carbonate. The aqueous phase was extracted with ethyl acetate. The organic layer was separated, dried over MgSO_{4,}, filtered and evaporated until dryness. The residue was purified by preparative LC (40 g SiOH 30µm Interchim, from 100% DCM to DCM 80%/ MeOH 20%/ NH₄OH 1%). Two fractions were obtained : F1 = 2 g and F2 = 0.5 g. F1 was crystallized in acetonitrile, filtered, washed with diethylether and dried to give F3. The filftrate and F2 were combined and purified by preparative LC (40 g SiOH 30µm Interchim, from 100% DCM to DCM 80%/ MeOH 20%/ NH₄OH 1%). The fractions were collected and evaporated until dryness to give F4 = 0.75 g. F4 and F3 were combined, taken up in DCM and a solution of potassium carbonate 10%. The organic layer was separated, dried over MgSO₄, filtered and evaporated until dryness. The compound was crystallized in acetonitrile, filtered and dried to give 1.54 g of Co. 1 (62%).[α]_{d}: - 35.95° (589 nm, c 0.194 w/v %, DMF, 20 °C); m.p. = 218°C (DSC).

The compounds listed in the table below have been prepared by an analogous reaction protocol as was used for the synthesis of Co. 1 (1^{st} approach).

| Compound | Structure | Quantity | Yield |
|---|---|---|---|
| Co. **2** | 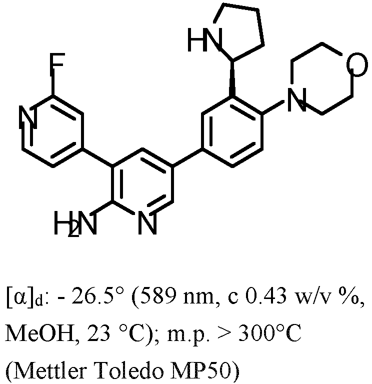 | 0.197 g | 95% |
| Starting from Intermediate 16 | | | |
| Co.**3** | 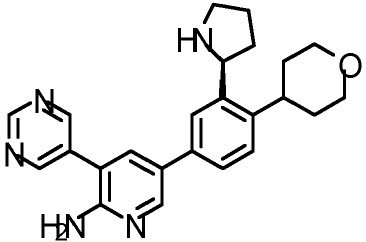 [α]_{d}: -34° (589 nm, c 0.11 w/v %, MeOH, 23 °C); m.p. = 245°C (Mettler Toledo MP50) | 0.105 g | 80% |
| Starting from Intermediate 23 | | | |
| Co.**4** | 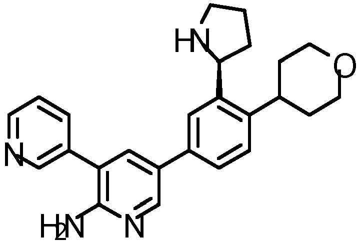 [α]_{d}: - 11.9° (589 nm, c 0.186 w/v %, MeOH, 23 °C); m.p. = 257°C (Mettler Toledo MP50) | 0.12 g | 92% |
| Starting from Intermediate 24 | | | |
| Co.**5a** | 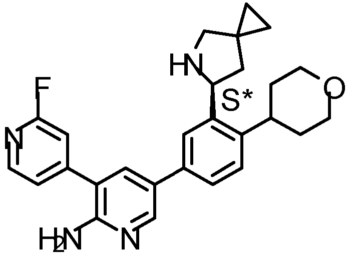 [α]_{d}: - 7.42° (589 nm, c 0.31 w/v %, DMF, 20 °C) | 0.064 g | 35% |
| Starting from Intermediate 103 | | | |
| Co.**5b** | 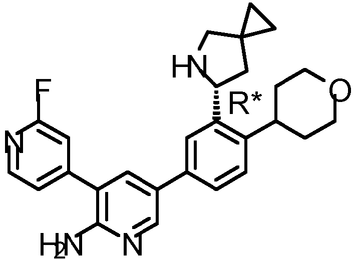 [α]_{d}: +7.2° (589 nm, c 0.25 w/v %, DMF, 20 °C) | 0.055 g | 30% |
| Starting from Intermediate 103 | | | |
| Co.**6** | 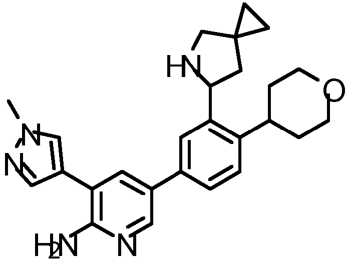 m.p. = 196.5°C (Mettler Toledo MP50) | 0.088 g | 32% |
| Starting from Intermediate 103 & 25 | | | |
| Co.**7a** | 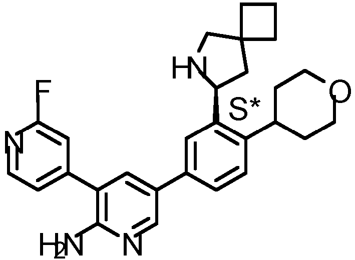 [α]_{d}: + 7.78° (589 nm, c 0.18 w/v %, DMF, 20 °C) | 0.024 g | 28% |
| Starting from Intermediate 104 & 18 | | | |
| Co.**7b** | 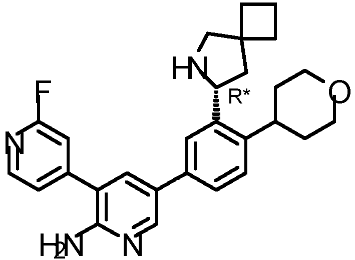 [α]_{d}: -10.43° (589 nm, c 0.23 w/v %, DMF, 20 °C) | 0.024 g | 28% |
| Starting from Intermediate 104 & 18 | | | |
| Co.**8a** | 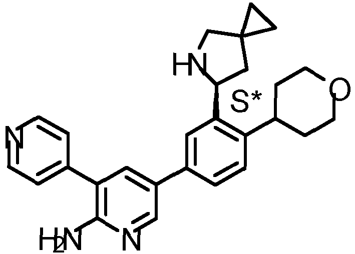 [α]_{d}: + 6.32° (589 nm, c 0.19 w/v %, DMF, 20 °C) | 0.024 g | 17% |
| Starting from Intermediate 103 & 26 | | | |
| Co.**8b** | 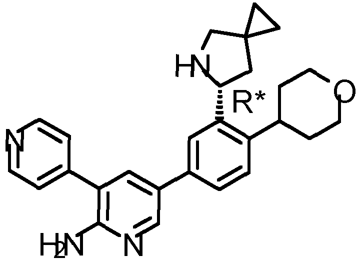 [α]_{d}: -6.09° (589 nm, c 0.23 w/v %, DMF, 20 °C) | 0.035 g | 25% |
| Starting from Intermediate 103 & 26 | | | |
| Co.**9** | Racemic mixture of 2 trans forms | 0.080 g | 36% |
| Starting from Intermediate 104 & 109 | 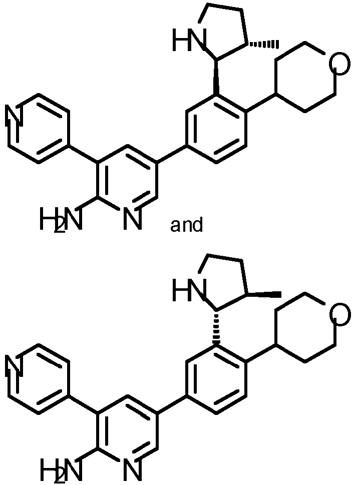 m.p. = 218.3°C (Mettler Toledo MP50) | | |
| Co.**10** | Racemic mixture of 2 cis forms | 0.014 g | 14% |
| Starting from Intermediate 104 & 108 | 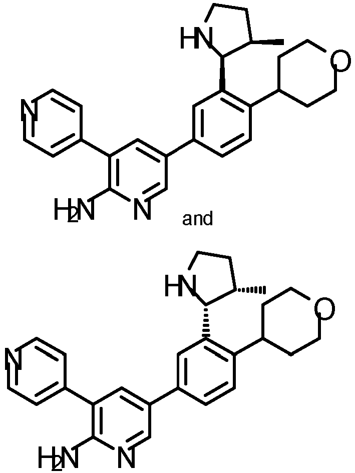 | | |
| Co.**11** | 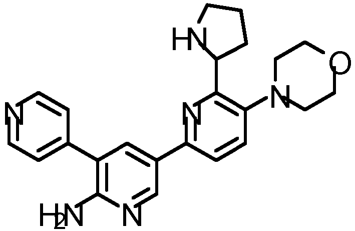 m.p. = 245°C (Mettler Toledo MP50) | 0.163 g | 43% |
| Starting from Intermediate 104 & 115 | | | |
| Co.**12** | 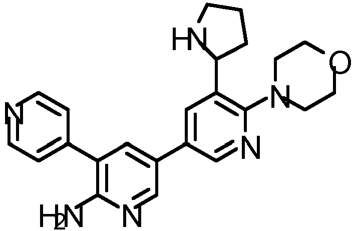 m.p. = 196°C (Mettler Toledo MP50) | 0.21 g | 36% |
| Starting from Intermediate 104 & 119 | | | |
| Co.**13** | 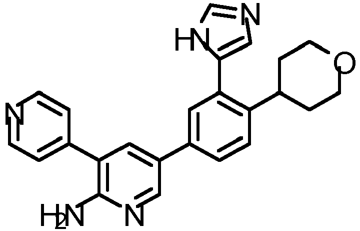 m.p. = 257°C (Mettler Toledo MP50) | 0.07 g | 21% |
| Starting from Intermediate 149 and 104 | | | |
| Co.**14** | 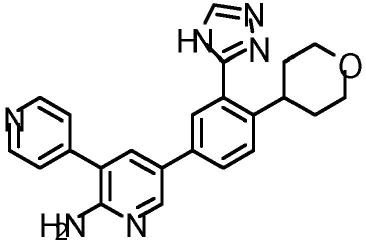 m.p. = 263°C (Mettler Toledo MP50) | 0.14 g | 54% |
| Starting from Intermediate 153 and 104 | | | |
| Co.**15** | 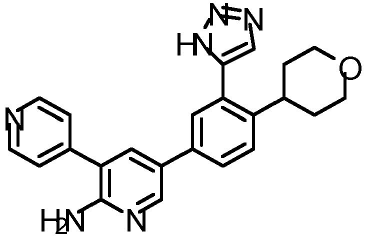 m.p. > 300°C (Mettler Toledo MP50) | 0.03 g | 16% |
| Starting from Intermediate 155 and 104 | | | |
| **Co. 15a** | 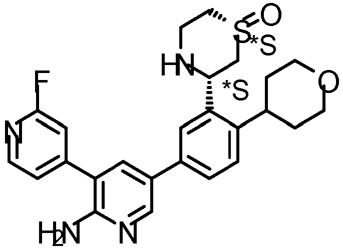 [α]_{d}: +29.6° (589 nm, c 0.22, w/v %, DMF, 20 °C) | 0.039 g | 10% |
| Starting from intermediates 145 and 18 | | | |
| **Co. 15b** | 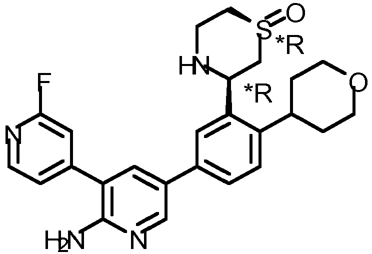 [α]_{d}: -9° (589 nm, c 0.2, w/v %, DMF, 20 °C) | 0.037 g | 9.5% |
| Starting from intermediates 145 and 18 | | | |
| **Co. 15c** | 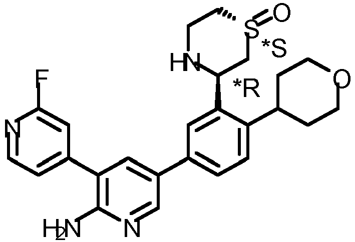 [α]_{d}: -4.4° (589 nm, c 0.23, w/v %, DMF, 20 °C) | 0.026 g | 6.7% |
| Starting from intermediates 145 and 18 | | | |
| **Co. 15d** | 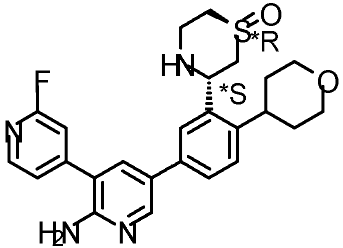 [α]_{d}: +9° (589 nm, c 0.19, w/v %, DMF, 20 °C) | 0.028 g | 7.2% |
| Starting from intermediates 145 and 18 | | | |
| Co.**16** | 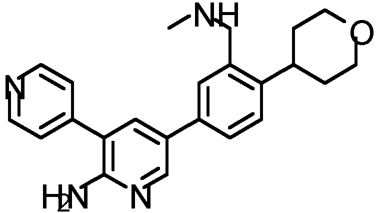 m.p. = 265°C (Mettler Toledo MP50) | 0.065 g | 32% |
| Starting from intermediate 167 and 104 | | | |

The compounds listed in the table below have been prepared by an analogous reaction protocol as was used for the synthesis of Co. 1 (2^{nd} approach).

| Compound | Structure | Quantity | Yield |
|---|---|---|---|
| Co. **17** | 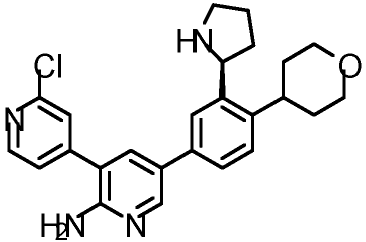 | 0.241 g | 70% |
| Starting from Intermediate 30 | | | |
| | [α]_{d}: - 36.4° (589 nm, c 0.25 w/v %, DMF, 20 °C) | | |
| Co. **18** | 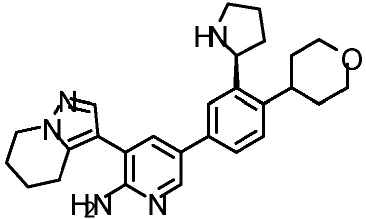 | 0.052 g | 40% |
| Starting from Intermediate 31 | | | |
| | [α]_{d}: - 45° (589 nm, c 0.24 w/v %, DMF, 20 °C) | | |
| Co. **19** | 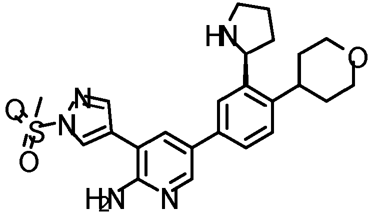 | 0.019 g | 23% |
| Starting from Intermediate 32 | | | |
| | [α]_{d}: - 55° (589 nm, c 0.18 w/v %, DMF, 20 °C) | | |
| Co. **20** | 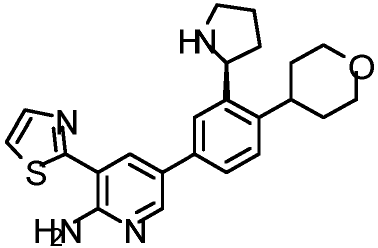 [α]_{d}: - 39.6° (589 nm, c 0.23 w/v %, DMF, 20 °C) | 0.026 g | 77% |
| Starting from Intermediate 33 | | | |
| Co. **21** | 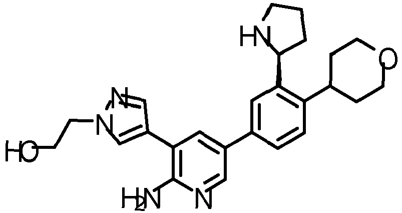 | 0.024 g | 45% |
| Starting from Intermediate 34 | | | |
| | [α]_{d}: - 41.8° (589 nm, c 0.22 w/v %, DMF, 20 °C) | | |
| Co. **22** | 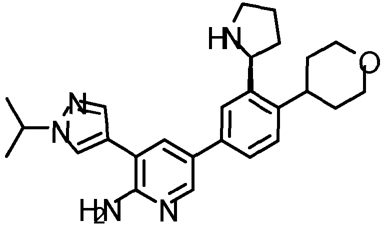 | 0.026 g | 55% |
| Starting from intermediate 35 | | | |
| | [α]_{d}: - 40.9° (589 nm, c 0.22 w/v %, DMF, 20 °C); m.p. = 191.7 °C (DSC) | | |
| Co. **23** | 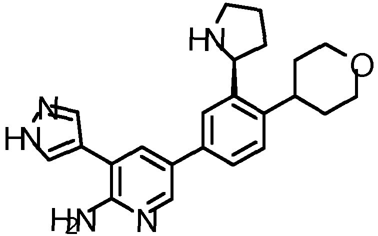 | 0.026 g | 38% |
| Starting from Intermediate 36 | | | |
| | [α]_{d}: - 46.5° (589 nm, c 0.2 w/v %, DMF, 20 °C); m.p. = 223.4° C (DSC) | | |
| Co. **24** | 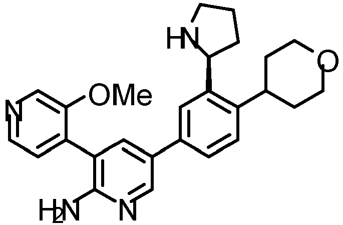 | 0.025 g | 42% |
| Starting from intermediate 37 | | | |
| | [α]_{d}: -36.1° (589 nm, c 0.31 w/v %, DMF, 20 °C); m.p. = 217.5° C (DSC) | | |
| Co. **25** | 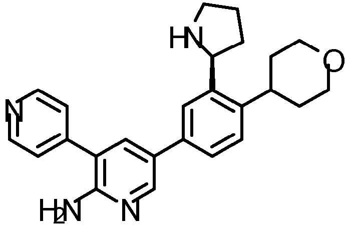 | 0.057 g | 56% |
| Starting from Intermediate 38 | | | |
| | [α]_{d}: - 44.4° (589 nm, c 0.25 w/v %, DMF, 20 °C); m.p. = 238.1° C (DSC) | | |
| Co.**25b** | 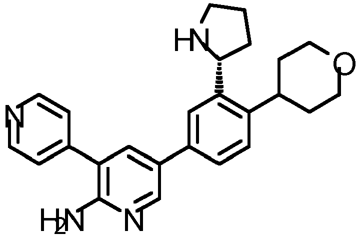 | 0.033 g | 26% |
| | | | |
| | [α]_{d}: +36° (589 nm, c 0.28 w/v %, DMF, 20 °C) | | |
| Co. **26** | 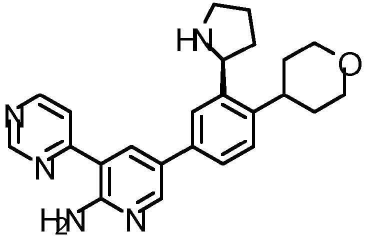 | 0.065 g | 85% |
| Starting from Intermediate 64 | | | |
| | [α]_{d}: - 21.7° (589 nm, c 0.12 w/v %, MeOH, 23 °C); m.p. = 201° C (Mettler Toledo MP50) | | |
| Co. **27** | 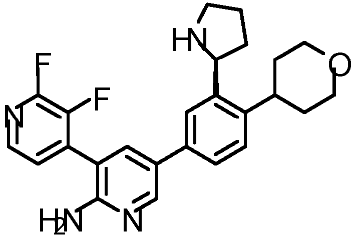 | 0.068 g | 83% |
| Starting from Intermediate 65 | | | |
| | [α]_{d}: -94.8° (589 nm, c 0.25 w/v %, DMF, 20 °C); m.p. > 300° C (Mettler Toledo MP50) | | |
| Co. **28** | 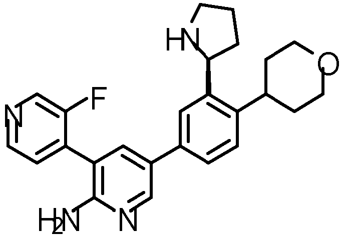 | 0.052 g | 46 % |
| Starting from Intermediate 66 | | | |
| | [α]_{d}: - 29.6° (589 nm, c 0.36 w/v %, MeOH, 23 °C); m.p. = 230° C (Mettler Toledo MP50) | | |
| Co. **29** | 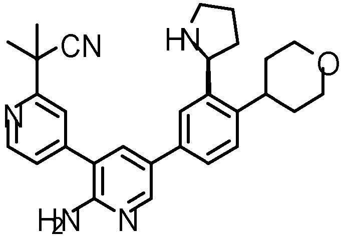 | 0.11 g | 68% |
| Starting from Intermediate 67 | | | |
| | [α]_{d}: - 18.2° (589 nm, c 0.39 w/v %, MeOH, 23 °C); m.p. = 228° C (Mettler Toledo MP50) | | |
| Co. **30** | 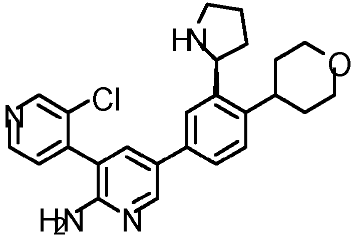 | 0.088 g | 71% |
| Starting from Intermediate 68 | | | |
| | [α]_{d}: - 23.9° (589 nm, c 0.27 w/v %, MeOH, 23 °C); m.p. > 300° C (Mettler Toledo MP50) | | |
| Co. **31** | 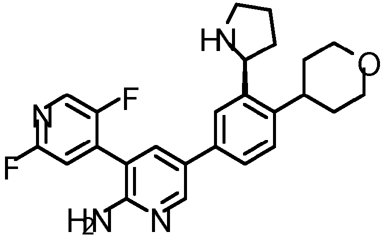 | 0.085 g | 68% |
| Starting from Intermediate 69 | | | |
| | [α]_{d}: - 25.5° (589 nm, c 0.093 w/v %, MeOH, 23 °C); m.p. = 225° C (Mettler Toledo MP50) | | |
| Co. **32** | 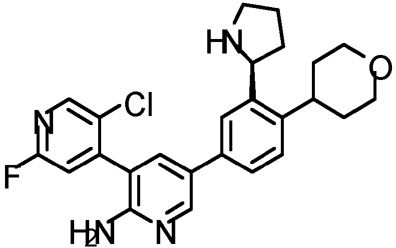 | 0.093 g | 49% |
| Starting from Intermediate 70 | | | |
| | [α]_{d}: - 28.1° (589 nm, c 0.73 w/v %, MeOH, 23 °C); m.p. = 221.6° C (Mettler Toledo MP50) | | |
| Co. **33** | 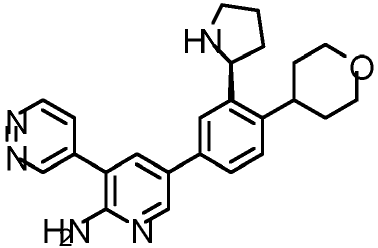 | 0.071 g | 39% |
| Starting from Intermediate 71 | | | |
| | [α]_{d}: - 23.1° (589 nm, c 0.73 w/v %, MeOH, 23 °C); m.p. = 240° C (Mettler Toledo MP50) | | |
| Co. **34** | 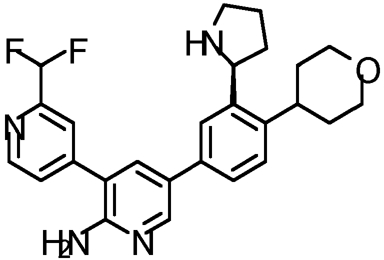 | 0.007 g | 21% |
| Starting from Intermediate 72 | | | |
| Co. **35** | 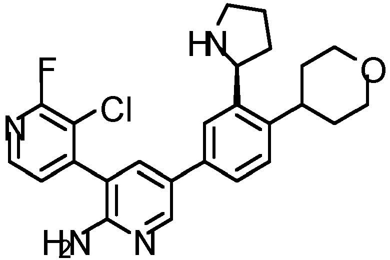 | 0.049 g | 82% |
| Starting from Intermediate 73 | | | |
| | [α]_{d}: - 16.6° (589 nm, c 0.53 w/v %, MeOH, 23 °C); m.p. = 218° C (Mettler Toledo MP50) | | |
| Co. **36** | 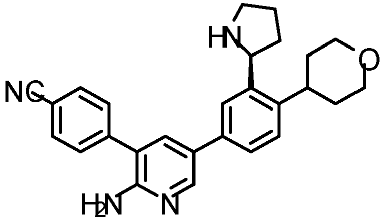 | 0.049 g | 82% |
| Starting from Intermediate 74 | | | |
| | [α]_{d}: - 22.6° (589 nm, c 0.1 w/v %, MeOH, 23 °C); m.p. = 231.6° C (Mettler Toledo MP50) | | |
| Co. **37** | 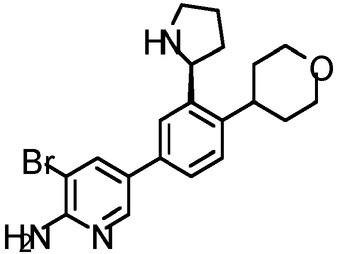 | 0.052 g | 65% |
| Starting from Intermediate 27 | | | |
| | [α]_{d}: - 42.1° (589 nm, c 0.24 w/v %, DMF, 20 °C); m.p. = 173° C (Mettler Toledo MP50) | | |
| Co. **38** | 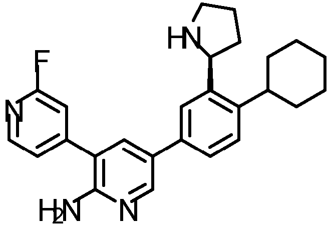 | | |
| Starting from Intermediate 75 | | 0.015 g | 37% |
| Co.**39** | 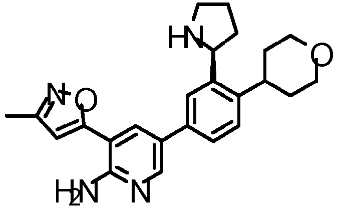 | | |
| Starting from Intermediate 76 | | 0.075 g | 64% |
| | [α]_{d}: - 18.7° (589 nm, c 0.13 w/v %, MeOH, 23 °C); m.p. = 184.8° C (Mettler Toledo MP50) | | |
| Co. **40** | 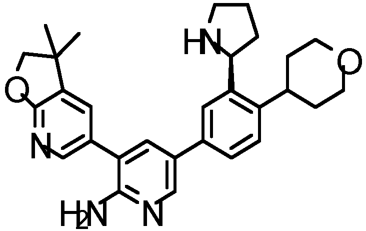 | | |
| Starting from Intermediate 77 | | 0.04 g | 22% |
| Co. **41** | 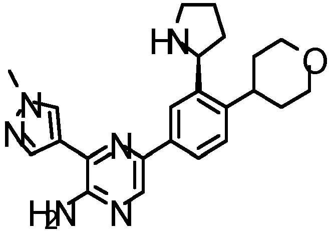 [α]_{d}: - 33.9° (589 nm, c 0.1 w/v %, MeOH, 23 °C); m.p. = 216.5° C (Mettler Toledo MP50) | | |
| Starting from Intermediate 78 | | 0.1 g | 49% |
| Co. **42** | 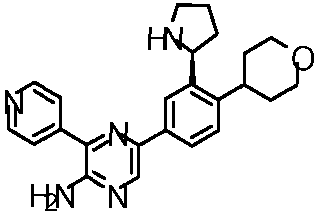 | | |
| Starting from Intermediate 79 | | 0.03 g | 68% |
| | [α]_{d}: - 37.47° (589 nm, c 0.13 w/v %, MeOH, 23 °C); m.p. = 262° C (Mettler Toledo MP50) | | |
| Co. **43** | 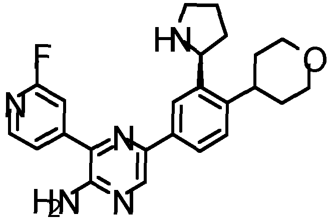 | | |
| Starting from Intermediate 80 | | 0.063 g | 80% |
| | [α]_{d}: - 24.8° (589 nm, c 0.08 w/v %, MeOH, 23 °C); m.p. = 217° C (Mettler Toledo MP50) | | |
| Co. **44** | 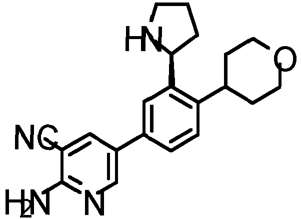 | | |
| Starting from Intermediate 82 | | 0.404 g | 27% |
| | [α]_{d}: - 56.8° (589 nm, c 0.19 w/v %, DMF, 20 °C); m.p. = 206°C (DSC) | | |
| Co. **45** | 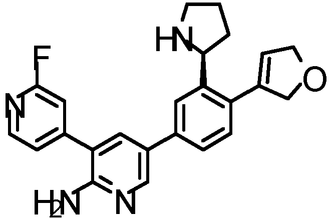 | 0.25 g | 95% |
| Starting from intermediate 122 | | | |
| | [α]_{d}: - 50.4° (589 nm, c 0.28 w/v %, DMF, 20 °C); m.p. = 206.7°C (DSC) | | |
| Co. **46** | 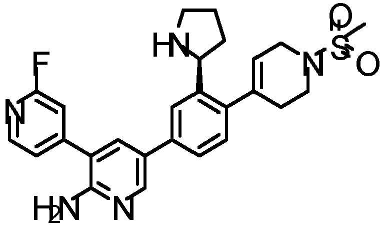 | 0.33 g | 53% |
| Starting from intermediate 123 | | | |
| | m.p. = 224°C (DSC) | | |
| Co. **47** | 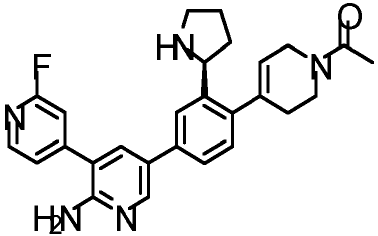 | 0.1 g | 22% |
| Starting from intermediate 124 | | | |
| Co. **48** | 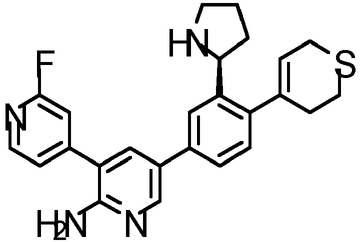 | 0.41 g | 97% |
| Starting from intermediate 125 | | | |
| | m.p. = 208°C (DSC) | | |
| Co. **49** | 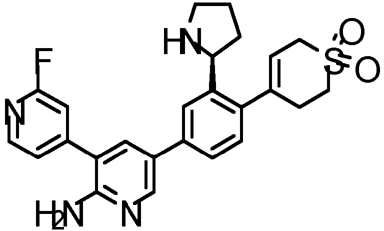 | 0.05 g | 29% |
| Starting from intermediate 126 | | | |
| Co. **50** | 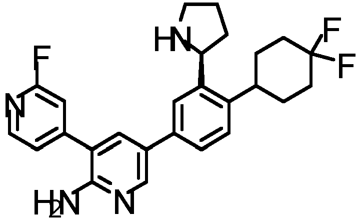 m.p. = 219°C (DSC) | 0.036 g | 38% |
| Starting from intermediate 129 | | | |
| Co. **51** | 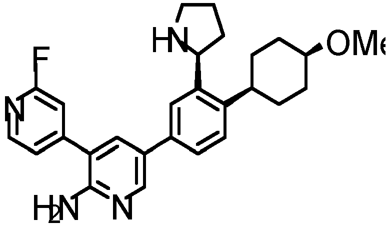 | 0.018 g | 49% |
| Starting from intermediate 130 | | | |
| Co. **52** | 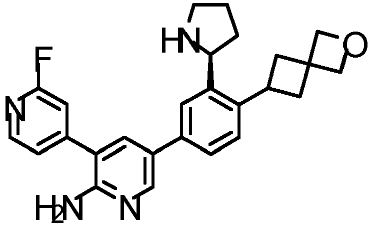 | 0.016 g | 26% |
| Starting from intermediate 131 | | | |
| | [α]_{d}: - 26.2° (589 nm, c 0.29 w/v %, DMF, 20 °C); m.p. = 222°C (DSC) | | |
| Co. **53** | 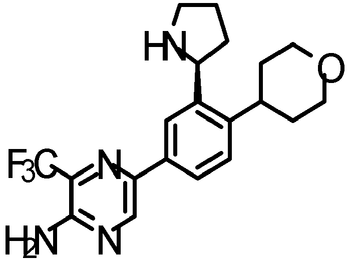 | 0.019 g | 40% |
| Starting from intermediate 133 | | | |
| | [α]_{d}: - 44.55° (589 nm, c 0.11 w/v %, DMF, 20 °C) | | |
| Co. **54** | 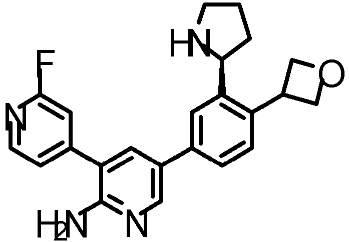 | 0.02 g | 13% |
| Starting from intermediate 132 | | | |
| | [α]_{d}: - 90° (589 nm, c 0.42 w/v %, DMF, 20 °C) | | |
| Co. **55** | 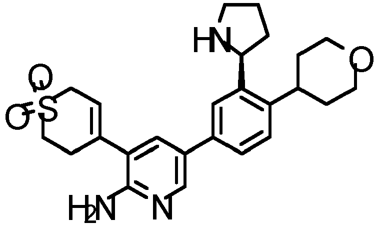 | 0.045 g | 20% |
| Starting from intermediate 39 | | | |
| | [α]_{d}: - 33.7° (589 nm, c 0.27 w/v %, DMF, 20 °C) | | |
| Co. **56** | 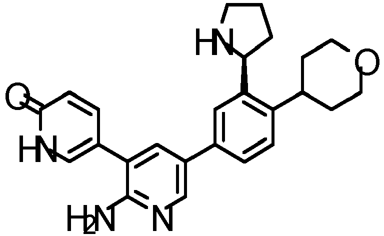 | 0.019 g | 20% |
| Starting from intermediate 40 | | | |
| Co. **57** | 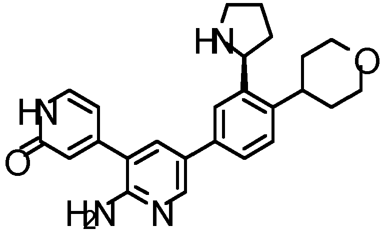 | 0.039 g | 40% |
| Starting from intermediate 41 | | | |
| | [α]_{d}: - 30.5° (589 nm, c 0.22 w/v %, DMF, 20 °C) | | |
| Co. **58** | 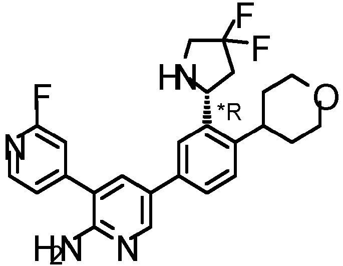 [α]_{d}: + 26.8° (589 nm, c 0.22 w/v %, DMF, 20 °C); m.p. = 216°C (DSC) | 0.039 g | 21% |
| Starting from intermediate 142 | | | |
| Co. **59** | 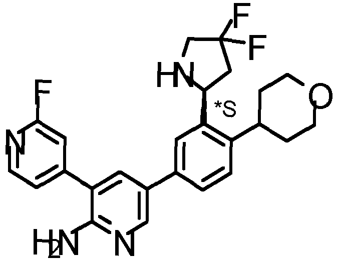 | 0.045 g | 24% |
| Starting from intermediate 142 | | | |
| | [α]_{d}: - 23.9° (589 nm, c 0.26 w/v %, DMF, 20 °C); m.p. = 215°C (DSC) | | |
| Co. **60** | 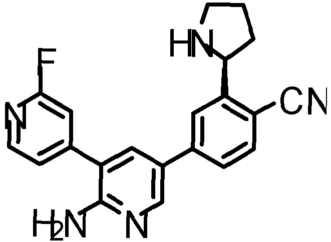 | 0.076 g | 49% |
| Starting from intermediate 143 | | | |
| Co. **61** | 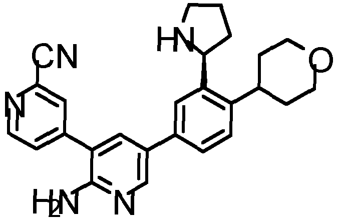 | 0.072 g | 39% |
| Starting from intermediate 42 | | | |
| | [α]_{d}: - 29.3° (589 nm, c 0.15, w/v %, DMF, 20 °C) | | |
| Co. **62** | 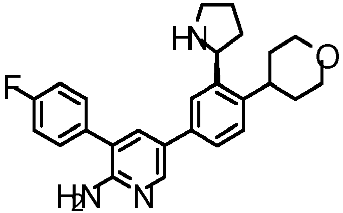 | 0.102 g | 52% |
| Starting from intermediate 43 | | | |
| | [α]_{d}: - 35.83° (589 nm, c 0.26 w/v %, DMF, 20 °C); m.p. = 216°C (DSC) | | |
| Co. **63** | 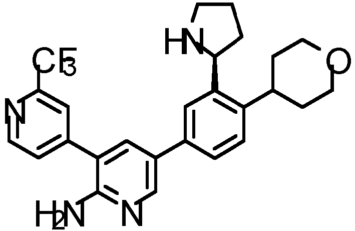 | 0.084 g | 46% |
| Starting from intermediate 44 | | | |
| | [α]_{d}: - 40° (589 nm, c 0.1, w/v %, DMF, 20 °C); m.p. = 213°C (DSC) | | |
| Co. **64** | 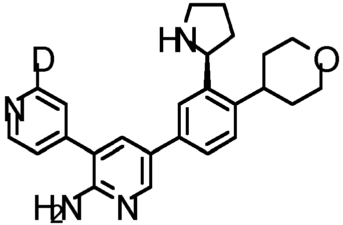 | 0.04 g | 33 % |
| Starting from intermediate 160 | | | |
| **Co. 65** | 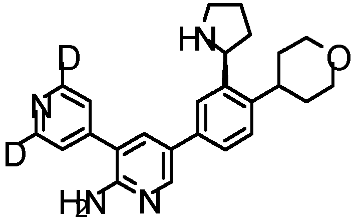 | 0.106 g | 65% |
| Starting from intermediate 162 | | | |
| **Co. 66** | 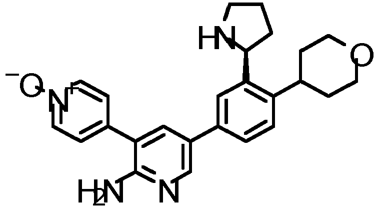 | 0.110 g | 48% |
| Starting from intermediate 84 | | | |
| | [α]_{d}: -32.2° (589 nm, c 0.27, w/v %, DMF, 20 °C); m.p. > 260°C (Kofler) | | |
| **Co.67** | 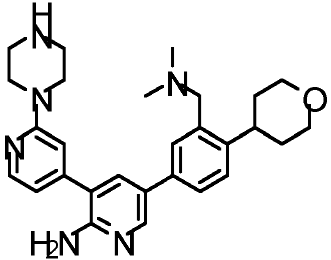 | 0.05 g | 48% |
| Starting from intermediate 166 | | | |
| | m.p. = 185°C (Kofler) | | |

### Synthesis of Co.68 :

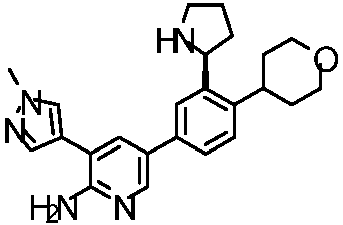

Sodium carbonate (119 mg, 1.12 mmol) was added to a solution of intermediate **89** (200 mg, 0.497 mmol), 1-Methyl-1H-pyrazole-4-boronic acid pinacol ester (124 mg, 0.6 mmol) in water (1.16 mL) and 1,4-dioxane (5.8 mL). The mixture was degassed with a stream of nitrogen for 5 min. , then PdCl₂(dppf) (34 mg, 0.041 mmol) was added. The reaction mixture was stirred at 90°C overnight in a sealed tube. The reaction mixture was cooled to rt, water and AcOEt were added. The aqueous layer was extracted with ethyl acetate. The organic layer was dried over MgSO₄, filtered and evaporated until dryness. The residue was purified by preparative LC (12 g SiOH 15-40 µm Interchim, mobile phase: DCM/MeOH/NH₄OH 97/3/0 to 90/10/0.1) The pure fractions were collected and evaporated until dryness to give 80 mg of **Co.68** (39%) which was taken up in Et₂O. The precipitate was filtered off and dried yielding 53 mg of **Co.68** (26%).

The compounds listed in the table below have been prepared by an analogous reaction protocol:

| Compound | Structure | Quantity | Yield |
|---|---|---|---|
| Co. **69** | 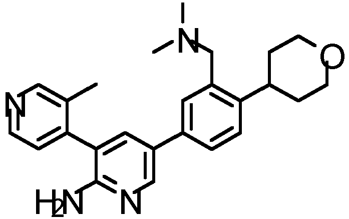 m.p. = 236°C (Kofler) | 0.023 g | 22% |
| Starting from Intermediate 165 | | | |
| Co. **70** | 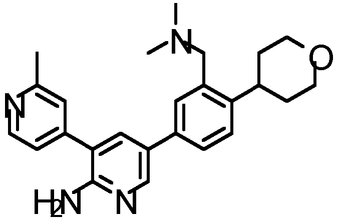 m.p. = 225°C | 0.057 g | 55% |
| Starting from Intermediate 165 | | | |
| Co. **71** | 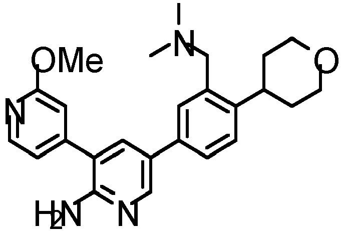 m.p. = 222°C (Kofler) | 0.056 g | 52% |
| Starting from Intermediate 165 | | | |
| **Co.72** | 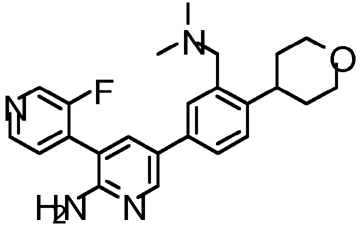 m.p. = 234°C (Kofler) | 0.063 g | 60% |
| Starting from Intermediate 165 | | | |
| **Co.73** | 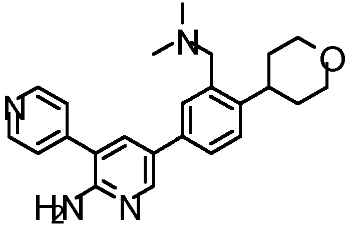 m.p. = 227°C (DSC) | 0.064 g | 80% |
| Starting from Intermediate 165 | | | |
| **Co.74** | 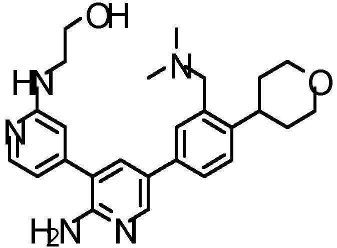 | 0.039 g | 34% |
| Starting from intermediate 165 and 2-(4-(4,4,5,5-tetramethyl-1,3,2-dioxaborolan-2-yl) pyridin-2-ylamino) ethanol | | | |
| **Co.75** | 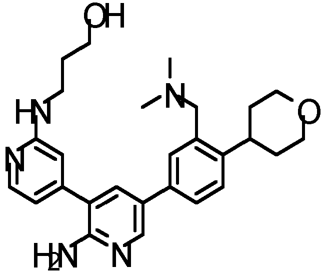 m.p. = 170°C (Kofler) | 0.071g | 60% |
| Starting from intermediate 165 and 3-(4-(4,4,5,5-tetramethyl-1,3,2-dioxaborolan-2-yl) pyridin-2-ylamino) propan-1-ol | | | |

### Synthesis of Co.76:

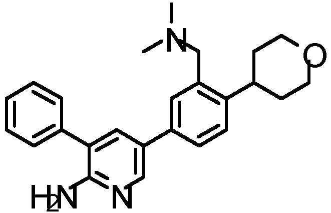

A mixture of intermediate **164** (0.119 g, 0.345 mmol), 5-bromo-3phenylpyridine-2-amine (0.08 g, 0.321 mmol), PdCl₂(dppf) (0.028 g, 0.0345 mmol) and sodium carbonate (0.11 g, 1.034 mmol) in 1,4-dioxane (1.5 mL) and water (0.4 mL) in a sealed tube was stirred at 120°C using one single mode microwave (Biotage Initiator EXP 60) with a power output ranging from 0 to 400 W for 30 min. The reaction mixture was poured into water and potassium carbonate, extracted with AcOEt. The organic layer was dried over MgSO₄, filtered and evaporated until dryness. The residue was purified by preparative LC (24 g of SiOH 30µm Interchim, graduent from 100% DCM to 90% DCM 10% CH₃OH 0.1% NH4OH). The fractions were collected and evaporated until dryness and the residue was cristallized in DIPE. The precipitate was filtered off and dried yielding **Co.76** (0.028g, 21%). m.p. = 173°C (Kofler).

### Synthesis of Co.77:

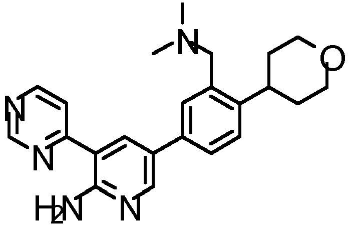

A mixture of intermediate **165** (0.100 g, 0.26 mmol), 4-(tributylstannyl)pyrimidine (0.114 g, 0.307 mmol), Copperiodide (11.7 mg, 0.062 mmol), Pd(PPh₃)₄ (0.021g, 0.018 mmol) and lithiumchloride (0.016 g, 0.384 mmol) in 1,4-dioxane (5 mL) was stirred at 130°C in a Biotage Microwave for 20 minutes. The reaction mixture was poured into water, extracted with AcOEt. The organic layer was dried over MgSO₄, filtered and evaporated until dryness. The residue was purified by preparative LC (40g of SiOH, 95% DCM 5% CH₃OH 0.1% NH₄OH). The fractions were collected and evaporated until dryness yielding **Co.77** (0.051g, 51%).

The compounds listed in the table below have been prepared by an analogous reaction protocol as described for the preparation of intermediate 27 :

| Compound | Structure | Quantity | Yield |
|---|---|---|---|
| Co. **78** | 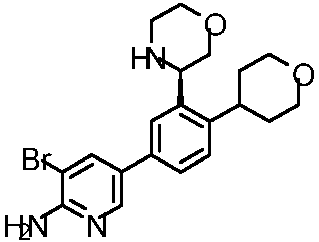 [α]_{d}: - 77.8° (589 nm, c 0.27 w/v %, DMF, 20 °C); m.p. = 208°C (DSC) | 0.5 g | 56% |
| Starting from Intermediate 96 | | | |
| Co. **79** | 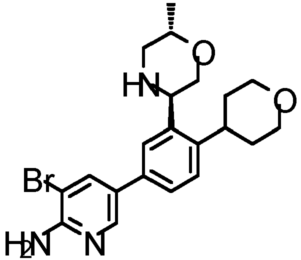 | 1.85 g | 66% |
| Starting from Intermediate 97 | | | |

The compounds listed in the table below have been prepared by an analogous reaction protocol as for preparation of intermediate 64 :

| Compound | Structure | Quantity | Yield |
|---|---|---|---|
| Co. **80a** | 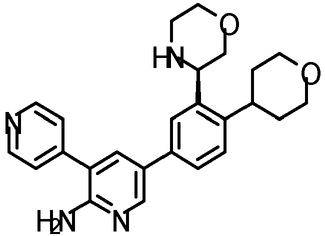 [α]_{d}: - 28.4° (589 nm, c 0.26 w/v %, DMF, 20 °C) | 0.07 g | 24% |
| Starting from Intermediate 98 and 5-bromo-[3,4'-bipyridin]-2-amine | | | |
| Co. **80b** | 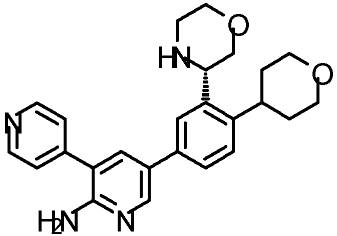 [α]_{d}: +48° (589 nm, c 0.11 w/v %, DMF, 20 °C) | 0.022 g | 17% |
| Co. **81** | 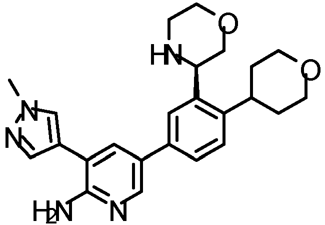 [α]_{d}: - 70° (589 nm, c 0.21 w/v %, DMF, 20 °C) | 0.025 g | 10% |
| Starting from Intermediate 98 and 5-bromo-3-(1-methyl-1H-pyrazol-4-yl) pyridin-2-amine | | | |
| Co. **82** | 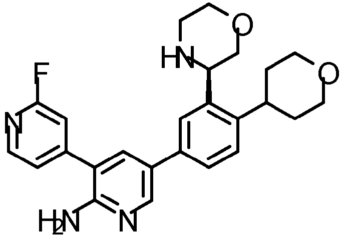 [α]_{d}: - 73.25° (589 nm, c 0.24 w/v %, DMF, 20 °C) | 0.072 g | 36% |
| Starting from Intermediate 98 and 17 | | | |
| Co.**83a** | 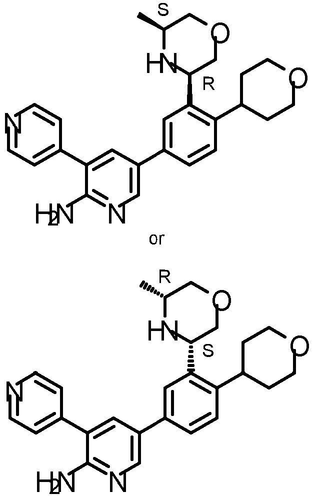 [α]_{d}: - 84.9° (589 nm, c 0.35 w/v %, DMF, 20 °C) | 0.04 g | 5% |
| Starting from Intermediate 99 and 5-bromo-[3,4'-bipyridin]-2-amine | | | |
| Co. **83b** | 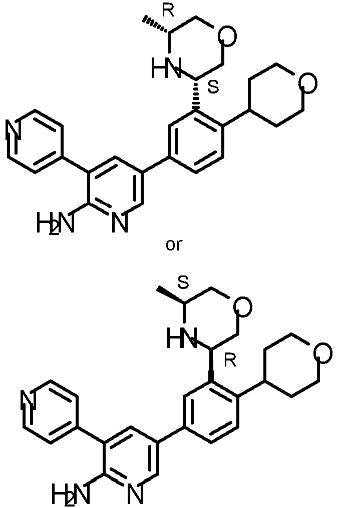 [α]_{d}: +82.2° (589 nm, c 0.35 w/v %, DMF, 20 °C) | 0.036 g | 4% |
| Co. **84** | 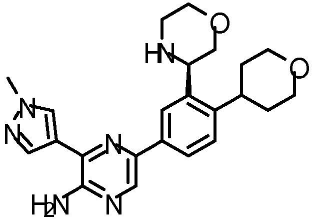 [α]_{d}: - 79.6° (589 nm, c 0.26 w/v %, DMF, 20 °C) m.p. = 243°C (DSC) | 0.06 g | 23% |
| Starting from Intermediate 98 and 2-pyrazinamine, 5-bromo-3-(1-methyl-1H-pyrazol-4-yl) | | | |
| Co. **85** | 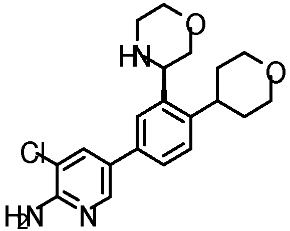 [α]_{d}: - 82.4° (589 nm, c 0.25 w/v %, DMF, 20 °C) m.p. = 213°C (DSC) | 0.148 g | 52% |
| Starting from Intermediate 98 and 2-pyridinamine, 5-bromo-3-chloro | | | |
| Co. **86** | 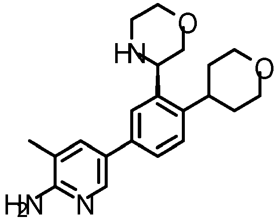 [α]_{d}: - 22.3° (589 nm, c 0.22 w/v %, DMF, 20 °C) m.p. = 266°C (DSC) | 0.069 g | 32% |
| Starting from Intermediate 98 and 2-Amino-5-bromo-3-methylpyridine | | | |
| Co. **87** | 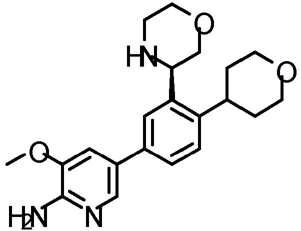 [α]_{d}: - 78.9° (589 nm, c 0.19 w/v %, DMF, 20 °C) m.p. = 239°C (DSC) | 0.053 g | 23% |
| Starting from Intermediate 98 and 5-bromo-3-methoxypyridin-2-amine | | | |
| Co. **88** | 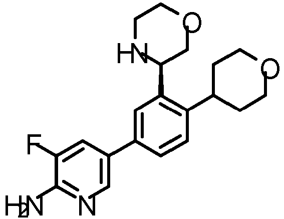 [α]_{d}: - 83.5° (589 nm, c 0.2 w/v %, DMF, 20 °C) m.p. = 225°C (DSC) | 0.089 g | 41% |
| Starting from Intermediate 98 and 5-bromo-3-fluoropyridin-2-amine | | | |
| Co. **89** | 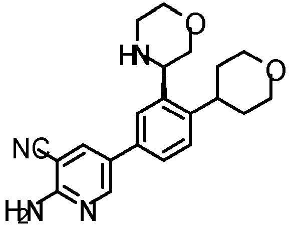 [α]_{d}: - 108.3° (589 nm, c 0.24 w/v %, DMF, 20 °C) m.p. = 258°C (DSC) | 0.086 g | 39% |
| Starting from Intermediate 98 and 2-Amino-5-bromonicotinonitrile | | | |
| Co. **90** | 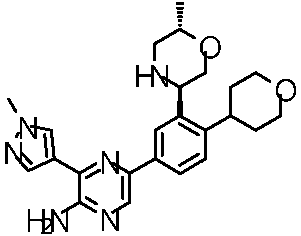 [α]_{d}: - 45.6° (589 nm, c 0.16 w/v %, DMF, 20 °C) m.p. = 246°C (DSC) | 0.195 g | 61% |
| Starting from Intermediate 100a and 5-bromo-3-(1-methyl-1H-pyrazol-4-yl)pyrazin-2-amine | | | |
| Co. **91** | 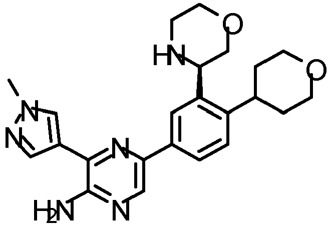 [α]_{d}: - 73.2° (589 nm, c 0.25 w/v %, DMF, 20 °C) m.p. = 242°C (DSC) | 0.76 g | 46% |
| Starting from Intermediate 98 and 5-bromo-3-(1-methyl-1H-pyrazol-4-yl) pyrazin-2-amine | | | |
| **Co.92** | 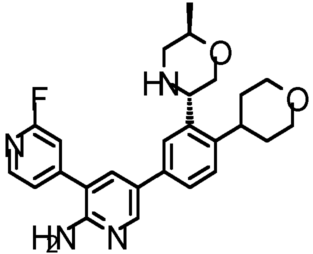 [α]_{d}: + 60.6° (589 nm, c 0.17 w/v %, DMF, 20 °C) m.p. = 209°C (DSC) | 0.052 g | 19% |
| Starting from Intermediate 100b and 17 | | | |
| **Co.93a** | 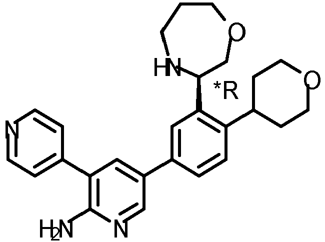 | 0.02 g | 3.5 % |
| Starting from Intermediate 101 and 5-bromo-[3,4'-bipyridin]-2-amine | | | |
| **Co.93b** | 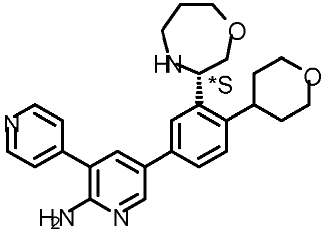 | 0.028 g | 5% |
| Starting from Intermediate 101 and 5-bromo-[3,4'-bipyridin]-2-amine | | | |

### Synthesis of Co.94 :

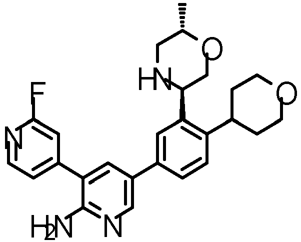

Sodium carbonate (0.402 g, 3.79 mmol) was added to a solution of **Co.79** (0.82 g, 1.90 mmol), 2-Fluoropyridine-4-boronic acid pinacol ester (0.508 g, 2.28 mmol) in water (1.8 mL) and 1,4-dioxane (18 mL). The mixture was degassed with a stream of nitrogen for 5 min. , then PdCl₂(dppf) (0.085 g, 0.104 mmol) was added. The reaction mixture was stirred at 90°C for 6 hours in a sealed tube. The reaction mixture was cooled to rt, poured into water and K₂CO₃. The aqueous layer was extracted with ethyl acetate. The organic layer was dried over MgSO₄, filtered and evaporated until dryness. The residue was purified by preparative LC (40 g SiOH 15 µm Interchim, mobile phase : DCM/MeOH/NH₄OH 100/0/0 to 90/10/0.1) The pure fractions were collected and evaporated until dryness. The compound was taken up in diisopropylether, stirred, filtered and dried yielding **Co.94** (0.735 g, 85%). [α]_{d}: - 82.7° (589 nm, c 0.22 w/v %, DMF, 20 °C).

### Synthesis of Co.95 :

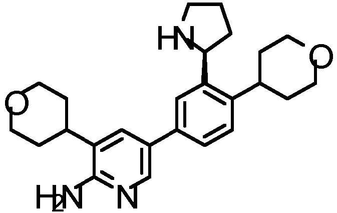

To a solution of intermediate **120** (0.240 g, 0.331 mmol) in DCM (5 mL) was added trifluoroacetic acid (2.54 mL) dropwise under an ice bath. The resulting reaction mixture was stirred at room temperature for 3 hours. The mixture was evaporated in vacuo and the crude mixture was neutralized with NaHCO₃ until basic-pH. The solvents were evaporated in vacuo and the crude was purified by reverse phase: Column: Brand Phenomenex; Type Gemini; I.D. (mm) 100 x 21.2; Particle size 5um (C18). Method: From 81 % of H₂O (25mM NH₄HCO₃)- 19 % (ACN: MeOH 1:1) until 45 % of H₂O (25mM NH₄HCO₃)- 55 % (ACN: MeOH 1:1). The desired fractions were collected and the solvents were evaporated in vacuo. The solid was washed with acetonitrile and evaporated under high vaccum three times at 60°C until a white solid appeared to give pure **Co.95** (0.062g, 45%). [α]_{d}: - 19.3° (589 nm, c 0.13 w/v %, methanol, 23°C). m.p. = 223° C (Mettler Toledo MP50)

The compound listed in the table below was prepared by an analogous reaction protocol:

| Compound | Structure | Quantity | Yield |
|---|---|---|---|
| Co. **96** | 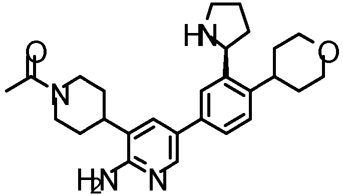 [α]_{d}: - 44.6° (589 nm, c 0.12 w/v %, MeOH, 23 °C) m.p. = 237° C (Mettler Toledo MP50) | 0.115 g | 75% |
| Starting from intermediate 159 | | | |

### Synthesis of Co.97:

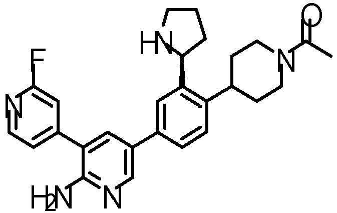

A mixture of **Co.47** (0.324 g, 0.709 mmol), Pd/C 10% (0.3g, 0.282 mmol) in THF (10 mL) was hydrogenated under 10 bars pressure at 55°C overnight. The reaction mixture was filtered over celite, washed with DCM and the filtrate was evaporated. The residue was purified by preparative LC (12 g SiOH 40 µm irregular, mobile phase : DCM/MeOH/NH₄OH 95/5/1 to 90/10/1) The pure fractions were collected and evaporated until dryness. The compound was taken up in diisopropylether, stirred, filtered and dried yielding **Co.97** (0.08 g, 25%). [α]_{d}: - 30.71° (589 nm, c 0.28 w/v %, DMF, 20 °C).

The compounds listed in the table below have been prepared by an analogous reaction protocol:

| Compound | Structure | Quantity | Yield |
|---|---|---|---|
| Co. **98** | 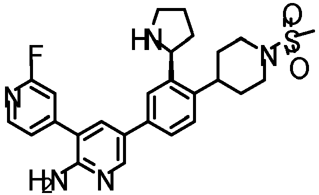 [α]_{d}: - 16.8° (589 nm, c 0.28 w/v %, DMF, 20 °C) | 0.1 g | 35% |
| Starting from Co.46 | | | |
| Co. **99** | 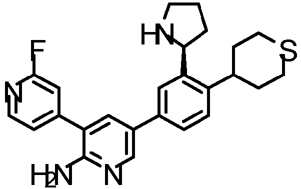 m.p. = 222°C (DSC) | 0.097 g | 27 % |
| Starting from Co.48 | | | |
| **Co. 100** | 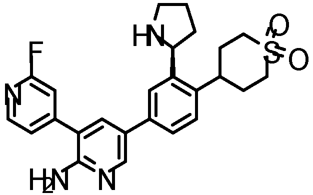 | 0.15 g | 45 % |
| Starting from Co. 49 | | | |
| **Co. 101** | 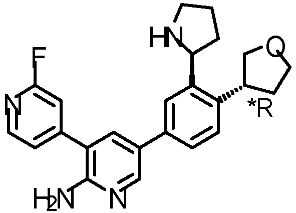 | 0.05 g | 20% |
| Starting from Co. 45 | | | |
| **Co. 102** | 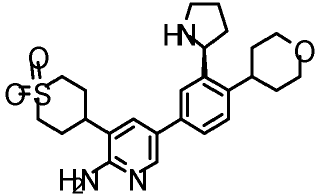 [α]_{d}: - 33.7° (589 nm, c 0.27 w/v %, DMF, 20 °C) | 0.024g | 28% |
| Starting from Co. 55 | | | |

### Synthesis of Co.103 :

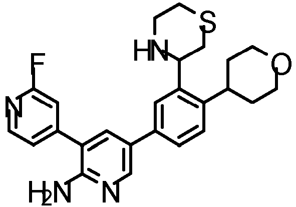

Intermediate **90** (1.45 g, 3.84 mmol) and molecular sieves 4A (0.2 g) were added to SLAP TM (0.78 g, 4.8 mmol) in DCM (16 mL). The reaction mixture was stirred at rt 5 h, filtered over celite^{©}. The filtrate was evaporated till dryness to afford the imine which was added to a solution of [Ir(dtbbpy)(ppy)₂]PF₆ (35 mg, 0.0384 mmol), Copper (II) trifluoromethanesulfonate (1.39 g, 3.85 mmol) and Bismuth(III) trifluoromethane sulfonate (1.26 g, 1.93 mmol) in MeCN (80 mL). The reaction mixture was stirred at rt for 16 h under blue light (34W - Hepatochem photoreactor - irraditated with a Kessil lamp).

The reaction mixture was poured into H₂O and K₂CO₃ and extracted with DCM. The organic layer was dried over MgSO4, filtered and evaporated until dryness. The residue was purified by preparative LC twice (Stationary phase: irregular SiOH 15-40µm 80 and 40g GraceResolv, graduent from DCM/MeOH/NH₄OH 98/2/0 to 95/5/0.1). The fractions were collected and evaporated until dryness yielding **Co.103** (1.09 g, 63%).

The compounds listed in the table below have been prepared by an analogous reaction protocol as was described for preparation of Co. 103:

| Compound | Structure |
|---|---|
| Co. **103a** | 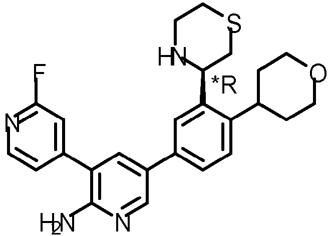 [α]_{d}: - 40.8° (589 nm, c 0.26 w/v %, DMF, 20 °C) |
| Co. **103b** | 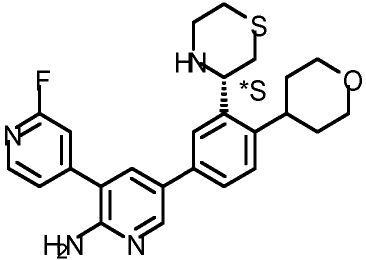 [α]_{d}: +77.7° (589 nm, c 0.26 w/v %, DMF, 20 °C) |

### Synthesis of Co.104 :

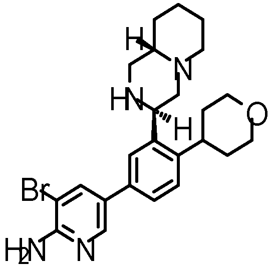

A mixture of intermediate **86** (100 mg, 0.28 mmol), SLAP hydropyridopyrazine (55 mg, 0.28 mmol), molecular sieves (30 mg) in DCM (7 mL) was stirred at rt for 5h. The imine was filtered off over celite, the filtrate was evaporated. Acetonitrile (4 mL), trifluoromethanol (1 mL) and DCM (3 mL) were added and then the photocalyst [Ir(dtbbpy)(ppy)₂]PF₆ (2.5mg). The reaction mixture was stirred at rt for 16h under blue light (34W - Hepatochem photoreactor - irraditated with a Kessil lamp). Water and AcOEt were added, the aqueous phase was extracted, the organic layer was separated, dried over MgSO₄, filtered and evaporated. A first purification was performed via preparative LC (Stationary phase: irregular SiOH 40 µm 4g, Mobile phase: 98/2 to 90/10/1 DCM/MeOH/NH₄OH) followed by a second one via reverse phase (Stationary phase: YMC-actus Triart C18 10µm 30*150mm, Mobile phase: Gradient from 65% NH₄HCO₃ 0.2% , 35% ACN to 25% NH4HCO3 0.2% , 75% ACN) yielding **Co. 104** (15 mg, 11%).

### Synthesis of Co.105 :

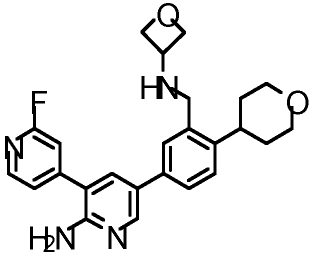

Sodium triacetoxyborohydride (0.168 g, 0.8 mmol) was added portionwise to a mixture of intermediate **90** (0.150 g, 0.4 mmol), 3-Oxetanamine (0.090 g, 1.2 mmol) in DCM (3mL) at room temperature. The reaction mixture was stirred for 15 hours. Water was added and the mixture was basified with potassium carbonate. The organic layer was extracted, dried over MgSO₄, filtered and evaporated until dryness. The residue was purified by preparative LC (12 g of SiOH 15µm Interchim, graduent from 100% DCM to 90% DCM 10% CH₃OH 0.1% NH₄OH). The fractions were collected and evaporated until dryness and the compound was cristallized in diisopropylether, filtered and dried to give of **Co.105** (0.085 g, 49%).

The compounds listed in the table below have been prepared by an analogous reaction protocol:

| Compound | Structure | Quantity | Yield |
|---|---|---|---|
| Co. **106** | 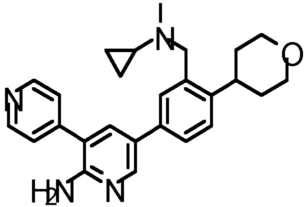 m.p. = 245°C (DSC) | 0.023 g | 13% |
| Starting from intermediate 91 | | | |
| Co. **107** | 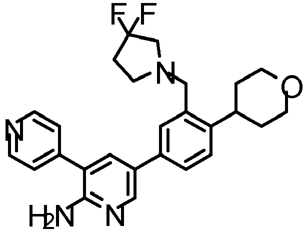 | 0.02 g | 11% |
| Starting from intermediate 91 | | | |
| Co. **108** | 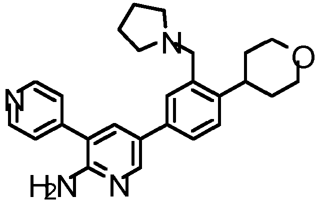 | 0.026 g | 26% |
| Starting from intermediate 91 | | | |

### Synthesis of Co.109 :

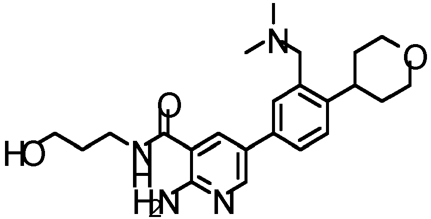

Aminopropanol (0.045 mL, 0.59 mmol) was added to a stirring solution of intermediate **169** (0.15 g, 0.422 mmol), DIPEA (0.147 mL, 0.84 mmol), 1-Ethyl-3-(3-dimethylaminopropyl) carbodiimide hydrochloride (0.121 g, 0.63 mmol), HOBt (0.086 g, 0.63 mmol) in DCM (3.2 mL) and DMSO (1.5 mL). The reaction mixture was stirred at rt overnight.To complete the reaction, 1.5 eq of 1-Ethyl-3-(3-dimethylaminopropyl) carbodiimide hydrochloride, 1.5 eq of HOBt, 1.4 eq of aminopropanol, 2 eq of DIPEA were added. After 14h at rt, the reaction mixture was diluted with water (5 mL) and DCM (20 mL). The layers were separated and the aqueous phase extracted with DCM (3 x 5 mL). The organic layer was dried over MgSO₄, filtered and evaporated until dryness. The residue was purified by flash chromatography (silica, gradient : 0-100% DCM-DCM/MeOH (4/1)). The desired fractions were collected and evaporated yielding Co. **109** (0.092 g, 52%). m.p. = 203°C

The compounds listed in the table below have been prepared by an analogous reaction protocol:

| Compound | Structure | Quantity | Yield |
|---|---|---|---|
| Co. **110** | 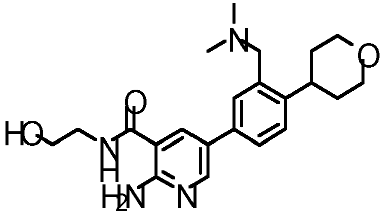 m.p. = 272°C (MP50 Mettler Toledo) | 0.060 g | 35% |
| Starting from intermediate 169 | | | |
| Co. **111** | 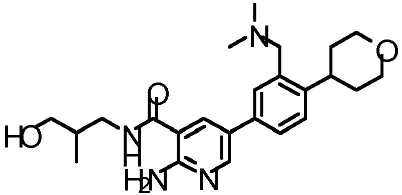 m.p. = 228°C (MP50 Mettler Toledo) | 0.075g | 41% |
| Starting from intermediate 169 | | | |

### Analytical Part

### OPTICAL ROTATION (OR)

Optical rotations were measured at 20°C or 23°C on a Perkin Elmer 341 digital polarimeter at λ = 589 nm (i.e., sodium D line), using a 0.2 mL cell (l = 1 dm), and are given as [α]_{D} (concentration in g/100 mL solvent).

### LCMS (Liquid Chromatography/Mass spectrometry)

### LCMS General procedure

The High Performance Liquid Chromatography (HPLC) measurement was performed using a LC pump, a diode-array (DAD) or a UV detector and a column as specified in the respective methods. If necessary, additional detectors were included (see table of methods below).

Flow from the column was brought to the Mass Spectrometer (MS) which was configured with an atmospheric pressure ion source. It is within the knowledge of the skilled person to set the tune parameters (e.g. scanning range, dwell time...) in order to obtain ions allowing the identification of the compound's nominal monoisotopic molecular weight (MW). Data acquisition was performed with appropriate software.

Compounds are described by their experimental retention times (Rt) and ions. If not specified differently in the table of data, the reported molecular ion corresponds to the [M+H]⁺ (protonated molecule) and/or [M-H]⁻ (deprotonated molecule). In case the compound was not directly ionizable the type of adduct is specified (i.e. [M+NH₄]⁺, [M+HCOO]⁻, etc...). For molecules with multiple isotopic patterns (Br, Cl..), the reported value is the one obtained for the lowest isotope mass. All results were obtained with experimental uncertainties that are commonly associated with the method used.

Hereinafter, "MSD" means Mass Selective Detector, "DAD" Diode Array Detector.

Table: LCMS Method codes (Flow expressed in mL/min; column temperature (T) in °C; Run time in minutes).

| Method code | Instrument | Column | Mobile phase | gradient | Flow Column T | Run time |
|---|---|---|---|---|---|---|
| 1 | Agilent: 1100-DAD and MSD | YMC: Pack ODS-AQ (3µm, 4.6x50mm) | A: HCOOH 0.1% in water, B: CH₃CN | 95% A to 5% A in 4.8min, held for 1min, back to 95% A in 0.2min. | 2.6 | 6 |
| | | | | | 35 | |
| | | | | | 60 | |
| 2 | Agilent 1290 Infinity DAD TOF-LC/MS G6224A | YMC-pack ODS-AQ C18 (50 x 4.6 mm, 3 µm) | A: 0.1% HCOOH in H₂O | ISET 2V1.0 Emulated Agilent Pump G1312A V1.0 From 94.51% A to 5% A in 4.8 min, held for 1.0 min, to 95% A in 0.2 min | 2.6 | 6 |
| | | | B: CH₃CN | | | |
| | | | | | 35 | |
| 3 | Waters: Acquity UPLC^{®}-DAD and Quattro Micro^{™} | Waters: BEH C18 (1.7µm, 2.1x100m m) | A: 95% CH₃COONH ₄ 7mM / 5% CH₃CN, B: CH₃CN | 84.2% A for 0.49min, to 10.5% A in 2.18min, held for 1.94min, back to 84.2% A in 0.73min, held for 0.73min. | 0.343 | 6.2 |
| | | | | | 40 | |
| 4 | Waters: Acquity^{®} H-Class - DAD and SQD2^{™} | Waters: BEH C18 (1.7µm, 2.1x100mm ) | A: 95% CH₃COONH ₄ 7mM / 5% CH₃CN, B: CH₃CN | 84.2% A to 10.5% A in 2.18 min, held for 1.96 min, back to 84.2% A in 0.73 min, held for 0.73 min. | 0.343 | 6.1 |
| | | | | | 40 | |
| 5 | Waters: Acquity^{®} UPLC - DAD and Quattro^{™} | Waters HSS^{®} T3 (1.8µm, 2.1x100mm) | A CH₃COONH₄ 7mM 95%/ CH₃CN 5%, B: CH₃CN | 99% A/1% B for 0.5min, to 15% A in 3.5min, held for2min, back to 99% A/1%B in 0.5min, held for 1.5min. | 0.35 | 8 |
| | | | | | 40 | |
| | | | | | 40 | |

### Melting Points

For a number of compounds, melting points (m.p.) were determined with a DSC 1 STAR^{e} System from Mettler Toledo (method 1) or a Mettler Toledo MP50 (method 2) or a Kofler bench (method 3). Melting points were measured with a temperature gradient of 10°C/minute up to 350 °C. Melting points are given by peak values.

The results of the analytical measurements are shown in table 3.

**Table 3: Retention time (Rt) in min., [M+H]⁺ peak (protonated molecule), LCMS method and m.p. (melting point in °C) (n.d. means not determined).**

| **Co. No.** | **Rₜ** | **[M+H]⁺** | **LC MS Meth od** | **m.p. (°C)** | **m.p. metho d** |
|---|---|---|---|---|---|
| 1 | 2.03 | 419.3 | 3 | 218 | 1 |
| 2 | 2.02 | 420.3 | 3 | >300 | 2 |
| 6 | 1.53 | 430.2 | 1 | 196 | 2 |
| 9 | 1.44 | 415.2 | 1 | 218 | 2 |
| 10 | 1.42 | 415.2 | 1 | n.d. | |
| 11 | 1.79 | 403.3 | 3 | 245 | 2 |
| 12 | 1.59 | 403.3 | 3 | 196 | 2 |
| 13 | 1.20 | 398.1 | 1 | 257 | 2 |
| 14 | 1.46 | 399.1 | 1 | 263 | 2 |
| 15 | 1.60 | 399.1 | 1 | >300 | 2 |
| 16 | 1.35 | 375.1 | 1 | 265 | 2 |
| 17 | 2.10 | 435.2 | 3 | n.d. | |
| 18 | 2.09 | 444.2 | 3 | n.d. | |
| 19 | 2.08 | 468.2 | 3 | n.d. | |
| 20 | 2.34 | 407.2 | 3 | n.d. | |
| 21 | 1.76 | 434.2 | 3 | n.d. | |
| 22 | 2.16 | 432.2 | 3 | 192 | 1 |
| 23 | 1.78 | 390.1 | 3 | 223 | 1 |
| 24 | 1.92 | 431.2 | 3 | 218 | 1 |
| 25 | 1.90 | 401.2 | 3 | 238 | 1 |
| 26 | 1.90 | 402.3 | 3 | 201 | 2 |
| 27 | 1.82 | 437.2 | 1 | >300 | 2 |
| 28 | n.d. | 419.2 | 1 | 230 | 2 |
| 29 | 1.79 | 468.2 | 1 | 228 | 2 |
| 31 | 1.79 | 437.2 | 1 | 225 | 2 |
| 32 | 1.86 | 453.1 | 1 | 222 | 2 |
| 33 | 1.37 | 402.2 | 1 | 240 | 2 |
| 34 | 2.17 | 451.2 | 3 | n.d. | |
| 35 | 1.88 | 453.1 | 1 | 218 | 2 |
| 36 | 1.74 | 425.1 | 1 | 232 | 2 |
| 37 | 2.16 | 404.0 | 3 | 173 | 1 |
| 38 | 2.84 | 417.2 | 3 | n.d. | |
| 39 | 2.07 | 405.3 | 3 | 185 | |
| 40 | 2.13 | 471.6 | 4 | n.d. | |
| 41 | 1.82 | 405.2 | 1 | 217 | 2 |
| 43 | 2.03 | 420 | 1 | 217 | 2 |
| 44 | 1.90 | 349.2 | 3 | 206 | 1 |
| 45 | 2.05 | 403.1 | 3 | 207 | 1 |
| 46 | 2.11 | 494.2 | 3 | 224 | 1 |
| 47 | 1.96 | 458.2 | 3 | 227 | 1 |
| 48 | 2.37 | 433.1 | 3 | 208 | 1 |
| 49 | 1.94 | 465.1 | 3 | n.d. | |
| 50 | 2.49 | 453.2 | 3 | 219 | 1 |
| 51 | 2.34 | 447.3 | 3 | n.d. | |
| 52 | 2.07 | 472.2 [M+ CH₃CN]⁺ | 3 | 222 | 1 |
| 53 | 2.21 | 393.1 | 3 | 219 | 1 |
| 54 | 1.86 | 391 | 3 | n.d. | |
| 55 | 1.67 | 454.5 | 4 | n.d. | |
| 56 | 1.44 | 417.5 | 4 | n.d. | |
| 57 | 1.47 | 417.5 | 4 | n.d. | |
| 58 | 2.57 | 455.5 | 4 | 216 | 1 |
| 59 | 2.55 | 455.5 | 4 | 215 | 1 |
| 60 | 1.85 | 360.1 | 3 | n.d. | |
| 61 | 2.06 | 426.3 | 3 | n.d. | |
| 62 | 2.33 | 418.2 | 3 | 216 | 1 |
| 63 | 2.27 | 469.3 | 3 | 213 | 1 |
| 64 | 1.91 | 402.2 | 3 | n.d. | |
| 65 | 1.78 | 403 | 4 | n.d. | |
| 66 | 2.56 | 417.4 | 5 | >260 | 3 |
| 67 | 1.79 | 473.6 | 4 | 185 | 3 |
| 68 | 1.73 | 404.6 | 4 | n.d. | |
| 69 | 2.02 | 403.6 | 4 | 236 | 3 |
| 70 | 2.19 | 403.1 | 3 | 225 | 3 |
| 71 | 2.45 | 419.1 | 3 | 222 | 3 |
| 72 | 2.04 | 407.5 | 4 | 234 | 3 |
| 73 | 2.04 | 389.1 | 3 | 227 | 1 |
| 74 | 1.81 | 448.6 | 4 | n.d. | |
| 75 | 2.00 | 462.2 | 3 | 170 | 3 |
| 76 | 2.67 | 388 | 3 | 173 | 3 |
| 77 | 2.12 | 390.1 | 3 | n.d. | |
| 78 | 2.32 | 418.1 | 3 | 208 | 1 |
| 79 | 2.45 | 474.9 [M+ CH₃CN]⁺ | 3 | 176 | 1 |
| 81 | 2.05 | 420.1 | 3 | n.d. | |
| 82 | 2.30 | 435.2 | 3 | n.d. | |
| 84 | 1.98 | 421.2 | 3 | 243 | 1 |
| 85 | 2.25 | 374 | 3 | 213 | 1 |
| 86 | 2.05 | 354 | 3 | 266 | 1 |
| 87 | 2.03 | 370 | 3 | 239 | 1 |
| 88 | 2.08 | 358 | 3 | 225 | 1 |
| 89 | 2.10 | 406.0 [M+ CH₃CN]⁺ | 3 | 258 | 1 |
| 90 | 2.11 | 435.2 | 3 | 246 | 1 |
| 92 | 2.46 | 449.2 | 3 | 209 | 1 |
| 94 | 2.46 | 449.2 | 3 | 205 | 1 |
| 95 | 1.91 | 408.4 | 3 | 223 | 2 |
| 96 | 1.37 | 449.4 | 1 | 237 | 2 |
| 98 | 2.11 | 496.4 | 3 | n.d. | |
| 99 | 2.41 | 435.1 | 3 | 222 | 1 |
| 100 | 1.94 | 467.1 | 3 | n.d. | |
| 101 | 1.99 | 405.1 | 3 | n.d. | |
| 102 | 1.80 | 456.2 | 3 | n.d. | |
| 105 | 2.25 | 435.2 | 3 | n.d. | |
| 106 | 2.91 | 415.2 | 3 | 245 | 1 |
| 107 | 2.75-2.79 | 451.2 | 3 | n.d. | |
| 108 | 2.06 | 415.2 | 3 | n.d. | |
| 109 | 1.16 | 413.3 | 2 | 203 | 2 |
| 110 | 1.05 | 399.2 | 2 | 272 | 2 |
| 111 | 1.26 | 427.3 | 2 | 228 | 2 |
| 103a | 2.55 | 451.3 | 3 | n.d. | |
| 103b | 2.55 | 451.3 | 3 | n.d. | |
| 15a | 1.97 | 508.3 [M+ CH₃CN]⁺ | 3 | n.d. | |
| 15b | 1.97 | 467.2 | 3 | n.d. | |
| 15c | 2.02 | 467.2 | 3 | n.d. | |
| 15d | 2.02 | 467.2 | 3 | n.d. | |
| 1b | 1.63 | 419 | 1 | n.d. | |
| 25b | 1.89 | 401.2 | 3 | n.d. | |
| 5a | 2.32 | 445.2 | 3 | n.d. | |
| 5b | 2.32 | 445.1 | 3 | n.d. | |
| 7a | 2.47 | 459.2 | 3 | n.d. | |
| 7b | 2.46 | 459.2 | 3 | n.d. | |
| 80a | 2.05 | 417.2 | 3 | n.d. | |
| 80b | 2.05 | 417.3 | 3 | n.d. | |
| 83a | 2.17 | 431.4 | 3 | n.d. | |
| 83b | 2.20 | 431.3 | 3 | n.d. | |
| 8a | 2.09 | 427.2 | 3 | n.d. | |
| 8b | 2.08 | 427.2 | 3 | n.d. | |
| 93a | 1.88 | 431.6 | 4 | n.d. | |
| 93b | 1.89 | 431.6 | 4 | n.d. | |

### NMR

Some NMR experiments were carried out using a Bruker Avance 500 spectrometer equipped with a Bruker 5mm BBFO probe head with z gradients and operating at 500 MHz for the proton and 125 MHz for carbon. Chemical shifts (d) are reported in parts per million (ppm). J values are expressed in Hz.

Some NMR experiments were carried out using a Bruker Avance III 400 spectrometer at ambient temperature (298.6 K), using internal deuterium lock and equipped with reverse double-resonance (¹H, ¹³C, SEI) probe head with z gradients and operating at 400 MHz for the proton. Chemical shifts (d) are reported in parts per million (ppm). J values are expressed in Hz.

**Table 4: ¹H NMR results**

| **Co. No.** | **¹H NMR result** |
|---|---|
| 1 | ¹H NMR (500 MHz, DMSO-d₆) δ 8.28-8.37 (m, 2H), 7.76 (d, *J*=1.98 Hz, 1H), 7.68 (d, *J*=2.44 Hz, 1H), 7.52 (td, *J*=1.56, 5.11 Hz, 1H), 7.44 (dd, *J*=2.06, 8.01 Hz, 1H), 7.35 (s, 1H), 7.29 (d, *J*=8.09 Hz, 1H), 6.08 (s, 2H), 4.32 (t, *J*=7.78 Hz, 1H), 3.91-4.02 (m, 2H), 3.43-3.54 (m, 2H), 3.33 (br s, 2H), 3.05-3.19 (m, 2H), 2.88 (td, *J*=7.67, 9.54 Hz, 1H), 2.71 (br s, 1H), 2.10-2.20 (m, 1H), 1.66-1.88 (m, 4H), 1.51-1.65 (m, 2H), 1.34-1.49 (m, 1H) |
| 25 | ¹H NMR (400 MHz, DMSO-*d*₆) δ ppm 8.63 - 8.68 (m, 2H), 8.36 (d, *J*=2.3 Hz, 1H), 7.77 (d, *J*=1.8 Hz, 1H), 7.70 (d, *J*=2.3 Hz, 1H), 7.52 - 7.59 (m, 3H), 7.36 (d, *J*=8.2 Hz, 1H), 6.00 (s, 2H), 4.62 (t, *J*=8.2 Hz, 1 H), 3.96 (br d, *J=*11.1 Hz, 2H), 3.45 - 3.55 (m, 2H), 3.20 - 3.30 (m, 1 H), 3.07-3.18 (m, 2H), 2.19 - 2.30 (m, 1H), 1.85 - 2.03 (m, 2H), 1.61 - 1.82 (m, 4H), 1.56 (br d, *J*=12.7 Hz, 1H) |
| 41 | ¹H NMR (500 MHz, DMSO-*d*₆) δ ppm 8.37 (s, 1H), 8.32 (s, 1H), 8.12 (s, 1H), 8.04 (s, 1H), 7.80 (br d, *J*=7.9 Hz, 1H), 7.32 (br d, *J*=8.2 Hz, 1H), 6.12 (br s, 2H), 4.39 (br t, *J*=7.6 Hz, 1H), 3.88 - 4.02 (m, 5H), 3.49 (br t, *J*=11.3 Hz, 2H), 3.09 - 3.21 (m, 2H), 2.89 - 3.02 (m, 1H), 2.11 - 2.23 (m, 1H), 1.68 - 1.96 (m, 4H), 1.54 - 1.68 (m, 2H), 1.43 - 1.54 (m, 1H) |
| 44 | ¹H NMR (500 MHz, DMSO-*d*₆) δ ppm 8.50 (d, *J*=2.5 Hz, 1H), 8.14 (d, *J*=2.5 Hz, 1H), 7.73 (d, *J*=1.9 Hz, 1H), 7.42 (dd, *J*=7.9, 1.9 Hz, 1H), 7.30 (d, *J*=8.2 Hz, 1H), 6.92 (s, 2H), 4.33 (t, *J*=7.7 Hz, 1H), 3.89 - 4.00 (m, 2H), 3.43 - 3.53 (m, 2H), 3.06 - 3.20 (m, 2H), 2.83 - 2.94 (m, 1H), 2.70 (br s, 1H), 2.08 - 2.22 (m, 1H), 1.66 - 1.90 (m, 4H), 1.50 - 1.65 (m, 2H), 1.44 (dq, *J*=11.7, 8.4 Hz, 1H) |
| 73 | ¹H NMR (500 MHz, DMSO-*d*₆) δ ppm 8.65 (d, *J*=5.7 Hz, 2H), 8.32 (d, *J*=2.2 Hz, 1H), 7.66 (d, *J*=1.9 Hz, 1H), 7.57 (d, *J*=5.7 Hz, 2H), 7.52 (dd, *J*=7.9, 1.3 Hz, 1H), 7.47 (s, 1H), 7.33 (d, J=8.2 Hz, 1H), 5.92 (s, 2H), 3.96 (br dd, *J*=11.0*,* 3.5 Hz, 2H), 3.38 - 3.52 (m, 4H), 3.12 - 3.26 (m, 1H), 2.17 (s, 6H), 1.70 (qd, *J*=12.3, 3.8 Hz, 2H), 1.58 - 1.64 (m, 2H) |
| 78 | ¹H NMR (500 MHz, DMSO-*d*₆) δ ppm 8.25 (d, *J*=1.9 Hz, 1H), 7.99 (d, *J*=1.9 Hz, 1H), 7.75 (d, *J*=1.9 Hz, 1H), 7.46 (dd, *J*=8.2, 1.9 Hz, 1H), 7.32 (d, *J*=8.2 Hz, 1H), 6.28 (s, 2H), 4.12 (dd, *J*=9.9, 2.7 Hz, 1H), 3.96 (td, *J*=10.7, 3.8 Hz, 2H), 3.76 (br d, *J*=9.5 Hz, 1 H), 3.65 (dd, *J*=10.9, 2.7 Hz, 1H), 3.46 - 3.56 (m, 3H), 3.25 (d, *J*=10.4 Hz 1H), 3.10 - 3.19 (m, 1H), 2.97 (td, *J*=12, 2.8 Hz, 1H), 2.91 (d, *J*=11 Hz, 1H), 2.77 (br s, 1H), 1.79 (qd, *J*=12.3, 4.3 Hz, 1H), 1.60 - 1.73 (m, 2H), 1.56 (br d, *J*=12.6 Hz, 1H) |
| 84 | ¹H NMR (500 MHz, DMSO-*d*₆) δ ppm 8.37 (s, 1H), 8.32 (s, 1H), 8.14 (d, *J*=1.6 Hz, 1H), 8.04 (s, 1H), 7.85 (dd, *J*=8.0, 1.7 Hz, 1H), 7.35 (d, *J*=8.2 Hz, 1H), 6.15 (s, 2H), 4.14 (br dd, *J*=9.8, 2.2 Hz, 1H), 3.89 - 4.02 (m, 5H), 3.78 (br d, *J*=10.7 Hz, 1H), 3.67 (dd, *J*=10.7, 2.5 Hz, 1H), 3.46 - 3.59 (m, 3H), 3.25-3.35 (m, 1H, partially obscured by solvent peak), 3.15 - 3.23 (m, 1H), 2.88 - 3.02 (m, 2H), 2.72 (br s, 1H), 1.81 (qd, *J*=12.3, 4.3 Hz, 1H), 1.62 - 1.74 (m, 2H), 1.58 (br d, *J*=12.9 Hz, 1H) |
| 94 | ¹H NMR (400 MHz, DMSO-*d*₆) δ ppm 8.28 - 8.34 (m, 2H), 7.78 (d, *J*=1.7 Hz, 1H), 7.69 (d, *J*=2.3 Hz, 1H), 7.52 (br d, *J*=6.4 Hz, 2H), 7.31 - 7.37 (m, 2H), 6.11 (s, 2H), 4.10 (br d, *J*=8.7 Hz, 1H), 3.91 - 4.01 (m, 2H), 3.68 (br dd, *J*=11.0, 2.4 Hz, 1H), 3.56 - 3.64 (m, 1H), 3.51 (br t, *J*=11.1 Hz, 2H), 3.26 - 3.26 (m, 1H, partially obscured by solvent peak), 3.07 - 3.21 (m, 1H), 2.98 (br d, *J*=11.2 Hz, 1H), 2.56 - 2.65 (m, 1H), 1.60 - 1.85 (m, 3H), 1.55 (br d, *J*=12.6 Hz, 1H), 1.09 (d, *J*=6.2 Hz, 3H) |

### PHARMACOLOGICAL PART

### HPK1 kinase assay

A recombinant fusion protein consisting of full-length human Hpk1 with an N-terminal Glutatione S-transferase (GST) tag was produced in a baculovirus/insect cell expression system. GST-Hpk1 protein was purified from cell lysates by glutathione sepharose affinity chromatography. Kinase inhibition was determined using the ADP Glo^{™} Kinase Assay kit (Promega, V9101). Test compounds were prepared by serial dilution in dimethyl sulfoxide (DMSO) and 0.1 µl of each dilution was spotted onto a 384-well white Proxiplate (Perkin Elmer, 6008289). 8 µl of kinase assay buffer (40 mM Tris pH 7.5, 2 mM dithiothreitol (DTT), 0.05% Bovine Serum Albumin, 5 mM MgCl₂) containing Hpk1 protein was transferred to each well and incubated for 30-45 minutes at room temperature. The enzymatic reaction was started by adding 2 µl of kinase assay buffer containing 75 µM ATP. After 120 minutes, the reaction was stopped by adding 3 µl of ADP Glo^{™} reagent (Promega, V9101) and incubating 45 minutes at room temperature. After adding 6 µl Kinase Detection Reagent (Promega, V9101) and incubating for at least 20 minutes at room temperature, the plate was transferred to an EnVision Plate Reader (Perkin Elmer) for luminescence detection and IC₅₀ values were determined.

### SLP76 phosphorylation assay

Hpk1 inhibition in cells was determined by detection of SLP76 phosphorylation at S376 in lysates from HEK293 cells engineered to express Hpk1 and SLP76. Serial dilutions of test compounds were prepared in cell culture medium and 20 µl was transferred into each well of a 96-well plate containing HEK293 cells in 180 µl of culture medium. After incubation for 4 hours at 37°C/5% CO₂, the culture medium was removed, plates were put on ice and cells washed with cold Minimal Essential Medium before lysing in 90 µl of cold Mammalian Protein Extraction Reagent (Thermo Scientific, 78501) for 30 minutes on ice. The cell lysates were transferred to another 96-well plate, centrifuged 10 minutes at 3000 rcf (4°C) and 30 µl transferred to a 96-well High-bind plate (MSD, L15XB-3). After coating overnight at 4°C, the cell lysate was removed, wells were washed with base buffer (TBS, 0.2% Tween-20), and blocking solution (base buffer containing 3% MSD blocker A) was added to each well for 1 hour at room temperature. The blocking solution was removed, the wells were washed with base buffer, and detection antibody (SLP76 pS376) was added in 25 µl base buffer containing 1% MSD blocker A to each well. After a 2h incubation at room temperature, the detection antibody was removed, wells were washed four times with base buffer, 25 µl SULFO-TAG labeled goat anti-rabbit secondary antibody (MSD, R32AB-1) in base buffer containing 1% MSD blocker A was added to each well and left for 1 hour at room temperature. Wells were washed 4 times with base buffer, 150 µl Read Buffer T was added to each well, and the plates were read in a Mesoscale plate reader (MSD). IC₅₀ values were determined by curve-fitting the electrochemiluminescent signals obtained from the lysates of HEK293 cells expressing Hpk1 and SLP76 treated with test compounds at various concentrations minus the signal obtained from HEK293 cells expressing SLP76 only.

Table: Data of the HPK1 kinase assay and the SLP76 phosphorylation assay. Co. No. means Compound Number.

| Co. No. | Hpk1 biochemical (pIC₅₀) | pSLP76 (HEK293) (pIC₅₀) |
|---|---|---|
| 1 | 8.12 | 6.42 |
| 2 | 7.38 | 6.06 |
| 3 | 7.27 | 5.25 |
| 4 | 7.37 | 5.43 |
| 5a | 7.99 | 6.11 |
| 5b | 6.04 | n.d. |
| 7a | 7.92 | 6.87 |
| 7b | 5.71 | 5.14 |
| 8a | 8.2 | 6.46 |
| 8b | 6.87 | 5.16 |
| 9 | 7.37 | 5.67 |
| 10 | 8.18 | 6.55 |
| 11 | 7.45 | 5.3 |
| 12 | 6.95 | 5.13 |
| 13 | 6.73 | 5.05 |
| 14 | 6.08 | <5 |
| 15 | 6.24 | <5 |
| 15a | 5.03 | <5 |
| 15b | 6.7 | <5 |
| 15c | 6.2 | <5 |
| 15d | 5.16 | <5 |
| 17 | 7.8 | 5.8 |
| 18 | 7.75 | 5.81 |
| 19 | 8.37 | 6.01 |
| 20 | 8.5 | 6.77 |
| 21 | 7.73 | 5.16 |
| 22 | 8.56 | 6.47 |
| 23 | 8.99 | 6.52 |
| 24 | 7.88 | 6.05 |
| 25 | 8.01 | 6.37 |
| 25b | 6.02 | <5 |
| 26 | 8.69 | 6.61 |
| 27 | 8.24 | 6.62 |
| 28 | 7.99 | 6.5 |
| 29 | 7.42 | 5.22 |
| 30 | 7.55 | 6.31 |
| 31 | 7.69 | 5.98 |
| 32 | 7.55 | 5.73 |
| 33 | 7.84 | 5.64 |
| 34 | 7.76 | 5.74 |
| 35 | 8.09 | 6.17 |
| 36 | 8.1 | 6.16 |
| 37 | 8.14 | 6.19 |
| 38 | 8.12 | 5.89 |
| 39 | 8.63 | 6.56 |
| 40 | 8.05 | 5.96 |
| 41 | 8.34 | 6.21 |
| 42 | 7.61 | 5.7 |
| 43 | 7.67 | <5 |
| 44 | 8.03 | 6.47 |
| 45 | 7.38 | 5.95 |
| 46 | 7.45 | 5.99 |
| 47 | 7.61 | 5.87 |
| 48 | 7.6 | 5.88 |
| 49 | 7.62 | 5.6 |
| 50 | 7.7 | 5.61 |
| 51 | 7.74 | 6.26 |
| 52 | 7.41 | 6.07 |
| 53 | 7.8 | 6.01 |
| 54 | 6.34 | <5 |
| 55 | 7.02 | <5 |
| 56 | 7.45 | <5 |
| 57 | 7.58 | <5 |
| 58 | 5.06 | <5 |
| 59 | 7.11 | 5.25 |
| 60 | 6.6 | <5 |
| 61 | 7.46 | 5.4 |
| 62 | 7.5 | 5.97 |
| 63 | 7.23 | 5.27 |
| 64 | 7.77 | 6.42 |
| 65 | 7.57 | 5.92 |
| 66 | 7.07 | <5 |
| 67 | 7.33 | 5.78 |
| 68 | 8.2 | 5.85 |
| 69 | 6.5 | n.d. |
| 70 | 6.94 | 5.36 |
| 71 | 7.38 | 5.69 |
| 72 | 7.39 | 5.64 |
| 73 | 7.37 | 5.81 |
| 74 | 7.25 | 5.73 |
| 75 | 7.38 | 5.64 |
| 76 | 7.46 | 5.71 |
| 77 | 7.85 | 5.59 |
| 78 | 8.09 | 6.04 |
| 79 | 7.59 | 6.1 |
| 80a | 7.84 | 6.07 |
| 80b | 5.43 | <5 |
| 81 | 7.81 | 6.09 |
| 82 | 8.06 | 6.9 |
| 83a | 7.35 | 5.87 |
| 83b | 5.24 | n.d. |
| 84 | 7.6 | 6.32 |
| 85 | 7.42 | 5.94 |
| 86 | 6.92 | 5.4 |
| 87 | 7.32 | 5.96 |
| 88 | 6.95 | 5.38 |
| 89 | 7.67 | 6.07 |
| 90 | 7.45 | 5.91 |
| 92 | 5.71 | <5 |
| 93a | 6.79 | 5.34 |
| 93b | 7.73 | 5.91 |
| 94 | 7.89 | 6.24 |
| 95 | 7.36 | 5.65 |
| 96 | 6.84 | 5.1 |
| 98 | 7.58 | 6.14 |
| 99 | 7.67 | 6.19 |
| 100 | 7.71 | 5.73 |
| 101 | 8.43 | 6.16 |
| 102 | 6.53 | <5 |
| 103a | 8.11 | 5.71 |
| 103b | 6.22 | <5 |
| 104 | 7.23 | 5.1 |
| 105 | 6.42 | <5 |
| 106 | 6.75 | 5.13 |
| 107 | 6.08 | n.d. |
| 108 | 6.93 | 5.12 |
| 110 | 7.05 | 5.33 |
| 111 | 6.96 | 5.23 |

## Claims

1. A compound of Formula (I) 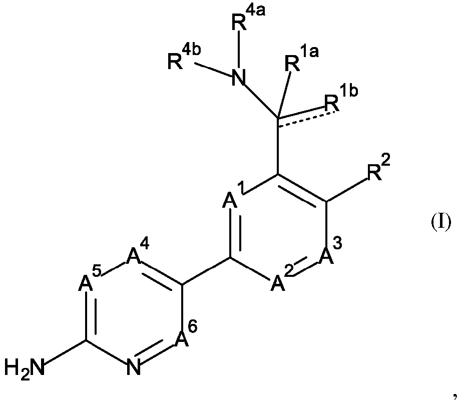
or a tautomer or a stereoisomeric form thereof, wherein
the dotted bond towards R^{1b} is an optional bond that may be present when R^{1b} and R^{4b} are taken together to form a monocyclic or bicyclic aromatic heterocyclyl as defined herein;
A¹ represents CH or N; A² represents CH; A³ represents CH or N;
provided that only one of A¹ and A³ represents N;
A⁴ represents CH or N; A⁵ represents CR^{3a}; A⁶ represents CH;
R^{1a} represents hydrogen;
R^{1b} represents hydrogen or CH₃;
R^{4a} represents hydrogen, C₁₋₄alkyl, or C₃₋₆cycloalkyl;
R^{4b} represents hydrogen, C₁₋₄alkyl, C₃₋₆cycloalkyl, or
a carbon linked monocyclic 4-, 5-, 6- or 7-membered non-aromatic heterocyclyl containing 1, 2 or 3 heteroatoms each independently selected from the group consisting of N, O, and S;
wherein said S-atom might be substituted to form S(=O), S(=O)₂, or S(=O)(=NR^{x});
or
R^{1b} and R^{4b} are taken together to form together with the atoms to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or
a bicyclic 6- to 12- membered aromatic or fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R ⁷, -NR^{6c}R^{6d}, -C(=O)-NR^{6a}R^{6b}, and Het^{c};
wherein said monocyclic or bicyclic, aromatic or fully saturated heterocyclyl might be substituted on the optional additional nitrogen atoms with in total 1 or 2 substituents each independently selected from the group consisting of R⁷, -S(=O)₂-R⁷, -C(=O)-R⁷, -C(=O)-NR^{6a}R^{6b}, and Het^{d};
provided that in case R^{1b} and R^{4b} are taken together, R^{4a} represents hydrogen; and
R^{1a} represents hydrogen or R^{1a} is absent when the dotted bond towards R^{1b} is a bond;
or
R^{4a} and R^{4b} are taken together to form together with the N-atom to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or
a bicyclic 6- to 12- membered aromatic or fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R ⁷, -NR^{6c}R^{6d}, -C(=O)-NR^{6a}R^{6b}, and Het^{c};
wherein said monocyclic or bicyclic, aromatic or fully saturated heterocyclyl might be substituted on the nitrogen atoms with in total 1 or 2 substituents each independently selected from the group consisting of R⁷, -S(=O)₂-R⁷, -C(=O)-R ⁷, -C(=O)-NR^{6a}R^{6b}, and Het^{d};
in case R^{4a} and R^{4b} are taken together, R^{1a} represents hydrogen, and R^{1b} represents hydrogen; R² is selected from the group consisting of cyano; halo; -C(=O)-NR^{8a}R^{8b}; -CH₂-NR^{8c}R^{8d}; Het^{b}; -P(=O)-(C₁₋₄alkyl)₂; -S(=O)₂-C₁₋₄alkyl; -S(=O)(=NR^{x})-C₁₋₄alkyl;
C₁₋₆alkyl optionally substituted with 1 or 2 substituents each independently selected from the group consisting of halo, -OH, cyano, and -O-C₁₋₄alkyl;
C₃₋₆cycloalkyl optionally substituted with 1 or 2 substituents each independently selected from the group consisting of halo, -OH, cyano and -O-C₁₋₄alkyl; and
C₃₋₆cycloalkenyl optionally substituted with 1 or 2 substituents each independently selected from the group consisting of halo, -OH, cyano and -O-C₁₋₄alkyl;
R^{3a} represents hydrogen, halo, R⁷, -O-R⁷, cyano, -C(=0)-NR^{6e}R^{6f}, Het^{a}, or phenyl optionally substituted with 1, 2 or 3 substituents each independently selected from the group consisting of halo, cyano, C₁₋₄alkyl, -O-C₁₋₄alkyl, C₁₋₄alkyl substituted with one cyano, and C₁₋₄alkyl substituted with 1, 2 or 3 halo atoms;
Het^{a} represents a carbon linked monocyclic 5-, 6- or 7-membered aromatic heterocyclyl or carbon linked monocyclic 4-, 5-, 6- or 7-membered non-aromatic heterocyclyl, each containing 1, 2 or 3 heteroatoms each independently selected from the group consisting of N, O or S; wherein said S-atom might be substituted to form S(=O), S(=O)₂, or S(=O)(=NR^{x}); or a carbon linked bicyclic 6- to 12-membered aromatic or non-aromatic heterocyclyl containing containing 1, 2 or 3 heteroatoms each independently selected from the group consisting of N, O or S; wherein said S-atom might be substituted to form S(=O), S(=O)₂, or S(=O)(=NR^{x}); wherein said monocyclic or bicyclic, aromatic or non-aromatic heterocyclyl might be substituted on one or more of the carbon atoms with in total 1, 2 or 3 substituents each independently selected from the group consisting of -OH, CN, halo, R⁷, -O-R⁷, -S(=O)₂-R⁷, -C(=O)-R ⁷, -NR^{6c}R^{6d}, -C(=O)-NR^{6a}R^{6b}, and Het^{c};
wherein said monocyclic or bicyclic, aromatic or non-aromatic heterocyclyl might be substituted on the nitrogen atoms with in total 1 or 2 substituents each independently selected from the group consisting of R⁷, -S(=O)₂-R⁷, -C(=O)-R ⁷, -C(=O)-NR^{6a}R^{6b} and Het^{d};
R^{6a}, R^{6b}, R^{6c}, R^{6d}, R^{6e}, and R^{6f} are each independently selected from the group consisting of hydrogen; C₃₋₆cycloalkyl optionally substituted with one -OR⁵; and
C₁₋₄alkyl optionally substituted with one -OR⁵, wherein two hydrogen atoms on the same carbon atom of said C₁₋₄alkyl might be taken together to form C₃₋₆cycloalkyl;
R⁵ represents hydrogen or C₁₋₄alkyl;
R^{8a}, R^{8c}, and R^{8d} are each independently selected from the group consisting of hydrogen; C₁₋₄alkyl optionally substituted with one -OH or -O-C₁₋₄alkyl; and C₃₋₆cycloalkyl optionally substituted with one -OH or -O-C₁₋₄alkyl;
R^{8b} is selected from the group consisting C₁₋₄alkyl optionally substituted with one -OH or -O-C₁₋₄alkyl; and C₃₋₆cycloalkyl optionally substituted with one -OH or -O-C₁₋₄alkyl;
or
R^{8a} and R^{8b}, or R^{8c} and R^{8d} are taken together to form together with the N-atom to which they are attached a monocyclic fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); wherein said monocyclic 4-, 5-, 6- or 7-membered fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, - C(=O)-R⁷, -NR^{6c}R^{6d}, and
-C(=O)-NR^{6a}R^{6b};
wherein said monocyclic fully saturated heterocyclyl might be substituted on the nitrogen atoms with in total 1 or 2 substituents each independently selected from the group consisting of R⁷, -S(=O)₂-R⁷, -C(=O)-R ⁷, and -C(=O)-NR^{6a}R^{6b};
each Het^{c} independently represents a monocyclic 4-, 5-, 6- or 7-membered fully saturated heterocyclyl containing 1, 2 or 3 heteroatoms each independently selected from the group consisting of N, O or S; wherein said S-atom might be substituted to form S(=O), S(=O)₂, or S(=O)(=NR^{x});
each Het^{d} independently represents a carbon linked monocyclic 4-, 5-, 6- or 7-membered fully saturated heterocyclyl containing 1, 2 or 3 heteroatoms each independently selected from the group consisting of N, O or S; wherein said S-atom might be substituted to form S(=O), S(=O)₂, or S(=O)(=NR^{x});
Het^{b} represents a monocyclic 4-, 5-, 6- or 7-membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x}); or
a bicyclic 6- to 12- membered non-aromatic heterocyclyl containing 1, 2 or 3 heteroatoms each independently selected from the group consisting of N, O, S; wherein said S-atom might be substituted to form S(=O), S(=O)₂, or S(=O)(=NR^{x});
wherein said monocyclic or bicyclic non-aromatic heterocyclyl might be substituted on one or more of the carbon atoms with in total 1, 2 or 3 substituents each independently selected from the group consisting of -OH, CN, halo, R⁷, -O-R⁷, -S(=O)₂-R⁷,
-C(=O)-R⁷, -NR^{6c}R^{6d}, and -C(=O)-NR^{6a}R^{6b};
wherein said monocyclic or bicyclic non-aromatic heterocyclyl might be substituted on the nitrogen atoms with in total 1 or 2 substituents each independently selected from the group consisting of R⁷, -S(=O)₂-R⁷, -C(=O)-R⁷, and -C(=O)-NR^{6a}R^{6b};
each R⁷ independently represents C₃₋₆cycloalkyl optionally substituted with 1, 2 or 3 substituents each independently selected from the group consisting of halo, -OH, -O-C₁₋₄alkyl and cyano; or C₁₋₄alkyl optionally substituted with 1, 2 or 3 substituents each independently selected from the group consisting of halo, -OH, -O-C₁₋₄alkyl and cyano;
each R^{x} independently represents hydrogen or C₁₋₄alkyl;
or a pharmaceutically acceptable addition salt, an N-oxide, or a solvate thereof.

2. The compound according to claim 1, wherein
the dotted bond towards R^{1b} is an optional bond that may be present when R^{1b} and R^{4b} are taken together to form a monocyclic aromatic heterocyclyl as defined herein;
A¹ represents CH or N; A² represents CH; A³ represents CH or N;
provided that only one of A¹ and A³ represents N;
A⁴ represents CH; A⁵ represents CR^{3a}; A⁶ represents CH;
R^{1a} represents hydrogen;
R^{1b} represents hydrogen;
R^{4a} represents hydrogen, or C₁₋₄alkyl;
R^{4b} represents C₁₋₄alkyl, C₃₋₆cycloalkyl, or
a carbon linked monocyclic 4-, 5-, 6- or 7-membered non-aromatic heterocyclyl containing 1, 2 or 3 oxygen atoms;
or
R^{1b} and R^{4b} are taken together to form together with the atoms to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O); or
a bicyclic 6- to 12-membered fully saturated heterocyclyl containing 1 N-atom and optionally 1 or 2 additional heteroatoms each independently selected from the group consisting of N, O, S; wherein said optional S-atom might be substituted to form S(=O);
wherein said monocyclic or bicyclic, aromatic or fully saturated heterocyclyl might be substituted on one or more of the carbon atoms with in total 1, 2 or 3 substituents each independently selected from the group consisting of halo and R⁷;
provided that in case R^{1b} and R^{4b} are taken together, R^{4a} represents hydrogen; and
R^{1a} represents hydrogen or R^{1a} is absent when the dotted bond towards R^{1b} is a bond;
or
R^{4a} and R^{4b} are taken together to form together with the N-atom to which they are attached a monocyclic 4-, 5-, 6- or 7-membered fully saturated heterocyclyl containing 1 N-atom; wherein said monocyclic fully saturated heterocyclyl might be substituted on one or more of the carbon atoms with in total 1, 2 or 3 halo substituents;
in case R^{4a} and R^{4b} are taken together, R^{1a} represents hydrogen, and R^{1b} represents hydrogen; R² is selected from the group consisting of cyano; Het^{b}; and C₃₋₆cycloalkyl optionally substituted with 1 or 2 substituents each independently selected from the group consisting of halo and -O-C₁₋₄alkyl;
R^{3a} represents halo, R⁷, -O-R⁷, cyano, -C(=0)-NR^{6e}R^{6f}, Het^{a}, or phenyl optionally substituted with 1, 2 or 3 substituents each independently selected from the group consisting of halo and cyano;
Het^{a} represents a carbon linked monocyclic 5-, 6- or 7-membered aromatic heterocyclyl or carbon linked monocyclic 4-, 5-, 6- or 7-membered non-aromatic heterocyclyl, each containing 1, 2 or 3 heteroatoms each independently selected from the group consisting of N, O or S; wherein said S-atom might be substituted to form S(=O) or S(=O)₂; or
a carbon linked bicyclic 6- to 12-membered non-aromatic heterocyclyl containing containing 1, 2 or 3 heteroatoms each independently selected from the group consisting of N or O;
wherein said monocyclic or bicyclic, aromatic or non-aromatic heterocyclyl might be substituted on one or more of the carbon atoms with in total 1, 2 or 3 substituents each independently selected from the group consisting of -OH, CN, halo, R⁷, -O-R⁷, -NR^{6c}R^{6d}, and Het^{c};
wherein said monocyclic or bicyclic, aromatic or non-aromatic heterocyclyl might be substituted on the nitrogen atoms with in total 1 or 2 substituents each independently selected from the group consisting of R⁷, -S(=O)₂-R⁷, and -C(=O)-R⁷;
R^{6c}, R^{6d}, R^{6e}, and R^{6f} are each independently selected from the group consisting of hydrogen; and C₁₋₄alkyl optionally substituted with one -OR⁵;
R⁵ represents hydrogen;
each Het^{c} independently represents a monocyclic 4-, 5-, 6- or 7-membered fully saturated heterocyclyl containing 1, 2 or 3 nitrogen atoms;
Het^{b} represents a monocyclic 4-, 5-, 6- or 7-membered non-aromatic heterocyclyl containing 1, 2 or 3 heteroatoms each independently selected from the group consisting of N, O, S; wherein said S-atom might be substituted to form S(=O) or S(=O)₂; or
a bicyclic 6- to 12- membered non-aromatic heterocyclyl containing 1, 2 or 3 heteroatoms each independently selected from the group consisting of N, O, S; wherein said S-atom might be substituted to form S(=O) or S(=O)₂;
wherein said monocyclic or bicyclic non-aromatic heterocyclyl might be substituted on the nitrogen atoms with in total 1 or 2 substituents each independently selected from the group consisting of -S(=O)₂-R⁷,and -C(=O)-R⁷;
each R⁷ independently represents C₁₋₄alkyl optionally substituted with 1, 2 or 3 substituents each independently selected from the group consisting of halo, -OH, and cyano.

3. The compound according to claim 1 or 2, wherein
the dotted bond towards R^{1b} is absent;
A¹ represents CH or N; A² represents CH; A³ represents CH;
A⁴ represents CH; A⁵ represents CR^{3a}; A⁶ represents CH;
R^{1b} and R^{4b} are taken together to form together with the atoms to which they are attached a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O);
wherein said monocyclic fully saturated heterocyclyl might be substituted on one of the carbon atoms with 1 substituent selected from the group consisting of halo and R⁷;
provided that R^{4a} represents hydrogen; and R^{1a} represents hydrogen;
R² represents Het^{b};
R^{3a} represents halo, cyano, or Het^{a};
Het" represents a carbon linked monocyclic 5-, 6- or 7-membered aromatic heterocyclyl containing 1, 2 or 3 heteroatoms each independently selected from the group consisting of N, O or S; wherein said S-atom might be substituted to form S(=O) or S(=O)₂;
wherein said monocyclic aromatic heterocyclyl might be substituted on one of the carbon atoms with a halo substituent;
wherein said monocyclic aromatic heterocyclyl might be substituted on one nitrogen atom with R⁷;
Het^{b} represents a monocyclic 4-, 5-, 6- or 7-membered non-aromatic heterocyclyl containing 1 oxygen atom;
each R⁷ represents C₁₋₄alkyl.

4. The compound according to claim 1, wherein
R^{1b} and R^{4b} are taken together to form together with the atoms to which they are attached a monocyclic 5-membered aromatic heterocyclyl or a monocyclic 4-, 5-, 6- or 7-membered fully saturated heterocyclyl, each containing 1 N-atom and optionally 1 or 2 additional heteroatoms each independently selected from the group consisting of N, O, S; wherein said optional S-atom might be substituted to form S(=O), S(=O)₂, or S(=O)(=NR^{x}); or
a bicyclic 6- to 12- membered aromatic or fully saturated heterocyclyl containing 1 N-atom and optionally 1 or 2 additional heteroatoms each independently selected from the group consisting of N, O, S; wherein said optional S-atom might be substituted to form S(=O), S(=O)₂, or S(=O)(=NR^{x});
wherein said monocyclic or bicyclic, aromatic or fully saturated heterocyclyl might be substituted on one or more of the carbon atoms with in total 1, 2 or 3 substituents each independently selected from the group consisting of -OH, CN, halo, R⁷, -O-R⁷, -S(=O)₂-R⁷, - C(=O)-R⁷, -NR^{6c}R^{6d}, -C(=O)-NR^{6a}R^{6b}, and Het^{c};
wherein said monocyclic or bicyclic, aromatic or fully saturated heterocyclyl might be substituted on the optional additional nitrogen atoms with in total 1 or 2 substituents each independently selected from the group consisting of R⁷, -S(=O)₂-R⁷, -C(=O)-R ⁷, -C(=O)-NR^{6a}R^{6b}, and Het^{d};
provided that R^{4a} represents hydrogen; and
R^{1a} represents hydrogen or R^{1a} is absent when the dotted bond towards R^{1b} is a bond.

5. The compound according to claim 1, wherein
the dotted bond towards R^{1b} is absent;
A¹ represents CH or N; A² represents CH; A³ represents CH;
A⁴ represents CH; A⁵ represents CR^{3a}; A⁶ represents CH;
R^{1b} and R^{4b} are taken together to form together with the atoms to which they are attached 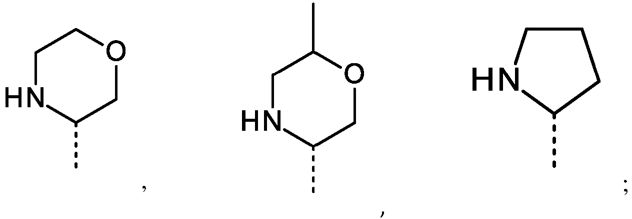
R^{4a} represents hydrogen; and R^{1a} represents hydrogen;
R² represents Het^{b};
R^{3a} represents halo, cyano, or Het^{a};
Het" represents 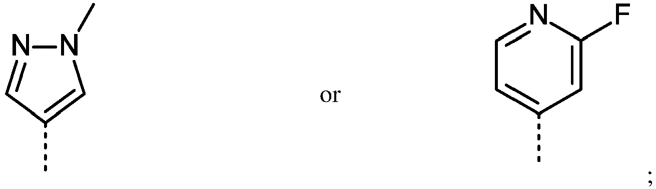
Het^{b} represents tetrahydropyranyl; in particular 4-tetrahydropyranyl.

6. The compound according to claim 1, wherein the compound is selected from the group consisting of 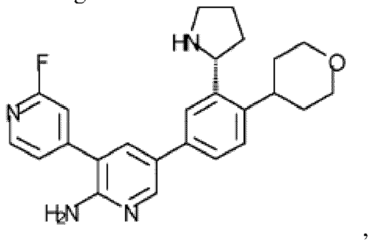 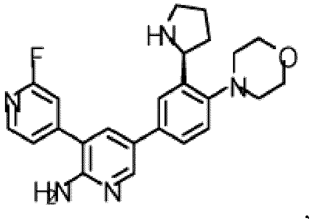 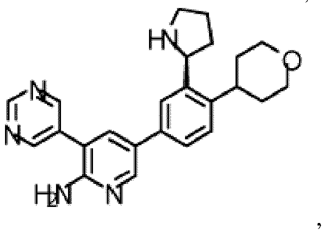 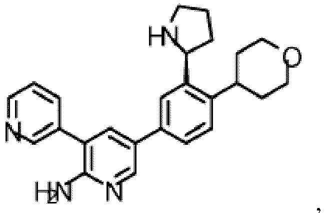 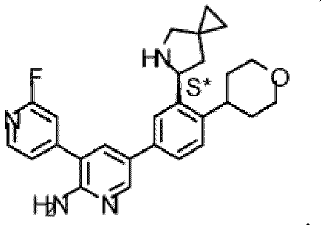 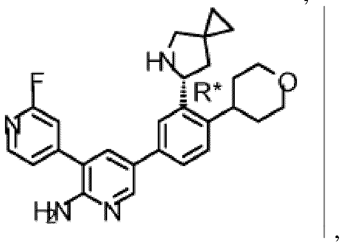 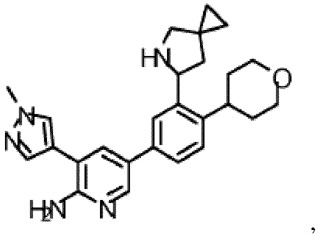 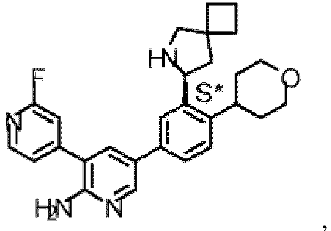 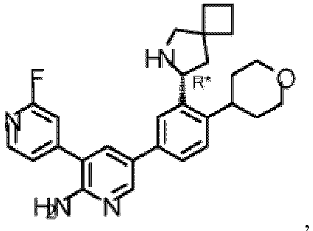 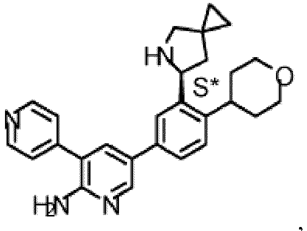 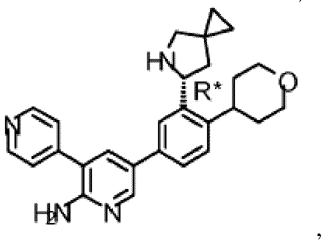 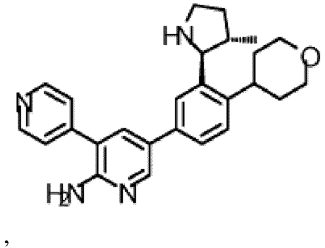 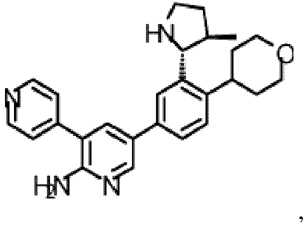 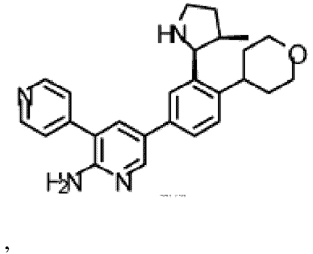 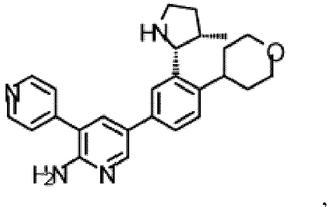 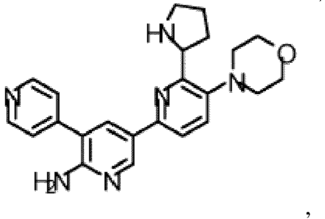 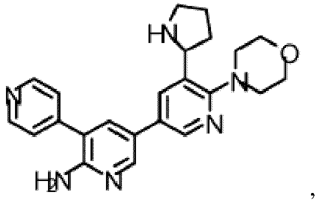 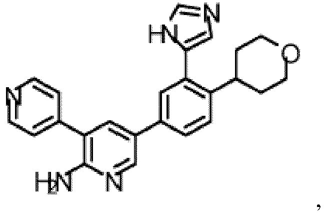 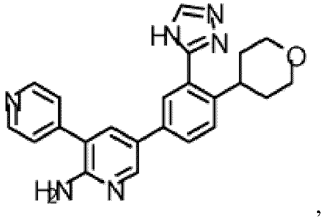 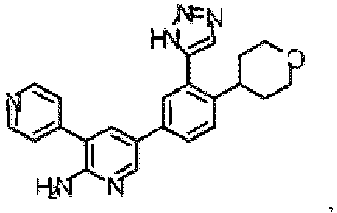 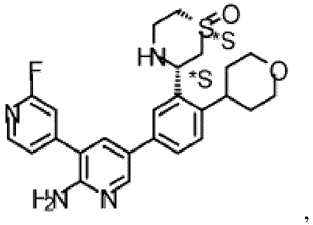 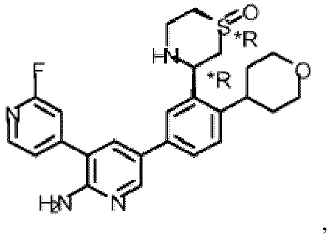 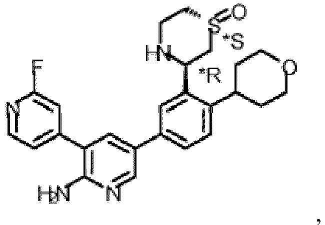 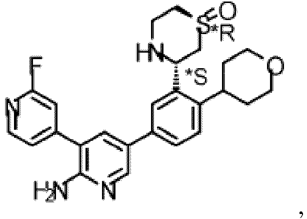 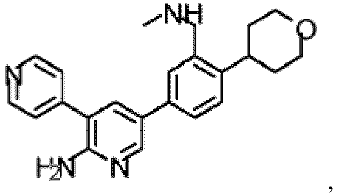 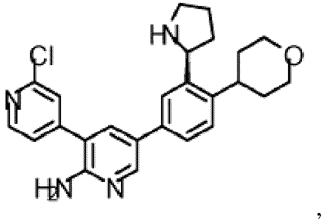 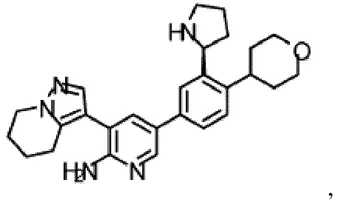 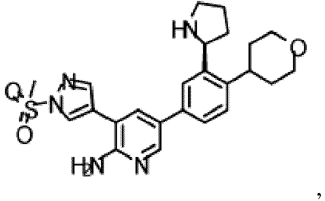 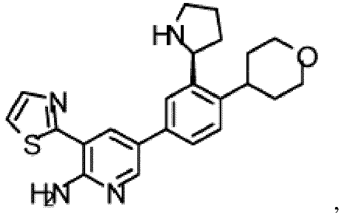 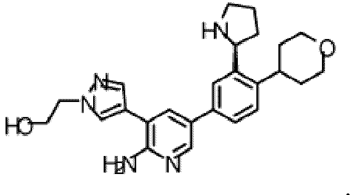 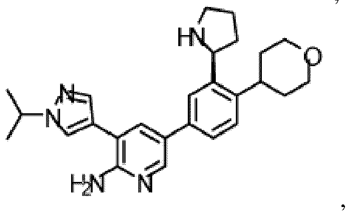 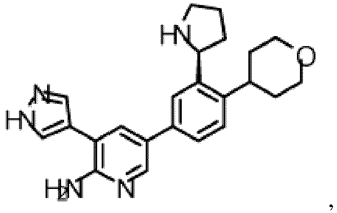 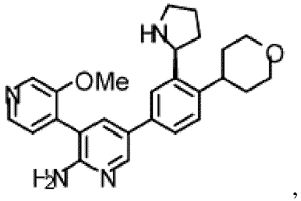 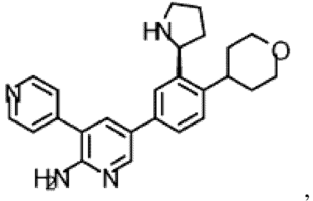 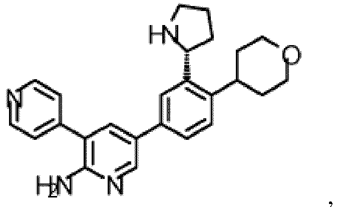 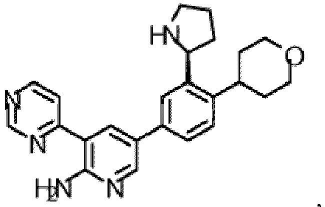 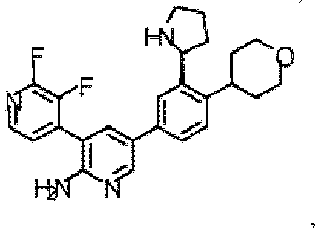 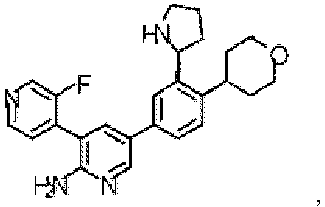 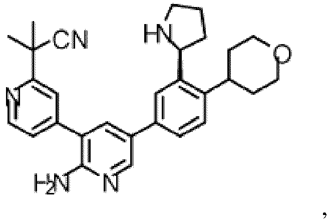 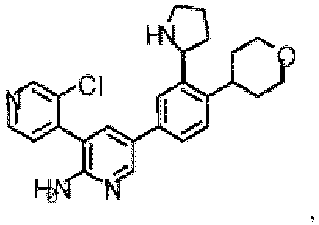 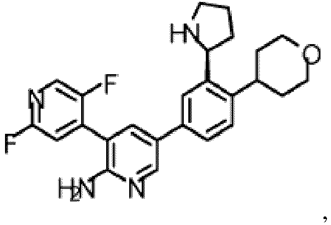 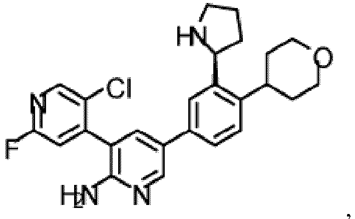 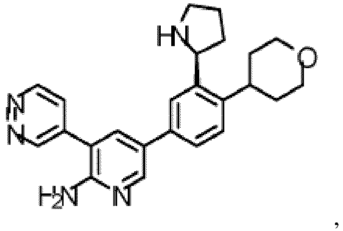 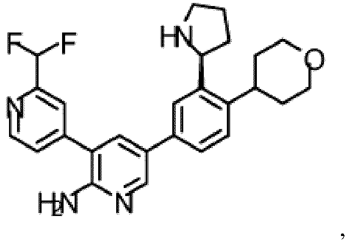 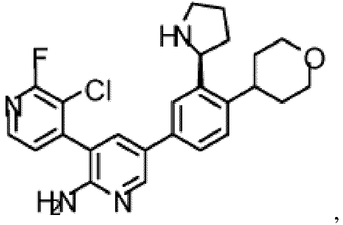 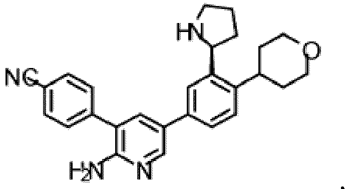 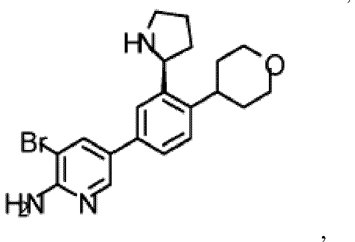 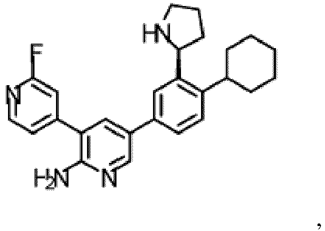 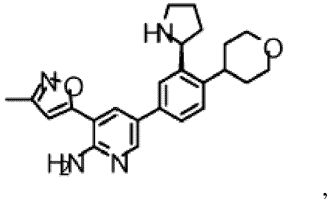 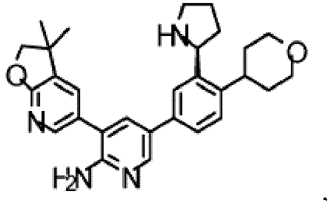 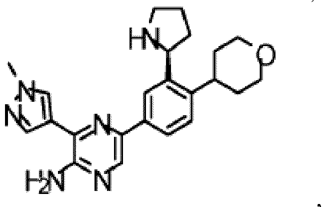 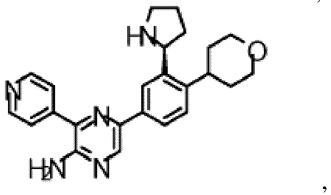 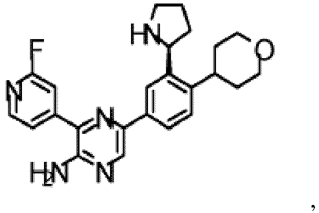 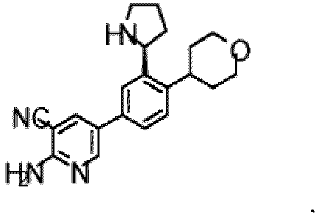 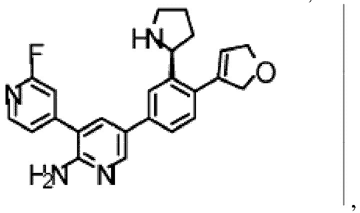 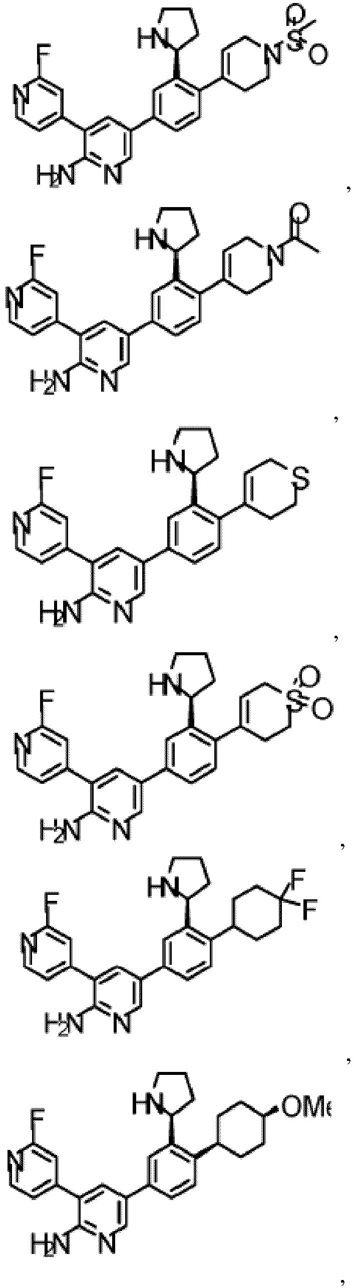 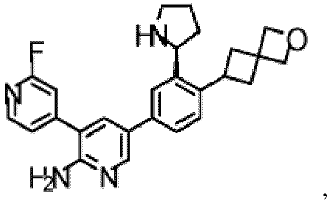 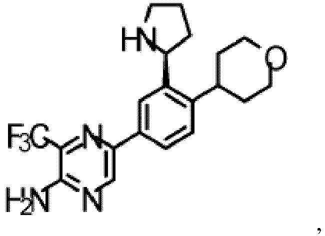 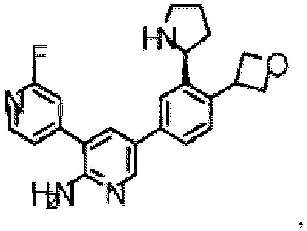 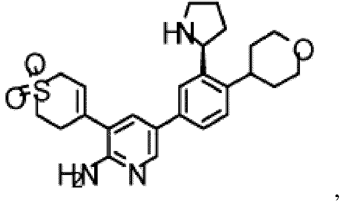 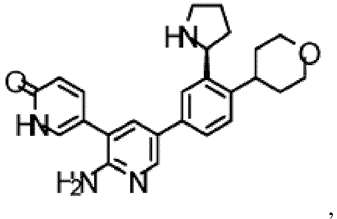 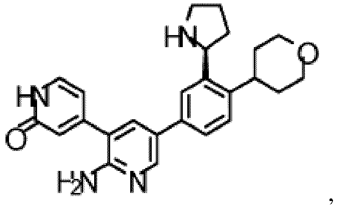 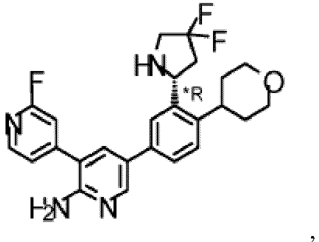 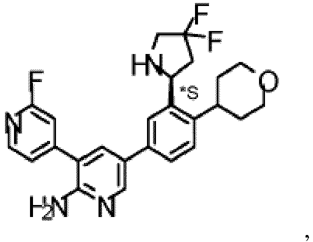 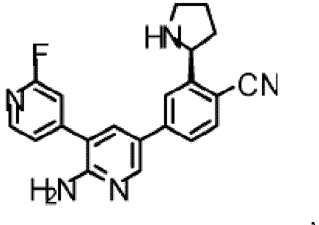 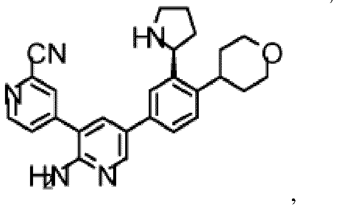 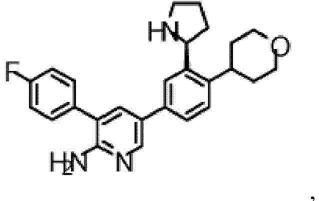 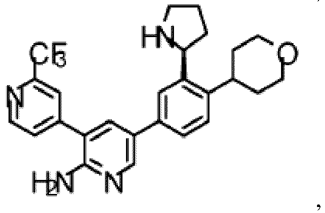 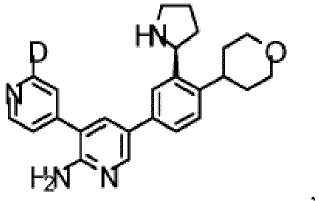 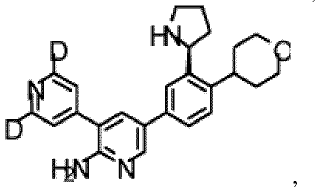 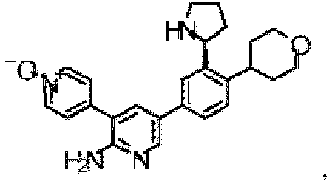 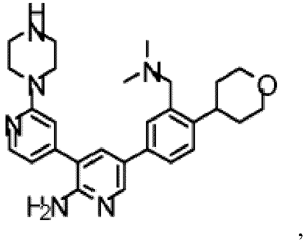 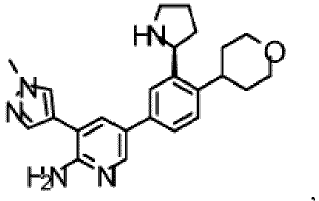 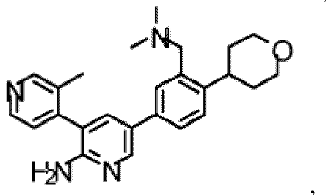 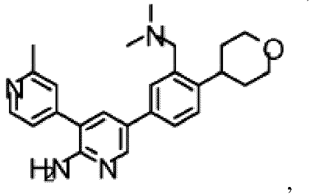 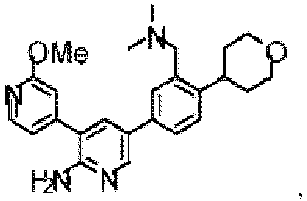 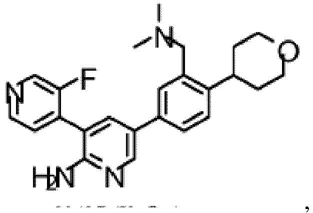 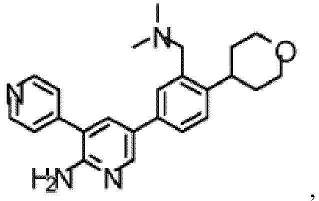 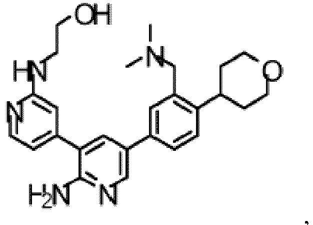 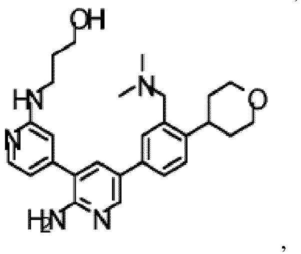 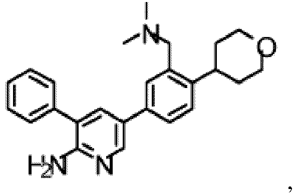 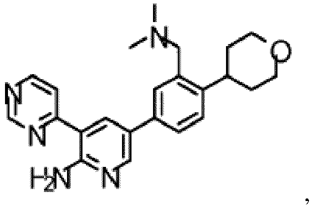 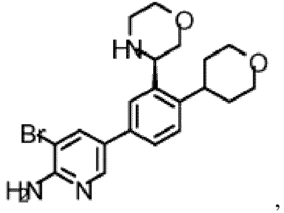 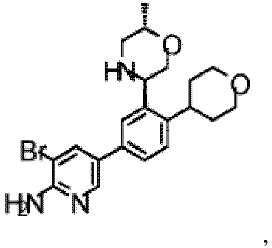 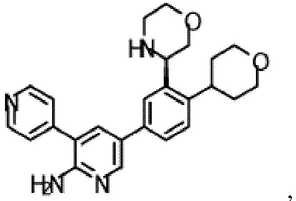 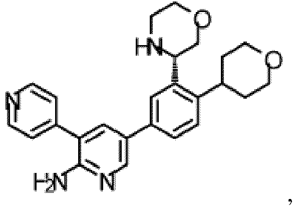 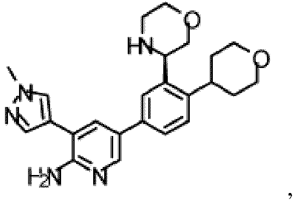 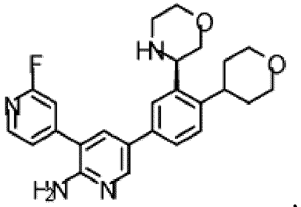 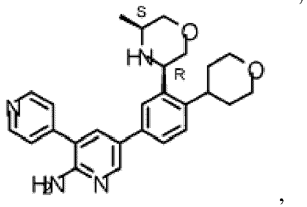 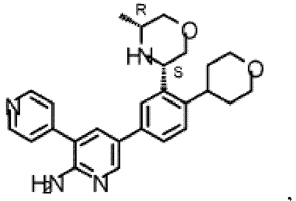 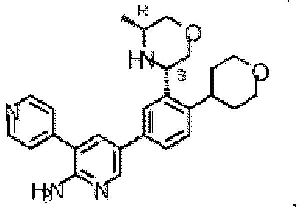 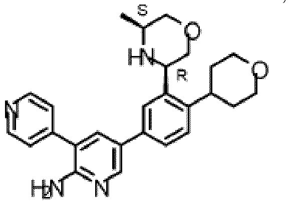 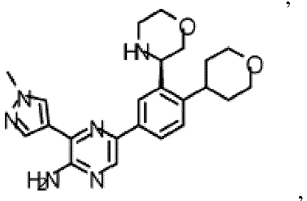 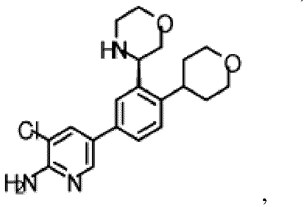 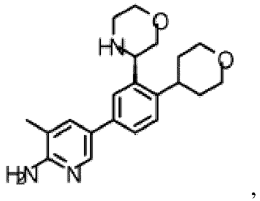 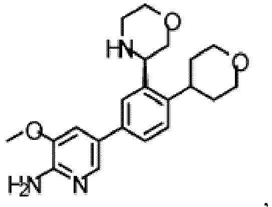 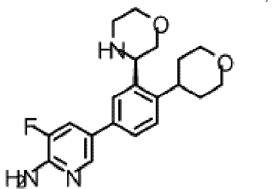 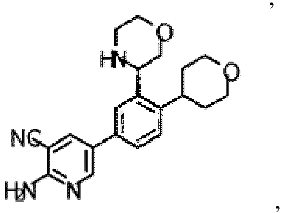 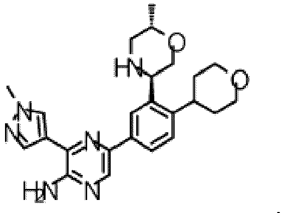 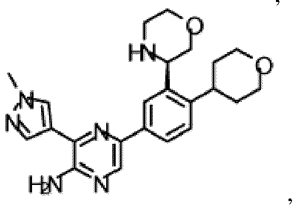 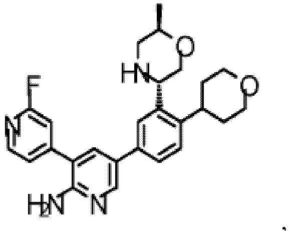 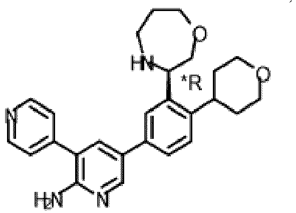 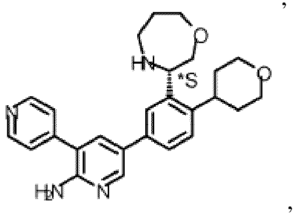 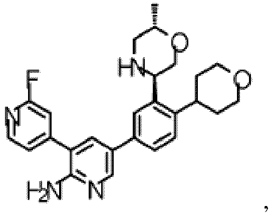 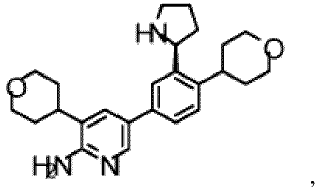 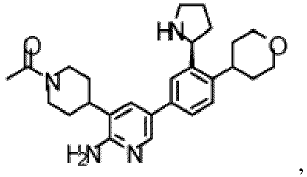 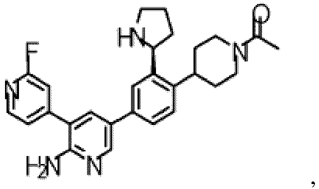 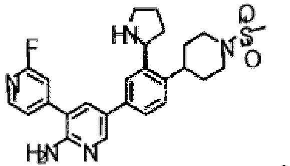 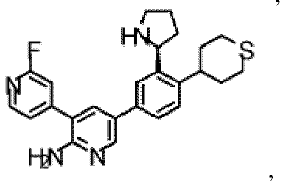 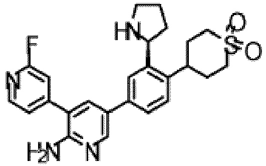 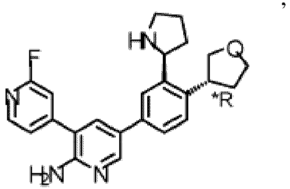 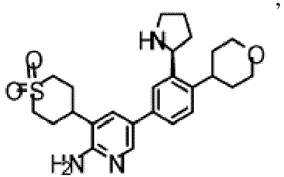 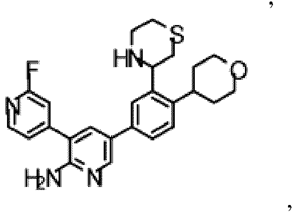 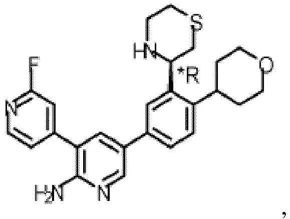 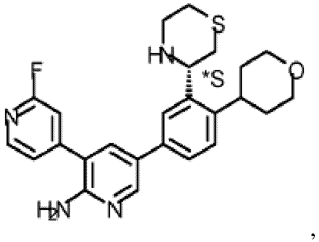 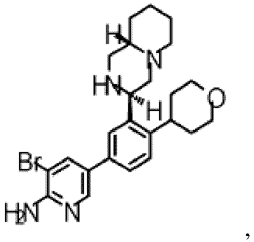 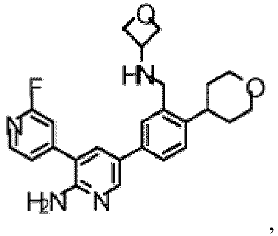 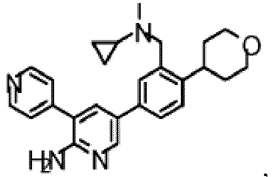 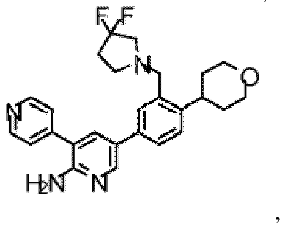 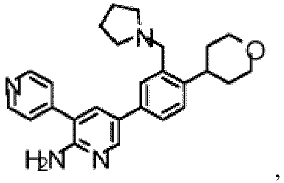 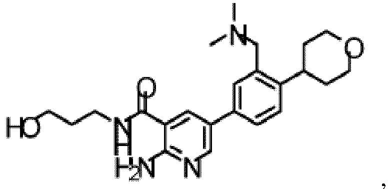 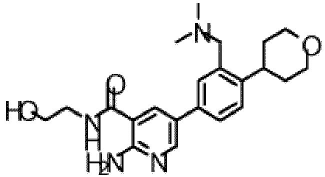 , or 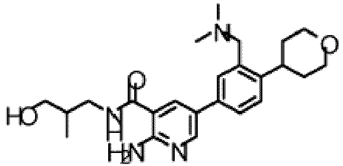
tautomers and stereoisomeric forms thereof,
and pharmaceutically acceptable addition salts, N-oxides, and solvates thereof.

7. The compound according to claim 1, wherein the compound is 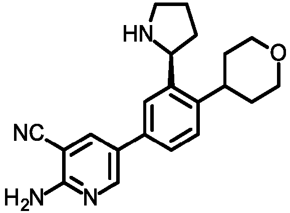 or a pharmaceutically acceptable addition salt, or a solvate thereof.

8. The compound according to claim 1, wherein the compound is

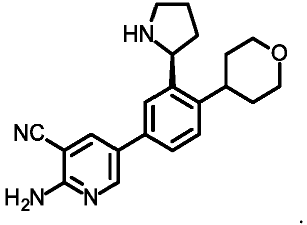

9. The compound according to claim 1, wherein the compound is 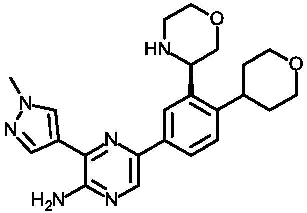 or a pharmaceutically acceptable addition salt, or a solvate thereof.

10. The compound according to claim 1, wherein the compound is

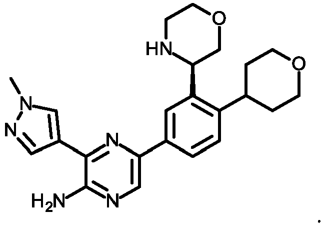

11. The compound according to claim 1, wherein the compound is 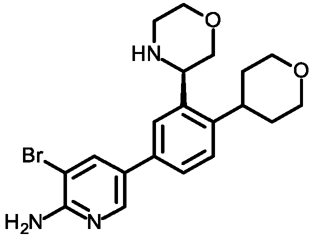 or a pharmaceutically acceptable addition salt, or a solvate thereof.

12. The compound according to claim 1, wherein the compound is

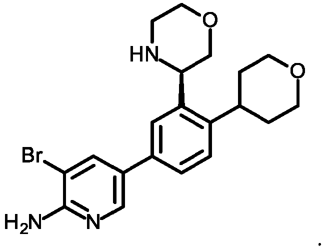

13. The compound according to claim 1, wherein the compound is 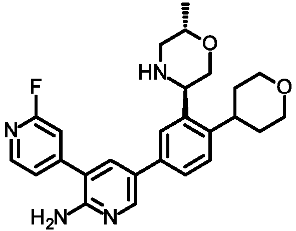 or a pharmaceutically acceptable addition salt, or a solvate thereof.

14. The compound according to claim 1, wherein the compound is

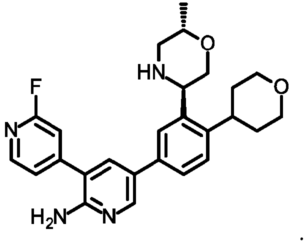

15. A pharmaceutical composition comprising a compound as claimed in any one of claims 1 to 14 and a pharmaceutically acceptable carrier or diluent.

16. A process for preparing a pharmaceutical composition as defined in claim 15 comprising mixing a pharmaceutically acceptable carrier with a therapeutically effective amount of a compound according to any one of claims 1 to 14.

17. A compound as claimed in any one of claims 1 to 14 or a pharmaceutical composition as claimed in claim 15 for use as a medicament.

18. A compound as claimed in any one of claims 1 to 14 or a pharmaceutical composition as claimed in claim 15 for use in the prevention or treatment of cancer and viral infection.

19. The compound or a pharmaceutical composition for use according to claim 18 in the prevention or treatment of cancer wherein cancer is selected from lung cancer, melanoma, head and neck cancer, esophageal cancer, bladder and urothelial cancer, liver cancer, kidney cancer, prostate cancer and hematopoietic cancer.

20. The compound or a pharmaceutical composition for use according to claim 18 in the prevention or treatment of a chronic viral infection.

## Patentansprüche

1. Verbindung gemäß Formel (I) 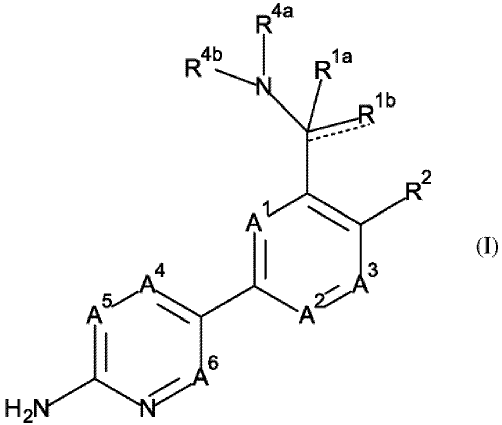
oder ein Tautomer oder eine stereoisomere Form davon, wobei
die gepunktete Bindung zu R^{1b} eine optionale Bindung ist, die vorhanden sein kann, wenn R^{1b} und R^{4b} zusammengenommen werden, um ein monocyclisches oder bicyclisches aromatisches Heterocyclyl auszubilden, wie hierin definiert;
A¹ für CH oder N steht; A² für CH steht; A³ für CH oder N steht;
vorausgesetzt, dass nur eines von A¹ und A³ für N steht;
A⁴ für CH oder N steht; A⁵ für CR^{3a} steht; A⁶ für CH steht;
R^{1a} für Wasserstoff steht;
R^{1b} für Wasserstoff oder CH₃ steht;
R^{4a} für Wasserstoff, C₁₋₄-Alkyl oder C₃₋₆-Cycloalkyl steht;
R^{4b} für Wasserstoff, C₁₋₄-Alkyl oder C₃₋₆-Cycloalkyl steht, oder
ein kohlenstoffverbundenes monocyclisches 4-, 5-, 6- oder 7-gliedriges nicht-aromatisches Heterocyclyl, das 1, 2 oder 3 Heteroatome enthält, die jeweils unabhängig ausgewählt sind aus der Gruppe bestehend aus N, O und S;
wobei das S-Atom substituiert sein kann, um S(=O), S(=O)₂ oder S(=O)(=NR^{×}) auszubilden;
oder
R^{1b} und R^{4b} zusammengenommen werden, um zusammen mit den Atomen, an die sie gebunden sind, ein monocyclisches 5-gliedriges aromatisches Heterocyclyl oder ein monocyclisches 4-, 5-, 6- oder 7-gliedriges vollständig gesättigtes Heterocyclyl auszubilden, die jeweils 1 N-Atom und optional 1 oder 2 zusätzliche Heteroatome enthalten, die jeweils unabhängig ausgewählt sind aus der Gruppe bestehend aus N, O, S; wobei das optionale S-Atom substituiert sein kann, um S(=O), S(=O)₂ oder S(=O)(=NR^{×}) auszubilden; oder ein bicyclisches 6-bis 12-gliedriges aromatisches oder vollständig gesättigtes Heterocyclyl, das 1 N-Atom und optional 1 oder 2 zusätzliche Heteroatome enthält, die jeweils unabhängig ausgewählt sind aus der Gruppe bestehend aus N, O, S; wobei das S-Atom substituiert sein kann, um S(=O), S(=O)₂ oder S(=O)(=NR^{×}) auszubilden;
wobei das monocyclische oder bicyclische, aromatische oder vollständig gesättigte Heterocyclyl an einem oder mehreren der Kohlenstoffatome mit insgesamt 1, 2 oder 3 Substituenten substituiert sein kann, die jeweils unabhängig ausgewählt sind aus der Gruppe bestehend aus -OH, CN, Halogen, R⁷, -O-R⁷, -S(=O)₂-R⁷, -C(=O)-R⁷, -NR^{6c}R^{6d}, -C(=O)-NR^{6a}R^{6b} und Het^{c};
wobei das monocyclische oder bicyclische, aromatische oder vollständig gesättigte Heterocyclyl an den optionalen zusätzlichen Stickstoffatomen mit insgesamt 1 oder 2 Substituenten substituiert sein kann, die jeweils unabhängig ausgewählt sind aus der Gruppe bestehend aus R⁷, -S(=O)₂-R⁷, -C(=O)-R⁷, -C(=O)-NR^{6a}R^{6b} und Het^{d};
vorausgesetzt, dass in einem Fall, dass R^{1b} und R^{4b} zusammengenommen werden, R^{4a} für Wasserstoff steht; und R^{1a} für Wasserstoff steht oder R^{1a} fehlt, wenn die gepunktete Bindung zu R^{1b} eine Bindung ist;
oder
R^{4a} und R^{4b} zusammengenommen werden, um zusammen mit dem N-Atom, an das sie gebunden sind, ein monocyclisches 5-gliedriges aromatisches Heterocyclyl oder ein monocyclisches 4-, 5-, 6- oder 7-gliedriges vollständig gesättigtes Heterocyclyl auszubilden, die jeweils 1 N-Atom und optional 1 oder 2 zusätzliche Heteroatome enthalten, die jeweils unabhängig ausgewählt sind aus der Gruppe bestehend aus N, O, S; wobei das optionale S-Atom substituiert sein kann, um S(=O), S(=O)₂ oder S(=O)(=NR^{×}) auszubilden; oder ein bicyclisches 6- bis 12-gliedriges aromatisches oder vollständig gesättigtes Heterocyclyl, das 1 N-Atom und optional 1 oder 2 zusätzliche Heteroatome enthält, die jeweils unabhängig ausgewählt sind aus der Gruppe bestehend aus N, O, S; wobei das optionale S-Atom substituiert sein kann, um S(=O), S(=O)₂ oder S(=O)(=NR^{×}) auszubilden;
wobei das monocyclische oder bicyclische, aromatische oder vollständig gesättigte Heterocyclyl an einem oder mehreren der Kohlenstoffatome mit insgesamt 1, 2 oder 3 Substituenten substituiert sein kann, die jeweils unabhängig ausgewählt sind aus der Gruppe bestehend aus -OH, CN, Halogen, R⁷, -O-R⁷, -S(=O)₂-R⁷, -C(=O)-R⁷, -NR^{6c}R^{6d}, -C(=O)-NR^{6a}R^{6b} und Het^{c},
wobei das monocyclische oder bicyclische, aromatische oder vollständig gesättigte Heterocyclyl an den Stickstoffatomen mit insgesamt 1 oder 2 Substituenten substituiert sein kann, die jeweils unabhängig ausgewählt sind aus der Gruppe bestehend aus R⁷, -S(=O)₂-R⁷, -C(=O)-R⁷, -C(=O)-NR^{6a}R^{6b} und Het^{d};
in dem Fall, dass R^{4a} und R^{4b} zusammengenommen werden, R^{1a} für Wasserstoff steht und R^{1b} für Wasserstoff steht;
R² aus der Gruppe ausgewählt ist, bestehend aus Cyano; Halogen; -C(=O)-NR^{8a}R^{8b};-CH₂-NR^{8c}NR^{8d}; Het^{b}; -P(=O)-(C₁₋₄-Alkyl)₂; -S(=O)₂-C₁₋₄-Alkyl; -S(=O)(=NR^{×})-C₁₋₄-Alkyl;
C₁₋₆-Alkyl, das optional mit 1 oder 2 Substituenten substituiert ist, die jeweils unabhängig ausgewählt sind aus der Gruppe bestehend aus Halogen, -OH, Cyano und -O-C₁₋₄-Alkyl;
C₃₋₆-Cycloalkyl, das optional mit 1 oder 2 Substituenten substituiert ist, die jeweils unabhängig ausgewählt sind aus der Gruppe bestehend aus Halogen, -OH, Cyano und -O-C₁₋₄-Alkyl; und
C₃₋₆-Cycloalkenyl, dasoptional mit 1 oder 2 Substituenten substituiert ist, die jeweils unabhängig ausgewählt sind aus der Gruppe bestehend aus Halogen, -OH, Cyano und -O-C₁₋₄-Alkyl;
R^{3a} für Wasserstoff, Halogen, R⁷, -OR⁷, Cyano, -C(=0)-NR^{6e}R^{6f}, Het^{a} steht oder für Phenyl steht, das optional mit 1, 2 oder 3 Substituenten substituiert ist, die jeweils unabhängig ausgewählt sind aus der Gruppe bestehend aus Halogen, Cyano, C₁₋₄-Alkyl, -O-C₁₋₄Alkyl, C₁₋₄-Alkyl, das mit einem Cyano substituiert ist, und C₁₋₄-Alkyl, das mit 1, 2 oder 3 Halogenatomen substituiert ist;
Het^{a} für ein kohlenstoffverbundenes monocyclisches 5-, 6- oder 7-gliedriges aromatisches Heterocyclyl oder ein kohlenstoffverbundenes monocyclisches 4-, 5-, 6- oder 7-gliedriges nicht-aromatisches Heterocyclyl steht, wobei jedes 1, 2 oder 3 Heteroatome enthält, die jeweils unabhängig ausgewählt sind aus der Gruppe bestehend aus N, O oder S; wobei das S-Atom substituiert sein kann, um S(=O), S(=O)₂ oder S(=O)(=NR^{×}) auszubilden; oder ein kohlenstoffverbundenes bicyclisches 6- bis 12-gliedriges aromatisches oder nicht-aromatisches Heterocyclyl, das 1, 2 oder 3 Heteroatome enthält, die jeweils unabhängig ausgewählt sind aus der Gruppe bestehend aus N, O oder S; wobei das S-Atom substituiert sein kann, um S(=O), S(=O)₂ oder S(=O)(=NR^{×}) auszubilden; wobei das monocyclische oder bicyclische, aromatische oder nicht-aromatische Heterocyclyl an einem oder mehreren der Kohlenstoffatome mit insgesamt 1, 2 oder 3 Substituenten substituiert sein kann, die jeweils unabhängig ausgewählt sind aus der Gruppe bestehend aus -OH, CN, Halogen, R⁷, -O-R⁷, -S(=O)₂-R⁷, -C(=O)-R⁷, -NR^{6c}R^{6d}, -C(=O)-NR^{6a}R^{6b} und Het^{c};
wobei das monocyclische oder bicyclische, aromatische oder nicht-aromatische Heterocyclyl an den Stickstoffatomen mit insgesamt 1 oder 2 Substituenten substituiert sein kann, die jeweils unabhängig ausgewählt sind aus der Gruppe bestehend aus R⁷, -S(=O)₂-R⁷, -C(=O)-R⁷, -C(=O)-NR^{6a}R^{6b} und Het^{d};
R^{6a}, R^{6b}, R^{6c}, R^{6d}, R^{6e} und R^{6f} jeweils unabhängig ausgewählt sind aus der Gruppe bestehend aus Wasserstoff; C₃₋₆-Cycloalkyl, das optional mit einem -OR⁵ substituiert ist; undC₁₋₄-Alkyl, das optional mit einem -OR⁵ substituiert ist, wobei zwei Wasserstoffatome auf dem gleichen Kohlenstoffatom des C₁₋₄-Alkyls zusammengenommen werden können, um C₃₋₆-Cycloalkyl auszubilden;
R⁵ für Wasserstoff oder C₁₋₄-Alkyl steht;
R^{8a}, R^{8c} und R^{8d} jeweils unabhängig ausgewählt sind aus der Gruppe bestehend aus Wasserstoff; C₁₋₄-Alkyl, das optional mit einem -OH oder -O-C₁₋₄-Alkyl substituiert ist; und C₃₋₆-Cycloalkyl, das optional mit einem -OH oder -O-C₁₋₄-Alkyl substituiert ist;
R^{8b} aus der Gruppe ausgewählt ist bestehend aus C₁₋₄-Alkyl, das optional mit einem -OH oder -O-C₁₋₄-Alkyl substituiert ist; und C₃₋₆-Cycloalkyl, das optional mit einem -OH oder -O-C₁₋₄-Alkyl substituiert ist;
oder
R^{8a} und R^{8b} oder R^{8c} und R^{8d} zusammengenommen werden, um zusammen mit dem N-Atom, an das sie gebunden sind, ein monocyclisches, vollständig gesättigtes Heterocyclyl auszubilden, das 1 N-Atom und optional 1 oder 2 zusätzliche Heteroatome enthält, die jeweils unabhängig ausgewählt sind aus der Gruppe bestehend aus N, O, S; wobei das optionale S-Atom substituiert sein kann, um S(=O), S(=O)₂ oder S(=O)(=NR^{×}) auszubilden; wobei das monocyclische 4-, 5-, 6- oder 7-gliedrige vollständig gesättigte Heterocyclyl an einem oder mehreren der Kohlenstoffatome mit insgesamt 1, 2 oder 3 Substituenten substituiert sein kann, die jeweils unabhängig ausgewählt sind aus der Gruppe bestehend aus -OH, CN, Halogen, R⁷, -O-R⁷, -S(=O)₂-R⁷, - C(=O)-R⁷, -NR^{6c}R^{6d}, und
-C(=O)-NR^{6a}R^{6b};
wobei das monocyclische, vollständig gesättigte Heterocyclyl an den Stickstoffatomen mit insgesamt 1 oder 2 Substituenten substituiert sein kann, die jeweils unabhängig ausgewählt sind aus der Gruppe bestehend aus R⁷, -S(=O)₂-R⁷, -C(=O)-R⁷ und -C(=O)-NR^{6a}R^{6b};
jedes Het^{c} unabhängig für ein monocyclisches 4-, 5-, 6- oder 7-gliedriges vollständig gesättigtes Heterocyclyl steht, das 1, 2 oder 3 Heteroatome enthält, die jeweils unabhängig ausgewählt sind aus der Gruppe bestehend aus N, O oder S; wobei das S-Atom substituiert sein kann, um S(=O), S(=O)₂ oder S(=O)(=NR^{×}) auszubilden;
jedes Het^{d} unabhängig für ein kohlenstoffverbundenes monocyclisches 4-, 5-, 6- oder 7-gliedriges vollständig gesättigtes Heterocyclyl steht, das 1, 2 oder 3 Heteroatome enthält, die jeweils unabhängig ausgewählt sind aus der Gruppe bestehend aus N, O oder S; wobei das S-Atom substituiert sein kann, um S(=O), S(=O)₂ oder S(=O)(=NR^{×}) auszubilden;
Het^{b} für ein monocyclisches 4-, 5-, 6- oder 7-gliedriges nicht-aromatisches Heterocyclyl steht, das 1, 2 oder 3 Heteroatome enthält, die jeweils unabhängig ausgewählt sind aus der Gruppe bestehend aus N, O, S; wobei das S-Atom substituiert sein kann, um S(=O), S(=O)₂ oder S(=O)(=NR^{×}) auszubilden; oder ein bicyclisches 6- bis 12-gliedriges nicht-aromatisches Heterocyclyl, das 1, 2 oder 3 Heteroatome enthält, die jeweils unabhängig ausgewählt sind aus der Gruppe bestehend aus N, O, S; wobei das S-Atom substituiert sein kann, um S(=O), S(=O)₂ oder S(=O)(=NR^{×}) auszubilden;
wobei das monocyclische oder bicyclische nicht-aromatische Heterocyclyl an einem oder mehreren der Kohlenstoffatome mit insgesamt 1, 2 oder 3 Substituenten substituiert sein kann, die jeweils unabhängig ausgewählt sind aus der Gruppe bestehend aus -OH, CN, Halogen, R⁷, -O-R⁷, -S(=O)₂-R⁷, -C(=O)-R⁷, -NR^{6c}R^{6d} und -C(=O)-NR^{6a}R^{6b};
wobei das monocyclische oder bicyclische nicht-aromatische Heterocyclyl an den Stickstoffatomen mit insgesamt 1 oder 2 Substituenten substituiert sein kann, die jeweils unabhängig ausgewählt sind aus der Gruppe bestehend aus R⁷, -S(=O)₂-R⁷, -C(=O)-R⁷ und -C(=O)-NR^{6a}R^{6b};
jedes R⁷ unabhängig für C₃₋₆-Cycloalkyl steht, das optional mit 1, 2 oder 3 Substituenten substituiert ist, die jeweils unabhängig ausgewählt sind aus der Gruppe bestehend aus Halogen, -OH, -O-C₁₋₄-Alkyl und Cyano; oder C₁₋₄-Alkyl, das optional mit 1, 2 oder 3 Substituenten substituiert ist, die jeweils unabhängig ausgewählt sind aus der Gruppe bestehend aus Halogen, -OH, -O-C₁₋₄-Alkyl und Cyano;
jedes R^{x} für Wasserstoff oder C₁₋₄-Alkyl steht;
oder ein pharmazeutisch verträgliches Additionssalz, ein N-Oxid oder ein Solvat davon.

2. Verbindung nach Anspruch 1, wobei
die gepunktete Bindung zu R^{1b} eine optionale Bindung ist, die vorhanden sein kann, wenn R^{1b} und R^{4b} zusammengenommen werden, um ein monocyclisches aromatisches Heterocyclyl auszubilden, wie hierin definiert;
A¹ für CH oder N steht; A² für CH steht; A³ für CH oder N steht;
vorausgesetzt, dass nur eines von A¹ und A³ für N steht;
A⁴ für CH steht; A⁵ für CR^{3a} steht; A⁶ für CH steht;
R^{1a} für Wasserstoff steht;
R^{1b} für Wasserstoff steht;
R^{4a} für Wasserstoff oder C₁₋₄-Alkyl steht;
R^{4b} für C₁₋₄-Alkyl, C₃₋₆-Cycloalkyl oder
ein kohlenstoffverbundenes monocyclisches 4-, 5-, 6- oder 7-gliedriges nicht-aromatisches Heterocyclyl steht, das 1, 2 oder 3 Sauerstoffatome enthält;
oder
R^{1b} und R^{4b} zusammengenommen werden, um zusammen mit den Atomen, an die sie gebunden sind, ein monocyclisches 5-gliedriges aromatisches Heterocyclyl oder ein monocyclisches 4-, 5-, 6- oder 7-gliedriges vollständig gesättigtes Heterocyclyl auszubilden, die jeweils 1 N-Atom und optional 1 oder 2 zusätzliche Heteroatome enthalten, die jeweils unabhängig ausgewählt sind aus der Gruppe bestehend aus N, O, S; wobei das optionale S-Atom substituiert sein kann, um S(=O) auszubilden; oder
ein bicyclisches 6- bis 12-gliedriges vollständig gesättigtes Heterocyclyl, das 1 N-Atom und optional 1 oder 2 zusätzliche Heteroatome enthält, die jeweils unabhängig ausgewählt sind aus der Gruppe bestehend aus N, O, S; wobei das optionale S-Atom substituiert sein kann, um S(=O) auszubilden;
wobei das monocyclische oder bicyclische, aromatische oder vollständig gesättigte Heterocyclyl an einem oder mehreren der Kohlenstoffatome mit insgesamt 1, 2 oder 3 Substituenten substituiert sein kann, die jeweils unabhängig ausgewählt sind aus der Gruppe bestehend aus Halogen und R⁷;
vorausgesetzt, dass in dem Fall, dass R^{1b} und R^{4b} zusammengenommen werden, R^{4a} für Wasserstoff steht; und R^{1a} für Wasserstoff steht oder R^{1a} fehlt, wenn die gepunktete Bindung zu R^{1b} eine Bindung ist;
oder
R^{4a} und R^{4b} zusammengenommen werden, um zusammen mit dem N-Atom, an das sie gebunden sind, ein monocyclisches 4-, 5-, 6- oder 7-gliedriges, vollständig gesättigtes Heterocyclyl ausbilden, das 1 N-Atom enthält; wobei das monocyclische, vollständig gesättigte Heterocyclyl an einem oder mehreren der Kohlenstoffatome mit insgesamt 1, 2 oder 3 Halogensubstituenten substituiert sein kann;
in dem Fall, dass R^{4a} und R^{4b} zusammengenommen werden, R^{1a} für Wasserstoff steht und R^{1b} für Wasserstoff steht; R² aus der Gruppe ausgewählt ist, bestehend aus Cyano; Het^{b}; und C₃₋₆-Cycloalkyl optional mit 1 oder 2 Substituenten substituiert ist, die jeweils unabhängig ausgewählt sind aus der Gruppe bestehend aus Halogen und -O-C₁₋₄-Alkyl;
R^{3a} für Halogen, R⁷, -O-R⁷, Cyano, -C(=0)-NR^{6e}R^{6f}, Het^{a} steht oder für Phenyl steht, das optional mit 1, 2 oder 3 Substituenten substituiert ist, die jeweils unabhängig ausgewählt sind aus der Gruppe bestehend aus Halogen und Cyano;
Het^{a} für ein kohlenstoffverbundenes monocyclisches 5-, 6- oder 7-gliedriges aromatisches Heterocyclyl oder ein kohlenstoffverbundenes monocyclisches 4-, 5-, 6- oder 7-gliedriges nicht-aromatisches Heterocyclyl steht, wobei jedes 1, 2 oder 3 Heteroatome enthält, die jeweils unabhängig ausgewählt sind aus der Gruppe bestehend aus N, O oder S; wobei das S-Atom substituiert sein kann, um S(=O) oder S(=O)₂ auszubilden; oder
ein kohlenstoffverbundenes bicyclisches 6- bis 12-gliedriges nicht-aromatisches Heterocyclyl, das 1, 2 oder 3 Heteroatome enthält enthält, die jeweils unabhängig ausgewählt sind aus der Gruppe bestehend aus N oder O;
wobei das monocyclische oder bicyclische, aromatische oder nicht-aromatische Heterocyclyl an einem oder mehreren der Kohlenstoffatome mit insgesamt 1, 2 oder 3 Substituenten substituiert sein kann, die jeweils unabhängig ausgewählt sind aus der Gruppe bestehend aus -OH, CN, Halogen, R⁷, -O-R⁷, -NR^{6c}R^{6d} und Het^{c};
wobei das monocyclische oder bicyclische, aromatische oder nicht-aromatische Heterocyclyl an den Stickstoffatomen mit insgesamt 1 oder 2 Substituenten substituiert sein kann, die jeweils unabhängig ausgewählt sind aus der Gruppe bestehend aus R⁷, -S(=O)₂-R⁷ und -C(=O)-R⁷;
R⁶°, R^{6d}, R^{6e} und R^{6f} jeweils aus der Gruppe unabhängig ausgewählt sind bestehend aus Wasserstoff; und C₁₋₄-Alkyl, das optional mit einem -OR⁵ substituiert ist;
R⁵ für Wasserstoff steht;
jedes Het^{c} für ein monocyclisches 4-, 5-, 6- oder 7-gliedriges vollständig gesättigtes Heterocyclyl unabhängig steht, das 1, 2 oder 3 Stickstoffatome enthält;
Het^{b} für ein monocyclisches 4-, 5-, 6- oder 7-gliedriges nicht-aromatisches Heterocyclyl steht, das 1, 2 oder 3 Heteroatome enthält, die jeweils unabhängig ausgewählt sind aus der Gruppe bestehend aus N, O, S;
wobei das S-Atom substituiert sein kann, um S(=O) oder S(=O)₂ auszubilden; oder
ein bicyclisches 6- bis 12-gliedriges nicht-aromatisches Heterocyclyl, das 1, 2 oder 3 Heteroatome enthält, die jeweils unabhängig ausgewählt sind aus der Gruppe bestehend aus N, O, S; wobei das S-Atom substituiert sein kann, um S(=O) oder S(=O)₂ auszubilden;
wobei das monocyclische oder bicyclische nicht-aromatische Heterocyclyl an den Stickstoffatomen mit insgesamt 1 oder 2 Substituenten substituiert sein kann, die jeweils unabhängig ausgewählt sind aus der Gruppe bestehend aus -S(=O)₂-R⁷ und -C(=O)-R⁷;
jedes R⁷ unabhängig für C₁₋₄-Alkyl steht, das optional mit 1, 2 oder 3 Substituenten substituiert ist, die jeweils unabhängig ausgewählt sind aus der Gruppe bestehend aus Halogen, -OH und Cyano.

3. Verbindung nach Anspruch 1 oder 2, wobei die gepunktete Bindung zu R^{1b} fehlt;
A¹ für CH oder N steht; A² für CH steht; A³ für CH steht;
A⁴ für CH steht; A⁵ für CR^{3a} steht; A⁶ für CH steht;
R^{1b} und R^{4b} zusammengenommen werden, um zusammen mit den Atomen, an die sie gebunden sind, ein monocyclisches 4-, 5-, 6- oder 7-gliedriges vollständig gesättigtes Heterocyclyl auszubilden, das 1 N-Atom und optional 1 oder 2 zusätzliche Heteroatome enthält, die jeweils unabhängig ausgewählt sind aus der Gruppe bestehend aus N, O, S; wobei das optionale S-Atom substituiert sein kann, um S(=O) auszubilden; wobei das monocyclische, vollständig gesättigte Heterocyclyl an einem der Kohlenstoffatome mit 1 Substituenten substituiert sein kann, der aus der Gruppe ausgewählt wird, bestehend aus Halogen und R⁷;
vorausgesetzt, dass R^{4a} für Wasserstoff steht; und R^{1a} für Wasserstoff steht;
R² für Het^{b} steht;
R^{3a} für Halogen, Cyano oder Het^{a} steht;
Het^{a} für ein kohlenstoffverbundenes monocyclisches 5-, 6- oder 7-gliedriges aromatisches Heterocyclyl steht, das 1, 2 oder 3 Heteroatome enthält, die jeweils unabhängig ausgewählt sind aus der Gruppe bestehend aus N, O oder S; wobei das S-Atom substituiert sein kann, um S(=O) oder S(=O)₂ auszubilden;
wobei das monocyclische aromatische Heterocyclyl an einem der Kohlenstoffatome mit einem Halogensubstituenten substituiert sein kann;
wobei das monocyclische aromatische Heterocyclyl an einem Stickstoffatom durch R⁷ substituiert sein kann;
Het^{b} für ein monocyclisches 4-, 5-, 6- oder 7-gliedriges nicht-aromatisches Heterocyclyl steht, das 1 Sauerstoffatom enthält;
jedes R⁷ für C₁₋₄-Alkyl steht.

4. Verbindung nach Anspruch 1, wobei
R^{1b} und R^{4b} zusammengenommen werden, um zusammen mit den Atomen, an die sie gebunden sind, ein monocyclisches 5-gliedriges aromatisches Heterocyclyl oder ein monocyclisches 4-, 5-, 6- oder 7-gliedriges vollständig gesättigtes Heterocyclyl auszubilden, die jeweils 1 N-Atom und optional 1 oder 2 zusätzliche Heteroatome enthalten, die jeweils unabhängig ausgewählt sind aus der Gruppe bestehend aus N, O, S; wobei das optionale S-Atom substituiert sein kann, um S(=O), S(=O)₂ oder S(=O)(=NR^{×}) auszubilden; oder
ein bicyclisches 6- bis 12-gliedriges aromatisches oder vollständig gesättigtes Heterocyclyl, das 1 N-Atom und optional 1 oder 2 zusätzliche Heteroatome enthält, die jeweils unabhängig ausgewählt sind aus der Gruppe bestehend aus N, O, S; wobei das optionale S-Atom substituiert sein kann, um S(=O), S(=O)₂ oder S(=O)(=NR^{×}) auszubilden;
wobei das monocyclische oder bicyclische, aromatische oder vollständig gesättigte Heterocyclyl an einem oder mehreren der Kohlenstoffatome mit insgesamt 1, 2 oder 3 Substituenten substituiert sein kann, die jeweils unabhängig ausgewählt sind aus der Gruppe bestehend aus -OH, CN, Halogen, R⁷, -O-R⁷, -S(=O)₂-R⁷, -C(=O)-R⁷, -NR^{6c}R^{6d}, -C(=O)-NR^{6a}R^{6b} und Het^{c},
wobei das monocyclische oder bicyclische, aromatische oder vollständig gesättigte Heterocyclyl an den optionalen zusätzlichen Stickstoffatomen mit insgesamt 1 oder 2 Substituenten substituiert sein kann, die jeweils unabhängig ausgewählt sind aus der Gruppe bestehend aus R⁷, -S(=O)₂-R⁷, -C(=O)-R⁷, -C(=O)-NR^{6a}R^{6b} und Het^{d};
vorausgesetzt, dass R^{4a} für Wasserstoff steht; und
R^{1a} für Wasserstoff steht oder R^{1a} fehlt, wenn die gepunktete Bindung zu R^{1b} eine Bindung ist.

5. Verbindung nach Anspruch 1, wobei
die gepunktete Bindung zu R^{1b} fehlt;
A¹ für CH oder N steht; A² für CH steht; A³ für CH steht;
A⁴ für CH steht; A⁵ für CR^{3a} steht; A⁶ für CH steht;
R^{1b} und R^{4b} zusammengenommen werden, um zusammen mit den Atomen, an die sie gebunden sind, auszubilden 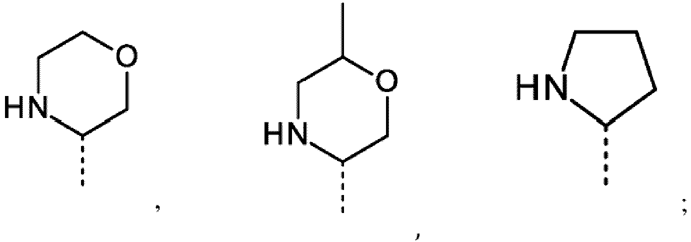
R^{4a} für Wasserstoff steht; und R^{1a} für Wasserstoff steht;
R² für Het^{b} steht;
R^{3a} für Halogen, Cyano oder Het^{a} steht;
Het^{a} für 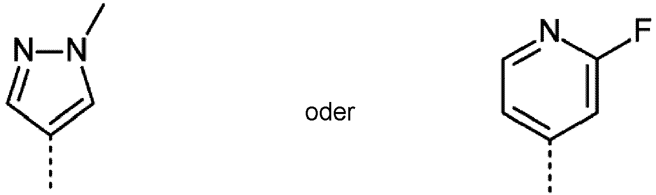 steht;
Het^{b} für Tetrahydropyranyl steht; insbesondere 4-Tetrahydropyranyl.

6. Verbindung nach Anspruch 1, wobei die Verbindung aus der Gruppe ausgewählt ist, bestehend aus 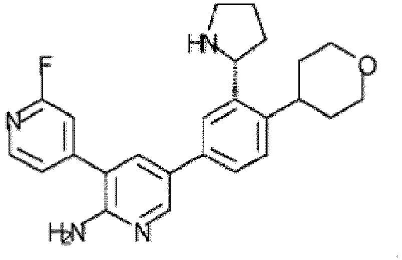 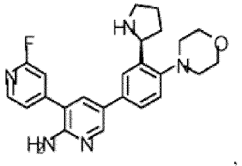 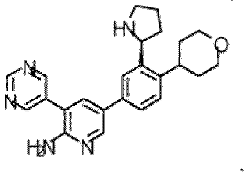 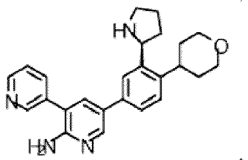 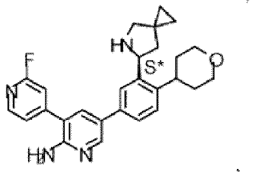 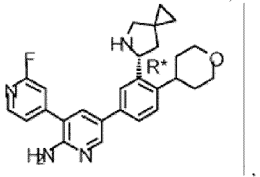 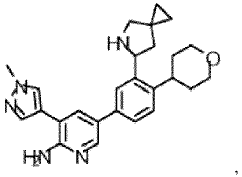 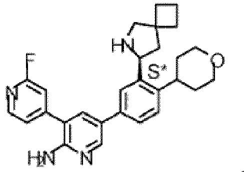 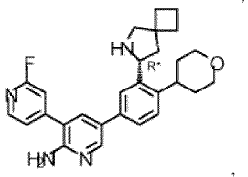 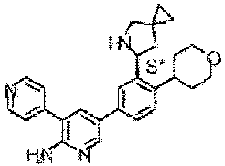 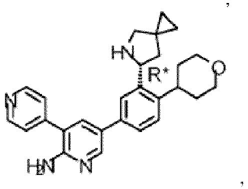 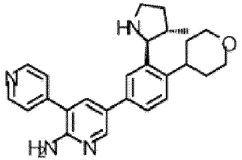 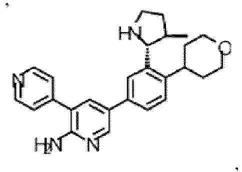 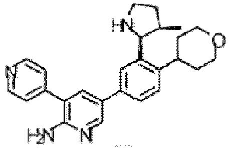 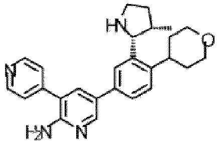 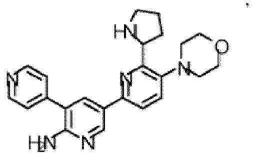 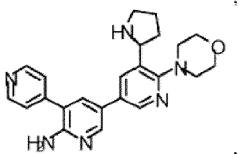 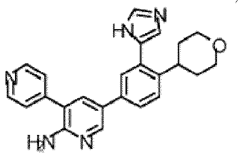 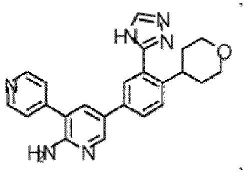 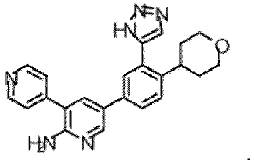 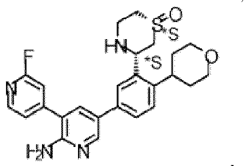 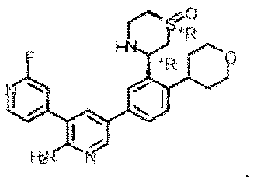 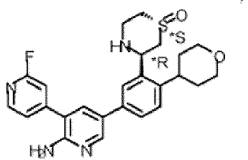 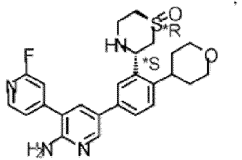 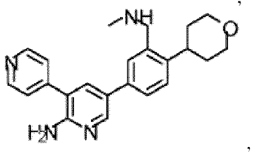 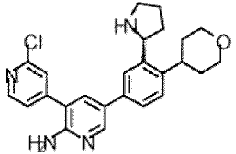 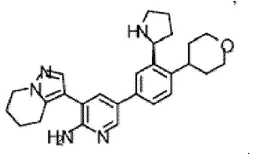 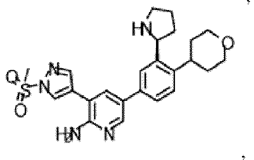 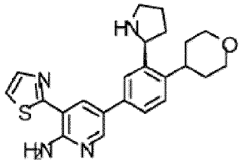 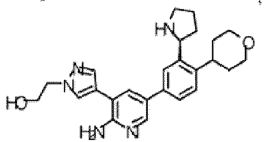 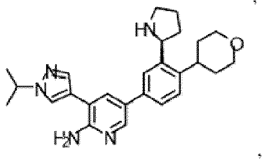 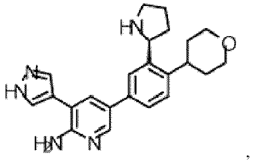 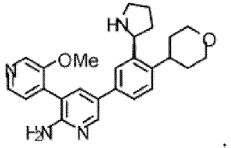 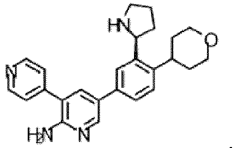 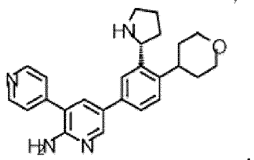 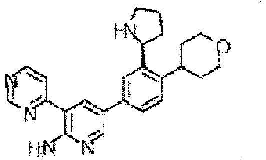 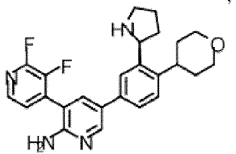 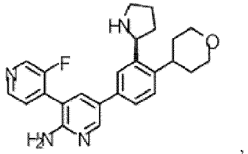 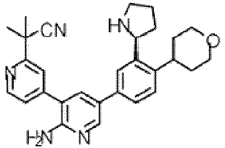 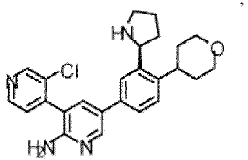 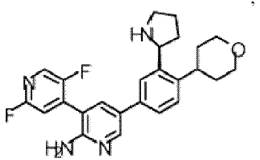 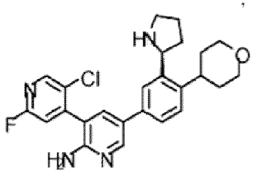 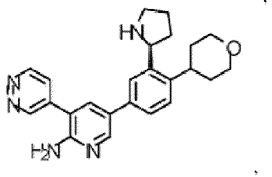 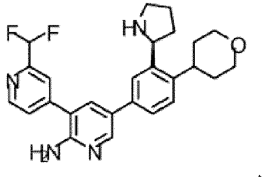 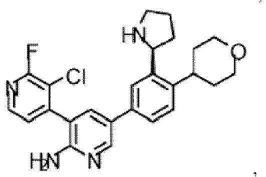 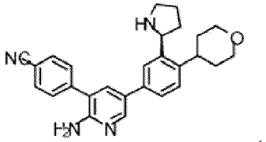 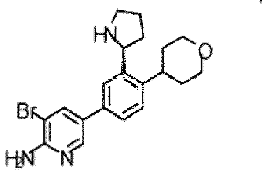 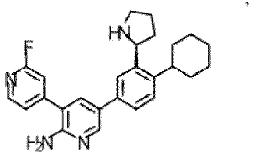 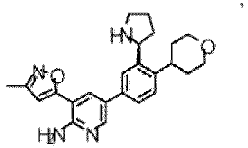 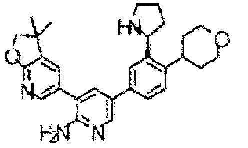 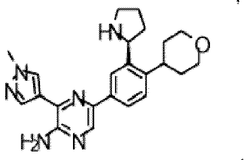 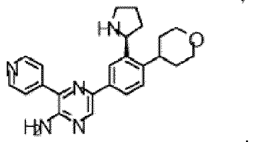 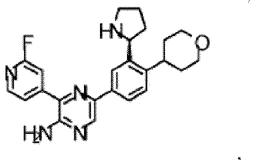 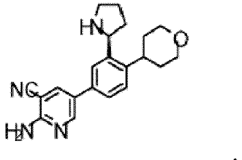 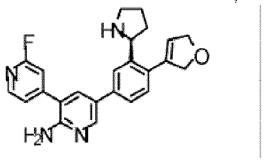 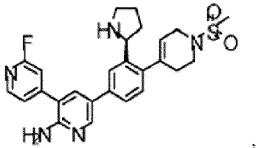 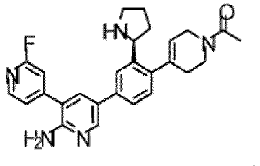 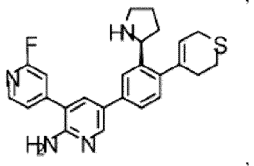 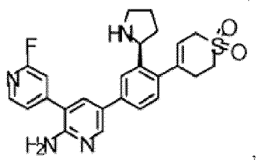 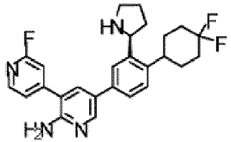 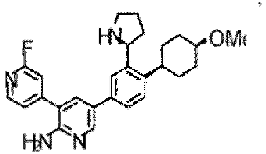 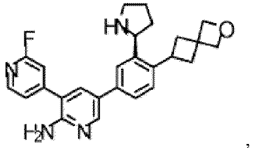 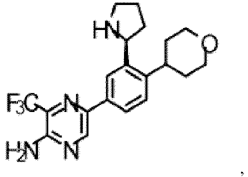 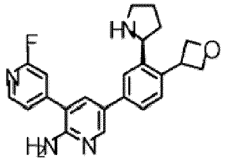 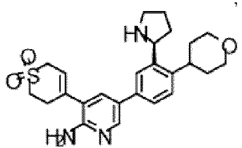 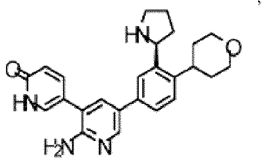 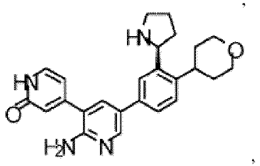 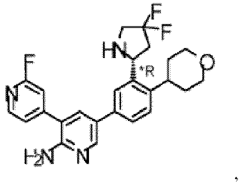 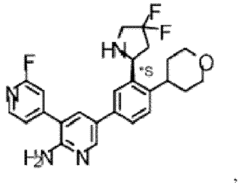 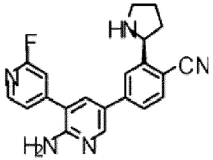 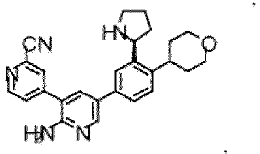 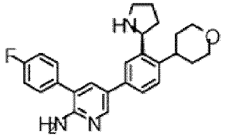 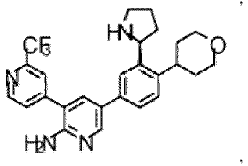 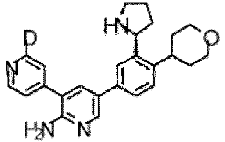 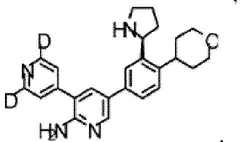 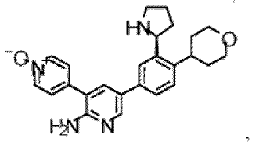 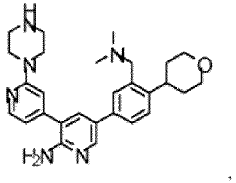 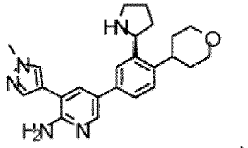 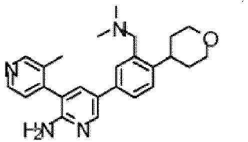 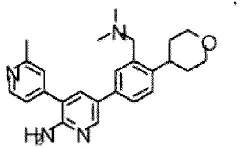 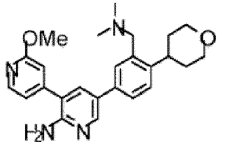 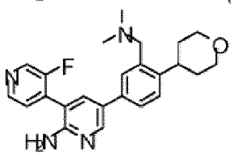 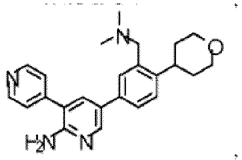 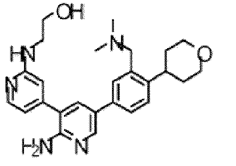 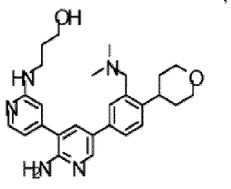 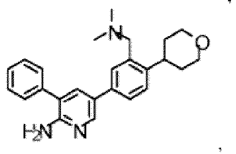 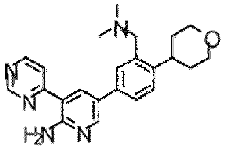 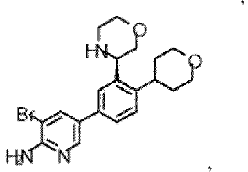 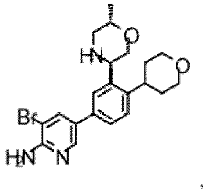 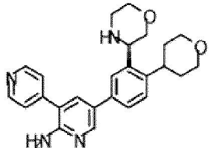 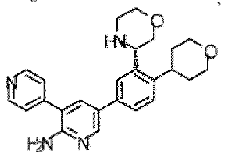 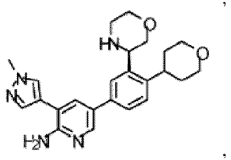 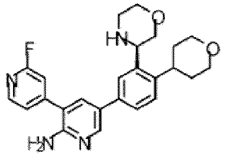 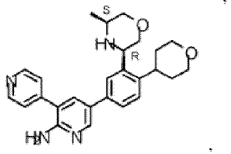 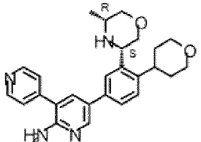 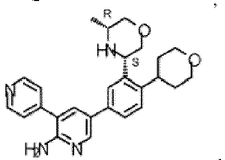 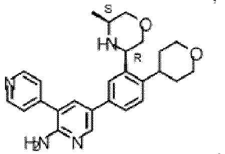 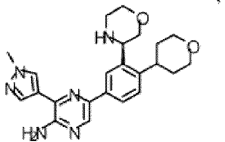 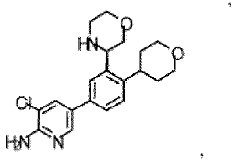 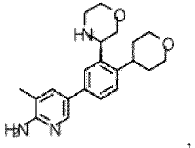 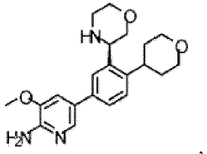 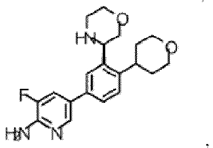 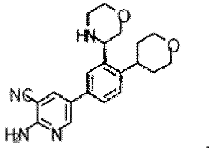 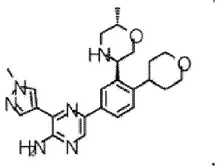 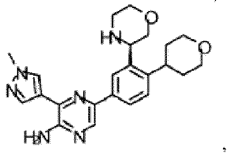 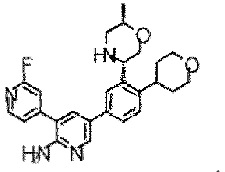 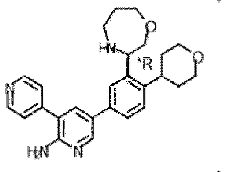 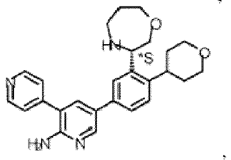 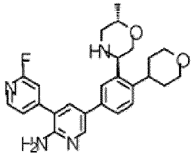 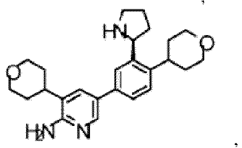 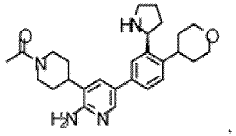 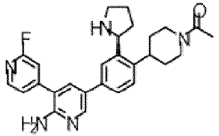 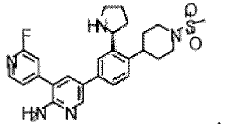 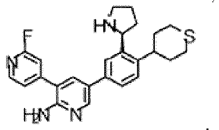 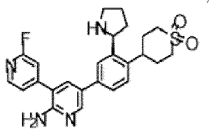 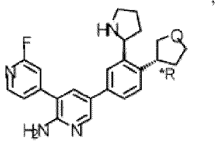 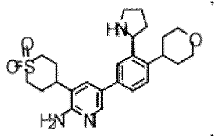 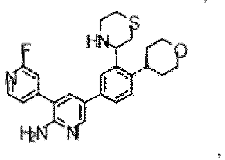 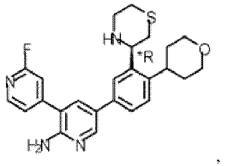 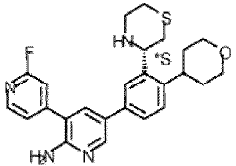 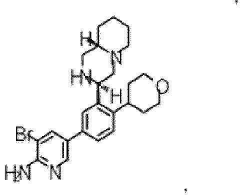 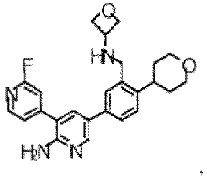 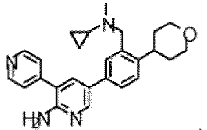 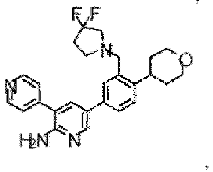 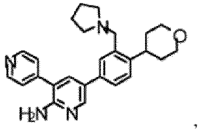 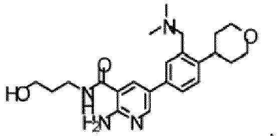 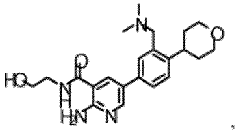 oder 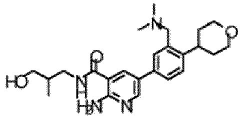
Tautomeren und stereoisomeren Formen davon,
und pharmazeutisch verträglichen Additionssalzen, N-Oxiden und Solvaten davon.

7. Verbindung nach Anspruch 1, wobei die Verbindung ist 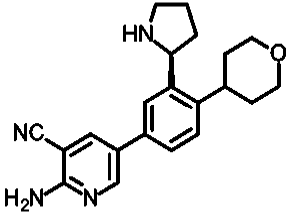 oder ein pharmazeutisch unbedenkliches Additionssalz oder ein Solvat davon.

8. Verbindung nach Anspruch 1, wobei die Verbindung ist

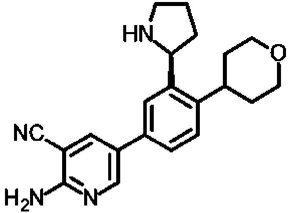

9. Verbindung nach Anspruch 1, wobei die Verbindung ist 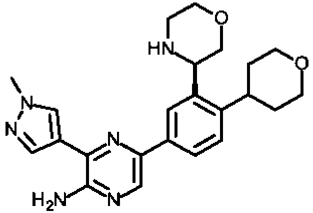 oder ein pharmazeutisch unbedenkliches Additionssalz oder ein Solvat davon.

10. Verbindung nach Anspruch 1, wobei die Verbindung ist

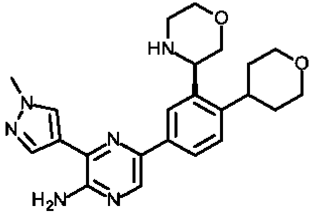

11. Verbindung nach Anspruch 1, wobei die Verbindung ist 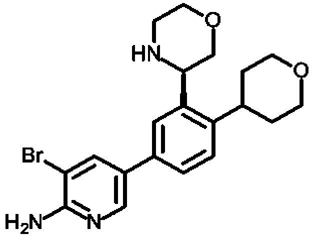 oder ein pharmazeutisch unbedenkliches Additionssalz oder ein Solvat davon.

12. Verbindung nach Anspruch 1, wobei die Verbindung ist

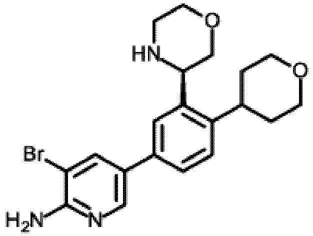

13. Verbindung nach Anspruch 1, wobei die Verbindung ist 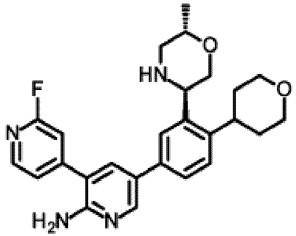 oder ein pharmazeutisch unbedenkliches Additionssalz oder ein Solvat davon.

14. Verbindung nach Anspruch 1, wobei die Verbindung ist

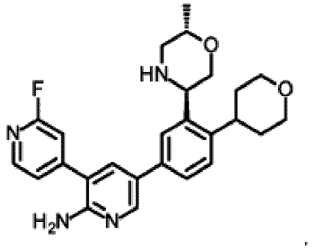

15. Pharmazeutische Zusammensetzung, umfassend eine Verbindung nach einem der Ansprüche 1 bis 14 und eine pharmazeutisch verträgliche Trägersubstanz oder ein pharmazeutisch unbedenkliches Verdünnungsmittel.

16. Verfahren zur Herstellung einer pharmazeutischen Zusammensetzung wie in Anspruch 15 definiert, umfassend ein Mischen einer pharmazeutisch verträglichen Trägersubstanz mit einer therapeutisch wirksamen Menge einer Verbindung nach einem der Ansprüche 1 bis 14.

17. Verbindung nach einem der Ansprüche 1 bis 14 oder pharmazeutische Zusammensetzung nach Anspruch 15 zur Verwendung als Arzneimittel.

18. Verbindung nach einem der Ansprüche 1 bis 14 oder eine pharmazeutische Zusammensetzung nach Anspruch 15 zur Verwendung bei der Vorbeugung oder der Behandlung von Krebs und einer viralen Infektion.

19. Verbindung oder pharmazeutische Zusammensetzung zur Verwendung nach Anspruch 18 bei der Vorbeugung oder der Behandlung von Krebs, wobei der Krebs aus Lungenkrebs, Melanom, Kopf- und Halskrebs, Speiseröhrenkrebs, Blasen- und Urothelkrebs, Leberkrebs, Nierenkrebs, Prostatakrebs und hämatopoetischem Krebs ausgewählt ist.

20. Verbindung oder pharmazeutische Zusammensetzung zur Verwendung nach Anspruch 18 bei der Vorbeugung oder der Behandlung einer chronischen Virusinfektion.

## Revendications

1. Composé de Formule (I) 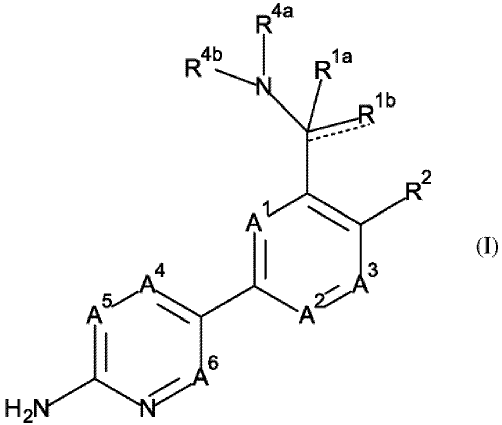
ou tautomère ou forme stéréoisomère de celui-ci, dans lequel
la liaison en pointillé vers R^{1b} est une liaison éventuelle qui peut être présente lorsque R^{1b} et R^{4b} sont pris ensemble pour former un hétérocycle aromatique monocyclique ou bicyclique tel que défini ici ;
A¹ représente CH ou N ; A² représente CH ; A³ représente CH ou N ;
à condition qu'un seul parmi A¹ et A³ représente N ;
A⁴ représente CH ou N ; A⁵ représente CR^{3a} ; A⁶ représente CH ;
R^{1a} représente hydrogène ;
R^{1b} représente hydrogène ou -CH₃ ;
R^{4a} représente hydrogène, C₁₋₄alkyle ou C₃₋₆cycloalkyle ;
R^{4b} représente hydrogène, C₁₋₄alkyle, C₃₋₆cycloalkyle, ou
un hétérocycle non aromatique monocyclique à 4, 5, 6 ou 7 chaînons à liaison carbone, contenant 1, 2 ou 3 hétéroatomes chacun choisi indépendamment dans le groupe constitué de N, O et S ;
dans lequel ledit atome S pourrait être substitué pour former S(=O), S(=O)₂ ou S(=O)(=NR^{×}) ;
ou
R^{1b} et R^{4b} sont pris ensemble pour former, avec les atomes auxquels ils sont attachés, un hétérocycle aromatique monocyclique à 5 chaînons ou un hétérocycle monocyclique entièrement saturé à 4, 5, 6 ou 7 chaînons, contenant chacun 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O), S(=O)₂ ou S(=O)(=NR^{×}) ; ou un hétérocycle bicyclique à 6 à 12 chaînons, aromatique ou entièrement saturé, contenant 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O), S(=O)₂ ou S(=O)(=NR^{×}) ;
dans lequel ledit hétérocycle monocyclique ou bicyclique, aromatique ou entièrement saturé, pourrait être substitué sur un ou plusieurs des atomes de carbone par au total 1, 2 ou 3 substituants chacun choisi indépendamment dans le groupe constitué de OH, CN, halo, R⁷, -O-R⁷, -S(=O)₂-R⁷, -C(=O)-R⁷, -NR^{6c}R^{6d}, - C(=O)-NR^{6a}R^{6b} et Het^{c} ;
dans lequel ledit hétérocycle monocyclique ou bicyclique, aromatique ou entièrement saturé, pourrait être substitué sur les atomes d'azote supplémentaires éventuels par au total 1 ou 2 substituants chacun choisi indépendamment dans le groupe constitué de R⁷, -S(=O)₂-R⁷, -C(=O)-R⁷, -C(=O)-NR^{6a}R^{6b} et Het^{d} ;
à condition que, dans le cas où R^{1b} et R^{4b} sont pris ensemble, R^{4a} représente hydrogène ; et R^{1a} représente hydrogène ou R^{1a} est absent lorsque la liaison en pointillé vers R^{1b} est une liaison ;
ou
R^{4a} et R^{4b} sont pris ensemble pour former, avec l'atome N auquel ils sont attachés, un hétérocycle aromatique monocyclique à 5 chaînons ou un hétérocycle monocyclique entièrement saturé à 4, 5, 6 ou 7 chaînons, chacun contenant 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O), S(=O)₂ ou S(=O)(=NR^{×}) ; ou un hétérocycle bicyclique à 6 à 12 chaînons, aromatique ou entièrement saturé, contenant 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O), S(=O)₂ ou S(=O)(=NR^{×}) ;
dans lequel ledit hétérocycle monocyclique ou bicyclique, aromatique ou entièrement saturé, pourrait être substitué sur un ou plusieurs des atomes de carbone par au total 1, 2 ou 3 substituants chacun choisi indépendamment dans le groupe constitué de OH, CN, halo, R⁷, -O-R⁷, -S(=O)₂-R⁷, -C(=O)-R⁷, -NR^{6c}R^{6d}, - C(=O)-NR^{6a}R^{6b} et Het^{c} ;
dans lequel ledit hétérocycle monocyclique ou bicyclique, aromatique ou entièrement saturé, pourrait être substitué sur les atomes d'azote par au total 1 ou 2 substituants chacun choisi indépendamment dans le groupe constitué de R⁷, - S(=O)₂-R⁷, -C(=O)-R⁷, -C(=O)-NR^{6a}R^{6b} et Het^{d} ;
dans le cas où R^{4a} et R^{4b} sont pris ensemble, R^{1a} représente hydrogène, et R^{1b} représente hydrogène ;
R² est choisi dans le groupe constitué de cyano ; halo ; -C(=O)-NR^{6a}R^{6b} ; -CH₂-NR^{8c}R^{8d} ; Het^{b} ; -P(=O)-(C₁₋₄alkyle)₂ ; -S(=O)₂-C₁₋₄alkyle ; -S(=O)(=NR^{×})-C₁₋₄alkyle ;
C₁₋₆alkyle éventuellement substitué par 1 ou 2 substituants chacun choisi indépendamment dans le groupe constitué de halo, -OH, cyano et -O-C₁₋₄alkyle ;
C₃₋₆cycloalkyle éventuellement substitué par 1 ou 2 substituants chacun choisi indépendamment dans le groupe constitué de halo, -OH, cyano et -O-C₁-₄alkyle ; et
C₃₋₆cycloalkényle éventuellement substitué par 1 ou 2 substituants chacun choisi indépendamment dans le groupe constitué de halo, -OH, cyano et -O-C₁-₄alkyle ;
R^{3a} représente hydrogène, halo, R⁷, -O-R⁷, cyano, -C(=O)-NR^{6e}R^{6f}, Het^{a}, ou phényle éventuellement substitué par 1, 2 ou 3 substituants chacun choisi indépendamment dans le groupe constitué de halo, cyano, C₁₋₄alkyle, -O-C₁-₄alkyle, C₁₋₄alkyle substitué par un cyano, et C₁₋₄alkyle substitué par 1, 2 ou 3 atomes d'halo ;
Het^{a} représente un hétérocycle aromatique monocyclique à 5, 6 ou 7 chaînons à liaison carbone ou un hétérocycle non aromatique monocyclique à 4, 5, 6 ou 7 chaînons à liaison carbone, chacun contenant 1, 2 ou 3 hétéroatomes chacun choisi indépendamment dans le groupe constitué de N, O ou S ; dans lequel ledit atome S pourrait être substitué pour former S(=O), S(=O)₂ ou S(=O)(=NR^{x}) ; ou un hétérocycle bicyclique, aromatique ou non aromatique, à 6 à 12 chaînons contenant 1, 2 ou 3 hétéroatomes, chacun choisi indépendamment dans le groupe constitué de N, O ou S ; dans lequel ledit atome S pourrait être substitué pour former S(=O), S(=O)₂ ou S(=O)(=NR^{x}) ; dans lequel ledit hétérocycle monocyclique ou bicyclique, aromatique ou non aromatique, pourrait être substitué sur un ou plusieurs atomes de carbone par au total 1, 2 ou 3 substituants chacun choisi indépendamment dans le groupe constitué de -OH, CN, halo, R⁷, -O-R⁷, -S(=O)₂-R⁷, -C(=O)-R⁷, -NR^{6c}R^{6d}, -C(=O)-NR^{6a}R^{6b} et Het^{c} ;
dans lequel ledit hétérocycle monocyclique ou bicyclique, aromatique ou non aromatique, pourrait être substitué sur les atomes d'azote par au total 1 ou 2 substituants chacun choisi indépendamment dans le groupe constitué de R⁷, - S(=O)₂-R⁷, -C(=O)-R⁷, -C(=O)-NR^{6a}R^{6b} et Het^{d} ;
R^{6a}, R^{6b}, R^{6c}, R^{6d}, R^{6e} et R^{6f} sont chacun choisi indépendamment dans le groupe constitué d'hydrogène ; C₃₋₆cycloalkyle éventuellement substitué par un - OR⁵ ; et
C₁₋₄alkyle éventuellement substitué par un -OR⁵, dans lequel deux atomes d'hydrogène sur le même atome de carbone dudit C₁₋₄alkyle pourraient être pris ensemble pour former C₃₋₆cycloalkyle ;
R⁵ représente hydrogène ou C₁₋₄alkyle ;
R^{8a}, R^{8c} et R^{8d} sont chacun choisi indépendamment dans le groupe constitué d'hydrogène ; C₁₋₄alkyle éventuellement substitué par un -OH ou -O-C₁-₄alkyle ; et C₃₋₆alkényle éventuellement substitué par un -OH ou -O-C₁₋₄alkyle ;
R^{8b} est choisi dans le groupe constitué de C₁₋₄alkyle éventuellement substitué par un -OH ou -O-C₁₋₄alkyle ; et C₃₋₆alkényle éventuellement substitué par un -OH ou -O-C₁₋₄alkyle ;
ou
R^{8a} et R^{8b}, ou R^{8c} et R^{8d} sont pris ensemble pour former, avec l'atome N auquel ils sont attachés, un hétérocycle monocyclique entièrement saturé contenant 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O), S(=O)₂ ou S(=O)(=NR^{x}) ; dans lequel ledit hétérocycle monocyclique entièrement saturé à 4, 5, 6 ou 7 chaînons pourrait être substitué sur un ou plusieurs des atomes de carbone par au total 1, 2 ou 3 substituants chacun choisi indépendamment dans le groupe constitué de -OH, CN, halo, R⁷, -O-R⁷, -S(=O)₂-R⁷, - C(=O)-R⁷, -NR^{6c}R^{6d}, et -C(=O)-NR^{6a}R^{6b} ;
dans lequel ledit hétérocycle monocyclique entièrement saturé pourrait être substitué sur les atomes d'azote par au total 1 ou 2 substituants chacun choisi indépendamment dans le groupe constitué de R⁷, -S(=O)₂-R⁷, -C(=O)-R⁷ et - C(=O)-NR^{6a}R^{6b} ;
chaque Het^{c} représente indépendamment un hétérocycle monocyclique entièrement saturé à 4, 5, 6 ou 7 chaînons contenant 1, 2 ou 3 hétéroatomes chacun choisi indépendamment dans le groupe constitué de N, O ou S ; dans lequel ledit atome S pourrait être substitué pour former S(=O), S(=O)₂ ou S(=O)(=NR^{x}) ;
chaque Het^{d} représente indépendamment un hétérocycle monocyclique entièrement saturé à 4, 5, 6 ou 7 chaînons à liaison carbone, contenant 1, 2 ou 3 hétéroatomes chacun choisi indépendamment dans le groupe constitué de N, O ou S ; dans lequel ledit atome S pourrait être substitué pour former S(=O), S(=O)₂ ou S(=O)(=NR^{x}) ;
Het^{b} représente un hétérocycle non aromatique monocyclique à 4, 5, 6 ou 7 chaînons contenant 1, 2 ou 3 hétéroatomes chacun choisi indépendamment dans le groupe constitué de N, O, S ; dans lequel ledit atome S pourrait être substitué pour former S(=O), S(=O)₂ ou S(=O)(=NR^{x}) ; ou un hétérocycle non aromatique bicyclique à 6 à 12 chaînons contenant 1, 2 ou 3 hétéroatomes chacun choisi indépendamment dans le groupe constitué de N, O, S ; dans lequel ledit atome S pourrait être substitué pour former S(=O), S(=O)₂ ou S(=O)(=NR^{x}) ;
dans lequel ledit hétérocycle non aromatique monocyclique ou bicyclique pourrait être substitué sur un ou plusieurs atomes de carbone par au total 1, 2 ou 3 substituants chacun choisi indépendamment dans le groupe constitué de -OH, CN, halo, R⁷, -O-R⁷, -S(=O)₂-R⁷, -C(=O)-R⁷, -NR^{6c}R^{6d} et -C(=O)-NR^{6a}R^{6b} ;
dans lequel ledit hétérocycle non aromatique monocyclique ou bicyclique pourrait être substitué sur les atomes d'azote par au total 1 ou 2 substituants chacun choisi indépendamment dans le groupe constitué de R⁷, -S(=O)₂-R⁷, - C(=O)-R⁷ et -C(=O)-NR^{6a}R^{6b} ;
chaque R⁷ représente indépendamment C₃₋₆cycloalkyle éventuellement substitué par 1, 2 ou 3 substituants chacun choisi indépendamment dans le groupe constitué de halo, -O-C₁₋₄alkyle et cyano ; ou C₁₋₄alkyle éventuellement substitué par 1, 2 ou 3 substituants chacun choisi indépendamment dans le groupe constitué de -OH, -O-C₁₋₄alkyle et cyano ; chaque R^{x} représente hydrogène ou C₁₋₄alkyle ;
ou un sel d'addition pharmaceutiquement acceptable, un N-oxyde ou un solvate de celui-ci.

2. Composé selon la revendication 1, dans lequel
la liaison en pointillé vers R^{1b} est une liaison éventuelle qui pourrait être présente lorsque R^{1b} et R^{4b} sont pris ensemble pour former un hétérocycle aromatique monocyclique tel que défini ici ;
A¹ représente CH ou N ; A² représente CH ; A³ représente CH ou N ;
à condition qu'un seul parmi A¹ et A³ représente N ;
A⁴ représente CH ; A⁵ représente CR^{3a} ; A⁶ représente CH ;
R^{1a} représente hydrogène ;
R^{1b} représente hydrogène ;
R^{4a} représente hydrogène, ou C₁₋₄alkyle ;
R^{4b} représente C₁₋₄alkyle, ou C₃₋₆cycloalkyle, ou
un hétérocycle non aromatique monocyclique à 4, 5, 6 ou 7 chaînons à liaison carbone, contenant 1, 2 ou 3 atomes d'oxygène ;
ou
R^{1b} et R^{4b} sont pris ensemble pour former, avec les atomes auxquels ils sont attachés, un hétérocycle aromatique monocyclique à 5 chaînons ou un hétérocycle monocyclique à 4, 5, 6 ou 7 chaînons entièrement saturé, contenant chacun 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O) ; ou
un hétérocycle bicyclique entièrement saturé à 6 à 12 chaînons, contenant 1 atome N et éventuellement 1 ou 2 hétéroatomes supplémentaires chacun choisi indépendamment dans le groupe constitué de N, O, S ; dans lequel ledit atome S éventuel peut être substitué pour former S(=O) ;
dans lequel ledit hétérocycle monocyclique ou bicyclique, aromatique ou entièrement saturé, pourrait être substitué sur un ou plusieurs des atomes de carbone par au total 1, 2 ou 3 substituants chacun choisi indépendamment dans le groupe constitué de halo et R⁷ ;
à condition que, dans le cas où R^{1b} et R^{4b} sont pris ensemble, R^{4a} représente hydrogène ; et R^{1a} représente hydrogène ou R^{1a} est absent lorsque la liaison en pointillé vers R^{1b} est une liaison ;
ou
R^{4a} et R^{4b} sont pris ensemble pour former, avec l'atome N auquel ils sont attachés, un hétérocycle monocyclique entièrement saturé à 4, 5, 6 ou 7 chaînons, contenant 1 atome N ; dans lequel ledit hétérocycle monocyclique entièrement saturé pourrait être substitué sur un ou plusieurs des atomes de carbone par au total 1, 2 ou 3 substituants halo ;
dans le cas où R^{4a} et R^{4b} sont pris ensemble, R^{1a} représente hydrogène, et R^{1b} représente hydrogène ; R² est choisi dans le groupe constitué de cyano ; Het^{b} ; et C₃₋₆cycloalkyle éventuellement substitué par 1 ou 2 substituants chacun choisi indépendamment dans le groupe constitué de halo et -O-C₁₋₄alkyle ;
R^{3a} représente halo, R⁷, -O-R⁷, cyano, -C(=O)-NR^{6e}R^{6f}, Het^{a} ou phényle éventuellement substitué par 1, 2 ou 3 substituants chacun choisi indépendamment dans le groupe constitué de halo et cyano ;
Het^{a} représente un hétérocycle aromatique monocyclique à 5, 6 ou 7 chaînons à liaison carbone ou un hétérocycle non aromatique monocyclique à 4, 5, 6 ou 7 chaînons à liaison carbone, chacun contenant 1, 2 ou 3 hétéroatomes chacun choisi indépendamment dans le groupe constitué de N, O ou S ; dans lequel ledit atome S pourrait être substitué pour former S(=O) ou S(=O)₂ ; ou
un hétérocycle bicyclique non aromatique à 6 à 12 chaînons à liaison carbone, contenant 1, 2 ou 3 hétéroatomes chacun choisi indépendamment dans le groupe constitué de N ou O ;
dans lequel ledit hétérocycle monocyclique ou bicyclique, aromatique ou non aromatique, pourrait être substitué sur un ou plusieurs des atomes de carbone par au total 1, 2 ou 3 substituants choisis indépendamment dans le groupe constitué de -OH, CN, halo, R⁷, -O-R⁷, -NR^{6c}R^{6d} et Het^{c} ;
dans lequel ledit hétérocycle monocyclique ou bicyclique, aromatique ou non aromatique, pourrait être substitué sur les atomes d'azote par au total 1 ou 2 substituants choisis indépendamment dans le groupe constitué de R⁷, -S(=O)₂-R⁷ et -C(=O)-R⁷ ;
R^{6c}, R^{6d}, R^{6e} et R^{6f} sont chacun choisi indépendamment dans le groupe constitué d'hydrogène ; et C₁₋₄alkyle éventuellement substitué par un -OR⁵ ;
R⁵ représente hydrogène ;
chaque Het^{c} représente indépendamment un hétérocycle monocyclique entièrement saturé à 4, 5, 6 ou 7 chaînons, contenant 1, 2 ou 3 atomes d'azote ;
Het^{b} représente un hétérocycle non aromatique monocyclique à 4, 5, 6 ou 7 chaînons contenant 1, 2 ou 3 hétéroatomes chacun choisi indépendamment dans le groupe constitué de N, O, S ;
dans lequel ledit atome S pourrait être substitué pour former S(=O) ou S(=O)₂ ; ou
un hétérocycle non aromatique bicyclique à 6 à 12 chaînons contenant 1, 2 ou 3 hétéroatomes chacun choisi indépendamment dans le groupe constitué de N, O, S ; dans lequel ledit atome S pourrait être substitué pour former S(=O) ou S(=O)₂ ;
dans lequel ledit hétérocycle non aromatique monocyclique ou bicyclique pourrait être substitué sur les atomes d'azote par au total 1 ou 2 substituants chacun choisi indépendamment dans le groupe constitué de -S(=O)₂-R⁷ et -C(=O)-R⁷ ;
chaque R⁷ représente indépendamment un C₁₋₄alkyle éventuellement substitué par 1, 2 ou 3 substituants chacun choisi indépendamment dans le groupe constitué de halo, -OH et cyano.

3. Composé selon la revendication 1 ou 2, dans lequel la liaison en pointillé vers R^{1b} est absente ;
A¹ représente CH ou N ; A² représente CH ; A³ représente CH ;
A⁴ représente CH ; A⁵ représente CR^{3a} ; A⁶ représente CH ;
R^{1b} et R^{4b} sont pris ensemble pour former, avec l'atome N auquel ils sont attachés, un hétérocyclyle monocyclique entièrement saturé à 4, 5, 6 ou 7 chaînons, contenant chacun 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O) ; dans lequel ledit hétérocycle monocyclique entièrement saturé pourrait être substitué sur l'un des atomes de carbone par 1 substituant choisi dans le groupe constitué de halo et R⁷ ;
à condition que R^{4a} représente hydrogène ; et R^{1a} représente hydrogène ;
R² représente Het^{b} ;
R^{3a} représente halo, cyano ou Het^{a} ;
Het^{a} représente un hétérocycle aromatique monocyclique à 5, 6 ou 7 chaînons à liaison carbone, contenant 1, 2 ou 3 hétéroatomes chacun choisi indépendamment dans le groupe constitué de N, O ou S ; dans lequel ledit atome S pourrait être substitué pour former S(=O) ou S(=O)₂ ;
dans lequel ledit hétérocycle aromatique monocyclique pourrait être substitué sur l'un des atomes de carbone par un substituant halo ;
dans lequel ledit hétérocycle aromatique monocyclique pourrait être substitué sur un atome d'azote par R⁷ ;
Het^{b} représente un hétérocycle monocyclique non aromatique à 4, 5, 6 ou 7 chaînons contenant 1 atome d'oxygène ;
chaque R⁷ représente C₁₋₄alkyle.

4. Composé selon la revendication 1, dans lequel
R^{1b} et R^{4b} sont pris ensemble pour former, avec les atomes auxquels ils sont attachés, un hétérocycle aromatique monocyclique à 5 chaînons ou un hétérocycle monocyclique entièrement saturé à 4, 5, 6 ou 7 chaînons, contenant chacun 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O), S(=O)₂ ou S(=O)(=NR^{x}) ; ou
un hétérocycle bicyclique à 6 à 12 chaînons, aromatique ou entièrement saturé, contenant 1 atome N et éventuellement 1 ou 2 hétéroatomes supplémentaires chacun choisi indépendamment dans le groupe constitué de N, O, S ; dans lequel ledit atome S éventuel pourrait être substitué pour former S(=O), S(=O)₂ ou S(=O)(=NR^{x}) ;
dans lequel ledit hétérocycle monocyclique ou bicyclique, aromatique ou entièrement saturé, pourrait être substitué sur un ou plusieurs des atomes de carbone par au total 1, 2 ou 3 substituants chacun choisi indépendamment dans le groupe constitué de -OH, CN, halo, R⁷, -O-R⁷, -S(=O)₂-R⁷,-C(=O)-R⁷, -NR^{6c}R^{6d}, - C(=O)-NR^{6a}R^{6b} et Het^{c} ;
dans lequel ledit hétérocycle monocyclique ou bicyclique, aromatique ou entièrement saturé, pourrait être substitué sur les atomes d'azote supplémentaires éventuels par au total 1 ou 2 substituants chacun choisi indépendamment dans le groupe constitué de R⁷, -S(=O)₂-R⁷,-C(=O)-R⁷, -C(=O)-NR^{6a}R^{6b} et Het^{d} ;
à condition que R^{4a} représente hydrogène ; et
R^{1a} représente hydrogène ou R^{1a} est absent lorsque la liaison en pointillé vers R^{1b} est une liaison.

5. Composé selon la revendication 1, dans lequel
la liaison en pointillé vers R^{1b} est absente ;
A¹ représente CH ou N ; A² représente CH ; A³ représente CH ;
A⁴ représente CH ; A⁵ représente CR^{3a} ; A⁶ représente CH ;
R^{1b} et R^{4b} sont pris ensemble pour former avec les atomes auxquels ils sont attachés 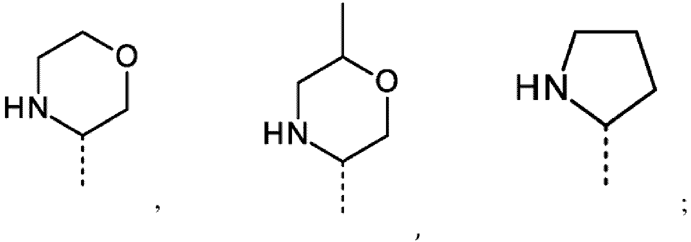
R^{4a} représente hydrogène ; et R^{1a} représente hydrogène ;
R² représente Het^{b} ;
R^{3a} représente halo, cyano ou Het^{a} ;
Het^{a} représente 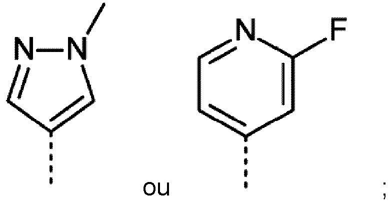
Het^{b} représente tétrahydropyranyle ; en particulier 4-tétrahydropyranyle.

6. Composé selon la revendication 1, dans lequel le composé est choisi dans le groupe constitué de 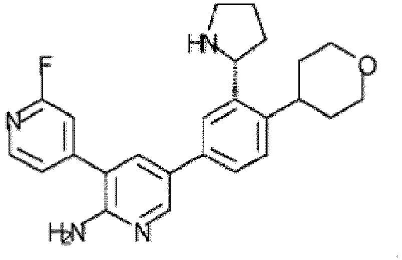 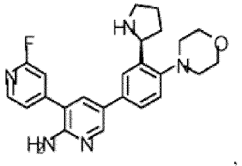 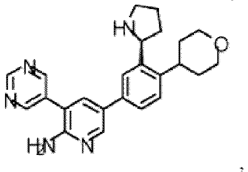 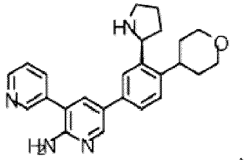 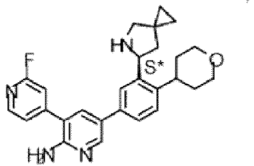 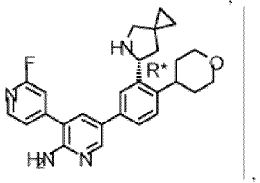 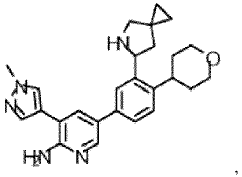 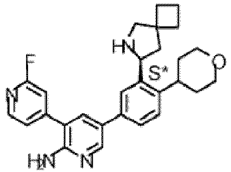 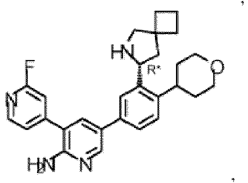 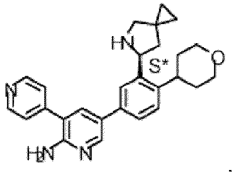 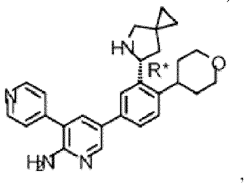 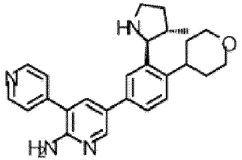 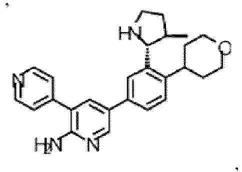 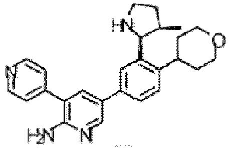 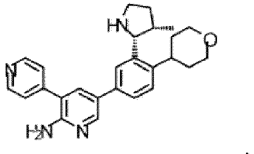 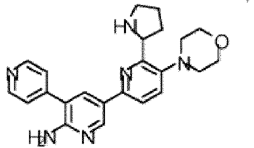 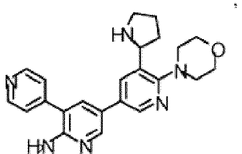 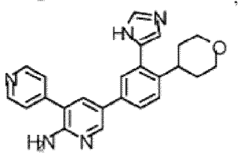 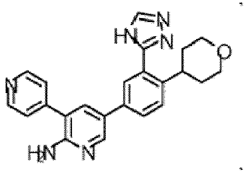 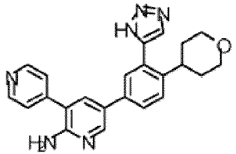 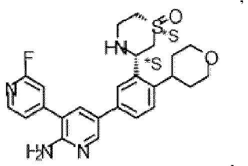 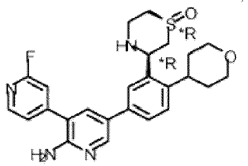 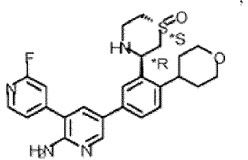 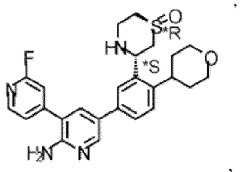 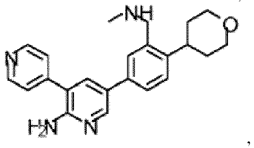 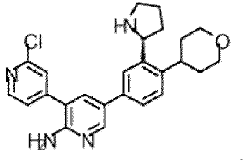 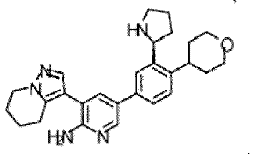 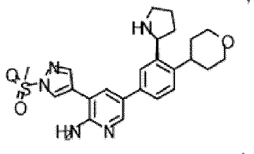 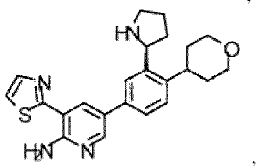 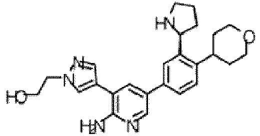 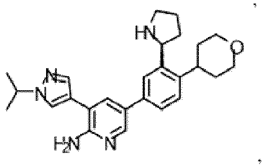 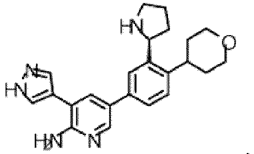 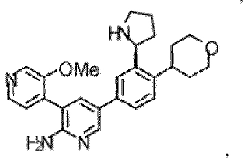 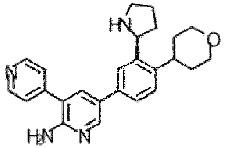 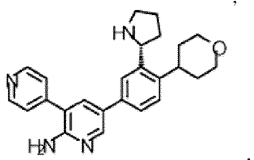 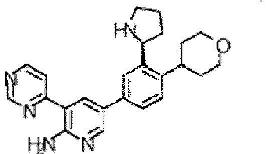 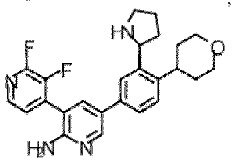 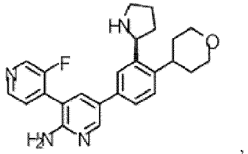 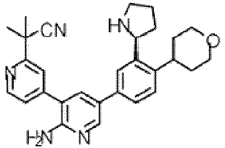 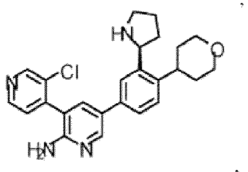 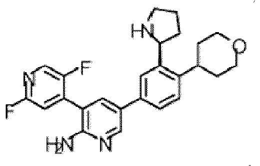 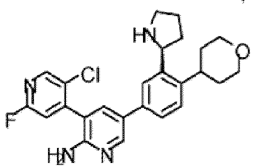 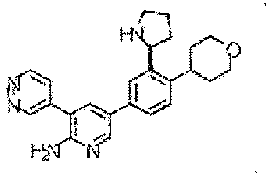 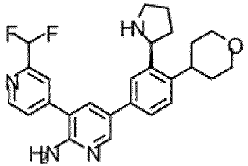 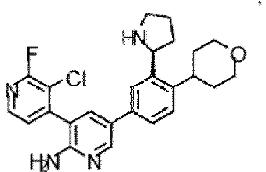 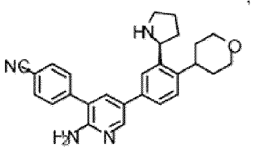 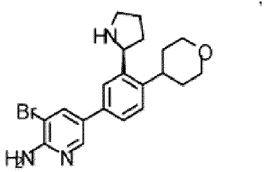 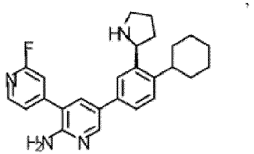 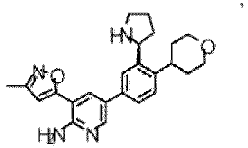 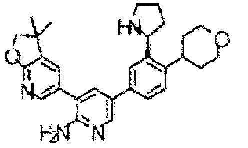 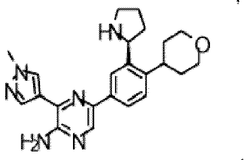 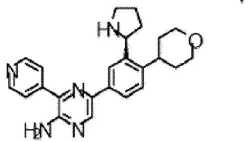 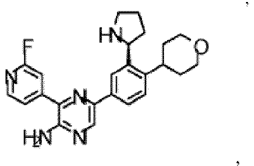 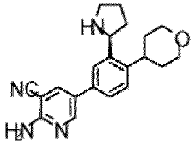 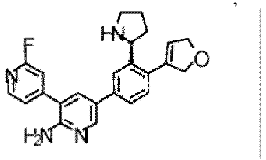 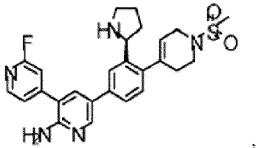 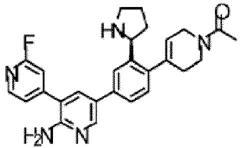 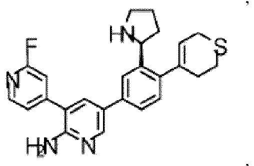 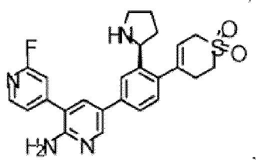 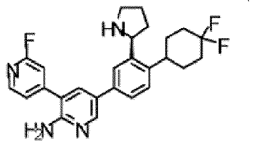 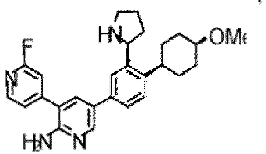 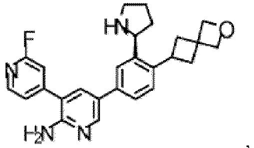 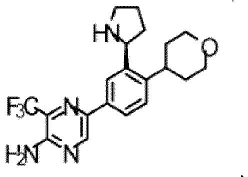 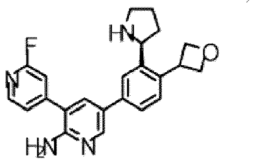 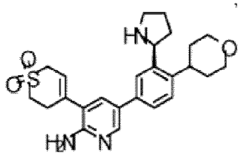 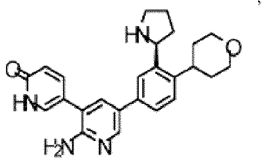 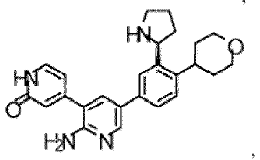 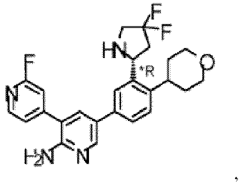 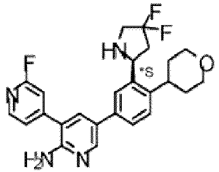 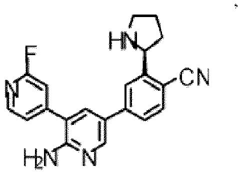 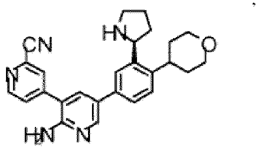 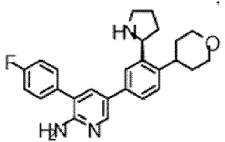 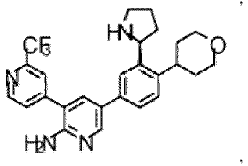 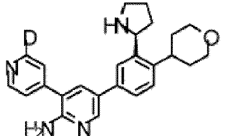 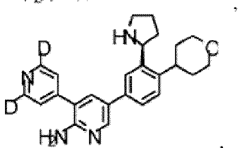 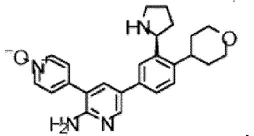 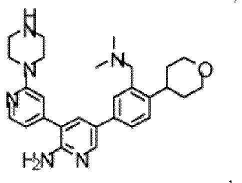 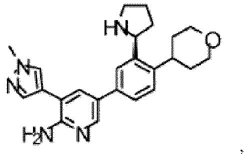 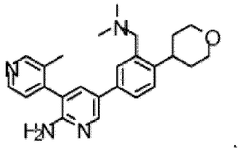 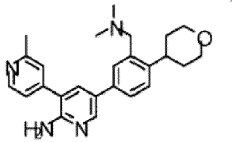 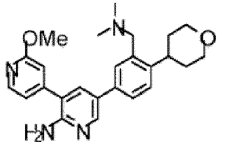 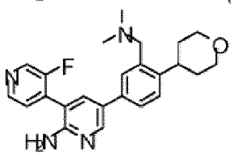 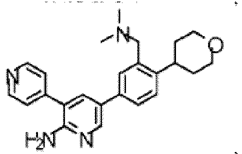 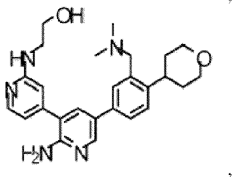 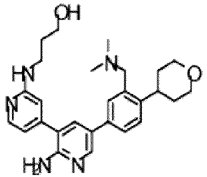 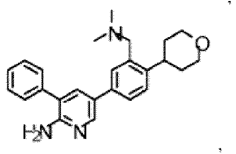 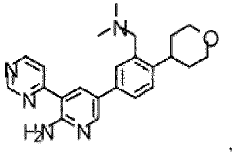 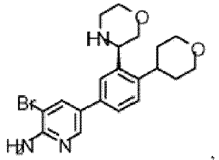 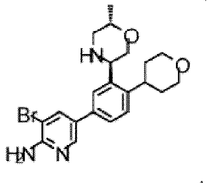 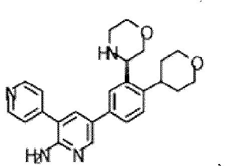 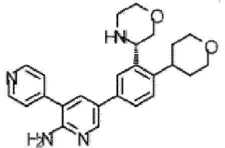 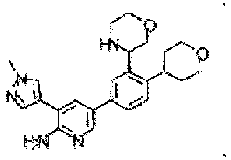 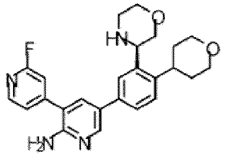 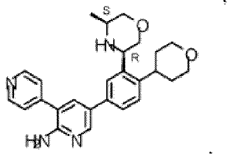 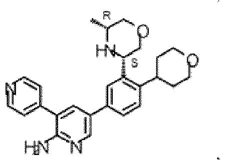 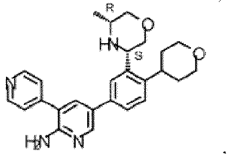 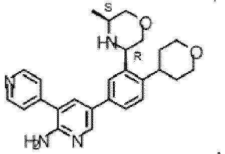 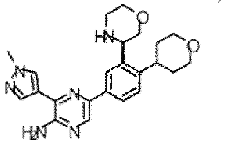 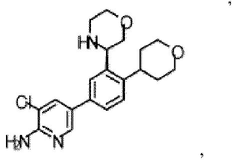 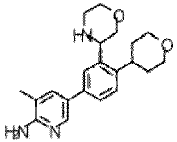 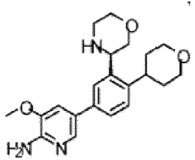 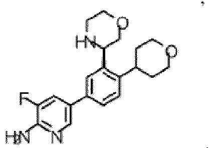 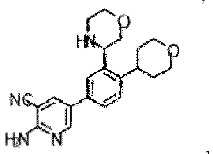 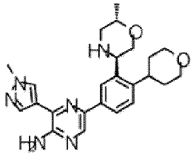 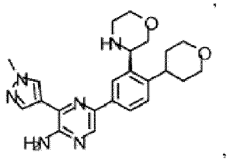 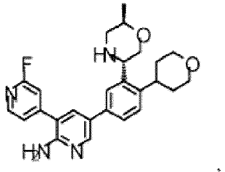 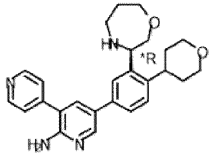 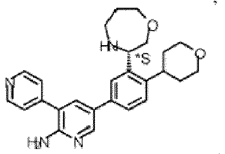 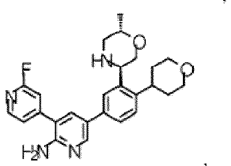 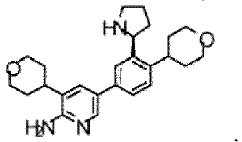 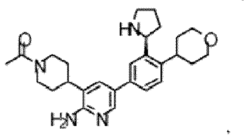 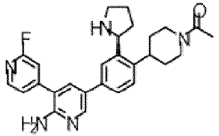 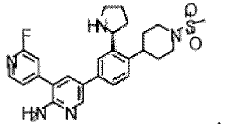 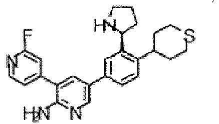 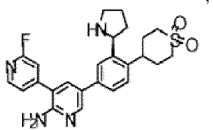 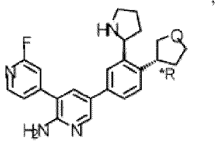 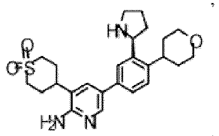 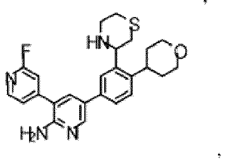 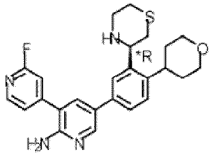 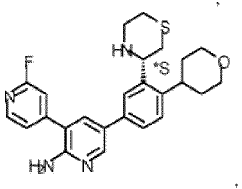 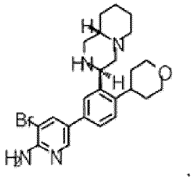 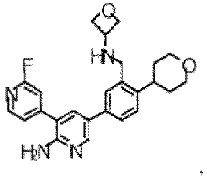 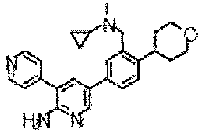 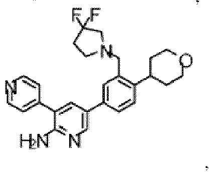 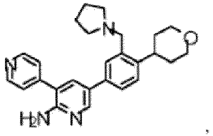 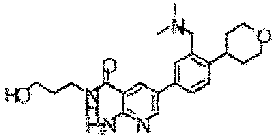 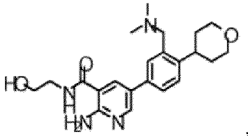 ou 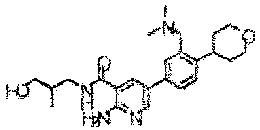
tautomères et formes stéréoisomères de ceux-ci,
et sels d'addition, N-oxydes et solvates pharmaceutiquement acceptables de ceux-ci.

7. Composé selon la revendication 1, dans lequel le composé est 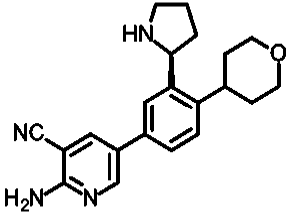 ou un sel d'addition pharmaceutiquement acceptable ou un solvate de celui-ci.

8. Composé selon la revendication 1, dans lequel le composé est

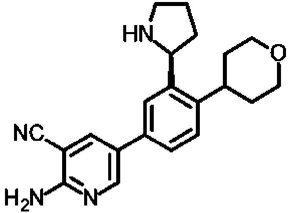

9. Composé selon la revendication 1, dans lequel le composé est 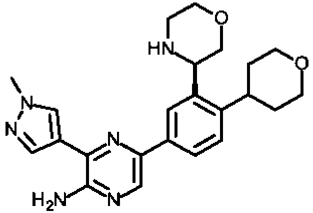 ou un sel d'addition pharmaceutiquement acceptable ou un solvate de celui-ci.

10. Composé selon la revendication 1, dans lequel le composé est

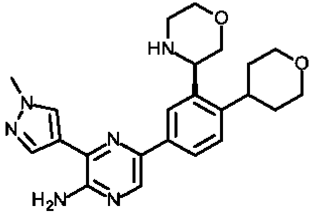

11. Composé selon la revendication 1, dans lequel le composé est 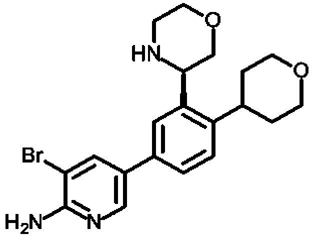 ou un sel d'addition pharmaceutiquement acceptable ou un solvate de celui-ci.

12. Composé selon la revendication 1, dans lequel le composé est

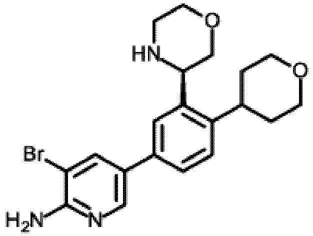

13. Composé selon la revendication 1, dans lequel le composé est 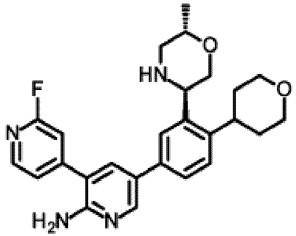 ou un sel d'addition pharmaceutiquement acceptable ou un solvate de celui-ci.

14. Composé selon la revendication 1, dans lequel le composé est

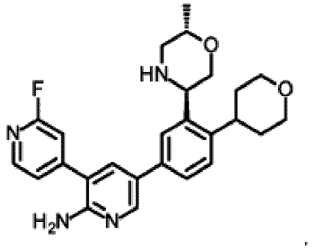

15. Composition pharmaceutique comprenant un composé selon l'une quelconque des revendications 1 à 14 et un support ou diluant pharmaceutiquement acceptable.

16. Procédé pour la préparation d'une composition pharmaceutique selon la revendication 15 comprenant le mélange d'un support pharmaceutiquement acceptable avec une quantité thérapeutiquement efficace d'un composé selon l'une quelconque des revendications 1 à 14.

17. Composé selon l'une quelconque des revendications 1 à 14 ou composition pharmaceutique selon la revendication 15 pour une utilisation comme médicament.

18. Composé selon l'une quelconque des revendications 1 à 14 ou composition pharmaceutique selon la revendication 15 pour une utilisation dans la prévention ou le traitement d'un cancer et d'une infection virale.

19. Composé ou composition pharmaceutique pour une utilisation selon la revendication 18 dans la prévention ou le traitement d'un cancer, dans lequel le cancer est choisi parmi cancer du poumon, mélanome, cancer de la tête et du cou, cancer de l'œsophage, cancer de la vessie et cancer urothélial, cancer du foie, cancer du rein, cancer de la prostate et cancer hématopoïétique.

20. Composé ou composition pharmaceutique pour une utilisation selon la revendication 18 dans la prévention ou le traitement d'une infection virale chronique.
